(19) Europäisches Patentamt
European Patent Office
Office européen des brevets

(11) EP 3 107 900 B1

(12) # FASCICULE DE BREVET EUROPEEN

(45) Date de publication et mention de la délivrance du brevet:
**15.11.2017 Bulletin 2017/46**

(21) Numéro de dépôt: **15709289.1**

(22) Date de dépôt: **20.02.2015**

(51) Int Cl.:
***C07D 217/02*** $^{(2006.01)}$ ***C07D 217/24*** $^{(2006.01)}$
***C07D 405/06*** $^{(2006.01)}$ ***A61K 31/472*** $^{(2006.01)}$
***A61P 9/00*** $^{(2006.01)}$

(86) Numéro de dépôt international:
**PCT/FR2015/050415**

(87) Numéro de publication internationale:
**WO 2015/124877 (27.08.2015 Gazette 2015/34)**

(54) **DÉRIVÉS DE 5-BENZYLISOQUINOLÉINE POUR LE TRAITEMENT DE MALADIES CARDIOVASCULAIRES**

5-BENZYLISOCHINOLIN-DERIVATE ZUR BEHANDLUNG VON KARDIOVASKULÄREN ERKRANKUNGEN

5-BENZYLISOQUINOLEINE DERIVATIVES FOR THE TREATMENT OF CARDIOVASCULAR DISEASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **21.02.2014 FR 1451389**

(43) Date de publication de la demande:
**28.12.2016 Bulletin 2016/52**

(73) Titulaire: **Les Laboratoires Servier**
**92284 Suresnes Cedex (FR)**

(72) Inventeurs:
- **CHIMENTI, Stefano**
  **F-75011 Paris (FR)**
- **COURCHAY, Christine**
  **F-91430 Igny (FR)**
- **DESSINGES, Aimée**
  **F-92500 Rueil-Malmaison (FR)**
- **GELLIBERT, Françoise**
  **F-91440 Bures-sur-Yvette (FR)**
- **GOUMENT, Bertrand**
  **F-78220 Viroflay (FR)**
- **KONNERT, Marc**
  **F-95170 Deuil-la-Barre (FR)**
- **PEGLION, Jean-Louis**
  **F-78110 Le Vesinet (FR)**
- **POITEVIN, Christophe**
  **F-75011 Paris (FR)**
- **VILAINE, Jean-Paul**
  **F-92290 Chatenay Malabry (FR)**
- **VILLENEUVE, Nicole**
  **F-92500 Rueil Malmaison (FR)**

(56) Documents cités:
**WO-A1-02/13827 WO-A1-2007/000240**

- **PHILIP V. LOGRASSO AND YANGBO FENG: "Rho Kinase (ROCK) inhibitors and their application to inflammatory disorders", CURRENT TOPICS IN MEDICINAL CHEMISTRY, vol. 9, no. 8, 2009, pages 704-723, XP055134546, ISSN: 1568-0266, DOI: 10.2174/156802609789044452**
- **CORRINE Y. WATSON ET AL: "Synthesis of 3-substituted benzamides and 5-substituted isoquinolin-1(2H)-ones and preliminary evaluation as inhibitors of poly(ADP-ribose)polymerase (PARP)", BIOORGANIC & MEDICINAL CHEMISTRY, vol. 6, no. 6, 1998, pages 721-734, XP002481784, ISSN: 0968-0896, DOI: 10.1016/S0968-0896(98)00029-7**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 107 900 B1

## Description

La présente invention concerne de nouveaux dérivés isoquinoléines, leur synthèse et leur utilisation dans la prévention et/ ou le traitement de pathologies qui résultent de l'activation de la voie RhoA/ROCK et de la phosphorylation de la chaîne légère de la myosine.

Sous l'effet d'agonistes tels que l'angiotensine II, la 5-hydroxytryptamine ou l'endothéline, la protéine RhoA membranaire, appartenant à la famille des petites protéines liées au GTP, acquière la configuration active liée au GTP, sous le contrôle de facteurs d'échange spécifiques des nucléotides adényliques. Cette forme active membranaire permet la liaison à la protéine Rho- kinase et l'activation de cette dernière.
Rho- kinase est une sérine/thréonine kinase avec une masse moléculaire de 160 kdaltons et une des nombreuses cibles de la protéine RhoA. Deux isoformes de Rho-kinase, Rho-kinase β/ROCK β/p160ROCK ou ROCK1 et Rho-kinase α/ROCK α ou ROCK2, codées par deux gènes différents, ont été identifiées. Les deux isoformes sont exprimées de manière ubiquitaire, ROCK2 particulièrement dans les cellules musculaires lisses vasculaires, le coeur et le cerveau. ROCK1 est exprimée préférentiellement sur les tissus non nerveux tels que le poumon, le foie, la rate, le rein et les testicules. Ces deux isoformes partagent 92% d'homologie dans leur domaine kinasique. L'activation de ROCK par RhoA-GTP conduit à la phosphorylation et l'inhibition d'une sous-unité régulatrice de la myosine phosphatase et permet ainsi de maintenir la chaîne légère de la myosine dans un état phosphorylé, indépendamment de la concentration en $Ca^{2+}$ intracellulaire (processus dit de sensibilisation au $Ca^{2+}$). La phosphorylation de la chaîne légère de la myosine est responsable de l'augmentation de la contractilité du cytosquelette d'actine, qui résulte d'un glissement entre les filaments d'actine et de myosine.

L'activation de la voie RhoA/ROCK est impliquée dans les dysfonctions suivantes et les pathologies qui leur sont associées :

- vasoconstriction par augmentation du tonus myogénique (Rattan et al, Pharmacological Sciences, 880 :1-10, 2011),
- formation des fibres de stress et contraction cellulaire (Kaibuchi et al, Sciences, 275 : 1308, 1997),
- hypertension artérielle systémique (Uehata et al, Nature, 389 : 990-993, 1997 ; Pacaud et al, P. Nat. Rev. Cardiol .,7(11) : 637-647, 2010),
- hypertension artérielle pulmonaire (Jankov et al, Am J Physiol Heart Circ Physiol, 299 :H1854-H1864, 2010 ; Fukumoto et al, Heart, 91 : 391-392, 2005) et la fibrose pulmonaire associée (Duong-Quy et al, J. Fran. Viet. Pneu., 03(08) : 1-74, 2012),
- augmentation de la pression intraoculaire, la rétinopathie et le glaucome qui en résulte (Acott et al, Curr Opin Ophtalmol, 23(2) : 135-43, 2012 ; Tanihara et al, Curr Eye Res, 36(10) : 964-70, 2011 ; Rossetti et al, Expert.Opin.Investig.Drugs, 20(7) :947-959, 2011 ; Chen et al, Clin. Ophtalmol, 5 : 667-677, 2011 ; Rao et al, J Glaucoma, 21 :530-538, 2012, Zhong et al, Int J Oncol, 43(5) :1357-67, 2013 ; Van de Velde, Acta Ophtalmologica, 91 : s252, 2013), dystrophie de la cornée due à une prolifération des cellules endothéliales (Kinoshita et al, Cornea, 32(8) : 1167-1170, 2013),
- vaso-spasme des coronaires, angine de poitrine, infarctus du myocarde, (Kandabashi et al, Circulation, 101 : 1319-1323, 2000 ; Shimokawa et al, Am. J. Physiol. Heart Circ. Physiol, 301 : H287-H296, 2011),
- dysfonction endothéliale par la régulation négative de la production de NO et athérosclérose (Shimokawa et al, Cardiovasc . Res. 51 : 169-177, 2001),
- anévrisme aortique, occlusion des artères périphériques (Shimokawa et al, Am J Physiol Heart Circ Physiol, 301 : H287-H296, 2011),
- dysfonction érectile (Chitaley et al, Int J Impot Res, 24(2) : 49-60,2012),
- prolifération, mobilité des cellules endothéliales et angiogénèse (Imamura et al, Biochem, Biophys Res, 269(2) : 633-640, 2000),
- viscosité du sang et taux de fibrinogène élevé (Zhang et al, Central South Pharmacy, 3,035, 2008),
- différenciation des fibroblastes cardiaques en myofibroblastes (Kalluri et al, J. Cell. Physiol. 225 :631-637, 2010 ; Sabbadini et al, Circ.Res., 82 : 303-312, 2009 ; Rohr, Heart Rhythm, 6(6) : 848-856, 2009),
- remodelage ventriculaire et fibrose cardiaque après infarctus du myocarde (Hattori et al, Circulation, 109 : 2234-2239, 2004 ; Krum et al, Am J Physiol Heart Circ Physiol, 294 : H1804-H1814, 2008 ; Entman et al, Cardiovasc.Res., 83 : 511-518, 2009 ; Liu et al, Toxicology Letters, 211 : 91-97, 2012) et insuffisance cardiaque (Kishi et al, Circulation, 111 : 2741-2747, 2005),
- prolifération des cellules musculaires lisses et resténose (Shimokawa et al, Am J Physiol Heart Circ Physiol, 301 : H287-H296, 2011),
- diabète, hyperglycémie, insulino-résistance, néphropathies diabétiques (Kikuchi et al, J. Endocrinol., 192 : 595-603, 2007 ; Kolavennu et al, Diabetes, 57 :714-723, 2008) et insuffisance rénale, fibrose rénale, néphrosclérose (Matsuoka et al, J Hypertens, 26(9) :1837-48, 2008),
- activation des astrocytes du foie et maladies du foie telles que cirrhose, hépatite et cancer (WO2000064478A1, 2000),

- différenciation des fibroblastes de derme humain dans la sclérose systémique cutanée (Distler et al, Arthritis and Rheumatism, 58(8) : 2553-2564, 2008),
- fibrose intestinale post-radiothérapie par différenciation des cellules musculaires lisses (Vozenin-Brotons et al, Gut, 54(3) :336-343, 2005),
- adhérence, migration, phagocytose des macrophages et maladies inflammatoires (Schwartz et al, the EMBO Journal, 26: 505-515, 2007 ; Doe et al, J.Pharmacol.Exp.Ther., 320 :89-98, 2007),
- vasospasme cérébral et l'ischémie qui en résulte avec dysfonction neurologique (Shibuya et al, J.Neurol.Science, 238 : 31-39, 2005),
- dégénérescence neuronale telle que la maladie d'Alzheimer (Zhou et al, Science, 302 : 1215-1217, 2003 ; Song et al, CNS Neurosci. Ther. 19, 603-610, 2013),
- douleurs neuropathiques (Xiao et al, Brain, Behaviour and Immunity, 23(8) : 1083-88, 2009),
- récupération neurologique après atteinte de la moelle épinière (Hara et al, J. Neurosurg. 93 (suppl.1) :94-101, 2000 ; Dergham et al, J. Neurosci. 22, 6570-6577, 2002 ; Yamashita et al, Ther. Clin. Risk Manag., 4(3) : 605-615, 2008),
- prolifération et migration cellulaire (Feng et al, Current Topics in Medical Chemistry, 9, 704-723, 2009 ; Utsunomiya et al, Biochemical and Biophysical Research Communication, 402 :725-730,2010),
- formation de métastases et développement du cancer du sein, du poumon, du côlon, du cerveau, de la tête et du cou (Liu et al, Cancer Res, 69 : 8742-8751, 2009 ; Li et al, FEBS Lett. 580 : 4252-4260, 2006 ; Vishnubhotla et al, Lab.Invest. 87 : 1149-1158, 2007 ; Zohrabian et al, Anticancer Res. 29 : 119-123, 2009 ; Torre et al, Arch. Otolaryngol. Head Neck Surg., 136 : 493-501, 2010 ; Ying et al, Mol.Cancer Ther., 5 : 2158-2164, 2006),
- activation des ostéoclastes (migration) et de la résorption osseuse (Hruska etal, J Biol chem, 278(31) : 29086-97,2003),
- contraction des cellules musculaires lisses bronchiques, maladies broncho-pulmonaires chroniques et asthme (Mori et al, Am. J. Resp. Cell. Mol. Biol., 20(6) : 1190-1200, 1999 ; Kanaide et al, Br J Pharmacol, 132 : 111-118, 2001),
- augmentation de la voie de signalisation de SREBP (Sterol Response Binding Element) sous l'effet du shear stress et activation du gène codant pour le récepteur aux LDL (Lin et al, Cir. Res., 92(12) : 1296-1304, 2003).

Ainsi, un composé qui aurait la capacité d'inhiber la Rho kinase et la phosphorylation de la chaîne légère de la myosine, pourrait prévenir ou traiter les maladies cardiovasculaires ou non-cardiovasculaires telles que : l'hypertension artérielle systémique, l'hypertension artérielle pulmonaire, le glaucome, les rétinopathies, les dégénérescences du nerf optique, les pathologies de la cornée, les maladies coronariennes telles que l'angor, l'infarctus du myocarde, la resténose post-angioplastie, l'anévrisme aortique, l'occlusion des artères périphériques, l'athérosclérose, la fibrose cardiaque et l'insuffisance cardiaque, la dysfonction érectile, les maladies pulmonaires broncho-obstructives telles que l'asthme ou le syndrome de détresse respiratoire chez l'adulte, la fibrose intestinale post-radiation, la sclérose systémique cutanée, la fibrose pulmonaire associée à l'hypertension artérielle pulmonaire, la prévention ou le traitement des maladies hépatiques, la fibrose et la glomérulo-sclérose rénale, les néphropathies diabétiques induites ou non par l'hypertension, les maladies thrombotiques, le vaso-spasme cérébral et l'ischémie cérébrale résultante, les douleurs neuropathiques, les maladies neuronales dégénératives telles que la maladie d'Alzheimer, les maladies inflammatoires, le développement du cancer et sa progression par les métastases, l'ostéoporose, le métabolisme lipidique.

Des inhibiteurs de Rho kinase possédant un squelette isoquinoléine sont décrits dans plusieurs demandes de brevet.

On peut citer par exemple la demande WO2005/035 503 qui décrit des inhibiteurs de Rho kinase pour le traitement du glaucome.

La demande EP 0 187 371 décrit des inhibiteurs de Rho kinase possédant un squelette isoquinoléine avec une fonction sulfonamide pour le traitement du glaucome, et en particulier le Fasudil.

On peut également citer les demandes de brevet WO2007/000 240, WO2007/012 421, WO2007/012 422, WO2008/077 550, WO2008/077 552, WO2008/077 553, WO2008/077 554, WO2008/077 555, WO2008/077 556, WO2009/156 092, WO2009/156 099 et WO2009/156 100 qui décrivent des inhibiteurs de Rho kinase utiles dans le traitement de l'hypertension et du glaucome.

La présente invention concerne les composés de formule (I)

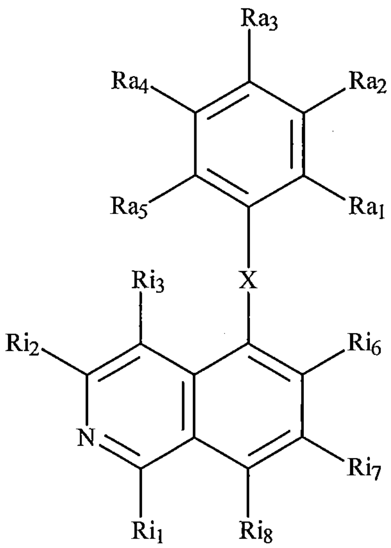

(I)

dans laquelle :

- X représente un groupement -C(=O), -CH(OH)- ou $-CH_2-$,
- $Ri_1$ représente un atome d'hydrogène ou un groupement hydroxyle, étant entendu que les composé de formule (I) pour lesquels $Ri_1$ représente un groupement hydroxyle peuvent être représentés sous la forme tautomère suivante :

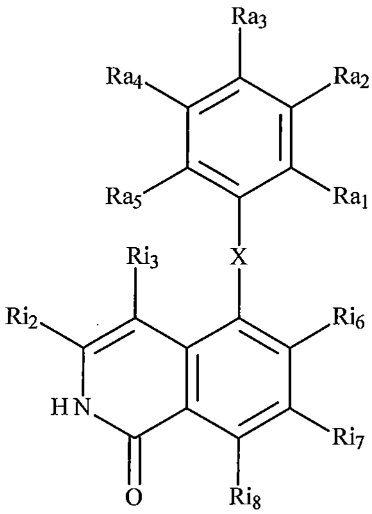

- $Ri_2$ et $Ri_3$, identiques ou différents, représentent chacun un atome d'hydrogène, un groupement $(C_1-C_6)$alkyle ou un atome d'halogène,
- $Ri_6$, $Ri_7$ et $Ri_8$, identiques ou différents, représentent chacun un atome d'hydrogène ou un atome d'halogène,
- $Ra_1$ et $Ra_5$, identiques ou différents, représentent chacun un atome d'hydrogène ou d'halogène, un groupement $-O(C_1-C_6)$alkyle ou un groupement $(C_1-C_6)$alkyle,
- $Ra_2$ représente un atome d'hydrogène ou d'halogène, un groupement hydroxyle, un groupement $-O(C_1-C_6)$alkyle, un groupement $-(C_1-C_6)$alkyle, un hétérocycle azoté possédant de 3 à 7 chaînons ou un groupement $-O-(CH2)_m-NR'R''$,
- $Ra_3$ représente un atome d'hydrogène, un groupement $-O(C_1-C_6)$alkyle, un groupement $-(C_1-C_6)$alkyle, un hétérocycle azoté possédant de 3 à 7 chaînons, ou un groupement $-CRy_1Ry_2NH(Ry_3)$
- $Ra_4$ représente un atome d'hydrogène ou d'halogène, un groupement $-O(C_1-C_6)$alkyle, un groupement $-(C_1-C_6)$alkyle, ou un groupement $-CRy_1Ry_2NH(Ry_3)$,
  étant entendu que :

  - $Ra_1$, $Ra_2$, $Ra_3$, $Ra_4$ et $Ra_5$ ne peuvent représenter simultanément un atome d'hydrogène,

- $Ra_3$ et $Ra_4$ ne peuvent représenter simultanément un groupement $-CRy_1Ry_2NH(Ry_3)$,
- $Ra_1$ et $Ra_2$ peuvent former ensemble avec les atomes de carbone qui les portent un hétérocycle possédant de 4 à 7 chaînons choisi parmi le tétrahydrofurane, le 1,4-dioxane, le tétrahydropyrane, la tétrahydro-2*H*-pyran-4-amine ou la 1-(tétrahydro-2*H*-pyran-4-yl)méthanamine, et
- $Ra_2$ et $Ra_3$ peuvent former ensemble avec les atomes de carbone qui les portent un cycle hydrogénocarboné possédant de 4 à 7 chaînons choisi parmi le cyclopentane, la cyclopentanamine, le *N*-cyclopentylglysinamide ou la 1-méthylcyclopentanamine,

- m est un nombre entier dont la valeur est fixée à 1,2 ou 3,
- R' et R", identiques ou différents, représentent chacun des groupements $-(C_1-C_6)$alkyle, ou bien forment ensemble avec l'atome d'azote qui les porte un hétérocycle possédant de 3 à 7 chaînons,
- $Ry_1$ représente un atome d'hydrogène, un groupement $-(C_1-C_6)$alkyle, un groupement $-CH_2$-cyclohexyle, ou un groupement 3-méthoxyphényle,
- $Ry_2$ représente un atome d'hydrogène ou un groupement $-(C_1-C_6)$alkyle,
- $Ry_3$ représente :

  - un atome d'hydrogène,
  - un groupement $-C(=O)-CHRy_4-NHRy_5$ avec $Ry_4$ représentant un atome d'hydrogène ou un groupement $(C_1-C_6)$alkyle et $Ry_5$ représentant un atome d'hydrogène ou un groupement méthyle, ou
  - un groupement $-(C_1-C_6)$alkyle qui peut être substitué par un groupement hydroxyle, un groupement $-O(C_1-C_3)$alkyle, un groupement cyclohexyle ou un groupement méthylsulfonyle,

ou $Ry_1$ et $Ry_2$ forment ensemble avec l'atome de carbone qui les porte un groupement cyclopropane, cyclobutane ou tétrahydropyrane,
ou $Ry_2$ et $Ry_3$ forment ensemble avec les atomes de carbone et d'azote qui les portent respectivement un groupement pyrrolidine ou pipéridine,
leurs isomères optiques lorsqu'ils existent, ainsi que leurs sels d'addition à un acide pharmaceutiquement acceptable et leurs hydrates.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, *para*-toluènesulfonique, benzènesulfonique, camphorique, pamoïque, 1,5-naphtalènedisulfonique.

Les groupements $(C_1-C_6)$alkyle peuvent être linéaires ou ramifiés.

Les cycles hydrogénocarbonés ou les hétérocycles présents dans les composés de formule (I) de la présente invention peuvent être substitués par un ou plusieurs atomes d'halogène et/ou par un ou plusieurs des groupements suivants : $-NH_2$, hydroxyle, $-(C_1-C_6)$alkyle, $-O(C_1-C_6)$alkyle, $-NH(C_1-C_6)$alkyle, $-(C_1-C_6)$alkyl$-NH_2$, $-NH-C(=O)-(C_1-C_6)$alkyl$-NH_2$

Lorsque R' et R" forment ensemble avec l'atome d'azote qui les porte un hétérocycle, substitué ou non, possédant de 3 à 7 chaînons, cet hétérocycle est préférentiellement choisi parmi la morpholine, la pyrrolidine, la pipéridine ou la *N*-méthylpipéridine.

Lorsque $Ra_2$ ou $Ra_3$ représente un hétérocycle azoté possédant de 3 à 7 chaînons, cet hétérocycle peut être choisi parmi la liste non limitative suivante : aziridine, azétidine, imidazoline, pyrrolidine, pipéridine, pipérazine, imidazole, pyrrole, pyridine, pyrimidine, pyridazine, 1,2,3,4-tétrahydropyrimidine, hexahydropyrimidine, hexahydropyridazine.

Un aspect de l'invention concerne les composés de formule (I) pour lesquels X représente un groupement $-C(=O)-$, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ri_1$ représente un groupement hydroxyle, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ri_2$ ou $Ri_3$ représente un groupement $(C_1-C_6)$alkyle, plus particulièrement un groupement méthyle ou éthyle, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ri_2$ et/ou $Ri_3$ représentent un atome d'hydrogène, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ri_6$ et/ou $Ri_7$ et/ou $Ri_8$ représentent un atome d'hydrogène, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ri_2$, $Ri_6$, $Ri_7$ et $Ri_8$ représentent chacun un atome d'hydrogène, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ra_1$ et/ou $Ra_5$ représentent un atome d'hydrogène ou un atome d'halogène, plus particulièrement un atome de chlore ou de fluor, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ra_1$ et $Ra_5$ représentent chacun un atome de fluor, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ra_2$ représente un atome d'hydrogène, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ra_3$ et $Ra_4$ représentent un atome d'hydrogène ou un groupement $-CRy_1Ry_2NH(Ry_3)$, étant entendu que $Ra_3$ et $Ra_4$ ne peuvent représenter simultanément un groupement $-CRy_1Ry_2NH(Ry_3)$, leurs isomères optiques lorsqu'ils existent, ainsi que leurs sels d'addition à un acide pharmaceutiquement acceptable et leurs hydrates.
Un autre aspect de l'invention concerne les composés de formule (I) pour lesquels $Ra_3$ ou $Ra_4$ représente un groupement $-CRy_1Ry_2NH(Ry_3)$, leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ra_3$ ou $Ra_4$ représente un groupement $-CRy_1Ry_2NH(Ry_3)$ et :

- $Ry_1$ représente un atome d'hydrogène ou un groupement $-(C_1-C_6)$alkyle,
- $Ry_2$ représente un groupement $-(C_1-C_6)$alkyle,
- $Ry_3$ représente un atome d'hydrogène,

leurs isomères optiques lorsqu'ils existent, ainsi que leurs sels d'addition à un acide pharmaceutiquement acceptable et leurs hydrates.

Un autre aspect de l'invention concerne les composés de formule (I) pour lesquels $Ra_3$ représente un groupement $-CRy_1Ry_2NH(Ry_3)$ et $Ra_1$ et $Ra_2$ forment ensemble avec les atomes de carbone qui les portent un hétérocycle possédant de 4 à 7 chaînons, leurs isomères optiques lorsqu'ils existent, ainsi que leurs sels d'addition à un acide pharmaceutiquement acceptable et leurs hydrates.

De façon préférentielle, un tel hétérocycle est choisi parmi le tétrahydrofurane, le 1,4-dioxane ou le tétrahydropyrane.

Un autre aspect de l'invention concerne les composés de formule (I) pour lesquels $Ra_3$ représente un atome d'hydrogène et $Ra_1$ et $Ra_2$ forment ensemble avec les atomes de carbone qui les portent un hétérocycle possédant de 4 à 7 chaînons, leurs isomères optiques lorsqu'ils existent, ainsi que leurs sels d'addition à un acide pharmaceutiquement acceptable et leurs hydrates.

De façon préférentielle un tel hétérocycle est choisi parmi la tétrahydro-2*H*-pyran-4-amine ou la 1-(tétrahydro-2*H*-pyran-4-yl)méthanamine.

Un autre aspect de l'invention concerne les composés de formule (I) pour lesquels $Ra_2$ et $Ra_3$ forment ensemble avec les atomes de carbone qui les portent un cycle hydrogénocarboné possédant de 4 à 7 chaînons, leurs isomères optiques lorsqu'ils existent, ainsi que leurs sels d'addition à un acide pharmaceutiquement acceptable et leurs hydrates.

De façon préférentielle un tel cycle hydrogénocarboné est choisi parmi le cyclopentane et ses dérivés, plus particulièrement la cyclopentanamine, le *N*-cyclopentylglysinamide ou la 1-méthylcyclopentanamine.

Un autre aspect de l'invention concerne les composés de formule (I) pour lesquels :

- X représente un groupement -C(=O)-,
- $Ri_1$ représente un atome d'hydrogène ou un groupement hydroxyle,
- $Ri_2$, $Ri_6$, $Ri_7$ et $Ri_8$ représentent chacun un atome d'hydrogène et $Ri_3$ représente un atome d'hydrogène ou un groupement $(C_1-C_6)$alkyle,
- $Ra_1$ et $Ra_5$, identiques ou différents, représentent chacun un atome d'hydrogène ou de fluor, ou un groupement $(C_1-C_6)$alkyle,
- $Ra_2$ représente un atome d'hydrogène ou un groupement $-(C_1-C_6)$alkyle,
- $Ra_3$ représente un atome d'hydrogène, un groupement pipéridine, ou un groupement $-CRy_1Ry_2NH(Ry_3)$,
- $Ra_4$ représente un atome d'hydrogène ou un groupement $-CRy_1Ry_2NH(Ry_3)$, étant entendu que $Ra_3$ et $Ra_4$ ne peuvent représenter simultanément un groupement$-CRy_1Ry_2NH(Ry_3)$, et que :

- $Ra_3$ représentant un groupement $-CRy_1Ry_2NH(Ry_3)$, $Ra_1$ et $Ra_2$ peuvent former ensemble avec les atomes de carbone qui les portent un groupement tétrahydrofurane, 1,4-dioxane ou tétrahydropyrane, ou
- $Ra_3$ représentant un atome d'hydrogène, $Ra_1$ et $Ra_2$ peuvent former ensemble avec les atomes de carbone qui les portent un groupement tétrahydro-2*H*-pyran-4-amine ou 1-(tétrahydro-2*H*-pyran-4-yl)méthanamine, ou
- $Ra_2$ et $Ra_3$ peuvent former ensemble avec les atomes de carbone qui les portent un groupement cyclopentanamine ou 1-méthylcyclopentanamine,

- $Ry_1$ représente un atome d'hydrogène, un groupement $-(C_1-C_6)$alkyle, ou un groupement $-CH_2$-cyclohexyle,
- $Ry_2$ représente un atome d'hydrogène ou un groupement $-(C_1-C_6)$alkyle,
- $Ry_3$ représente un atome d'hydrogène ou un groupement $-(C_1-C_6)$alkyle qui peut être substitué par un groupement hydroxyle,

leurs isomères optiques lorsqu'ils existent, ainsi que leurs sels d'addition à un acide pharmaceutiquement acceptable et leurs hydrates.

Un autre aspect de l'invention concerne les composés de formule (I) choisi parmi :

- la [4-(1-aminoéthyl)-2,6-difluorophényl](isoquinoléin-5-yl)méthanone ainsi que ses isomères optiques et leurs sels d'addition à un acide pharmaceutiquement acceptable et leurs hydrates,
- la [4-((1*R*)-1-aminoéthyl)-2,6-difluorophényl](isoquinoléin-5-yl)méthanone ainsi que ses sels d'addition à un acide pharmaceutiquement acceptable et leurs hydrates,
- la [4-(1-aminoéthyl)-2,6-difluorophényl](1-hydroxyisoquinoléin-5-yl)méthanone ainsi que ses isomères optiques et leurs sels d'addition à un acide pharmaceutiquement acceptable et leurs hydrates,
- la 1-[3,5-difluoro-4-(isoquinoléin-5-ylméthyl)phényl]éthanamine ainsi que ses isomères optiques et ses sels d'addition à un acide pharmaceutiquement acceptable et leurs hydrates,
- le {4-[(1*S*)-1-aminoéthyl]-2,6-difluorophényl}(isoquinoléin-5-yl)méthanol ainsi que ses sels d'addition à un acide pharmaceutiquement acceptable et leurs hydrates,
- la [4-(2-aminopropan-2-yl)-2,6-difluorophényl](isoquinoléin-5-yl)méthanone ainsi que ses sels d'addition à un acide pharmaceutiquement acceptable et leurs hydrates,
- la 5-[4-(2-aminopropan-2-yl)-2,6-difluorobenzoyl]isoquinoléin-1(2*H*)-one ainsi que ses sels d'addition à un acide pharmaceutiquement acceptable et leurs hydrates,
- la 5-[4-(1-aminoéthyl)-2-fluoro-3-méthoxybenzoyl]isoquinoléin-1(2*H*)-one ainsi que ses isomères optiques et leur sels d'addition à un acide pharmaceutiquement acceptable et leurs hydrates,
- la 5-({5-[(1*R*)-1-aminoéthyl]-3,4-dihydro-2*H*-chromén-8-yl}carbonyl)isoquinoléin-1(2*H*)-one ainsi que ses sels d'addition à un acide pharmaceutiquement acceptable et leurs hydrates,
- la 5-{4-[(1*R*)-1-aminoéthyl]-2-méthylbenzoyl}isoquinoléin-1(2*H*)-one ainsi que ses sels d'addition à un acide pharmaceutiquement acceptable et leurs hydrates,
- la 5-(2,6-difluoro-4-{1-[(2-hydroxyéthyl)amino]éthyl}benzoyl)isoquinoléin-1(2*H*)-one ainsi que ses isomères optiques et leurs sels d'addition à un acide pharmaceutiquement acceptable et leurs hydrates,
- la 5-{4-[(1*R*)-1-aminoéthyl]-2,6-difluorobenzoyl}-4-méthylisoquinoléin-1(2*H*)-one ainsi que ses sels d'addition à un acide pharmaceutiquement acceptable et leurs hydrates,
- la 5-{3-[(1*R*)-1-aminoéthyl]-2,6-difluorobenzoyl}isoquinoléin-1(2*H*)-one ainsi que ses sels d'addition à un acide pharmaceutiquement acceptable et leurs hydrates,
- la 5-[(1-amino-4,6-difluoro-2,3-dihydro-1*H*-indén-5-yl)carbonyl]isoquinoléin-1(2*H*)-one ainsi que ses isomères optiques et leurs sels d'addition à un acide pharmaceutiquement acceptable et leurs hydrates,
- la 5-{[(3*R*)-3-amino-4,6-difluoro-2,3-dihydro-1*H*-indén-5-yl]carbonyl} isoquinoléin-1(2*H*)-one ainsi que ses sels d'addition à un acide pharmaceutiquement acceptable et leurs hydrates,
- la 5-({8-[(1*R*)-1-aminoéthyl]-2,3-dihydro-1,4-benzodioxin-5-yl}carbonyl) isoquinoléin-1(2*H*)-one ainsi que ses sels d'addition à un acide pharmaceutiquement acceptable,
- la 5-[2,6-difluoro-4-(pipéridin-2-yl)benzoyl]isoquinoléin-1(2*H*)-one ainsi que ses isomères optiques et leurs sels d'addition à un acide pharmaceutiquement acceptable et leurs hydrates,
- la 5-[4-(1-amino-2-cyclohexyléthyl)-2,6-difluorobenzoyl]isoquinoléin-1(2*H*)-one ainsi que ses isomères optiques et leurs sels d'addition à un acide pharmaceutiquement acceptable et leurs hydrates,
- la 5-{[4-(aminométhyl)-3,4-dihydro-2*H*-chromén-8-yl]carbonyl}isoquinoléin-1(2*H*)-one ainsi que ses isomères optiques et leurs sels d'addition à un acide pharmaceutiquement acceptable et leurs hydrates.

Un autre aspect de l'invention concerne un procédé de synthèse des composés de formule (Ia), cas particuliers des composés de formule (I) pour lesquels X représente un groupement -C(=O), à partir d'un composé de formule (II) :

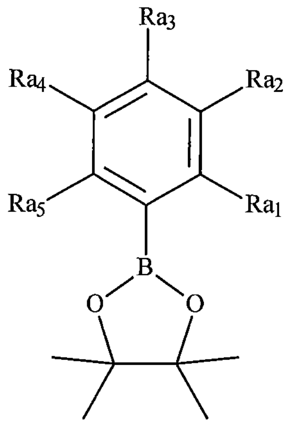

(II)

qui est soumis à une réaction de couplage avec le composé de formule (III) :

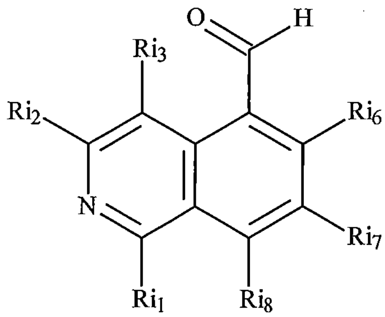

(III)

en présence d'un catalyseur au rhodium ou au palladium, d'une phosphine et d'une base dans un solvant organique, pour conduire au composé de formule (Ia) :

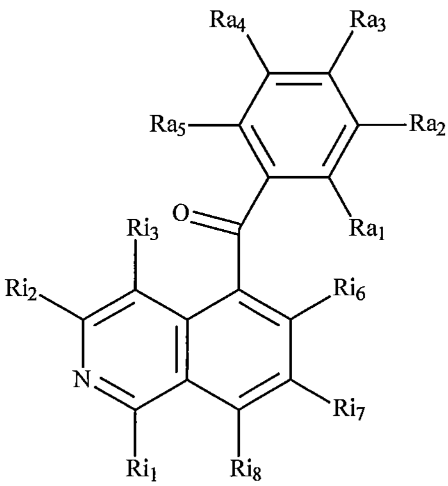

(Ia)

Parmi les catalyseurs au rhodium et au palladium pouvant être utilisés pour effectuer la réaction de couplage entre le composé de formule (II) et le composé de formule (III) on peut citer à titre non limitatif les catalyseurs suivants : ($[Rh(CH_2CH_2)_2Cl]_2$, Rh(acac)(coe)2 (coe = cyclooctène) et le complexe de tris(dibenzylidéneacétone)dipalladium/chloroforme ($Pd_2dba_3$-$CHCl_3$).

Parmi les phosphines pouvant être utilisées pour effectuer la réaction de couplage entre le composé de formule (II) et le composé de formule (III) on peut citer à titre non limitatif la tri-butylphosphine, le 1,3-bis(diphénylphosphino)propane (dppp) et 1,1'-Bis(diphénylphosphino)ferrocene (dppf).

Parmi les bases pouvant être utilisées pour effectuer la réaction de couplage entre le composé de formule (II) et le composé de formule (III) on peut citer à titre non limitatif le carbonate de postassium et l'hydrogénocarbonate de

potassium.

Parmi les solvants organiques pouvant être utilisés pour effectuer la réaction de couplage entre le composé de formule (II) et le composé de formule (III) on peut citer à titre non limitatif le 1,4-dioxane, le diméthoxyéthane et le toluène.

Les composés de formule (Ia) ainsi obtenus peuvent être ensuite transformés en composés de formule (Ib), cas particuliers de composés de formule (I) pour lesquels X représente - CH(OH)-, par une réaction de réduction :

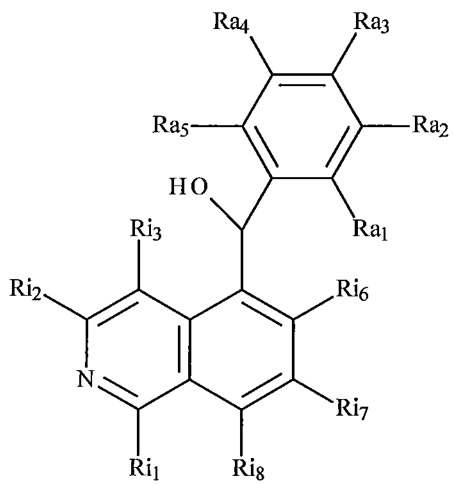

(Ib)

Cette réaction de réduction peut être effectuée en présence de donneurs d'hydrures, tels que le tétraborohydrure de sodium ($NaBH_4$).

Les composés de formule (Ib) ainsi obtenus peuvent être ensuite transformés en composés de formule (Ic), cas particuliers de composés de formule (I) pour lesquels X représente - $CH_2$-, par une nouvelle réaction de réduction, qui peut être effectuée en présence d'acide trifluoroacétique et de triéthylsilane :

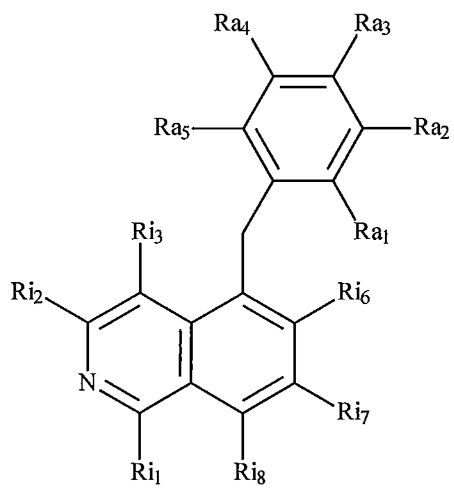

(Ic)

Les formes optiquement actives des composés de formule (I) sont obtenues, soit à partir de formes optiquement actives du composé de formule (III), soit par dédoublement des formes racémiques des composés de formule (I), selon des méthodes connues de la littérature.

La présente invention a également pour objet les compositions pharmaceutiques contenant comme principe actif un composé de formule (I), ou son sel d'addition à un acide pharmaceutiquement acceptable, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre le composé de formule (I), les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :

- ♦ *pour les diluants* : le lactose, le dextrose, le sucrose, le mannitol, le sorbitol, la cellulose, la glycérine,
- ♦ *pour les lubrifiants* : la silice, le talc, l'acide stéarique et ses sels de magnésium et de calcium, le polyéthylène glycol,
- ♦ *pour les liants* : le silicate d'aluminium et de magnésium, l'amidon, la gélatine, la tragacanthe, la méthylcellulose, la carboxyméthylcellulose de sodium et la polyvinylpyrrolidone,
- ♦ *pour les désintégrants* : l'agar, l'acide alginique et son sel de sodium, les mélanges effervescents.

Le pourcentage de principe actif de formule (I) dans la composition pharmaceutique est préférentiellement compris entre 5% et 50% en poids.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent l'invention.

**Liste des abréviations utilisées**

| | |
|---|---|
| AcOEt : | acétate d'éthyle |
| $Boc_2O$ : | dicarbonate de di tert-butyle |
| DMF : | diméthylformamide |
| DMSO : | diméthylsulfoxyde |
| DTT : | dithiothréitol |
| EDTA : | acide éthylène diamine tétraacétique |
| EGTA | Acide éthylènebis(oxyéthylènenitrilo)tétraacétique |
| ESI : | electrospray ionization (ionisation par éléctronébuliseur) |
| éq.: | équivalent(s) |
| GC : | gas chromatography (chromatographie en phase gazeuse) |
| Hepes : | acide 4-(2-hydroxyéthyl)-1-pipérazine éthanesulfonique |
| HRMS : | high resolution mass spectrometry (spectrométrie de masse haute résolution) |
| IR : | infrarouge |
| i.v.: | intraveineuse |
| KHMDS : | hexaméthyl-disilazane de potassium |
| LCMS : | liquid chromatography-mass spectrometry (chromatographie en phase liquide-spectrométrie de masse) |
| LDA : | diisopropylamidure de lithium |
| *m*-CPBA : | acide méta-chloroperbenzoïque |
| MS: | mass spectrometry (spectrométrie de masse) |
| $NEt_3$: | triéthylamine |
| TMSCN : | cyanure de triméthylsilyle |
| SFC : | chromatographie en phase supercritique |
| RMN : | résonance magnétique nucléaire |
| tBuOK | *tert*-butanoate de potassium |
| THF : | tétrahydrofurane |
| TMS : | tétraméthylsilane |
| TMSCN | triméthylsilanecarbonitrile |
| Tris : | trishydroxyméthylaminométhane ou 2-amino-2-hydroxyméthyl-1,3-propanediol |

La présence du signe « * » à proximité d'un atome de carbone dans les formules chimiques souligne que ce carbone a une configuration absolue fixée, sans que la nature de cette configuration ait été identifiée.

Les spectres infrarouges ont été enregistrés à l'aide d'un spectromètre à transformée de Fourrier Bruker TENSOR 27 en mode ATR.

Les spectres RMN du proton ont été enregistrés sur des spectromètres Bruker DPX 400-B. Les déplacements chimiques sont exprimés en ppm et sont déterminés par rapport au TMS utilisé comme référence. Les abréviations utilisées sont :

- s : singulet
- d : doublet
- dd : doublet dédoublé
- dt : doublet triplé
- t : triplet
- td : triplet dédoublé
- quad : quadruplet
- quint : quintuplet
- m : multiplet

Les spectres de masse sont enregistrés sur un spectromètre TSQ 7000.

Les contrôles GC ont été effectués sur une colonne HPS-J&W Scientific 0.53 x 15 m avec un chromatographe GC Agilent 4890 à détection par ionisation de flamme (FID).

Les contrôles par HPLC ont été effectués sur des colonnes Acquity UPLC BEH C18 1.7 μm 2.1 x 30 mm sur des HPLC 1200 Agilent avec détecteur à barrettes de diodes (DAD).

SFC : Les analyses de pureté énantiomèrique sont réalisées sur un UPC2 (Waters).

Les chromatographies sur couche mince (CCM) ont été effectuées sur des plaques de silice MERCK 60F-254.

Les chromatographies ont été effectuées avec un gel de silice MERCK 60 (0.040-0.063 mm) ou avec des colonnes de silice prépackées Interchim ou Grace.

Les séparations en phase inverse ont été réalisées sur des colonnes Interchim FHP RP C18 15 μm 275 x 60 mm avec détection UV.

Les filtrations ont été effectuées sur des filtres de type GVHP (0.22 μm) Millipore pour les phases organiques et sur des filtres de la marque Whatman GF/A cat. No1820-070 pour les phases aqueuses.

**Préparation des composés de l'invention**

*Réparation des précurseur isoguinoléines*

**Protocole I : Préparation d'intermédiaires 5-halo-isoquinoléines**

Les intermédiaires suivants ont été préparés par bromation des isoquinoléines commerciales selon le protocole décrit par Brown, W. D. ; Gouliaev, A. H., Synthesis, 2002, 1, 83-86 et Organic Syntheses, 2004, 81, 98-104.

**Intermédiaire 2 :**

**RMN $^1$H** (300MHz-DMSO-$d_6$): δ 9,40 (s,1H), 8,70 (d,1H), 8,22 (d,1H), 8,18 (d,1H), 7,93 (d,1H), 7,65 (t,1H)
**IR(cm$^{-1}$):** 1621-1579, 819-629;

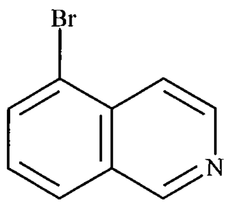

**2**

**Intermédiaire 290 :**

**RMN $^1$H** (400MHz-CDCl$_3$): δ 9,15 (s, 1H), 7,90 (2d, 2H), 7,80 (s, 1H), 7,35 (t, 1H), 2,75 (s, 3H)
**IR (cm$^{-1}$):** 1621-1584, 666
**GC-EI** (70 eV): M+. = 221.

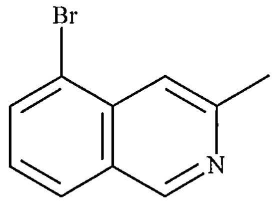

**290**

Intermédiaire 299 :

**RMN $^1$H** (400MHz-DMSO-$d_6$): δ 9,30 (s, 1H), 8,40 (s, 1H), 8,17 (t, 1H), 7,55 (t, 1H), 2,95 (s, 3H)
**IR (cm$^{-1}$):** 1609-1579

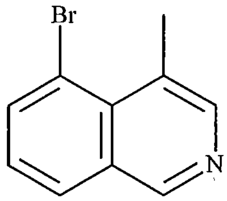

**299**

L'intermédiaire **754** a été préparé à partir de l'intermédiaire 2 selon le protocole suivant : A une solution d'$H_2SO_4$ concentré (48 mL) à température ambiante est ajouté l'intermédiaire **2** (10 g, 48 mmoles), puis du *N*-chlorosuccinimide (25 g, 187 mmoles). Le mélange est chauffé à 80°C pendant 5 jours. Le milieu réactionnel est versé sur un mélange glace/eau (33 g/300 mL) puis une solution de $NH_4OH$ 28% est ajoutée jusqu'à pH 8. Le précipité formé est filtré, puis dissous dans de l'AcOEt, la phase organique est séchée sur $MgSO_4$, la concentration sous vide conduit à l'obtention de l'intermédiaire **754** sous forme d'un solide beige (11 g) qui peut être utilisé sans traitement supplémentaire dans l'étape suivante.
**RMN $^1$H** (300MHz-DMSO-$d_6$): δ 9,60 (s, 1H), 8,80 (d, 1H), 8,20 (d, 1H), 8,00 (d, 1H), 7,80 (d, 1H);
**IR (cm$^{-1}$):** 1607, 1568, 830, 631.

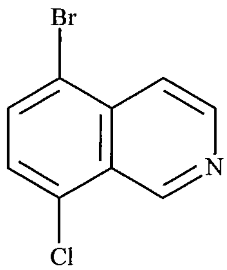

**754**

Protocole II : Préparation de dérivés 5-halo-1-alkoxysoquinoléine

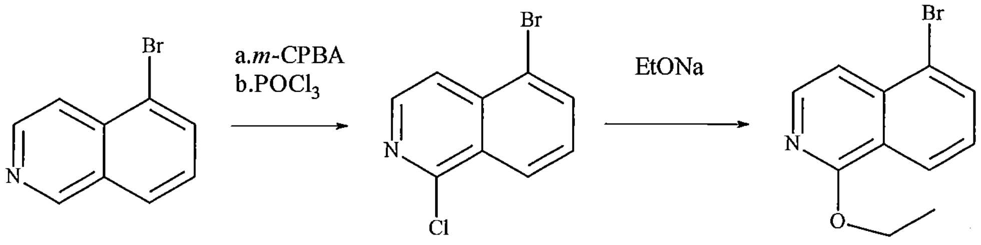


**2** **123** **124**

**Intermédiaire 123 :**

A une solution de **2** (60 g, 288 mmoles) dans du chlorure de méthylène (1,5 L) sont ajoutés (75 g, 436 mmoles) de *m*-CPBA à 75 %. Le mélange est chauffé à 40°C pendant 20 heures. Après contrôle HPLC et retour à température ambiante, 75 g de sodium thiosulfate sont ajoutés en 10 minutes, puis 300 mL d'eau. L'ensemble est décanté, la phase organique est lavée par une solution de soude 1N, la phase organique est séchée par passage sur $MgSO_4$. L'évaporation sous pression réduite conduit à l'obtention d'un solide blanc (42 g) qui est utilisé sans traitement supplémentaire dans l'étape suivante. Une solution de l'intermédiaire formé (37 g, 165 mmoles) dans du chlorure de méthylène (900 mL) et du $POCl_3$ (37 mL) est agité 18 heures à 45°C. Après contrôle HPLC et/ou GC le mélange réactionnel est concentré sous vide. Le résidu est traité avec précaution par de l'eau, la phase aqueuse est extraite par du chlorure de méthylène. La phase organique est lavée avec précaution par une solution de $NaHCO_3$ saturée, puis par une solution aqueuse saturée en NaCl. L'évaporation sous pression réduite conduit à l'obtention de l'intermédiaire **123** (33 g) sous la forme d'un solide beige qui peut être utilisé sans traitement supplémentaire dans l'étape suivante.
**RMN $^1$H** (400MHz-$CDCl_3$): δ 8,35 (2d, 2H), 8,00 (2d, 2H), 7,55 (t, 1H)
**GC-EI (70 eV):** M+. = 241

**Intermédiaire 124 :**

A une solution d'éthylate de sodium, préparée par ajout de sodium (10,3 g) dans de l'éthanol (323 mL), est ajouté l'intermédiaire **123** par portions (15 g, 62 mmoles). Le mélange est chauffé pendant 2 heures. Après contrôle HPLC et retour à température ambiante, le milieu réactionnel est coulé sur un mélange d'eau et de glace (3 kg): le produit précipite. La filtration du solide conduit à l'obtention d'un solide qui par recristallisation dans de l'acétonitrile conduit à l'obtention de l'intermédiaire **124** (8,06 g).
**RMN $^1$H** (40OMHz-DMSO-$d_6$): δ 8,20 (d, 1H), 8,15 (d, 1H), 8,10 (d, 1H), 7,55 (m, 1H), 7,45 (d, 1H), 4,55 (quad, 2H), 1,45 (t, 3H)
**GC-EI** (70 eV): $M^+$ = 251
Cette procédure a été utilisée pour préparer les intermédiaires **293** et **302.**

**Intermédiaire 293 :**

Obtenu à partir de l'intermédiaire **290** selon le **protocole II**
**RMN $^1$H** (400MHz-DMSO-$d_6$): δ 8,20 (td, 1H), 7,85 (dd, 1H), 7,35 (m, 1H), 7,30 (t, 1H), 4,55 (quad, 2H), 2,55 (s, 3H), 1,50 (t, 3H)

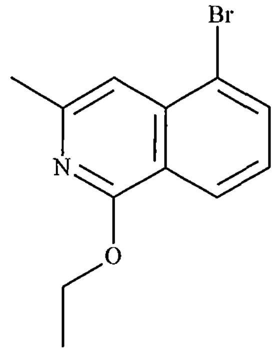

**293**

**Intermédiaire 302 :**

Obtenu à partir de **299** selon le **protocole II**

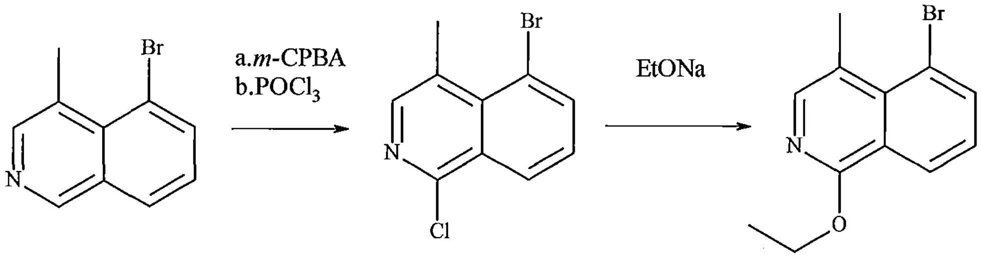


**299** **301** **302**

**Intermédiaire 301 :**

**RMN $^1$H** (400/500 MHz, dmso-d6): δ 8.29 (d, 1 H), 8.26 (d, 1 H), 8.22 (s, 1 H), 7.66 (m, 1 H), 2.94 (s, 3 H).
**IR ($cm^{-1}$):** 1603.
**GC-EI (70 eV):** M+. = 254.9

**Intermédiaire 302 :**

**RMN $^1$H** (400MHz-DMSO-$d_6$): δ 8,30 (dd, 1H), 8,10 (dd, 1H), 7,90 (s large, 1H), 7,50 (t, 1H), 4,50 (quad, 2H), 2,80 (s, 3H), 1,40 (t, 3H)

**Protocole III: Préparation d'intermédiaires isoquinoléine carbonylés selon une réaction d'échange halogène métal suivie d'une formylation.**

A titre d'exemple la synthèse de l'intermédiaire 125 est décrite ci après :

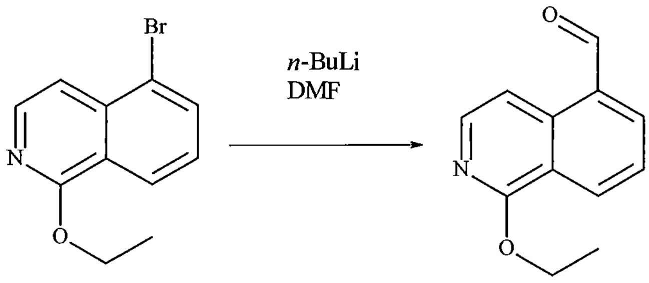


124 125

Intermédiaire 125 :

Une solution de l'intermédiaire **124** (21 g, 84 mmoles) dans du THF (106 mL) est ajoutée en 35 minutes sur une solution de *n*-BuLi 2,5N dans l'hexane (67 mL, 168mmoles) préalablement introduite dans un mélange THF/éther éthylique (270 mL/270 mL) à -78°C. Le mélange réactionnel est agité 1 heure à -78 C, puis une solution de DMF (10mL) dans du THF (30mL) préalablement refroidie à -70 C est alors cannulée sur le milieu en 5min. Le mélange réactionnel est agité pendant 35 minutes. Addition de 73 mL d'éthanol, puis 73 mL d'une solution aqueuse saturée en $NH_4Cl$. Retour à température ambiante. La phase aqueuse est extraite par de l'éther éthylique, séchée sur $MgSO_4$ et l'évaporation sous pression réduite conduit à l'obtention d'un solide jaune qui purifié sur gel de silice (éluant AcOEt/chlorure de méthylène 10/90). L'intermédiaire **125** (9 g) est obtenu sous forme d'un solide jaune.
**RMN $^1$H** (400MHz ; DMSO-$d_6$): δ 10,41 (s, 1H), 8,53 (ddd, 1H), 8,44 (dd,1H), 8,41 (dd,1H), 8,19 (d,1H), 7,85 (dd,1H), 4,55 (quad, 2H), 1,45 (t, 3H).
**IR ($cm^{-1}$):** 1691.

Ce protocole a été utilisé pour préparer les intermédiaires du tableau ci-dessous :

| Int. | Issu de | Nomenclature<br>Description analytique |
|---|---|---|
| 3 | 2 | **isoquinoléine-5-carbaldéhyde**<br>**RMN $^1$H** (300MHz ; DMSO-$d_6$): δ 10,50 (s, 1H), 9,50 (d, 1H), 8,90 (d,1H), 8,70 (d,1H), 8,50 (t et d,1H), 7,95 (dd,1H)<br>**IR ($cm^{-1}$):** 1693-1679 |
| 294 | 293 | **1-éthoxy-3-méthyl-isoquinoléine-5-carbaldéhyde**<br>**RMN $^1$H** (400MHz ; DMSO-$d_6$): δ 10,37 (s, 1H), 8,46 (d,1H), 8,34 (d,1H), 8,30 (s,1H), 7,73 (dd, 1H), 4,53 (quad, 2H), 2,53 (s, 3H), 1,45 (t, 3H)<br>**IR ($cm^{-1}$):** 1684 |
| 303 | 302 | **1-éthoxy-4-méthyl-isoquinoléine-5-carbaldéhyde**<br>**RMN $^1$H** (400MHz ; DMSO-$d_6$): δ 10,90 (s, 1H), 8,50 (dd,1H), 8,20 (dd,1H), 8,00 (s,1H), 7,75 (t, 1H), 4,50 (quad, 2H), 2,60 (s, 3H), 1,45 (t, 3H)<br>**IR ($cm^{-1}$):** 1677 |
| 321 | 299 | **4-méthyl-isoquinoléine-5-carbaldéhyde**<br>**RMN $^1$H** (400MHz ; DMSO-$d_6$): δ 10,90 (s, 1H), 9,20 (s,1H), 8,50 (s,1H), 8,25-8,20 (2d, 2H), 7,70 (t,1H), 2,80 (s, 3H) |
| 755 | 754 | **8-chloroisoquinoléine-5-carbaldéhyde**<br>**RMN $^1$H** (400MHz ; DMSO-$d_6$): δ 10,50 (s, 1H), 9,70 (s, 1H), 9,00 (d, 1H), 8,40 (d, 1H), 8,35 (d, 1H), 8,10 (d 1H)<br>**IR ($cm^{-1}$):** 1679 |

**Protocole IV: Préparation de l'intermédiaire 655**

**Intermédiaire 653 :**

A une solution (dégazée à l'azote) de l'intermédiaire **2** (45 g, 216 mmoles) dans le DMF (450 mL) sont ajoutés du $Zn(CN)_2$ (30 g) et du $Pd(PPh_3)_4$ (9,8 g), le mélange est chauffé à 100°C pendant 2 heures. Après retour à température ambiante, le milieu est repris par de l'AcOEt (200 mL) et de l'eau (2 L), le mélange est amené à pH>8 par ajout d'une solution aqueuse de NaOH 20%. Après ajout d'AcOEt (200 mL) la phase organique est recupérée par décantation, filtrée sur Celite®, lavée par une solution aqueuse saturée en NaCl puis séchée sur $MgSO_4$ avant d'être concentrée sous vide. Le solide est broyé et séché sous vide, repris par une solution aqueuse d'HCl 1N (1 L) et la phase aqueuse acide est lavée par de l'AcOEt, puis traitée par une solution aqueuse de NaOH 20%, le précipité formé est collecté et dissous dans du chlorure de méthylène. La solution est séchée sur $MgSO_4$, et l'évaporation sous vide conduit à l'intermédiaire **653** sous forme d'un solide beige (24 g).
**RMN $^1$H** (400MHz; DMSO-$d_6$): $\delta$ 9,53 (s, 1H), 8,76 (d, 1H), 8,54 (d, 1H), 8,45 (d, 1H), 7,96 (d, 1H), 7,87 (t, 1H)
**IR ($cm^{-1}$):** 2226.

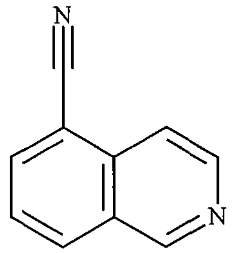

**653**

**Intermédiaire 654 :**

A une solution d'HCl 37% (137 mL) est ajouté l'intermédiaire **653** et le mélange est chauffé au reflux pendant 20h. Après retour à température ambiante, le précipité est collecté sur fritté, lavé par de l'acétone et séché à l'étuve à 50°C sous vide ($10^{-2}$ mbar). Le chlorhydrate de l'intermédiaire **654** est obtenu sous forme d'un solide blanc (33 g) utilisé dans l'étape suivante sans purification supplémentaire.
**RMN $^1$H** (300MHz; DMSO-$d_6$): $\delta$ 10,00 (s, 1H), 9,20 (d, 1H), 8,00 (m, 3H), 8,10 (t, 1H) **IR ($cm^{-1}$):** 1695, 1214

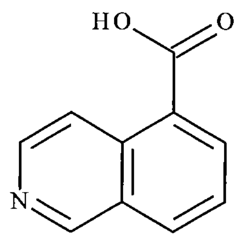

**654**

**Intermédiaire 655 :**

L'intermédiaire **654** (20 g, 95mmoles) est ajouté avec précaution à du chlorure de thionyle (200 mL). Le mélange est chauffé pendant 15 heures à 80°C. Après retour à température ambiante, le mélange est filtré, la concentration sous vide (avec précaution) du filtrat conduit au chlorhydrate de **655** sous la forme d'un solide marron qui est utilisé rapidement dans l'étape de substitution électrophile aromatique (**Protocole XXIII**). Le produit peut être analysé sous sa forme ester méthylique (par dérivation avec du méthanol)

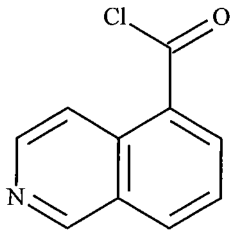

**655**

*Réparation des précurseurs phényles - protocoles généraux*

**Protocole V : Préparation d'intermédiaires aminés à partir d'intermédiaires phénylacétonitriles**

A titre d'exemple, la synthèse de l'intermédiaire 601 est décrite ci après :

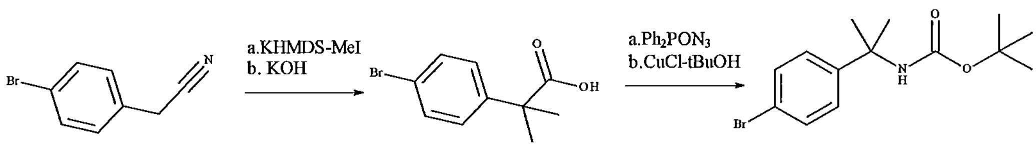


**599** **601**

**Intermédiaire 599 :**

Etape 1

A une solution de KHMDS (50 g) dans le THF (300 mL) refroidie à 0°C est ajouté lentement une solution de (4-bromophényl)acétonitrile commercial (16 g, 81 mmoles) dans le THF (90 mL) en maintenant une température inférieure à 3°C. Le mélange est agité 40 minutes à 0°C puis de l'iodure de méthyle (11,7 mL) est ajouté en 50 minutes avec une température inférieure à 8°C. Le milieu est agité à température ambiante pendant 20 heures, puis versé avec précaution sur de l'eau glacée (1,5 L). La phase aqueuse est extraite à l'$Et_2O$, la phase organique est lavée par une solution aqueuse saturée en NaCl, séchée et concentrée sous vide. Le résidu est chromatographié sur gel de silice (éluant $CH_2Cl_2$/Cyclohexane (60/40)). L'intermédiaire attendu (13 g) est obtenu sous forme d'un solide blanc.
**RMN $^1$H** (300MHz, $CDCl_3$): δ 7,52 (d, 2H), 7,36 (d, 2H), 1,75 (s, 6H)
**IR ($cm^{-1}$):** 2237

Etape 2

Une solution de l'intermédiaire obtenu ci-dessus (5 g, 22mmoles) et de KOH (2,4 g) dans un mélange d'éthanol (25 mL) et d'eau (7,5 mL) est chauffée au reflux pendant 20 heures. Le milieu réactionnel est concentré sous vide et le résidu est repris par de l'éther éthylique (100 mL) et de l'eau (60 mL). La phase aqueuse (exempte de solvant organique) est refroidie à 10°C, puis acidifiée par une solution d'HCl à 37%. Le précipité formé est collecté sur fritté et séché sous vide. L'intermédiaire **599** (5,3 g) est obtenu sous la forme d'un solide blanc.
**RMN $^1$H** (400MHz, $CDCl_3$): δ 9,00-8,00 (1H), 7,75 (d, 2H), 7,25 (d, 2H), 1,60 (s, 6H)
**IR ($cm^{-1}$):** 3347-2235, 1697

**Intermédiaire 601 :**

Etape 1

A une solution de l'intermédiaire **599** (2,6 g, 10,7mmoles) dans du toluène (60 mL) sont ajoutés de la $NEt_3$ (1,6 mL) et du $PhO_2PON_3$ (2,3 mL). Le milieu résultant est chauffé au reflux 20 heures Après retour à température ambiante, une solution aqueuse de $NaHCO_3$ saturée est ajoutée. La phase organique est extraite à l'$Et_2O$, lavée par une solution aqueuse de NaCl saturée, séchée et concentrée sous vide. Le résidu est chromatographié sur gel de silice (éluant $CH_2Cl_2$/Cyclohexane (50/50)). L'intermédiaire attendu (1,8 g) est obtenu sous forme d'une huile incolore.

**RMN $^1$H** (300MHz, $CDCl_3$): δ 7,50 (d, 2H), 7,35 (d, 2H), 1,70 (s, 6H)
**IR ($cm^{-1}$):** 2248

Etape 2

A un mélange de *tert*-butanol (3,5 mL) et de CuCl (0,74 g) dans le DMF (30 mL) est ajoutée une solution de l'intermédiaire obtenu ci-dessus (1,8 g, 7,5mmoles) dans DMF (10 mL). Le mélange est agité à température ambiante pendant 5 heures. Le milieu réactionnel est extrait à l'$Et_2O$. La phase organique est lavée par une solution saturée en NaCl, séchée et concentrée sous vide. Le résidu est chromatographié sur gel de silice (éluant $CH_2Cl_2$/AcOEt (100/0 à 95/5)). L'intermédiaire **601** (1,2 g) est obtenu sous la forme d'un solide blanc.
**RMN $^1$H** (300 MHz; $CDCl_3$): δ 7,45 (d, 2H); 7,30 (d, 2H) ; 4,90 (m, 1H) ; 1,60 (s, 6H) ; 1,35 (s large, 9H)
**IR ($cm^{-1}$) :** 3265 ; 1698

**Protocole VI: Préparation d'intermédiaires aminés protégés à partir d'intermédiaires benzonitriles**

A titre d'exemple la synthèse de l'intermédiaire 158 (*tert*-butyl [2-(4-bromo-3,5-difluorophényl)propan-2-yl]carbamate) est décrite ci après :

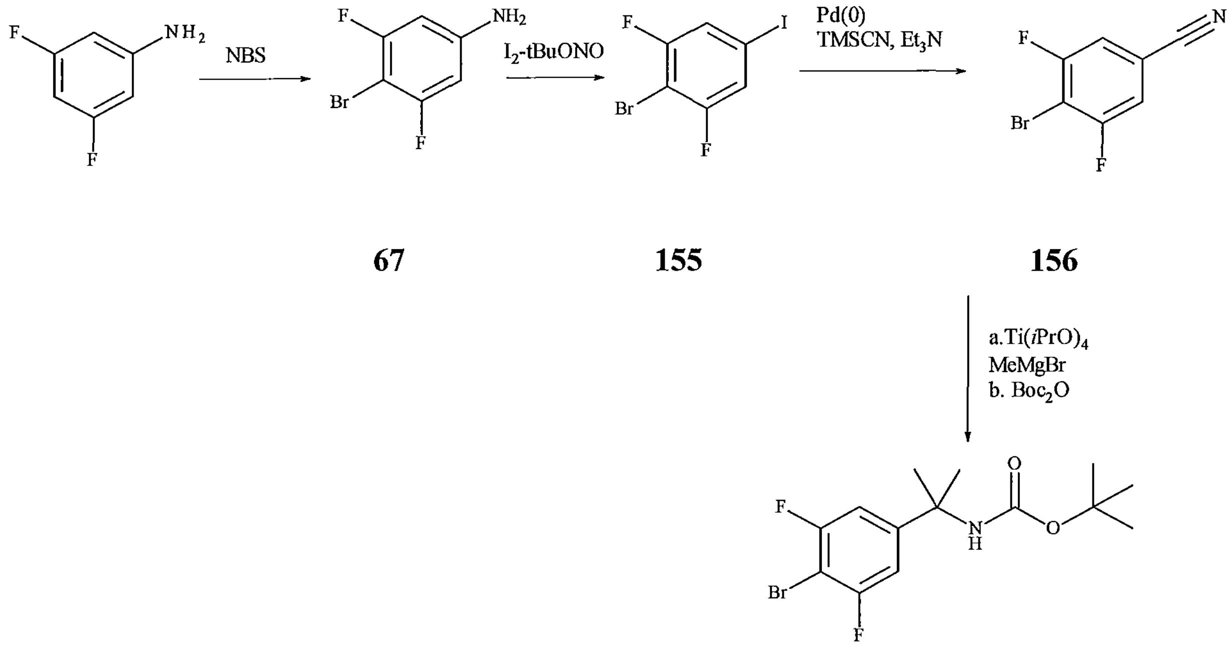


**67** **155** **156**

**158**

**Intermédiaire 67 :**

**2-bromo-1,3-difluoro-5-aniline**

A une solution de 3,5-difluoronaniline commerciale (100 g, 770 mmoles) dans le DMF (310 mL), est ajoutée une solution de *N*-bromosuccinimide (140 g, 786 mmoles) dans le DMF (310 mL) en 40 minutes. La solution résultante est agitée à température ambiante pendant 1 heure 30. L'ensemble est transféré sur 8 L d'eau, ce qui provoque la précipitation de l'intermédiaire désiré. Le solide est filtré sur fritté, puis rincé abondamment avec de l'eau. Le solide obtenu est séché à l'air 48 heures : 148 g de l'intermédiaire attendu sous forme d'un solide blanc sont obtenus, et utilisés sans traitement supplémentaire dans l'étape suivante.
**RMN $^1$H** (400MHz, DMSO-$d_6$): δ 6,3 (2d, 2H), 3,90 (m, 2H)
**IR ($cm^{-1}$):** 3479-3392;

**Intermédiaire 155 :**

**2-bromo-1,3-difluoro-5-iodobenzène**

Le diiode (365,48 g; 1.44 moles) ainsi que le *tert*-butyl nitrite (85 mL) sont mis en solution dans de l'acétonitrile (320 mL). Une solution de l'intermédiaire obtenu ci-dessus (100 g ; 0,48 mole) dans l'acétonitrile (210 mL) est additionnée lentement au milieu réactionnel (Tmax : 35°C). Le mélange est agité à température ambiante pendant 50 minutes. Une solution aqueuse de $Na_2S_2O_3$ est ensuite additionnée jusqu'à décoloration du milieu réactionnel. La phase aqueuse est ensuite extraite par $Et_2O$, puis séchée sur $MgSO_4$ et concentrée sous pression réduite. Le résidu est purifié par chromatographie sur silice (dépôt solide (100 % cyclohexane)). L'intermédiaire **155** (143 g) est obtenu sous forme d'un solide jaune.
**RMN $^1$H** (400MHz, DMSO-$d_6$): $\delta$ 7,72 (d, 2H)
**IR (cm$^{-1}$):** 3080

**Intermédiaire 156 :**

**4-bromo-3,5-difluorobenzonitrile**

A une solution de l'intermédiaire **155** (10 g, 31 mmoles) dans de la triéthylamine (63 mL), sont ajoutés du TMSCN (6,2 mL), puis du $Pd(PPh_3)_4$ (1,8 g). Le mélange réactionnel est porté à 80°C, 1,8 g de $Pd(PPh_3)_4$ sont ajoutés à nouveau. La solution se colore et un précipité se forme. Après contrôle GC, le milieu réactionnel est ramené à température ambiante puis 50 mL de toluène sont ajoutés. Le mélange est filtré, le filtre est rincé 2 fois par 50 mL de toluène. Le filtrat est traité par 300 mL d'HCl 1N, puis une solution aqueuse saturée en NaCl. L'évaporation sous pression réduite conduit à l'obtention de 19 g d'un solide. Le résidu est purifié par chromatographie sur silice (dépôt solide (100 % cyclohexane)). L'intermédiaire **156** (5,6 g) est obtenu sous forme d'un solide jaune.
**RMN $^1$H** (300MHz, DMSO-$d_6$): $\delta$ 7,98 (m, 2H)
**IR (cm$^{-1}$):** 2236, 1032; **GC-EI** (70 eV): 216,9

**Intermédiaire 158 :**

<u>Etape 1</u>

A une solution de l'intermédiaire **156** (13,6 g, 62 mmoles) dans l'éther éthylique (330 mL) est ajouté du $CH_3MgBr$ (3M dans l'$Et_2O$) (65 mL, 195 mmoles). Le mélange est agité 35 minutes à température ambiante puis du $Ti(OiPr)_4$ (19 mL, 64 mmoles) est ajouté. Le mélange réactionnel est agité la nuit à température ambiante, puis traité avec précaution par une solution aqueuse de NaOH 20% (50 mL). Le mélange a été décanté en présence d'AcOEt et d'une solution aqueuse saturée en NaCl, la phase organique est séchée sur $MgSO_4$, puis concentrée sous vide. Le résidu est chromatographié sur silice (éluant $CH_2Cl_2$/EtOH (100/0 à 95/05)). L'intermédiaire **157** attendu (6,2 g) est obtenu sous forme d'une huile
**RMN $^1$H** (400MHz, DMSO-$d_6$): $\delta$ 7,41 (d, 2H), 2,0 (s large, 2H), 1,34 (s, 6H)
**IR (cm$^{-1}$):** 3375-3288, 1021.

<u>Etape 2</u>

A une solution de l'intermédiaire obtenu ci-dessus (4,2 g, 16,8 mmoles) dans le chlorure de méthylène (80 mL) est ajouté avec précaution du di-*tert*-butyl-dicarbonate (3,56 g, 16,3 mmoles). Le milieu réactionnel est agité à température ambiante pendant 3 jours avant d'être traité par une solution d'HCl 1N. La phase organique est séchée, puis concentrée sous vide. Le résidu est chromatographié sur gel de silice en utilisant un mélange éluant $CH_2Cl_2$/Cyclohexane (50/50 à 100/0). L'intermédiaire 158 (4,3 g) est obtenu sous forme d'un solide amorphe.
**RMN $^1$H** (300 MHz ; DMSO-$d_6$) : $\delta$ 7,13 (d, 2H) ; 6,85 (m, 1H) ; 1,50 (s, 6H) ; 1,30 (s, 9H)
**IR (cm$^{-1}$) :** 3318 ; 1683

<u>**Protocole VII : Préparation des cétones par réaction de Sandmeyer**</u>

A titre d'exemple la synthèse de l'intermédiaire **681** est décrite ci après :

**Intermédiaire 681 :**

A un mélange de 4-bromo-3-méthoxyaniline commerciale (10,2 g, 50,6 mmoles) dans $HCl_{cc}/H_2O$ (11/25 mL) préalablement refoidi à -5°C est ajouté par portions du $NaNO_2$ (3,48 g). Le milieu réactionnel est agité 1h à 0°C, avant d'être transféré sur un mélange d'acetaldoxime (6,02 g), de $CuSO_4$ (2,52 g), d'AcONa.3$H_2O$ (36,64 g) dans de l'eau (20,5 mL) à 0°C. Le mélange résultant est agité entre 0°C et 10°C pendant 2h puis de l'HCl à 37% (23 mL) est ajouté et le milieu porté au reflux 2 heures. Après retour à température ambiante, le mélange est extrait à l'heptane, la phase organique est séchée sur $MgSO_4$. L'évaporation de la phase organique sous vide conduit à l'obtention d'un résidu qui est chromatographié sur gel de silice (éluant $CH_2Cl_2$/Cyclohexane (50/50 à 90/10)). L'intermédiaire **681** (5,9 g) est obtenu sous forme d'un solide amorphe.
**RMN $^1$H** (400 MHz ; $CDCl_3$) : δ 7,55 (d, 1H) ; 7,50 (d, 1H) ; 7,40 (dd, 1H) ; 3,95 (s, 3H) ; 2,60 (s, 3H)
**IR ($cm^{-1}$) :** 1681

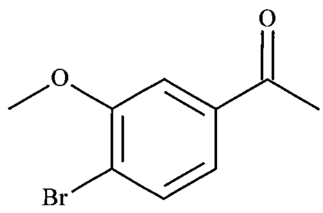

**681**

**Protocole VIII: Obtention des cétones par réaction d'un magnésien sur des benzonitriles**

A titre d'exemple la synthèse de l'intermédiaire 9 est décrite ci après :

**Intermédiaire 9 :**

A température ambiante, de l'iodure de méthyl magnésium (3 M dans l'éther diéthylique) (17 mL, 51 mmoles est ajouté goutte à goutte à une solution de 4-bromo-3-méthyl benzonitrile commercial (10 g, 51 mmoles) dans l'éther diéthylique (100 mL)). Le milieu réactionnel est chauffé au reflux pendant 16 heures. Après retour à température ambiante, 60 mL d'acide chlorhydrique 6N sont ajoutés, le milieu est ensuite chauffé au reflux pendant 6 heures. Après refroidissement, les phases aqueuse et organiques sont séparées et la phase aqueuse est extraite avec 40 mL d'acétate d'éthyle. Les phases organiques sont combinées, lavées avec une solution aqueuse saturée en NaCl (2 x 40 mL), séchées sur $MgSO_4$ puis concentrées sous pression réduite. 4,5 g de l'intermédaire **9** sont obtenus sous forme d'une huile marron.
**RMN $^1$H** (300MHz, $CDCl_3$): δ 7,80 (s, 1H), 7,62 (s, 2H), 2,60 (s, 3H), 2,45 (s, 3H)
**IR ($cm^{-1}$) :** 1681

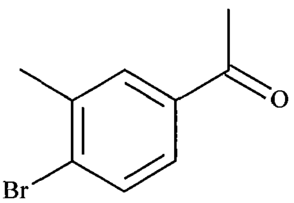

**9**

**Protocole IX : Obtention des cétones par réaction de Weinreb**

A titre d'exemple la synthèse de l'intermédiaire **37** est décrite ci après :

**Intermédiaire 36 :**

A une suspension de l'acide 4-bromo-3-fluoro-benzoïque commercial (5 g, 22,8 mmoles) dans du chlorure de méthylène (70 mL) sont ajoutés du DMF (0,1 mL), puis du chlorure d'oxalyle (2,1 mL). Le mélange est agité à température ambiante pendant 4 heures, puis il est concentré sous vide. Au résidu repris par du chlorure de méthylène (220 mL) est

ajouté du chlorhydrate de N-méthyl-méthoxylamine, le mélange est refroidi à 5°C et de la pyridine est ajoutée (4 mL). Le milieu a été agité 2 heures, puis lavé par une solution aqueuse d'HCl 2N, la phase organique est séchée sur $MgSO_4$ puis concentrée sous vide. L'intermédiaire **36** est obtenu sous forme d'un solide (5,4 g).
**RMN $^1$H** (300MHz, DMSO-$d_6$): δ 7,85 (dd, 1H), 7,60 (dd, 1H), 7,40 (dd, 1H), 3,59 (s, 3H), 3,29 (s, 3H)
**IR (cm$^{-1}$) :** 1657

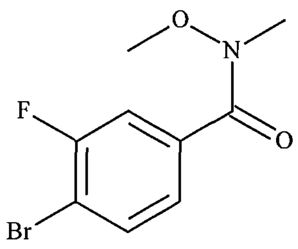

**36**

**Intermédiaire 37 :**

A -70°C, l'intermédiaire **36** (5,4 g, 20,6 mmoles) en solution dans du THF (75 mL) est traité par une solution de bromure de méthyl magnésium (3 M dans l'éther diéthylique) (8,1 mL, 24 mmoles). Le millieu est agité avec retour à température ambiante pendant 3 heures avant d'être versé sur une solution aqueuse d'HCl 1N à 0°C. Le produit est extrait par de l'AcOEt, la phase organique est lavée par une solution aqueuse saturée en NaCl, séchée sur $MgSO_4$ puis concentrée sous vide. L'intermédiaire 37 est obtenu sous forme d'un solide (3,6 g).
**RMN $^1$H** (300 MHz ; $CDCl_3$): δ 7,7-7,6 (m, 3H), 2,58 (s,3H)
**IR (cm$^{-1}$) :** 1679

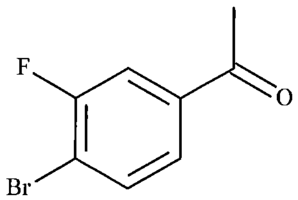

**37**

**<u>Protocole X : Obtention des cétones par réaction de Stille</u>**

A titre d'exemple la synthèse du composé **61** est décrite ci après :

**Intermédiaire 61 :**

A une solution dégazée à l'azote de 44 g (137 mmoles, 1 éq.) de l'intermédiaire **155** dans 1,7 L de DMF, sont ajoutés du tri-butyl-(1-éthoxyvinyl)étain (65 mL, 179 mmoles), puis du $PdCl_2(PPh_3)_2$ (13 g, 0,18 mmoles). Le mélange est chauffé à 80°C jusqu'à disparition de l'intermédiaire de départ (suivi GC). Après retour à température ambiante le mélange réactionnel est décanté avec 3 L d'eau et 1 L d'$Et_2O$. La phase organique est séchée sur $MgSO_4$, puis concentrée. Le résidu ainsi obtenu repris dans 300 mL de THF est agité 1 heure en présence d'une solution aqueuse d'HCl 1N (100 mL). Le milieu est alors décanté en présence de 1 L d'$Et_2O$, la phase organique est séchée par sur $MgSO_4$. L'évaporation sous pression réduite conduit à l'obtention de 56 g d'une huile. Cette huile est chromatographiée sur gel de silice (cyclohexane/chlorure de méthylène 80/20 à 50/50). L'intermédiaire **61** (27 g) est obtenu sous forme d'un solide beige qui est utilisé sans traitement supplémentaire dans l'étape suivante
**RMN $^1$H** (300MHz, $CDCl_3$): δ 7,50 (d, 2H), 2,60 (s, 3H)
**IR (cm$^{-1}$):** 1694

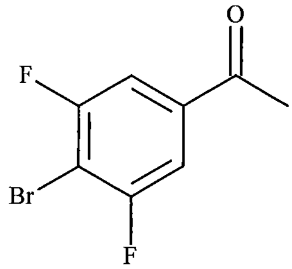

**61**

**Protocole XI : Obtention des cétones par réarrangement de Fries**

A titre d'exemple la synthèse de l'intermédiaire **547** est décrite ci après :

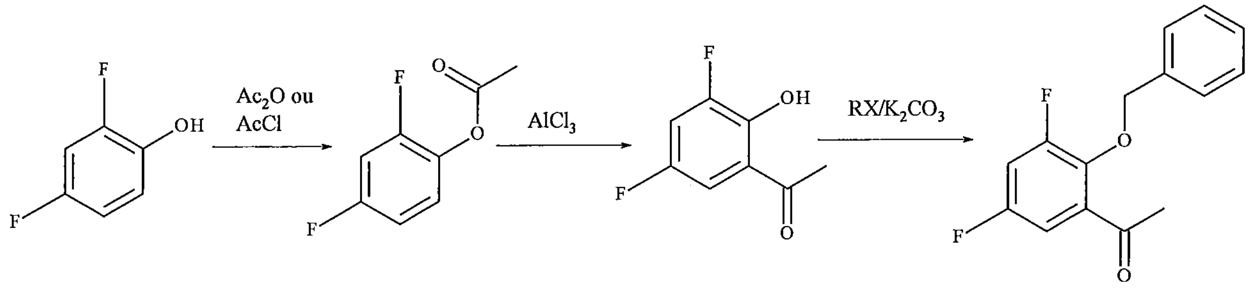


**362** **363** **547**

**Intermédiaire 362 :**

A un mélange de 2,4-difluorophénol commercial (15 g, 115 mmoles) et de pyridine (10,2 mL) dans du chlorure de méthylène (156 mL) est ajouté du chlorure d'acétyle (8,6 mL) en maintenant une température inférieure à 30°C. Le mélange résultant (formation d'un précipité) est agité 1 heure à température ambiante avant d'être hydrolysé. Le milieu est décanté, la phase organique est lavée successivement par une solution aqueuse d'HCl 1N, une solution aqueuse saturée en $NaHCO_3$ puis séchée sur $MgSO_4$. L'évaporation sous pression réduite conduit à l'obtention de l'intermédiaire **362** sous forme d'une huile (19,4 g) qui est utilisée dans l'étape suivante.
**RMN $^1$H** (300MHz, $CDCl_3$): δ 7,10 (m, 1H), 6,90 (m, 2H), 2,30 (s, 3H)
**IR ($cm^{-1}$) :** 1770

**Intermédiaire 363 :**

Un mélange de l'intermédiaire **362** (2 g, 11,6 mmoles) et d'$AlCl_3$ (2,8 g) est chauffé sous argon à 150°C pendant 30 minutes. Après retour à température ambiante, sont ajoutées avec précaution de la glace puis une solution aqueuse d'HCl 1N. Le milieu est décanté en présence de toluène, la phase organique est lavée à l'eau puis par une solution aqueuse saturée en NaCl. Après séchage, l'évaporation sous pression réduite conduit à l'obtention de l'intermédiaire **363** sous forme d'un solide (1,5 g).
**RMN $^1$H** (300MHz, $CDCl_3$): δ 12,00 (m, 1H), 7,25 (m, 1H), 7,10 (m, 1H), 2,65 (s, 3H)
**IR ($cm^{-1}$) :** 1651

**Intermédiaire 547 :**

A une solution de l'intermédiaire **363** (15 g, 87 mmoles) dans l'acétone (150 mL) est ajouté du $K_2CO_3$ (24 g) puis du bromure de benzyle (10,8 mL, 91 mmoles). Le mélange est agité à température ambiante pendant 24 heures. Les sels sont filtrés et le filtrat est évaporé. Le résidu est repris par de l'$Et_2O$ puis lavé à l'eau et par une solution aqueuse saturée en NaCl. Après séchage sur $MgSO_4$, l'évaporation sous pression réduite conduit à l'obtention de l'intermédiaire **547** sous forme d'une huile (22,5 g).

**RMN $^1H$** (400MHz, $CDCl_3$): δ 7,40 (m, 5H), 7,15 (m, 1H), 7,00 (m, 1H), 5,10 (s, 2H), 2,50 (s, 3H)
**IR ($cm^{-1}$) :** 1687

**Protocole XII : Transformation des cétones en amines racémiques**

A titre d'exemple la synthèse de l'intermédiaire **48** est décrite ci après :

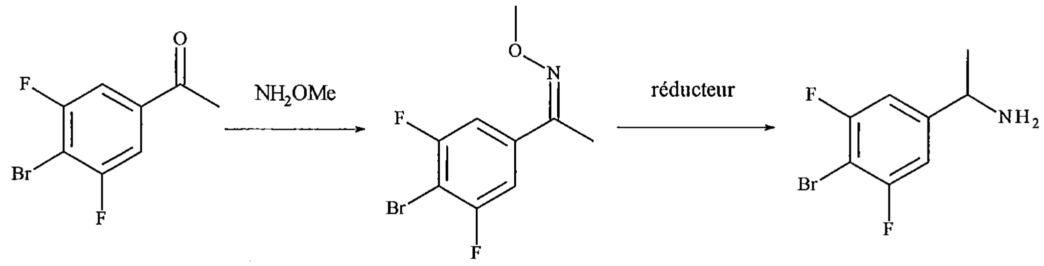


**61** **62** **48**

**Intermédiaire 62 :**

A un mélange de 26 g (110 mmoles) de l'intermédiaire **61** dans 53 mL d'éthanol, sont ajoutés 107 mL d'eau, 11,8 g (141 mmoles) de chlorhydrate de méthoxylamine et 11,8 g (142 mmoles) d'acétate de sodium. Le mélange est chauffé 3 heures à 70°C. Le milieu est ramené à température ambiante, puis extrait par de l'AcOEt (0,5 L) en présence d'une solution aqueuse saturée en NaCl. La phase organique est séchée sur $MgSO_4$. L'évaporation sous pression réduite conduit à l'obtention de 27,7 g du mélange d'oximes **62** sous forme d'un solide marron clair, qui est utilisé sans traitement supplémentaire dans l'étape suivante.
**IR ($cm^{-1}$) :** 1025

**Intermédiaire 48 :**

A température ambiante, 210 mL (210 mmoles, 2 éq.) d'une solution de $BH_3$. THF 1M dans le THF sont ajoutés en 10 minutes à une solution de 27 g (105 mmoles, 1 éq.) du mélange d'oximes **62** dans 114 mL de THF. La solution obtenue est chauffée à 70°C pendant 2 h 30. Après contrôle HPLC le milieu est ramené à température ambiante. Une solution 2N d'HCl dans l'éther (2 éq.) est ajoutée au milieu avec précaution. Le mélange est chauffé 1 h à 40°C. La filtration du solide conduit à l'obtention de 11,8 g du chlorhydrate de l'intermédiaire **48.**
**RMN $^1H$** (400MHz ; DMSO-$d_6$): δ 8,65 (m, 3H), 7,53 (d, 2H), 4,46 (quad, 1H), 1,51 (d, 3H)
**IR ($cm^{-1}$):** 3200-2500

Cette procédure est utilisée pour préparer les amines racémiques sous la forme de chlorhydrates ou de bases libres.

**Protocole XIIb : procédé alternatif de transformation des cétones en amines racémiques**

A titre d'exemple, la synthèse de l'intermédiaire **341** est décrite ci dessous:

**Intermédiaire 341 :**

A une solution de la 5,7-difluoro-2,3-dihydro-1*H*-indén-1-one commerciale (6 g, 35 mmoles) dans la pyridine (60 mL) à température ambiante, est ajouté du chlorhydrate de méthoxylamine (3,0 g, 37 mmoles). Le mélange réactionnel est agité 20 heures à température ambiante. La pyridine est évaporée sous vide, le résidu est agité dans l'eau (30 mL) pendant 1 heure, puis collecté sur fritté. Le solide est rincé à l'eau puis séché sous vide à 50°C. L'intermédiaire oxime est obtenu sous forme d'un solide blanc (6,5 g) qui est ensuite réduit en intermédiaire **341** selon le procédé décrit pour l'intermédiaire **48.**
**RMN $^1H$** (300MHz ; DMSO-$d_6$): δ 7,10 (m, 2H), 6,50 (m, 3H), 4,80 (m, 1H), 3,25 (m, 1H), 2,95 (m, 1H), 2,50 (m, 1H), 2,15 (m, 1H)
**RMN $^{19}F$:** -107,6, -110,8 (quad et dd, 2F)
**IR ($cm^{-1}$):** 3380-2500

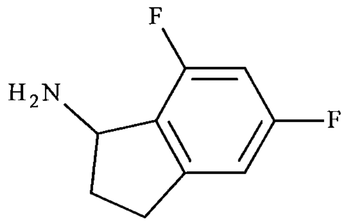

**341**

**Protocole XIII : Transformations des cétones en intermédiaires *tert*-butanesulfinyl amines chirales**

Référence bibliographique: John T. Colyer, Neil G. Andersen,* Jason S. Tedrow, Troy S. Soukup, et Margaret M. Faul. J. Org. Chem. 2006, 71, 6859-6862

A titre d'exemple la synthèse de l'intermédiaire **286** est décrite ci après :

**Intermédiaire 286 :**

A une solution de 1-(3,5-difluorophényl)éthanone commerciale (20 g, 120 mmoles) dans du THF (332 mL) sont ajoutés successivement du $Ti(OEt)_4$ (34 mL, 163 mmoles) puis du (*R*)-(+)-2-méthyl-2-propanesulfinamide (14,5 g, 119 mmoles). Le milieu est chauffé 24 heures à 70°C. Le milieu refroidi à -40°C est transféré par cannulation sur une suspension de $NaBH_4$ (18,1 g; 374 mmoles) dans du THF (220 mL). Le milieu réactionnel à température ambiante, est traité avec précaution par du méthanol (56 mL), puis dilué par de l'AcOEt (300 mL) et une solution aqueuse de NaCl (700 mL). Le mélange résultant est filtré sur de la Célite®, qui est rincée par du THF et de l'AcOEt. Le filtrat est décanté, la phase organique est séchée sur $MgSO_4$. L'évaporation sous pression réduite conduit à l'obtention d'un solide blanc, qui est purifié sur gel de silice en utilisant un gradient d'élution AcOEt/chlorure de méthylène 0/100 à 40/60. Le diastéréoisomère **286** (18 g) est isolé sous la forme d'un solide blanc.

**RMN $^1$H** (400MHz; DMSO-$d_6$): $\delta$ 7,15 (m, 2H), 7,08 (m, 1H), 5,29 (d, 1H), 4,40 (m, 1H), 1,38 (d, 3H), 1,10 (s, 9H)

**IR (cm$^{-1}$):** 3146, 1043

**GC-EI** (70 eV): $M^{+\cdot}$= 261,1

Pureté diastéréoisomèrique : de>99%

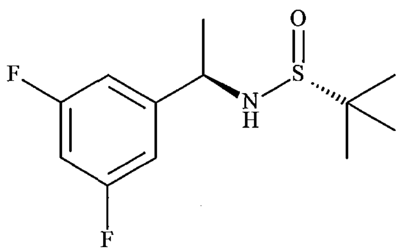

**286**

**Protocole XIIIb : Méthode alternative pour la préparation des intermédiaires *tert*-butanesulfinyl amines chiraux**

La préparation des intermédiaires *tert*-butanesulfinyl amines chiraux peut être décomposée selon la séquence décrite pour l'intermédiaire **331.**

**Intermédiaire 330 :**

A une solution de 1-(2,4-difluorophényl)éthanone commerciale (4 g, 25,6 mmoles) dans 80 mL de THF sont ajoutés successivement du $Ti(OEt)_4$ (13,1 g, 46 mmoles) puis du (*R*)-(+)-2-méthyl-2-propane sulfinamide (3,1 g, 25 mmoles). Le milieu est chauffé 24 heures à 70°C. Le milieu réactionnel refroidi vers 15°C est versé sur une solution aqueuse saturée en NaCl puis de l'acétate d'éthyle est ajouté. Après agitation (30 minutes) le mélange est filtré sur Célite®, lavé par 2 fois 80 mL d'acétate d'éthyle. Le filtrat est décanté, la phase organique est séchée sur $MgSO_4$, filtrée et évaporée à sec. Le résidu est purifié par flash-chromatographie sur silice (éluant : $CH_2Cl_2$/AcOEt : 95/5). L'intermédiaire **330** (5,2 g) est obtenu sous la forme d'un liquide jaune.
**RMN $^1$H** (400MHz; DMSO-$d_6$): δ 7,80 (m, 1H), 7,40 (m, 1H), 7,20 (m, 1H), 2,70 (s, 3H), 1,20 (s, 9H)
**IR (cm$^{-1}$):** 1603, 1080
**GC-EI** (70 eV): $M^{+\cdot}$= 259,1

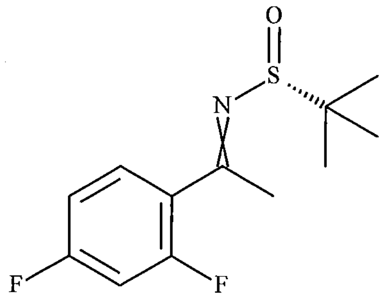

**330**

**Intermédiaire 331 :**

A une solution de l'intermédiaire **330** (2,5 g, 9,64 mmoles) dans 50 mL de THF et refroidie à -60°C est ajouté du $NaBH_4$ (366 mg, 9,64 mmoles). Après retour à température ambiante, le milieu réactionnel est traité avec précaution par du méthanol puis concentré sous vide. Le résidu est repris à l'eau et extrait à l'éther. La phase organique est lavée par une solution aqueuse saturée de NaCl, puis séchée sur $MgSO_4$. Le résidu est purifié par flash-chromatographie sur silice (éluant : $CR_2Cl_2$/AcOEt: 80/20). L'intermédiaire **331** est obtenu (1,85 g) sous la forme d'un solide blanc cristallin.
**RMN $^1$H** (300MHz; DMSO-$d_6$): δ 7,60 (m, 1H), 7,20 (m, 1H), 7,10 (m, 1H), 5,75 (d, 1H), 4,60 (quint, 1H), 1,4 (d, 3H), 1,1 (s, 9H)
**IR (cm$^{-1}$):** 3243, 1603, 853, 814
Pureté diastéréoisomèrique : de>99%

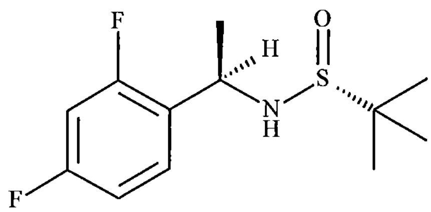

**331**

**<u>Protocole XIV: Transformation des aldéhydes commerciaux en intermédiaires *tert*-butanesulfinylamines chirales.</u>**

A titre d'exemple la synthèse de l'intermédiaire **497** *N*-[1-(3,5-difluorophényl)-2-méthylpropyl]-2-méthylpropane-2-sulfinamide) est décrite ci après :

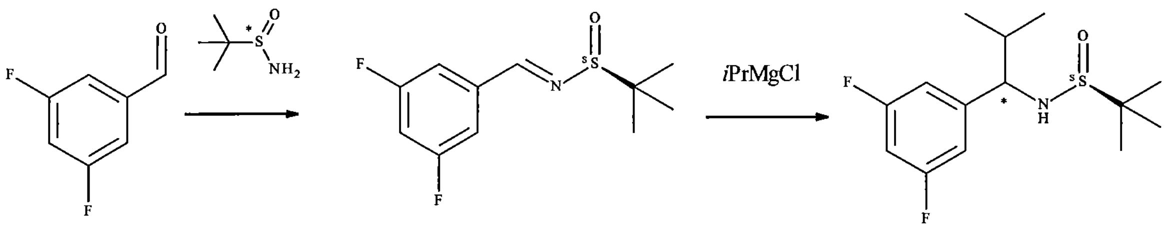


**(495)** **496** **497**

**Intermédiaire 496 :**

A une solution de (*S*)-(-)-2-méthylpropane-2-sulflnamide (44,7 g, 368 mmoles) dans du méthanol (500 mL), est ajouté à température ambiante du tBuOK (3,93 g). Après 15 minutes d'agitation à température ambiante, le 3,5-difluoro-benzaldéhyde commercial (50 g, 0,35 mole) est ajouté. Le mélange réactionnel est agité à température ambiante pendant 1 heure puis il est hydrolysé par une solution aqueuse saturée de $NH_4Cl$. L'évaporation du méthanol sous vide permet la cristallisation d'un solide qui est repris dans 300 mL d'eau, filtré et lavé à l'eau. Le solide est solubilisé dans l'éther, lavé avec une solution aqueuse saturée en NaCl, séché sur $MgSO_4$, filtré et concentrée sous vide. L'intermédiaire **496** (74,5 g) est obtenu sous la forme d'une huile qui cristallise.
**RMN $^1$H** (400MHz, DMSO-$d_6$): δ 8,58 (s, 1H), 7,70 (d large, 2H), 7,50 (t, 1H), 1,25 (s, 9H)
**IR (cm$^{-1}$):** 1620, 1142,1078

**Intermédiaire 497 :**

A une solution de l'intermédiaire **496** (3 g, 12 mmoles) dans du THF (60 mL), refroidie à - 65°C est ajouté une solution de isopropylMgBr (3M/éther) (9 mL, 27mmoles) en 20 minutes. Après controle le mélange réactionnel est hydrolysé à -40°C par une solution aqueuse saturée de $NH_4Cl$. Le milieu est décanté en présence d'éther éthylique, la phase organique est lavée par une solution saturée en NaCl, séchée sur $MgSO_4$ puis concentrée. Une chromatographie sur silice (éluant $CH_2Cl_2$/AcOEt 99/1 à 85/15) conduit à l'obtention de de l'intermédiaire **497** (2,7 g) sous forme d'une huile.
**RMN $^1$H** (400MHz, DMSO-$d_6$): δ 7,05 (m, 3H), 5,35 (d, 1H), 4,0 (t, 1H), 2,0 (m, 1H), 1,05 (s, 9H), 1,0-0,8 (2d, 6H)
**IR (cm$^{-1}$):** 3189, 1116, 1040

**Protocole XV : Préparation des dérivés carbamate de *tert*-butyle**

L'auxiliaire chiral a été clivé en milieu acide selon le protocole suivant :

**Intermédiaire 287 :**

Une solution de l'intermédiaire **286** (18 g, 69mmoles) dans de l'éther éthylique (580 mL) est traitée par de l'acide chlorhydrique dans $Et_2O$ (solution 2M, 59 mL). Le mélange réactionnel est agité 20 heures à température ambiante. Le précipité est filtré sur fritté, puis séché sous vide. Le chlorhydrate de **287** (11,5 g, ee > 99%) est obtenu sous forme d'un solide blanc.
**RMN $^1$H** (300MHz, DMSO-$d_6$): δ 8,68 (s large, 3H), 7,32 (m, 2H), 7,27 (m, 1H), 4,45 (quad, 1H), 1,50 (d, 3H)
**IR (cm$^{-1}$):** 3100-2500

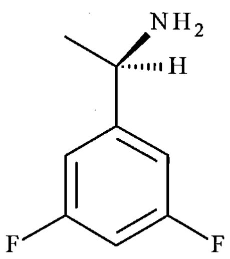

**287**

Les amines obtenues sont protégées sous forme de *tert*-butyl carbamates selon la procédure décrite pour l'intermédiaire **158** (**protocole VI**)
Lorsque des chlorhydrates sont obtenus, ils sont traités à la soude IN et les amines obtenues sont protégées comme indiqué ci-dessus.

**Protocole XVI : Préparation des dérivés trifluoroacétamides**

A titre d'exemple, la procédure pour préparer l'intermédiaire **17** est décrite ci-dessous :

**Intermédiaire 17 :**

A une solution de la (*R*)-(+)-1-(3-méthoxyphényl)éthylamine commerciale (50 g, 330 mmoles) dans le chlorure de méthylène (400 mL) à température ambiante, est ajouté lentement une solution d'anhydride trifluoroacétique (46 mL, 330 mmoles). Le mélange est agité à température ambiante pendant 2 heures 30. Le milieu réactionnel est lavé par une solution aqueuse d'HCl 1N (400 mL). L'ensemble est décanté, la phase organique est séchée par passage sur $MgSO_4$. L'évaporation sous pression réduite conduit à l'obtention de l'intermédiaire **17** (84 g) sous la forme d'un solide qui est utilisé sans traitement supplémentaire dans l'étape suivante.
**RMN $^1$H** (400 MHz, $CDCl_3$) : δ 7,30 (m, 1H), 6,90 (2d et s, 3H), 6,45 (s large, 1H), 5,10 (m, 1H), 3,80 (s, 3H), 1,60 (d, 3H)
**IR (cm$^{-1}$):** 3293, 1696, 1612, 1588

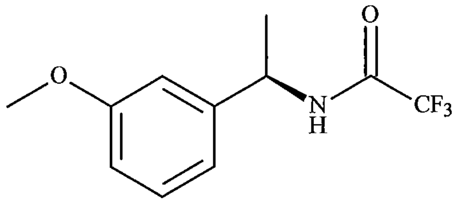

**17**

**Protocole XVII**

A titre d'exemple la préparation de **94** est décrite ci-dessous :

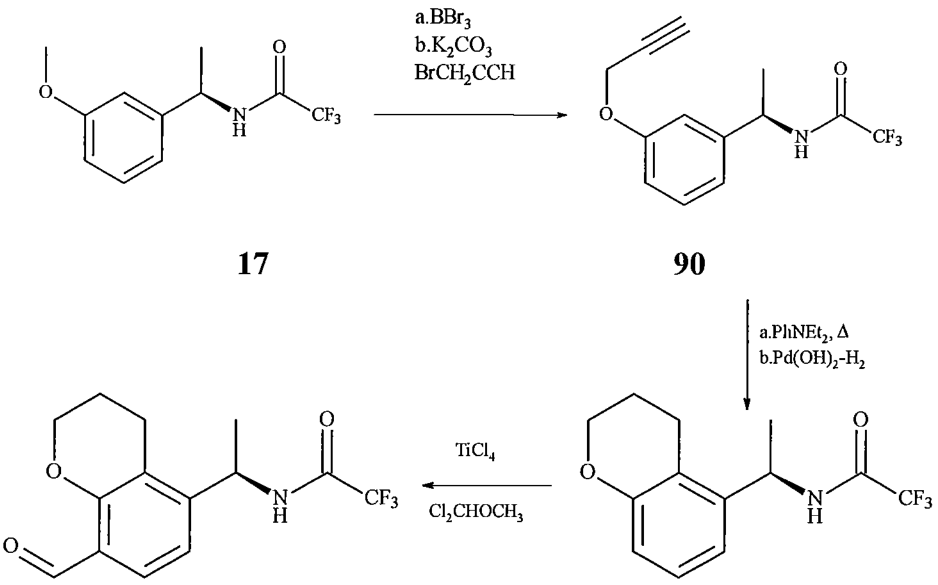


**94** **93**

**Intermédiaire 90 :**

A une solution de l'intermédiaire **17** (65 g, 262 mmoles) dans le chlorure de méthylène (800 mL) à -70°C, est ajouté lentement une solution de $BBr_3$ (1M dans $CH_2Cl_2$) (480 mL, 480 mmoles,). Le mélange est amené à température ambiante sur une durée de 2heures. Après contrôle HPLC, le milieu réactionnel est refroidi à -70°C et traitée avec précaution par du méthanol (200 mL). La solution est concentrée sous vide, le résidu est repris avec précaution par de l'eau (300 mL), le mélange est traité par du NaOAc jusqu'à pH 4-5. L'ensemble est décanté en présence de chlorure de méthylène (500 mL), la phase organique est séchée par passage sur $MgSO_4$. L'évaporation sous pression réduite conduit à l'obtention de l'intermédiaire **90** (64 g) sous la forme d'un solide beige qui est utilisé sans traitement supplémentaire dans l'étape suivant.
**RMN $^1$H** (300 MHz, DMSO-$d_6$) : δ 9,70 (m, 1H), 9,20 (s, 1H), 7,12 (t, 1H), 6,75 (s, 1H), 6,78 (m, 1H), 6,65 (dd, 1H), 4,90

(quint, 1H), 1,42 (d, 3H)
**IR ($cm^{-1}$):** 3298, 1702, 1153

**Intermédiaire 93 :**

Etape 1

A une solution de l'intermédiaire **90** (21 g, 90 mmoles) dans de la DMF (450 mL) sont ajoutés du $K_2CO_3$ (15 g, 108 mmoles) puis du bromure de propargyle (11 mL, 98 mmoles). Le mélange est agité à 60°C pendant 4 heures. Après contrôle HPLC le milieu réactionnel refroidi à température ambiante est versé sur un mélange eau/glace (1 L/1 kg). Le solide est filtré sur fritté, lavé par de l'eau. Un séchage sous vide conduit à l'obtention de l'intermédiaire attendu (24 g) sous la forme d'un solide beige qui est utilisé sans traitement supplémentaire dans l'étape suivante.
**RMN $^1$H** (300 MHz, DMSO-$d_6$) : $\delta$ 9,70 (s large, 1H), 7,28 (t, 1H), 6,92 (m, 3H), 5,00 (quad, 1H), 4,78 (s, 2H), 3,42 (t, 1H), 1,45 (d, 3H)
**IR ($cm^{-1}$):** 3301, 1693, 1158

Etape 2

Une suspension de l'intermédiaire obtenu ci-dessus (5 g, 11 mmoles, 1éq) dans de la diéthylaniline (7 mL) est chauffé au four à micro-ondes (CEM, DISCOVER, mode standard) 40 minutes à 210°C. Le milieu réactionnel est versé sur un mélange eau/glace/AcOEt (0,2 L/0,2 kg/0,2 L) sous agitation, puis traité par du HCl 12N jusqu'à pH 1 stable, la phase organique est lavée par une solution de NaCl saturée (1L), puis séchée par passage sur $MgSO_4$. L'évaporation sous pression réduite conduit à l'obtention d'une huile qui est chromatographiée sur gel de silice en utilisant un mélange éluant $CH_2Cl_2$/Cyclohexane (30/70 à 50/50). Le mélange de deux composés **a et b** (2,3 g) est obtenu sous forme d'une huile jaune (ratio **a/b :** 56/43).

**Intermédiaire a :**

**RMN $^1$H** (400 MHz, DMSO-$d_6$) : $\delta$ 9,90 (s, 1H), 7,15 (t, 1H), 6,9-6,7 (2d, 2H), 6,80 (d, 1H), 6,00 (m, 1H), 5,20 (m, 1H), 4,70 (m, 2H), 1,40 (d, 3H)
**IR ($cm^{-1}$):** 3284, 1692

Etape 3

A une solution de **a/b** (7,1 g, 26,1 mmoles, 1éq) dans du méthanol (700 mL) est ajouté du $Pd(OH)_2$ (2,9 g, 40% en poids). Le mélange résultant est agité à pression atmosphérique et à température ambiante jusqu'à disparition de la matière première (suivi GC). Le milieu réactionnel est filtré. L'évaporation sous pression réduite du filtrat conduit à l'obtention d'un mélange des intermédiaires **93a/93b** (5,8 g) qui est utilisé sans traitement supplémentaire dans l'étape suivante.

**Intermédiaire 93a :**

**RMN $^1$H** (300 MHz, DMSO-$d_6$): $\delta$ 9,80 (d, 1H), 7,10 (t, 1H), 6,90 (d large, 1H), 6,65 (d large, 1H), 5,10 (quint, 1H), 4,10 (t, 2H), 2,85-2,6 (m, 2H), 1,95 (m, 2H), 1,40 (d, 3H)
**IR ($cm^{-1}$):** 3276, 1691, 1181

**Intermédiaire 94 :**

**2,2,2-trifluoro-*N*-[(1*R*)-1-(8-formyl-3,4-dihydro-2*H*-chromén-5-yl)éthyl]acétamide**

A une solution de **93a/93b** (2,58 g, 9,4 mmoles) dans du chlorure de méthylène (40 mL) à 0°C sont ajoutés du $TiCl_4$ (1,8 mL, 16 mmoles) puis du $Cl_2CHOMe$ (0,78 mL, 8,6 mmoles,). Le mélange résultant est agité à température ambiante 20 heures, puis versé sur un mélange eau/glace (250 mL/250 g). L'ensemble est décanté en présence de chlorure de méthylène (500 mL), la phase organique est séchée par passage sur $MgSO_4$. L'évaporation sous pression réduite conduit à l'obtention d'une huile qui est chromatographiée sur gel de silice (éluant $CH_2Cl_2$/AcOEt (97/3)). L'intermédiaire **94** (0,9 g) est obtenu sous forme d'une huile
**RMN $^1$H** (500MHz, $CDCl_3$): $\delta$ 10,30 (s, 1H), 10,00 (1H), 7,55 (d, 1H), 7,05 d, 1H), 5,10 (quad, 1H), 4,28 (t, 2H), 2,9-2,78 (m, 2H), 2,05 (m, 2H), 1,40 (dd, 3H)

**IR** (**cm**$^{-1}$): 3303, 1716, 1660

*Réparation des précurseurs phényles - Exemples*

**Intermédiaire 64 :**

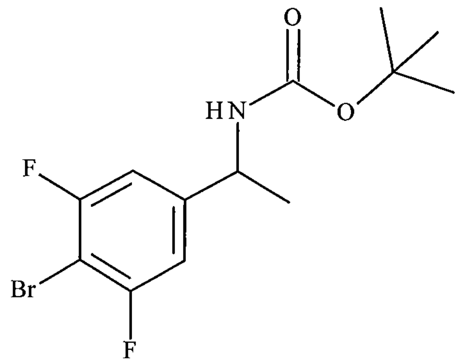

**64**

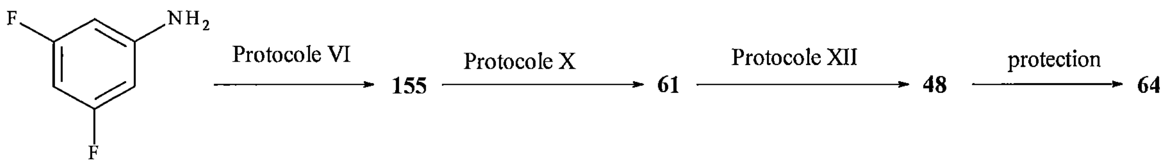


Obtenu par protection de l'amine **48** selon le protocole décrit pour l'intermédaire **158**
**RMN** $^{1}$**H** (300MHz, $CDCl_3$): δ 6,90 (d, 2H), 4,70 (2m, 2H), 1,40 (m et d, 12H)
**IR** (**cm**$^{-1}$): 3420, 1680

**Intermédiaires 145 et 146 :**

L'intermédiaire **64** (11 g) a été chromatographié par chromatographie haute pression sur support chiral (Colonne ChiralPak IC, éluant heptane/THF100/5, détction UV à 270 nm) pour donner les intermédiaires **145** (4 g) et **146** (4 g).

**Intermédiaire 145 :**

**[(1*R*)-1-(4-bromo-3,5-difluorophényl)éthyl]carbamate de *tert*-butyle**

**RMN** $^{1}$**H** (400MHz, DMSO-$d_6$): 7,45 (dlarge, 1H), 7,20 (d, 2H), 4,65 (m, 1H), 1,40 (s, 9H), 1,30 (d, 3H)
**IR** (**cm**$^{-1}$): 3368, 1678
Attribution de chiralité réalisée par IR-VCD à partir des spectres infra-rouges de la (1*R*)-1-(4-bromophényl)éthanamine.

**Intermédiaire 146 :**

**[(1*S*)-1-(4-bromo-3,5-difluorophényl)éthyl]carbamate de *tert*-butyle**

**RMN** $^{1}$**H** (300MHz, DMSO-$d_6$): 7,14 (m, 2H), 7,01 (m, 1H), 4,62 (m, 1H), 1,35 (s, 9H), 1,32 (d, 3H)
**IR** (**cm**$^{-1}$): 3366, 1678
Attribution de chiralité réalisée par IR-VCD à partir des spectres infra-rouges de la (1*S*)-1-(4-bromophényl)éthanamine.

Les intermédiaires **145** et **146** peuvent également être obtenus respectivement à partir des intermédiaires **166** et **161** en utilisant les conditions du **protocole XV.**

**Intermédiaire 166 :**

Obtenu par réaction de l'intermédiaire **61** et du (*R*)-(+)-2-méthyl-2-propanesulfinamide selon le **protocole XIIIb**
LC/MS : [M+H]+ mesuré 339
pureté chimique 85%.

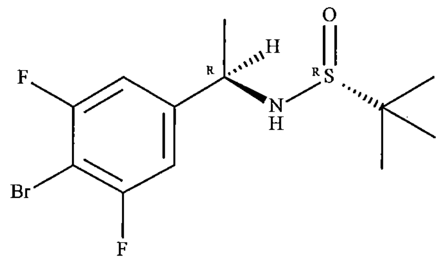

**166**

**Intermédiaire 161 :**

Obtenu par réaction de l'intermédiaire **61** et du (*S*)-(-)-2-méthyl-2-propanesulfinamide selon le **protocole XIIIb**
**RMN $^1$H** (400 MHz, DMSO-$d_6$): δ 7,34 (d, 2H), 5,81 (d, 1H), 4,41 (quint, 1H), 1,38 (d, 3H), 1,12 (s, 9H)
**IR (cm$^{-1}$):** 3174

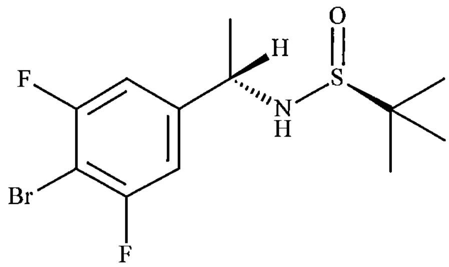

**161**

**Intermédiaire 5 :**

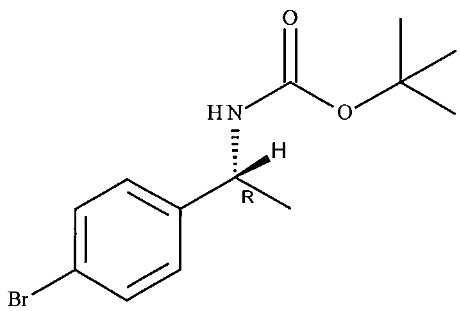

**5**

Obtenu par protection de la (1*R*)-1-(4-bromophényl)éthanamine commerciale selon le protocole décrit pour l'intermédiaire **158**
**RMN $^1$H** (300MHz, DMSO-$d_6$): δ 7,50 (d, 2H), 7,25 (d, 2H), 7,40 (d, 1H), 4,60 (m, 1H), 1,40 (s, 9H), 1,30 (d, 3H)
**IR (cm$^{-1}$)**: 3373, 1681.

**Intermédiaires 13 et 313 :**

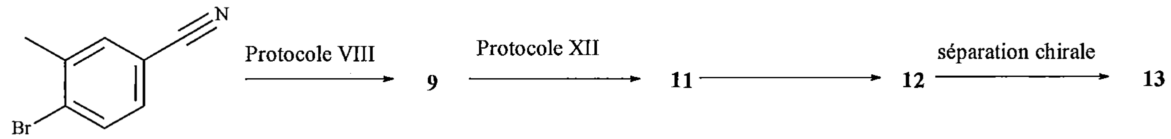


**Intermédiaire 11 :**

Obtenu à partir de l'intermédiaire **9** selon le **protocole XII**
**RMN $^1$H** (300 MHz; $CDCl_3$) : 7,45 (d, 1H), 7,20 (d, 1H); 7,05 (dd, 1H); 4,05 (quad, 1H); 2,40 (s, 3H); 1,35 (d, 3H); 1,55 (m, 2H)

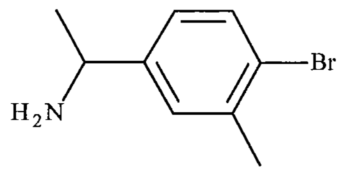

**11**

**Intermédiaire 12 :**

A une solution de l'intermédiaire **11** (28 g, 131 mmoles) dans du chlorure de méthylène (1,4 L) à température ambiante, est ajouté une solution de di-*tert*-butyl-dicarbonate (28 g, 131 mmoles) dans du chlorure de méthylène (0,36L). Le milieu réactionnel est agité 3 heures 30, puis une solution aqueuse d'HCl 1N est ajoutée. La phase organique est séchée sur $MgSO_4$, puis concentrée sous vide. Le résidu solide est agité dans du pentane pendant 1 heure, le solide est collecté sur fritté. L'intermédiaire **12** est obtenu sous forme d'un solide blanc.
**RMN $^1$H** (400MHz, DMSO-$d_6$): $\delta$ 7,50 (d, 1H), 7,37 (d, 1H), 7,26 (d, 1H), 7,05 (dd, 1H), 4,54 (m, 1H), 2,32 (s, 3H), 1,36 (s, 9H), 1,27 (d, 3H)
**IR ($cm^{-1}$):** 3374, 1684

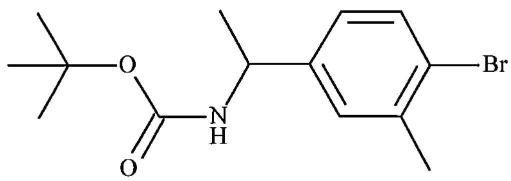

**12**

L'intermédiaire **12** (122 g) a ensuite été chromatographié par chromatographie haute pression sur support chiral (Colonne ChiralPak WHELK (R,R), éluant iPrOH, détection : 220nm) pour conduire aux énantiomères **313** (48 g) et **13** (57 g).

**Intermédiaire 313 :**

$\alpha_D$ (589nM) = - 69,9 (c = 0,010 g/mL, MeOH) à 20°C
**Pureté optique:** >99%, intermédiaire **13**< à 1%.

**Intermédiaire 13 :**

**RMN $^1$H** (400MHz, DMSO-$d_6$): 7,50 (d, 1H), 7,37 (d, 1H), 7,26 (d, 1H), 7,05 (dd, 1H), 4,54 (m, 1H), 2,32 (s, 3H), 1,36 (s, 9H), 1,27 (d, 1H)

**IR (cm$^{-1}$):** 3374, 1684
$\alpha_D$ (589nM) = + 71,80 (c = 0,010 g/mL, MeOH) à 20°C
**Pureté optique:** >99%, intermédiaire **313**< à 1%

L'intermédiaire **13** peut également être obtenu à partir de l'intermédiaire **459** en utilisant les conditions du **protocole XV.**

**Intermédiaire 459 :**

Obtenu par réaction de l'intermédiaire **9** et du (*R*)-(+)-2-méthyl-2-propanesulfinamide selon le **protocole XIIIb**
**RMN $^1$H** (400 MHz, DMSO-$d_6$): $\delta$ 7,50 (d, 1H), 7,35 (d, 1H), 7,15 (dd, 1H), 5,60 (d, 1H), 4,30 (quint, 1H), 2,30 (s, 3H), 1,40 (d, 3H), 1,10 (s, 9H)
**IR (cm$^{-1}$)** : 3207, 1052

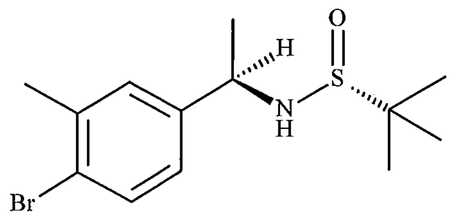

**459**

**Intermédiaire 32 :**

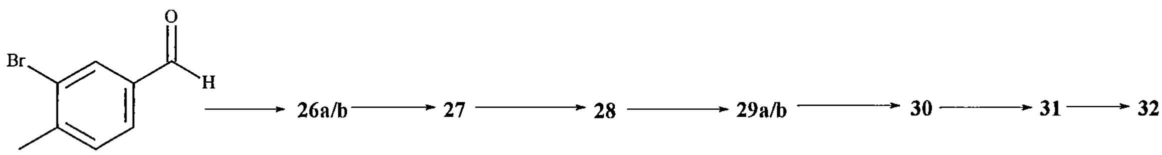


**Intermédiaires 26a/b :**

A une solution d'éthylate de sodium préparée par ajout de sodium (9,1 g, 396 mmoles) dans de l'éthanol (400 mL) préalablement refroidie à 0°C, est ajouté du $(EtO)_2POCH_2CN$ (63 mL, 396 mmoles). Le mélange résultant est agité à 0°C pendant 30 minutes puis le 3-bromo-4-methylbenzaldéhyde commercial (77 g, 391 mmoles) est ajouté lentement (environ 20 minutes). Le mélange réactionnel est agité à température ambiante jusqu'à disparition du produit de départ, puis il est versé sur de l'eau (4 L). Le précipité est filtré sur fritté, rincé à l'eau et séché sous vide. Le mélange d'intermédiaires **26a/b** (mélange des formes Z et E) est obtenu (81 g) et utilisé dans l'étape suivante.

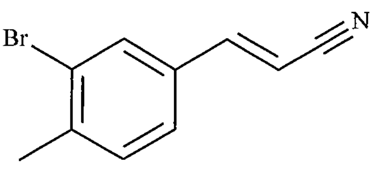

**26a**

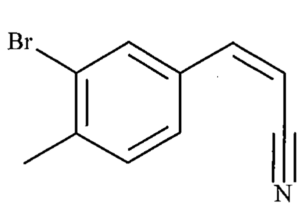

**26b**

**Intermédiaire 27 :**

A une solution des intermédiaires **26a/b** (75 g, 338 mmoles) dans l'isopropanol (1 L) est ajouté du $NaBH_4$ (51 g, 1,35 mole). Le mélange réactionnel est chauffé à 90-100°C pendant 48 heures et agité avec retour à température ambiante pendant 2 jours. Le solvant est évaporé, le résidu est repris par de l'eau, neutralisé avec précaution par de

l'HCl concentré et extrait par de l'AcOEt. La phase organique est lavée successivement par de l'eau, par une solution aqueuse saturée par du NaCl puis elle est séchée sur $MgSO_4$ et concentrée sous vide. Cette huile est chromatographiée sur gel de silice (chlorure de méthylène 100%). L'intermédiaire **27** (58 g) est obtenu sous forme d'une huile.
**RMN $^1$H** (400 MHz ; $CDCl_3$) : δ 7,40 (d, 1H); 7,20 (d, 1H); 7,10 (dd, 1H); 2,90 (t, 2H); 2,60 (t, 2H); 2,40 (s, 3H)
**IR ($cm^{-1}$)** : 2247, 1040.

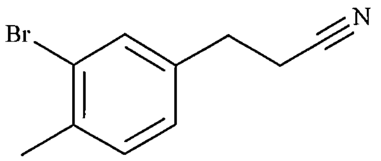

**27**

**Intermédiaire 28 :**

L'intermédiaire **27** traité selon le protocole décrit pour l'intermédiaire **599 (protocole V)** conduit à l'obtention de l'intermédiaire **28** (60 g).
**RMN $^1$H** (400 MHz ; $CDCl_3$) : δ 11,0 (m, 1H); 7,40 (d, 1H); 7,15 (d, 1H); 7,00 (dd, 1H); 2,90 (t, 2H); 2,65 (t, 2H); 2,35 (s, 3H).
**IR ($cm^{-1}$)** : 3400, 2100, 1697.

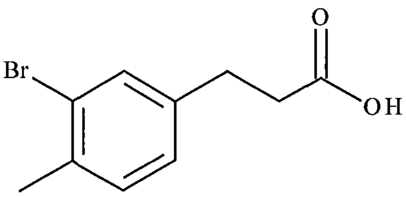

**28**

**Intermédiaires 29a/b :** A un mélange de $P_2O_5$ (14 g, 100 mmoles) et d'acide méthane sulfonique (142 mL) préalablement chauffé à 60°C pendant 2 heures, est ajouté l'intermédiaire **28** (12 g, 50 mmoles). Le mélange réactionnel est agité 35 minutes à 60°C, puis il est versé avec précaution sur de la glace. Le mélange est extrait par de l'AcOEt, la phase organique est lavée successivement par de l'eau, par une solution aqueuse de NaOH 4N, à nouveau de l'eau et une solution d'HCl. La phase organique est séchée sur $MgSO_4$ et concentrée sous vide. Le résidu est concrétisé dans de l'éther isopropylique. Le mélange d'intermédiaires **29a/b** (5 g) obtenu est engagé dans l'étape suivante.

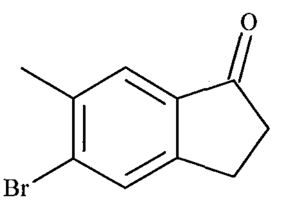

**29a**

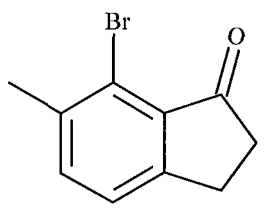

**29b**

**Intermédiaire 30 :** le mélange d'intermédiaires **29a/b** (5,2 g, 23 mmoles) est traité selon le protocole décrit pour l'exemple **341** en substituant de chlorhydrate de méthoxylamine par le chlorhydrate de o-benzylhydroxylamine. Après traitement et chromatographie ($SiO_2$, éluant : toluène/$CH_2Cl_2$ 80/20), l'intermédiaire **30** (2,5 g) est obtenu.
**RMN $^1$H** (300 MHz ; $CDCl_3$) : δ 7,55 (s, 1H); 7,50 (s, 1H); 7,4-7,25 (m, 5H); 5,20 (s, 2H); 2,90 (2m, 4H); 2,40 (s, 3H).

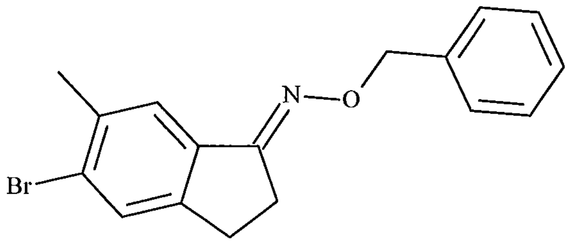

**30**

**Intermédiaire 31:**

Obtenu à partir de l'intermédiaire **30** selon le procédé décrit pour l'intermédiaire **48 (protocole XII)** **RMN $^1$H** (300 MHz ; $CDCl_3$) : 7,40 (s, 1H), 7,20 (s, 1H) ; 4,30 (t, 1H); 2,90 (m, 1H) ; 2,75 (m, 1H); 2,50 (m, 1H); 2,40 (s, 3H) ; 1,70 (m, 1H); 1,50 (m, 2H)

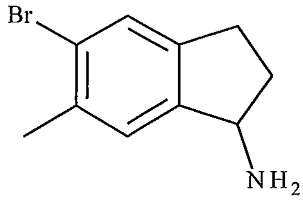

**31**

**Intermédiaire 32 :**

Obtenu par protection de l'intermédiaire **31** selon le protocole décrit pour l'intermédaire **158 (protocole VI)** **RMN $^1$H** (400MHz, $CDCl_3$): δ 7,40 (s, 1H), 7,20 (s, 1H), 5,10 (quad, 1H), 4,70 (d large, 1H), 2,90 (m, 1H), 2,80 (m, 1H), 2,55 (m, 1H), 2,40 (s, 3H), 1,80 (m, 1H), 1,50 (s, 9H) **IR (cm$^{-1}$):** 3299, 1674

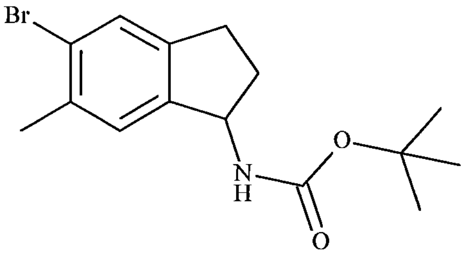

**32**

**Intermédiaire 40 :**

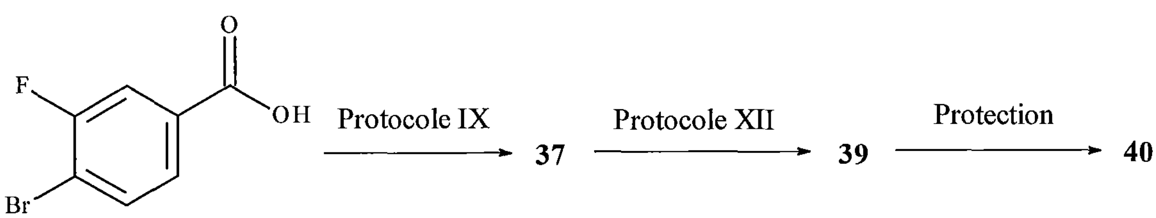


La synthèse de l'intermédiaire **37** est décrite à titre d'exemple pour le **protocole IX.**

**Intermédiaire 39 :**

Obtenu à partir de l'intermédiaire **37** selon le **protocole XII**
**RMN $^1$H** (300MHz ; $CDCl_3$): 7,49 (t, 1H), 7,18 (dd, 1H), 7,01 (dd, 1H), 4,11 (quad, 1H), 1,50 (s, 2H), 1,35 (d, 3H)
**IR (cm$^{-1}$):** 3372, 3288

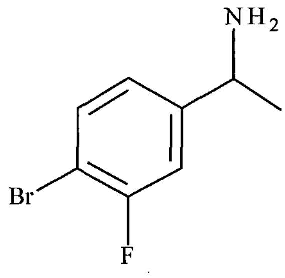

**39**

**Intermédiaire 40 :**

Obtenu par protection de l'intermédiaire **39** selon le protocole décrit pour l'intermédaire **158 (protocole VI)**
**RMN $^1$H** (300MHz, DMSO-$d_6$): δ 7,65 (t, 1H), 7,45 (d, 1H), 7,30 (dd, 1H), 7,09 (dd, 1H), 4,60 (m, 1H), 1,40 (s, 9H), 1,30 (d, 3H)
**IR (cm$^{-1}$)**: 3365, 1678

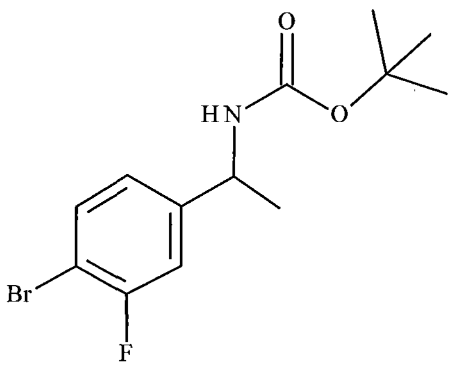

**40**

**Intermédiaire 676:**

Obtenu à partir de l'intermédiaire **39** selon le **protocole XVI**
**RMN $^1$H** (300 MHz, DMSO-$d_6$) : δ 9,80 (d, 1H), 7,70 (dd, 1H), 7,40 (dd, 1H), 7,15 (dd, 1H), 5,00 (quint, 1H), 1,45 (d, 3H)
**IR (cm$^{-1}$)** : 3267, 1702, 1556, 1205, 1146

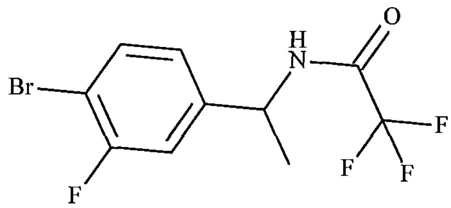

**676**

Intermédiaire 60b :

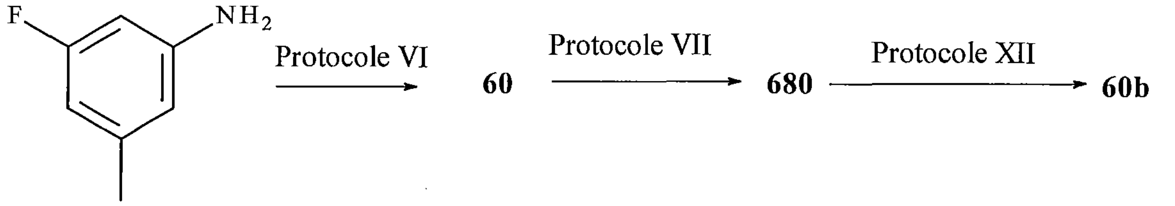


**Intermédiaire 60 :**

**4-bromo-3-fluoro-5-méthylaniline**

Obtenu par bromation de la 3-fluoro-5-méthylaniline commerciale selon le protocole décrit pour l'intermédiaire **67 (protocole VI)**

**Intermédiaire 680 :**

Obtenu à partir de la 4-bromo-3-fluoro-5-méthylaniline **60** selon le **protocole VII**
**RMN $^1$H** (300 MHz ; $CDCl_3$) : δ 7,60 (dd, 1H); 7,50 (dd, 1H); 2,60 (s, 3H); 2,50 (s large, 3H)
**IR (cm$^{-1}$)** : 1687

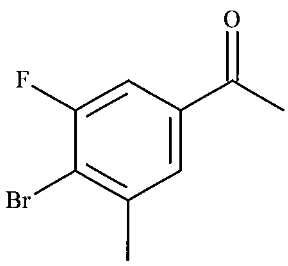

**680**

**Intermédiaire 60b :**

Obtenu à partir de l'intermédiaire **680** selon le **protocole XII**
**RMN $^1$H** (300MHz ; DMSO-$d_6$): 7,20 (m, 2H), 3,90 (quad, 1H), 2,40 (s, 3H), 1,85 (m, 2H), 1,20 (d, 3H)
**IR (cm$^{-1}$):** 3650-3030

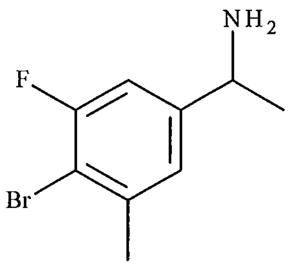

**60b**

Intermédiaire 73 :

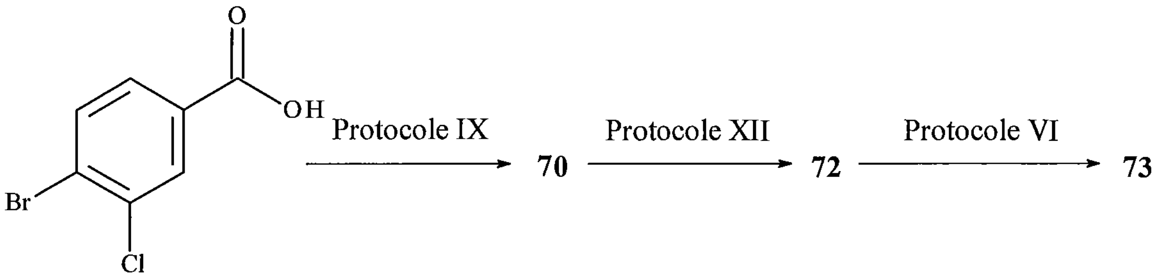


**Intermédiaire 70 :**

Obtenu à partir de l'acide 4-bromo-3-chloro-benzoïque commercial et du bromure de méthylmagnésium selon le **protocole IX**
**RMN $^1$H** (400 MHz ; $CDCl_3$): δ 8,00 (d, 1H), 7,70 (d, 1H), 7,65 (dd, 1H), 2,60 (s,3H)
**IR (cm-1):** 1680

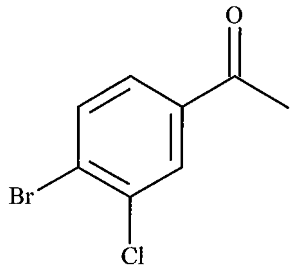

**70**

**Intermédiaire 72 :**

Obtenu à partir de l'intermédiaire **70** selon le **protocole XII**
**RMN $^1$H** (400MHz ; $CDCl_3$): 7,55 (d, 1H), 7,50 (sd, 1H); 7,10 (dd, 1H ;) 4,10 (quad, 1H); 1,60 (m, 2H); 1,35 (d, 3H)
**IR (cm$^{-1}$):** 3650, 3000

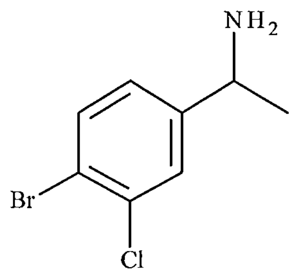

**72**

**Intermédiaire 73 :**

Obtenu par protection de l'intermédiaire **72** selon le protocole décrit pour l'intermédaire **158 (protocole VI)**
**RMN $^1$H** (300MHz, $CDCl_3$): δ 7,55 (d, 1H), 7,40 (sd, 1H), 7,05 (dd, 1H), 4,70 (m, 2H), 1,40 (m, 12H)
**IR (cm$^{-1}$):** 3365, 1681

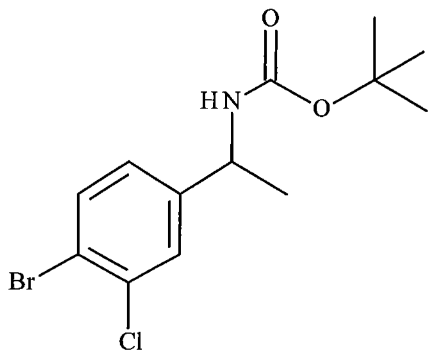

73

Intermédiaire 87 :

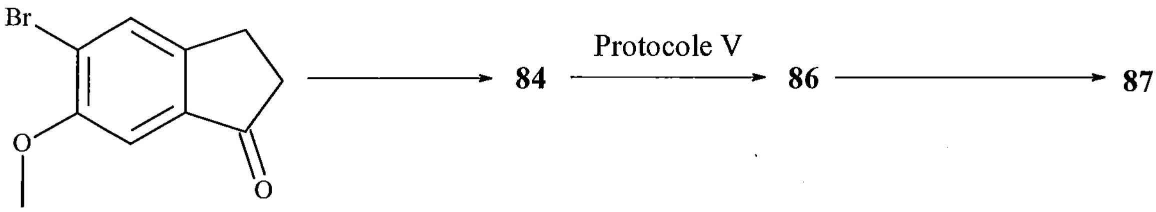


Intermédiaire 84 :

A un mélange de la 5-bromo-6-méthoxy-2,3-dihydro-1H-indén-1-one (10 g, 41mmoles) et de LiCN (0,27 g, 8,3mmoles) dans le THF (105 mL) est ajouté du $(Et_2O)_2POCN$ (9,5 mL, 62mmoles). Le mélange est agité à température ambiante pendant 5 heures, puis concentré sous vide. Le résidu est repris par de l'AcOEt, lavé par de l'eau puis par une solution aqueuse saturée en NaCl. La phase organique est séchée sur $MgSO_4$ et concentrée sous vide. Le résidu est repris par du toluène (100 mL) puis traité par du $BF_3.OEt_2$ (10,2 mL, 82,96 mmoles) pendant 5 heures. Le milieu est lavé par de l'eau puis par une solution aqueuse saturée en NaCl. La phase organique est séchée sur $MgSO_4$ puis concentrée sous vide. L'intermédiaire **83** (5-bromo-6-méthoxy-3H-indène-1-carbonitrile) ainsi obtenu (11,9 g) est utilisé dans l'étape suivante sans purification supplémentaire. Une solution de **83** (10 g, 41 mmoles) dans du THF (70 mL) est ajouté en 10 minutes sur un mélange de $NaBH_4$ (4,7 g, 124 mmoles) dans du THF (100 mL), cette addition est exothermique (Tmax 48°C). Le mélange est chauffé à 50°C pendant 2 heures 30, puis refroidi vers 0°C. Une solution d'HCl 4N (30 mL) est ajoutée, puis de l'$Et_2O$ (250 mL) et de l'eau (50 mL). La phase organique est extraite, séchée sur $MgSO_4$ et concentrée sous vide. Le résidu est purifié par chromatographie sur de la silice (éluant $CH_2Cl_2$/cyclohexane 50/50). L'intermédiaire **84** (3 g) est obtenu sous forme d'un solide jaune.
**RMN $^1$H** (300 MHz ; $CDCl_3$) : $\delta$ 7,45 (s, 1H) ; 6,95 (s, 1H), 4,05 (t, 1H), 3,90 (s, 3H), 3,0-2,9 (2m, 2H), 2,6-2,4 (2m, 2H)
**IR ($cm^{-1}$)** : 2238

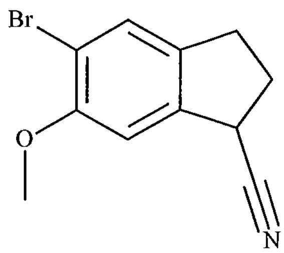

84

**Intermédiaire 86 :**

Obtenu à partir de l'intermédiaire **84** selon le protocole décrit pour la préparation de l'intermédiaire **599 (protocole V)**
**RMN $^1$H** (300 MHz ; DMSO-$d_6$) : δ 13,0-11,0 (m, 1H), 7,40 (s, 1H) ; 6,95 (s, 1H), 3,80 (s, 3H), 3,0-2,75 (m, 2H), 2,55 (m, 1H), 1,90 (m, 1H), 1,45 (s,3H).

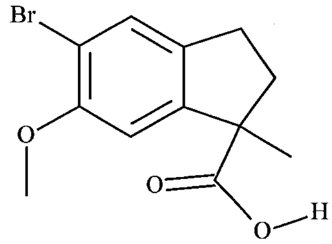

**86**

**Intermédiaire 87 :**

A une solution de *t*BuOH (18,3 mL) et de $Boc_2O$ (1,56 g, 7,1 mmoles) préalablement chauffée 2 heures à 90°C puis ramenée à température ambiante sont ajoutés successivement l'intermédiaire **86** (2,31 g, 8 mmoles), de la $NEt_3$ (1,13 mL, 8 mmoles) et du $PhO_2PON_3$ (1,75 mL). Le mélange est chauffé à 90°C pendant 3 jours puis il est concentré sous vide. Le résidu est purifié par chromatographie sur silice (éluant $CH_2Cl_2$ 100%) pour donner l'intermédiaire **87** (1,5 g).
**RMN $^1$H** (300 MHz ; DMSO-$d_6$) : δ 7,35 (s, 1H) ; 7,00 (m, 1H), 6,95 (d, 1H), 3,80 (s, 3H), 2,85-2,65 (m, 2H), 2,45 (m, 1H), 2,00 (m, 1H), 1,40 (s, 3H), 1,30 (s large, 9H)
**IR ($cm^{-1}$)** : 3356, 1694

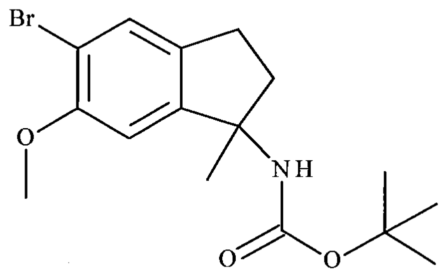

**87**

**Intermédiaire 120 :**

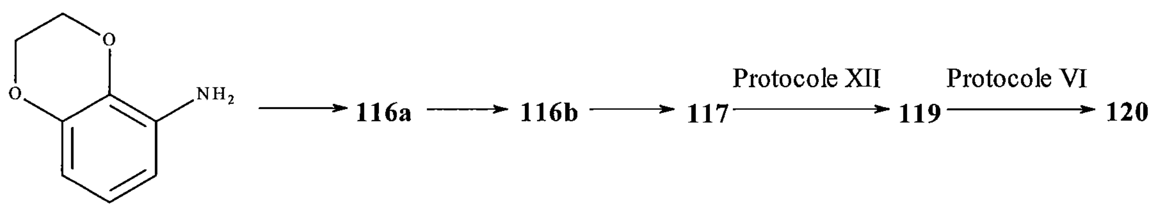


**Intermédiaire 116a :**

A une solution de 2,3-dihydro-1,4-benzodioxin-5-amine (40 g, 260 mmoles) dans le DMF (750 mL) refroidie à -30°C est ajouté goutte à goutte une solution de *N*-bromosuccinimide (47 g) dans le DMF (250 mL). Le milieu est agité 1 heure, puis il est versé sur un mélange eau/glace (500 mL/500 g) ; le précipité formé est solubilisé dans du chlorure de méthylène. La phase organique est séchée sur $MgSO_4$, puis concentré sous vide. L'intermédiaire **116a** est obtenu

sous forme d'un solide violet (30 g).
**RMN $^1$H** (400MHz, DMSO-$d_6$): δ 6,78 (d, 1H), 6,20 (d, 1H), 4,25 (m, 4H), 4,90 (s, 2H) **IR (cm$^{-1}$):** 3480

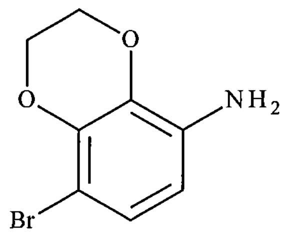

**116a**

**Intermédiaire 116b** :

A un mélange de l'intermédiaire **116a** (7 g, 30 mmoles) dans de l'eau (20 mL) est ajouté du $HCl_{cc}$ (20 mL), le mélange résultant est refroidi à 0°C et une solution de $NaNO_2$ (2,2 g) dans de l'eau (10 mL) est ajoutée. Le milieu est agité 1h30 à 0°C, puis une solution aqueuse de KI (5 g dans 7 mL) est ajoutée, l'ensemble est chauffé progressivement à 90°C. Après retour à température ambiante, le milieu est versé sur de la glace, le précipité formé est solubilisé dans de l'AcOEt. La phase organique est lavée par une solution de thiosulfate de sodium 0,1N, séchée sur $MgSO_4$, puis évaporée sous vide. L'intermédiaire **116b** est obtenu sous forme d'un solide marron (7 g) (qui peut être chromatographié sur gel de silice (cyclohexane/chlorure de méthylène 80/20 à 0/100)).
**RMN $^1$H** (400MHz, DMSO-$d_6$): δ 7,25 (d, 1H), 6,95 (d, 1H), 4,40 (s, 4H)
**IR (cm$^{-1}$)**: 1734

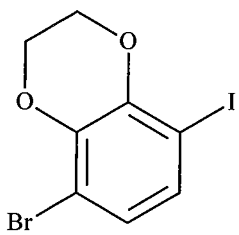

**116b**

**Intermédiaire 117 :**

A une solution dégazée à l'azote de l'intermédiaire **116b** (15 g, 44 mmoles) dans du DMF (500 mL), est ajouté du tri-butyl-(1-éthoxyvinyl)-étain (15 mL, 44 mmoles). La solution est amenée à 70°C puis du $PdCl_2(PPh_3)_2$ (3,7 g) est ajouté. Le mélange est chauffé à 70°C jusqu'à disparition de l'intermédiaire de départ. Après retour à température ambiante, le mélange réactionnel est traité successivement par 10 g de KF dans de l'eau (1 L) et par de l'éther éthylique. Les sels sont filtrés et la phase organique est concentrée sous vide. Le résidu ainsi obtenu solubilisé dans du THF est agité 1 heure en présence de 30 mL d'une solution aqueuse d'HCl 1N. Le milieu est alors décanté en présence de 1 L d'$Et_2O$, la phase organique est séchée sur $MgSO_4$. L'évaporation sous pression réduite conduit à l'obtention d'une huile qui est chromatographiée sur gel de silice en utilisant un gradient d'élution cyclohexane/chlorure de méthylène (50/50 à 20/80). L'intermédiaire **117** est obtenu sous forme d'un solide orangé (4,6 g).
**RMN $^1$H** (400MHz, DMSO-$d_6$): δ 7,2-7,1 (2d, 2H), 4,40 (s, 4H), 2,55 (s, 3H);7,25 (d, 1H), 6,95 (d, 1H), 4,40 (s, 4H)
**IR (cm$^{-1}$):** 1664

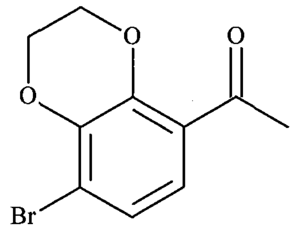

**117**

**Intermédiaire 119 :**

Obtenu à partir de l'intermédiaire **117** selon le **protocole XII**
**RMN $^1$H** (400MHz ; DMSO-$d_6$): 7,08 (d, 1H), 6,95 (d, 1H), 4,30 (m, 4H), 4,20 (quad, 1H), 1,20 (d, 3H)

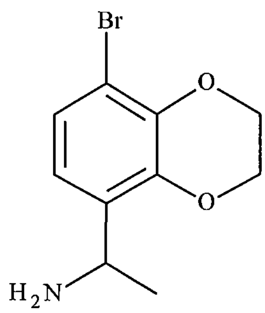

**119**

**Intermédiaire 120 :**

Obtenu par protection de l'intermédiaire **119** selon le protocole décrit pour l'intermédaire **158 (protocole VI)**
**RMN $^1$H** (400MHz, DMSO-$d_6$): δ 7,33 (d, 1H), 7,09 (d, 1H), 6,78 (d, 1H), 4,82 (m, 1H), 4,30 (m, 4H), 1,35 (s, 9H), 1,20 (d, 3H)
**IR (cm$^{-1}$):** 3368, 1684

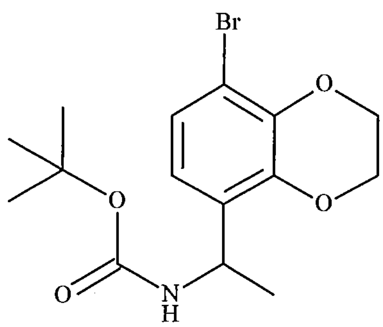

**120**

**<u>Intermédiaire</u> <u>371 :</u>**

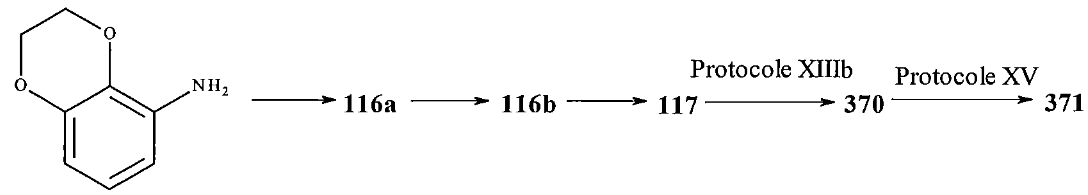


**Intermédiaire 370 :**

Obtenu à partir de l'intermédiaire **117** et du (*S*)-(-)-2-méthyl-2-propanesulfinamide selon le **protocole XIIIb**
**RMN $^1$H** (400 MHz, DMSO-$d_6$): δ 7,12 (d, 1H), 6,95 (d, 1H), 5,59 (d, 1H), 4,63 (m, 1H),4,32 (m, 4H), 1,33 (d, 3H), 1,10 (s, 9H)
**IR (cm$^{-1}$)** : 3500, 3000, 1056

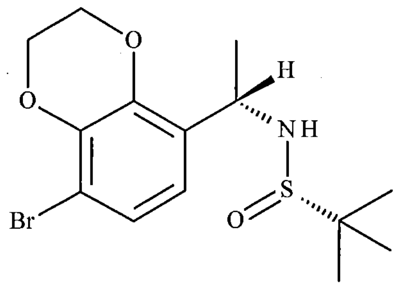

**370**

**Intermédiaire 371 :**

Obtenu à partir de l'intermédiaire **370** selon le **protocole XV**
**RMN $^1$H** (400MHz, DMSO-d6): δ 7,35 (d, 1H), 7,09 (d, 2H), 6,79 (d, 2H), 4,85 (m, 1H), 4,33 (m, 4H), 1,37 (s, 9H), 1,21 (d, 3H)
**IR (cm$^{-1}$)**: 3260, 1695, 1676
**Excès énantiomérique** > 99%

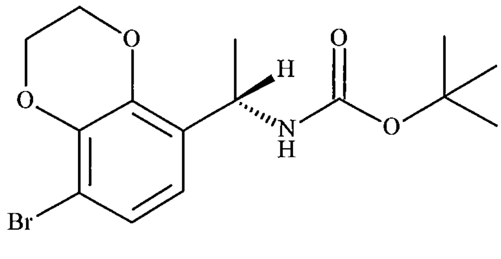

**371**

**Intermédiaire 349b :**

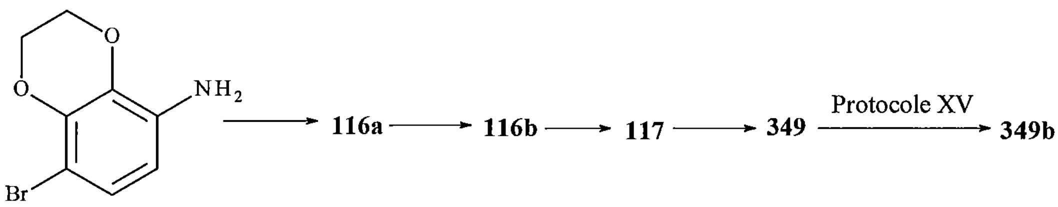

**Intermédiaire 349 :**

A une solution de l'intermédiaire **117** (10,7 g, 41 mmoles) dans 160 mL de THF sont ajoutés successivement du $Ti(OEt)_4$ (30 mL, 143 mmoles) puis du (R)-(+)-2-méthyl-2-propanesulfinamide (5 g, 41 mmoles). Le milieu est chauffé 48 heures à 55°C. Le milieu refroidi à -40°C est transféré par cannulation sur une suspension de $NaBH_4$ (3,1 g ; 82 mmoles) dans 46 mL de THF. Le milieu réactionnel à température ambiante, est traité avec précaution par du méthanol, puis dilué par 300 mL d'acétate d'éthyle et 700 mL d'une solution aqueuse saturée de NaCl. Le mélange résultant est filtré sur de la Célite® qui est rincée par du THF et de l'AcOEt. Le filtrat est décanté, la phase organique est séchée par passage sur $MgSO_4$. L'évaporation sous pression réduite conduit à l'obtention d'un solide blanc qui est purifié sur silice en utilisant un gradient d'élution AcOEt/chlorure de méthylène 0/100à 40/60. Le diastéréoisomère **349** (7 g) est isolé sous la forme d'un solide blanc.
**RMN $^1$H** (400MHz; DMSO-$d_6$): $\delta$ 7,12 (d, 1H), 6,95 (d, 1H), 5,59 (d, 1H), 4,63 (m, 1H), 4,32 (m, 4H), 1,33 (d, 3H); 1,10 (s, 9H)
**IR (cm$^{-1}$):** 3265, 1057
**GC-EI** (70 eV): $M^+$ = 361
Pureté diastéréoisomèrique : de>99%

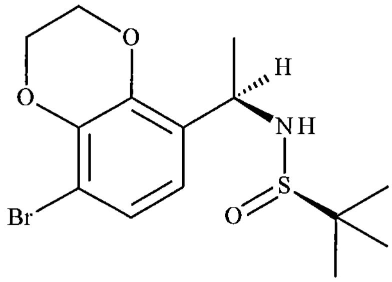

**349**

**Intermédiaire 349b :**

Obtenu à partir de l'intermédiaire **349** selon le **protocole XV**
**RMN $^1$H** (400MHz, DMSO-$d_6$): $\delta$ 7,33 (d large, 1H), 7,08 (d, 1H), 6,78 (d, 1H), 4,83 (m, 1H), 4,32 (m large, 4H), 1,35 (s large, 9H), 1,21 (d, 3H)
**Excès énantiomérique** > 99%

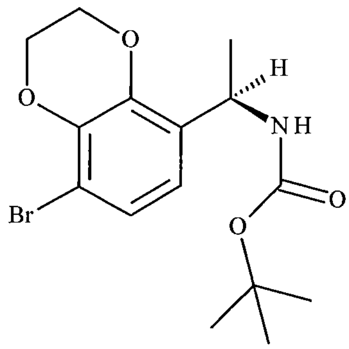

**349b**

**Intermédiaire 135 :**

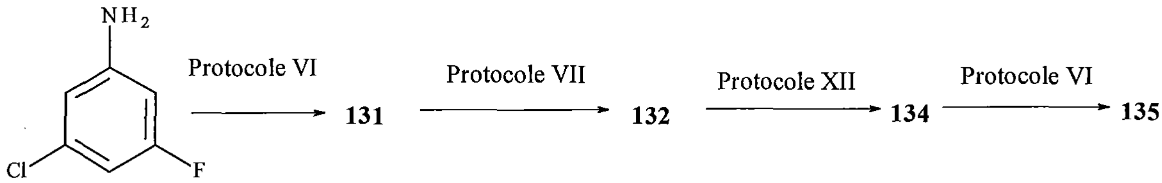


**Intermédiaire 131**

**4-bromo-3-chloro-5-fluoroaniline**

Obtenu par bromation de la 3-chloro-5-fluoroaniline commerciale selon le protocole décrit pour l'intermédiaire **67(protocole VI)**

**Intermédiaire 132 :**

Obtenu à partir de l'intermédiaire **131** selon le **protocole VII**
**RMN $^1$H** (300MHz, $CDCl_3$) : δ 7,85(s, 1H), 7,60 (d, 1H), 2,60 (s,3H)
**IR ($cm^{-1}$)** : 1692, 1206.

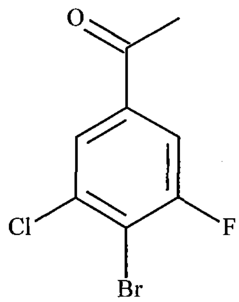

**132**

**Intermédiaire 134 :**

Obtenu à partir de l'intermédiaire **132** selon le **protocole XII**
**RMN $^1$H** (300MHz ; $CDCl_3$): 7,30 (s, 1H), 7,05(d, 1H) 4,10 (quad, 1H); 1,35 (d, 3H)
**IR ($cm^{-1}$):** 3600, 2500

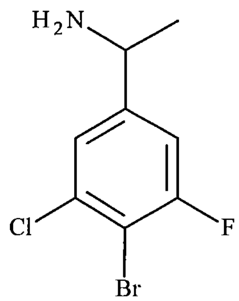

**134**

**Intermédiaire 135 :**

Obtenu par protection de l'intermédiaire **134** selon le protocole décrit pour l'intermédaire **158 (protocole VI)**
**RMN $^1$H** (400MHz, $CDCl_3$): δ 7,20 (s, 1H), 7,00 (d, 1H), 4,75 (slarge, 1H), 4,70 (m, 1H), 1,40 (s et d, 12H)
**IR ($cm^{-1}$):** 3367, 1682, 1163

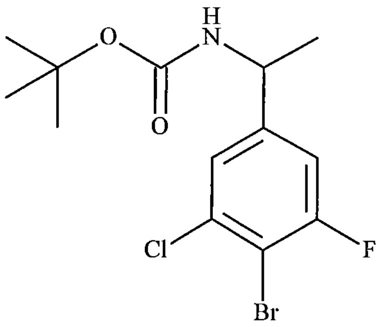

**135**

<u>**Intermédiaire 174 :**</u>

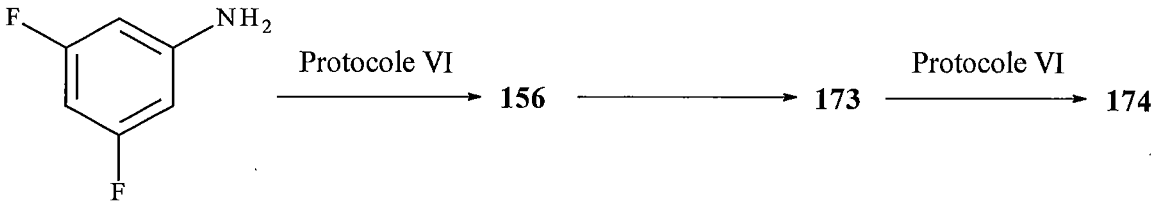


**Intermédiaire 173 :**

L'intermédiaire **173** a été préparé par réduction au $BH_3$.THF de l'intermédiaire **156** (4-bromo-3,5-difluorobenzonitrile).
**RMN $^1$H** (300MHz, DMSO-$d_6$): δ 8,60 (m, 3H), 7,50 (d, 2H), 4,05 (s, 2H)
**IR (cm$^{-1}$)**: 3450-2400.

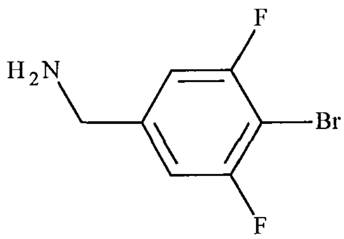

**173**

**Intermédiaire 174 :**

Obtenu par protection de l'intermédiaire **173** selon le protocole décrit pour l'intermédaire **158 (protocole VI)**
**RMN $^1$H** (300MHz, DMSO-$d_6$): δ 7,50 (t, 1H), 7,10 (d, 2H), 4,10 (d, 2H), 1,40 (s, 9H)
**IR (cm$^{-1}$):** 3323, 1681

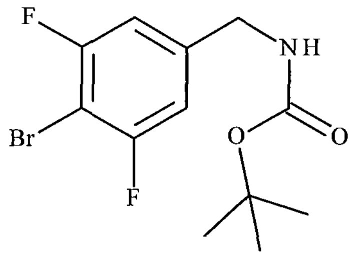

**174**

<u>**Intermédiaire 112 :**</u>

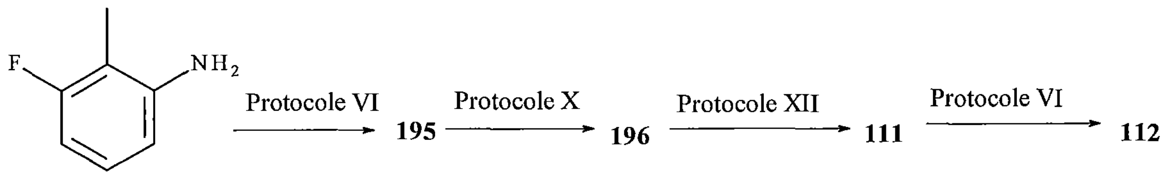


**Intermédiaire 195 :**

**1-bromo-2-fluoro-4-iodo-3-méthylbenzène**

Obtenu à partir de la 3-fluoro-2-méthylaniline commerciale selon le protocole décrit pour l'intermédiaire **155 (protocole VI)**

**Intermédiaire 196 :**

Obtenu à partir de l'intermédiaire **195** selon le **protocole X**
**RMN $^1$H** (400MHz, $CDCl_3$): δ 7,45 (dd, 1H), 7,30 (d, 1H), 2,60 (s, 3H), 1,40 (s, 3H)
**IR (cm$^{-1}$)** : 1684

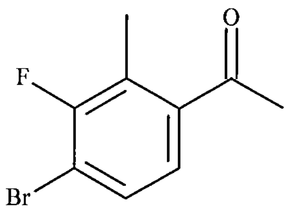

**196**

**Intermédiaire 111 :**

Obtenu à partir de l'intermédiaire **196** selon le **protocole XII**
**RMN $^1$H** (400MHz ; $CDCl_3$): 7,40 (dd, 1H), 7,20 (d, 1H) ; 4,30 (quad, 1H) ; 2,30 (s, 3H), 1,60 (m, 2H) ; 1,30 (d, 3H)
**IR (cm$^{-1}$):** 3750, 2900

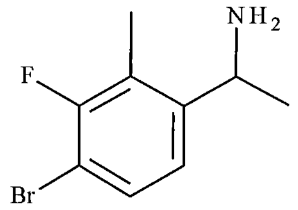

**111**

**Intermédiaire 112 :**

Obtenu par protection de l'intermédiaire **111** selon le protocole décrit pour l'intermédaire **158 (protocole VI)**
**RMN $^1$H** (300MHz, DMSO-$d_6$): δ 7,50 (dd, 1H), 7,10 (d, 1H), 4,75 (quint, 1H), 2,25 (m, 3H), 1,35 (m, 9H), 1,25 (d, 3H)
**IR (cm$^{-1}$):** 3301, 1690-1664

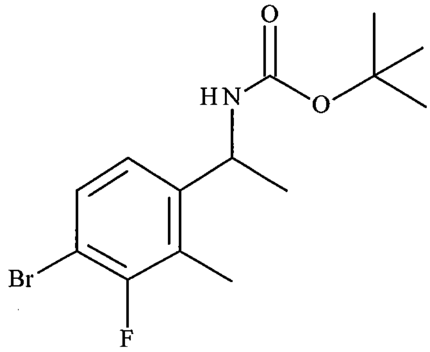

**112**

**Intermédiaire 187 :**

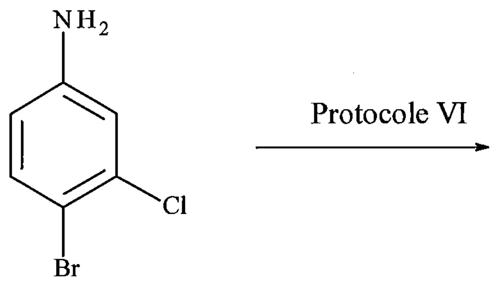


**187**

Obtenu à partir de la 4-bromo-3-chloroaniline commerciale selon le **protocole VI**
**RMN $^1$H** (400 MHz ; DMSO-$d_6$) : δ 7,70 (d, 1H) ; 7,50 (d, 1H) ; 7,30 (s, 1H) ; 7,20 (dd, 1H) ; 1,45 (s, 6H) ; 1,30 (s, 9H)
**IR (cm$^{-1}$)** : 3264-2972 ; 1698.

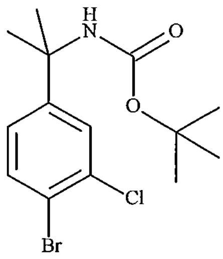

187

Intermédiaire 193 :

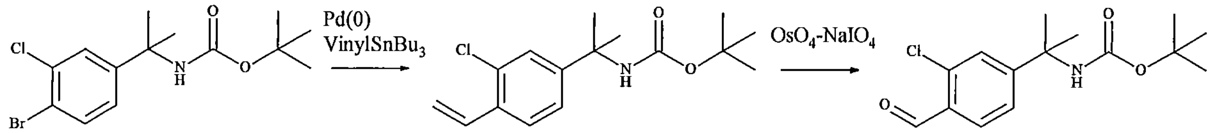


187 192 193

Intermédiaire 192 :

A une solution (dégazée à l'azote) de l'intermédiaire **187** (12 g, 34 mmoles) dans le DMF (240 mL) sont ajoutés du tri-butyl-vinyl étain (10 mL, 34 mmoles) et du $Pd(PPh_3)_4$ (2,47 g). Le mélange réactionnel est chauffé à 100°C pendant 20h. Après retour à température ambiante, le mélange est traité par une solution aqueuse à 10% de KF, les sels résultants sont filtrés, le filtrat est extrait par de l'acetate d'éthyle. La phase organique est lavée par une solution aqueuse saturée en NaCl, séchée sur $MgSO_4$ et concentrée sous vide. Le résidu est chromatographié sur gel de silice (éluant $CH_2Cl_2$ (100%)). L'intermédiaire **192** (4,7 g) est obtenu sous forme d'un solide jaune.
**RMN $^1$H** (400 MHz, DMSO-$d_6$) : δ 7,60 (d, 1H), 7,36 (d, 1H), 7,30 (dd, 1H), 6,99 (dd, 1H), 6,85 (m, 1H), 5,82 (d, 1H), 5,40 (d, 1H), 1,52 (s, 6H), 1,31 (s, 9H)
**IR (cm$^{-1}$)** : 3327, 1689

Intermédiaire 193 :

A un mélange de l'intermédiaire **192** (4,7 g, 15 mmoles) dans du dioxane (95 mL) et de l'eau (5 mL) sont ajoutés de l'$OsO_4$ (2,5% en poids dans le butanol) (3,2 g) de la 2,6-lutidine (3,7 g, 31 mmoles) et du $NaIO_4$ (13,6 g, 63 mmoles). Le mélange réactionnel est agité 1h à température ambiante. Après ajout d'AcOEt et d'une solution de NaCl saturée, le solide est filtré, le filtrat est extrait par de l'acetate d'éthyle. La phase organique est lavée par une solution de NaCl saturée, séchée sur $MgSO_4$ et concentrée sous vide. Le résidu est chromatographié sur gel de silice en utilisant un éluant $CH_2Cl_2$ (100). L'intermédiaire **193** (4,2 g) est obtenu sous forme d'une huile incolore.
**RMN $^1$H** (400 MHz, DMSO-$d_6$) : δ 10,40 (s 1H), 7,81 (d, 1H), 7,50 (m, 2H), 7,40 (m, 1H), 1,50 (s, 6H), 1,35 (m, 9H)
**IR (cm$^{-1}$)** : 3350, 1688193

Intermédiaire 200 :

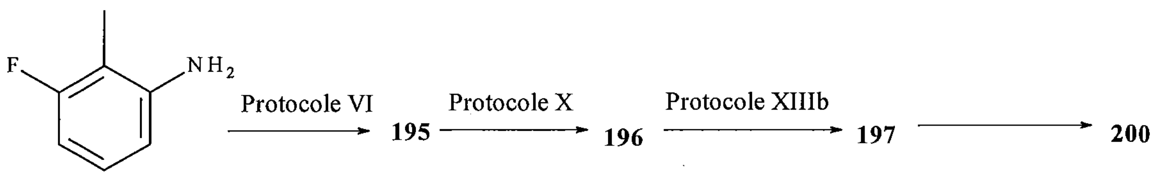

**Intermédiaire 195 :**

**1-bromo-2-fluoro-4-iodo-3-méthylbenzène**

Obtenu à partir de la 3-fluoro-2-méthylaniline commerciale selon le protocole décrit pour l'intermédiaire **155 (protocole VI)**

**Intermédiaire 196 :**

Obtenu à partir de l'intermédiaire **195** selon le **protocole X**
**RMN $^1$H** (400MHz, $CDCl_3$): δ 7,45 (dd, 1H), 7,30 (d, 1H), 2,60 (s, 3H), 1,40 (s, 3H)
**IR ($cm^{-1}$)** : 1684

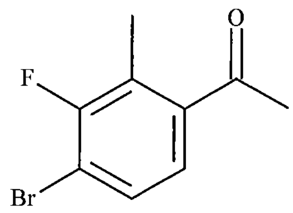

**196**

**Intermédiaire 197 :**

Obtenu par réaction de l'intermédiaire **196** et du (*R*)-(+)-2-méthyl-2-propanesulfinamide selon le **protocole XIIIb**
**RMN $^1$H** (400 MHz, $CDCl_3$): δ 7,40 (m, 1H), 7,05 (d, 1H), 4,75 (m, 1H), 2,30 (d, 3H), 1,45 (d, 3H), 1,20 (s, 9H), 3,30 (m, 1H)
**IR ($cm^{-1}$):** 3378, 3290, 1620

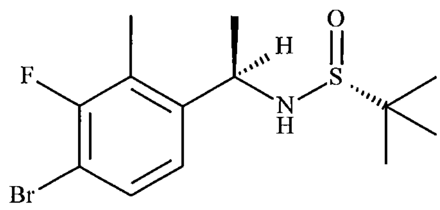

**197**

**Intermédiaire 200 :**

Obtenu à partir de l'intermédiaire **197** selon le **protocole XV**
**RMN $^1$H** (400MHz, DMSO-$d_6$): δ 7,50 (2m, 2H), 7,10 (d, 1H), 4,75 (m, 1H), 2,25 (d, 3H), 1,35 (s, 9H), 1,25 (d, 3H)
**IR ($cm^{-1}$):** 3373, 1681
**Excès énantiomérique** > 99%

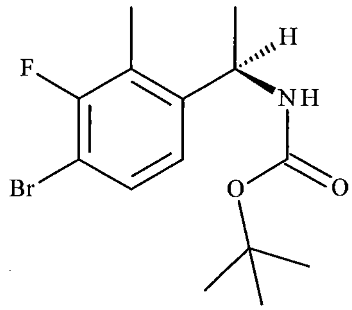

**200**

__Intermédiaire 211 :__

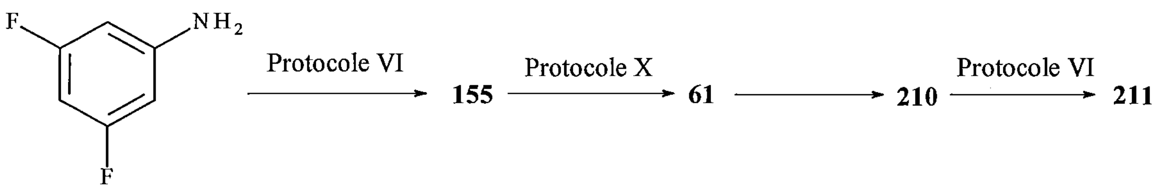


**Intermédiaire 210 :**

A un mélange de $TiOiPr_4$ (16 mL, 54 mmoles) dans de l'éthanol (70 mL) sont ajoutés de la $NEt_3$ (7,7 mL, 55,3 mmoles), du chlorhydrate de méthylamine (3,7 g, 54,8 mmoles) et l'intermédiaire **61** (7 g, 27,8 mmoles). Le milieu est agité à température ambiante pendant 40 heures, puis du $NaBH_4$ (1,56 g, 41,4 mmoles) est ajouté par portions. Après 20 heures d'agitation le mélange réactionnel est versé avec précaution sur une solution aqueuse de $NH_4OH$ 2N, le précipité résultant est filtré et rincé par du chlorure de méthylène. Le filtrat est décanté, la phase organique est lavée par une solution aqueuse d'HCl 2N, la phase acide est ramenée à pH basique par une solution de soude à 20%. Le produit est extrait au chlorure de méthylène, la phase organique est séchée sur $MgSO_4$, l'évaporation sous pression réduite conduit à l'obtention d'une huile, qui est purifiée sur gel de silice en utilisant un gradient d'élution chlorure de méthylène/éthanol 100/0 à 95/5. L'intermédiaire **210** est isolé sous la forme d'une huile (1,5 g).
**RMN $^1$H** (400MHz; DMSO-$d_6$): $\delta$ 7,25 (d, 2H), 3,6 (q, 1H), 2,5-2,15 (m, 1H), 2,10 (s, 3H), 1,20 (d, 3H)
**IR (cm$^{-1}$):** 3280-3360

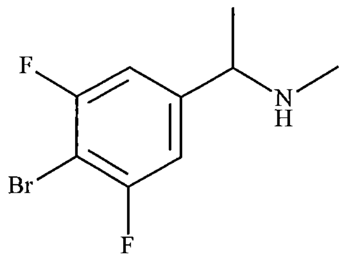

**210**

__Intermédiaire 211 :__

Obtenu par protection de l'intermédiaire **210** selon le protocole décrit pour l'intermédaire **158 (protocole VI)**
**RMN $^1$H** (400MHz, DMSO-$d_6$): $\delta$ 7,12 (m, 2H), 5,20 (s large, 1H), 2,63 (s, 3H), 1,45 (d, 3H), 1,39 (slarge, 9H)
**IR (cm$^{-1}$):** 1686

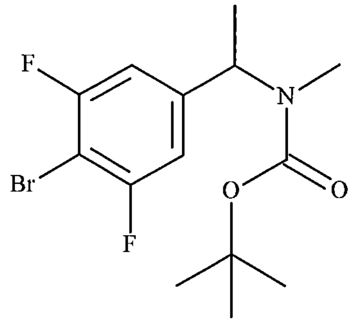

**211**

**Intermédiaire 217 :**

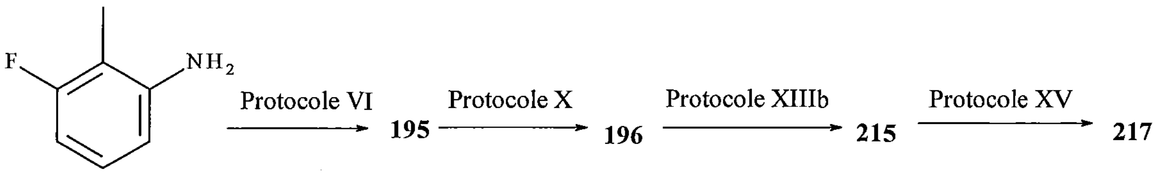


**Intermédiaire 195 :**

**1-bromo-2-fluoro-4-iodo-3-méthylbenzène**

Obtenu à partir de la 3-fluoro-2-méthylaniline commerciale selon le protocole décrit pour l'intermédiaire **155 (protocole VI)**

**Intermédiaire 196 :**

Obtenu à partir de l'intermédiaire **195** selon le **protocole X**
**RMN $^1$H** (400MHz, $CDCl_3$): δ 7,45 (dd, 1H), 7,30 (d, 1H), 2,60 (s, 3H), 1,40 (s, 3H)
**IR ($cm^{-1}$)** : 1684

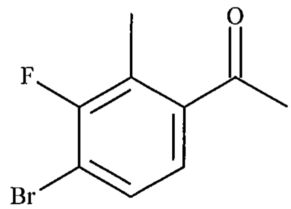

**196**

**Intermédiaire 215 :**

Obtenu par réaction de l'intermédiaire **196** et du (*S*)-(-)-2-méthyl-2-propanesulfinamide selon le **protocole XIIIb**
**RMN $^1$H** (300 MHz, DMSO-$d_6$): δ 7,50 (dd, 1H), 7,25 (d, 1H), 5,70 (d, 1H), 4,55 (quint, 1H), 2,25 (d, 3H), 1,35 (d, 3H), 1,10 (s, 9H)
**IR ($cm^{-1}$)** : 3353, 3298, 1121

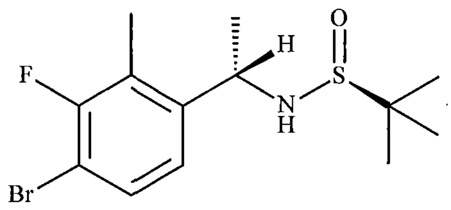

**215**

**Intermédiaire 217 :**

Obtenu à partir de l'intermédiaire **215** selon le **protocole XV**
**RMN $^1$H** (400MHz, DMSO-$d_6$): δ 7,50 (t et m, 2H), 7,10 (d, 1H), 4,75 (m, 1H), 2,25 (d, 3H), 1,35 (s, 9H), 1,25 (d, 3H)
**IR (cm$^{-1}$):** 3373, 1681
**Excès énantiomérique** > 99%

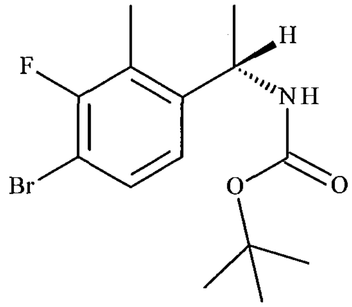

**217**

**Intermédiaires 615a et 615b :**

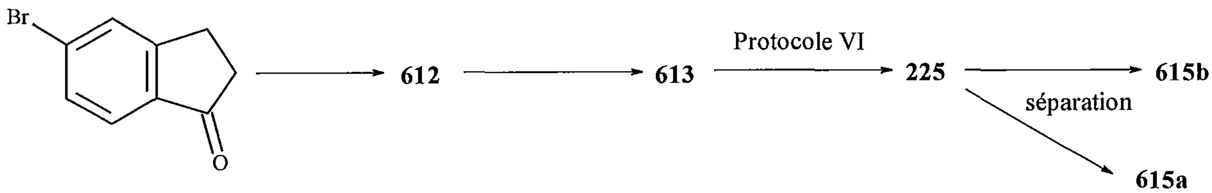


**Intermédiaire 612 :**

A une solution de la 5-bromo-2,3-dihydro-1H-indén-1-one commerciale (5 g, 23 mmoles) dans de l'éthanol (47 mL) sont ajoutés successivement du chlorhydrate de hydroxylamine (3,1 g, 44 mmoles) et de la pyridine (9,5 mL). Le mélange réactionnel est agité 8 heures à 80°C. La pyridine est évaporée sous vide, le résidu est repris dans l'eau, puis extrait par du chlorure de méthylène. Après séchage de la phase organique sur $MgSO_4$ et concentration l'intermédiaire **612** est obtenu sous forme d'un solide (5,13 g) utilisé sans traitement supplémentaire dans l'étape suivante.
**RMN $^1$H** (300MHz; DMSO-$d_6$): δ 11,0 (s, 1H), 7,60 (s, 1H), 7,45 (2d, 2H), 3,0-2,8 (2m, 4H)
**IR (cm$^{-1}$):** 3100

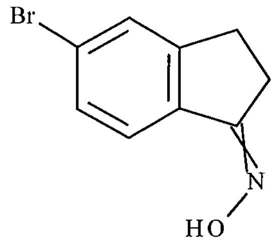

**612**

**Intermédiaire 613 :**

A 0°C, un mélange de **612** (0,5 g, 2,2 mmoles), de $MoO_3$ (0,42 g, 2,9 mmoles) dans du méthanol (22 mL) est traité avec précaution par du $NaBH_4$ (0,84 g, 2,2 mmoles) en maintenant une température inférieure à 36°C. Le milieu réactionnel est agité 1 heure à 0°C, puis à température ambiante 18 heures, avant d'être concentré sous vide. Le résidu est repris avec précaution par une solution aqueuse d'HCl 1N et de l'acétate d'éthyle, les sels sont filtrés et le filtrat décanté. La phase aqueuse est basifiée puis extraite par du chlorure de méthylène. Après séchage sur $MgSO_4$ et concentration sous vide, l'intermédiaire **613** est obtenu (0,28 g).
**RMN $^1$H** (300MHz ; DMSO-$d_6$): $\delta$ 7,35 (s, 1H), 7,35 (d, 1H), 4,15 (t, 1H), 2,85-2,7 (m, 2H), 2,3-1,55 (m, 2H), 2,00 (m, 2H)
**IR (cm$^{-1}$):** 3400-3300

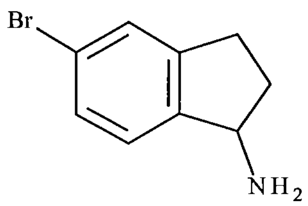

**613**

**Intermédiaire 225 :**

Obtenu par protection de l'intermédiaire **613** selon le protocole décrit pour l'intermédaire **158 (protocole VI)**
**RMN $^1$H** (400MHz, $CDCl_3$): $\delta$ 7,36 (s large, 1H), 7,33 (d large, 1H), 7,19 (d, 1H), 5,12 (quad, 1H), 4,69 (m, 1H), 2,93 (ddd, 1H), 2,82 (ddd, 1H), 2,57 (m, 1H), 1,79 (m, 1H), 1,48 (s, 9H)
**IR (cm$^{-1}$):** 3316, 1682

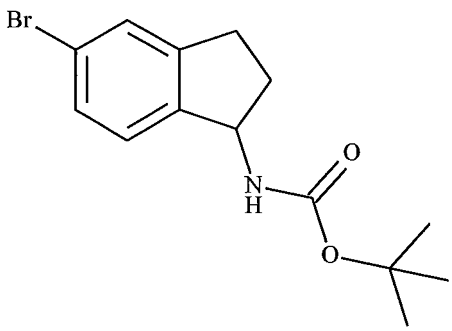

**225**

L'intermédiaire **225** (18 g) a été purifié par chromatographie haute pression sur support chiral (Colonne ChiralPak T101, éluant iPrOH/$CH_3CN$ 10/90, détection: 275nm) pour donner les énantiomères **615a** (9,8 g) et **615b** (7,4 g).
**Intermédiaire 615a:** $\alpha_D$ (589nM) = 76,78 (c = 0,011 g/mL, MeOH) à 20°C
**Intermédiaire 615b:** $\alpha_D$ (589nM) = -77,52 (c = 0,011 g/mL, MeOH) à 20°C

Intermédiaire 245 :

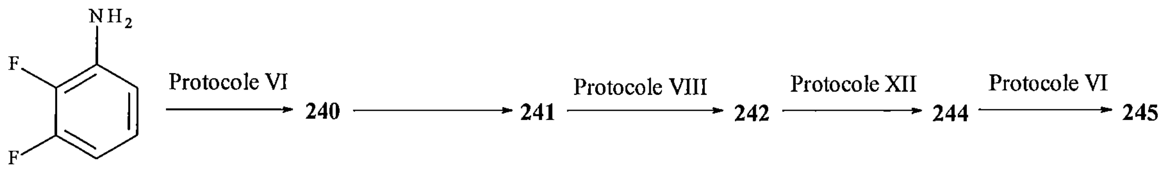


Intermédiaire 240 :

4-bromo-2,3-difluorobenzonitrile

Obtenu à partir de la 2,3-difluoroaniline commerciale selon le protocole décrit pour la préparation de l'intermédiaire **156 (protocole VI)**

Intermédiaire 241 :

Obtenu par traitement de l'intermédiaire **240** (0,5 g, 2,3 mmoles) dans du méthanol (12 mL) à 0°C en présence de méthylate de sodium (0,25 g, 4,6 mmoles).

Le milieu réactionnel est agité 72 heures à température ambiante, puis il est versé sur de l'eau. Le précipité collecté sur fritté est séché sous vide. L'intermédiaire **241** (0,3 g) est obtenu.

**RMN $^1$H** (400MHz, $CDCl_3$): δ 7,30 (dd, 1H), 7,22 (d, 1H), 4.16 (s, 3H)
**IR ($cm^{-1}$):** 3091, 2233, 1674
**GC-EI** (70 eV): M+. = 229

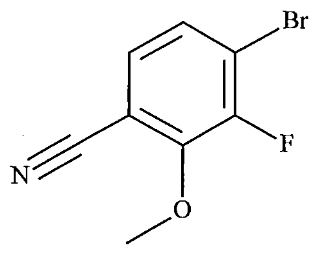

**241**

Intermédiaire 242 :

Obtenu à partir de l'intermédiaire **241** selon le **protocole VIII**
**RMN $^1$H** (400 MHz ; DMSO-$d_6$) : δ 7,52 (dd, 1H), 7,39 (dd, 1H), 3,97 (s, 3H), 2,55 (s, 3H)
**IR ($cm^{-1}$)** : 1681

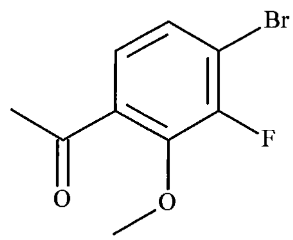

**242**

**Intermédiaire 244 :**

Obtenu à partir de l'intermédiaire **242** selon le **protocole XII**
**RMN $^1$H** (400MHz ; DMSO-$d_6$): 8,45 (s, 3H), 7,55 (m, 1H), 7,35 (d, 1H), 4,60 (quad, 1H), 3,95 (s, 3H), 1,50 (d, 3H)
**IR (cm$^{-1}$):** 3170-2400

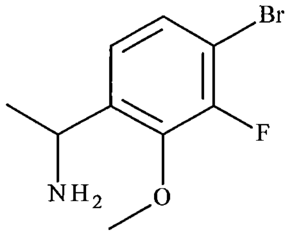

**244**

**Intermédiaire 245 :**

Obtenu par protection de l'intermédiaire **244** selon le protocole décrit pour l'intermédaire **158 (protocole VI)**
**RMN $^1$H** (300MHz, DMSO-$d_6$): δ 7,47 (d large, 1H), 7,41 (dd, 1H), 7,10 (d, 1H), 4,88 (m, 1H), 3,90 (s, 3H), 1,36 (s, 9H), 1,25 (d, 3H)
**RMN $^{19}$F:** -125

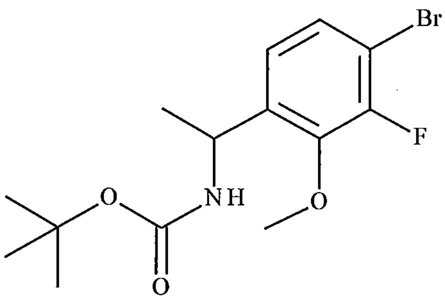

**245**

**Intermédiaire 255 :**

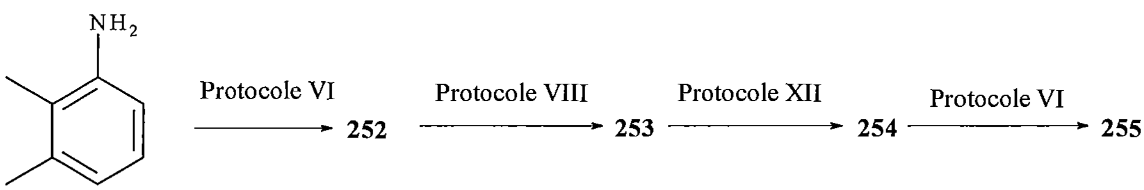


**Intermédiaire 252 :**

Obtenu à partir de la 2,3-diméthylaniline commerciale selon la procédure décrite pour l'intermédiaire **156 (protocole VI)**

**Intermédiaire 253 :**

Obtenu à partir de l'intermédiaire **252** selon le **protocole VIII**
**RMN $^1$H** (300 MHz ; DMSO-$d_6$) : δ 7,55 (d, 1H), 7,40 (d, 1H), 2,55 (s, 3H), 2,40 (s, 3H), 2,30 (s, 3H)
**IR (cm$^{-1}$):** 1684

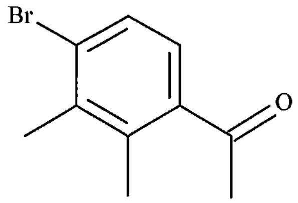

**253**

**Intermédiaire 254 :**

Obtenu à partir de l'intermédiaire **253** selon le **protocole XII**
**RMN $^1$H** (400MHz ; DMSO-$d_6$): 8,45 (s, 3H), 7,55 (d, 1H), 7,35 (d, 1H), 4,60 (quad, 1H), 2,4-2,3 (2s, 6H), 1,45 (d, 3H)
**IR (cm$^{-1}$):** 3200, 2430

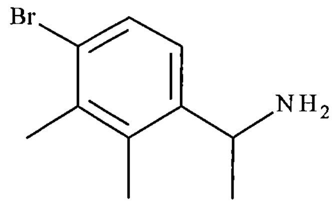

**254**

**Intermédiaire 255 :**

Obtenu par protection de l'intermédiaire **254** selon le protocole décrit pour l'intermédaire **158 (protocole VI)**
**RMN $^1$H** (300MHz, DMSO-$d_6$): δ 7,45 (dl, 1H), 7,41 (d, 1H), 7,10 (d, 1H), 4,81 (m, 1H), 2,35-2,28 (2s, 6H), 1,35 (s, 9H), 1,20 (d, 3H)

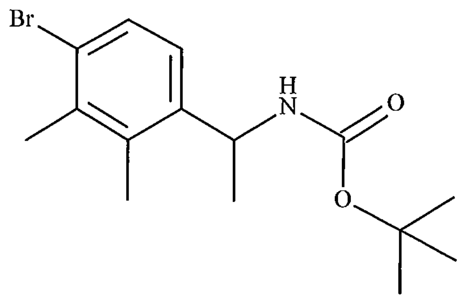

**255**

**Intermédiaire 310 :**

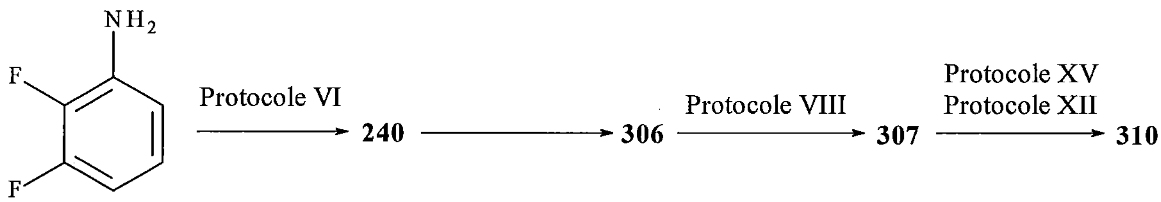

**Intermédiaire 240 :**

**4-bromo-2,3-difluorobenzonitrile**

Obtenu à partir de la 2,3-difluoroaniline commerciale selon le protocole décrit pour la préparation de l'intermédiaire **156 (protocole VI)**

**Intermédiaire 306 :**

**4-bromo-2-éthoxy-3-fluorobenzonitrile**

Obtenu par traitement de l'intermédiaire **240** (0,5 g, 2,3mmoles) dans de l'éthanol (12 mL) à 0°C en présence d'éthylate de sodium (0,31 g, 4,6 mmoles).

Le milieu réactionnel est agité 72 heures à température ambiante, puis il est versé sur de l'eau. Le précipité collecté sur fritté est séché sous vide.
**RMN $^1$H** (400MHz, DMSO-d6): δ 7,60 (s, 2H), 4.35 (q, 2H), 1.35 (t, 3H)
**IR (cm$^{-1}$):** 3085, 2237

**Intermédiaire 307 :**

Obtenu à partir de l'intermédiaire **306** selon le **protocole VIII**
**RMN $^1$H** (300 MHz ; $CDCl_3$) : δ 7,33 (d, 1H), 7,28 (dd, 1H), 4,26 (quad, 2H), 2,62 (s, 3H), 1,45 (t, 3H)
**IR (cm$^{-1}$)** : 1685

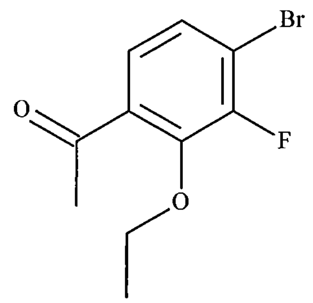

**307**

**Intermédiaire 310 :**

Obtenu à partir de l'intermédiaire **307** selon le **protocole XV**, intermédiaire **307** qui est au préalable converti en amine (non isolée) selon le **protocole XII**
**RMN $^1$H** (400MHz, DMSO-$d_6$): δ 7,45 (d, 1H), 7,40 (dd, 1H), 7,10 (d, 1H), 4,90 (m, 1H), 4,10 (m, 2H), 1,35 (m, 12H), 1,20 (d, 3H)
**IR (cm$^{-1}$):** 3480-3280, 1694

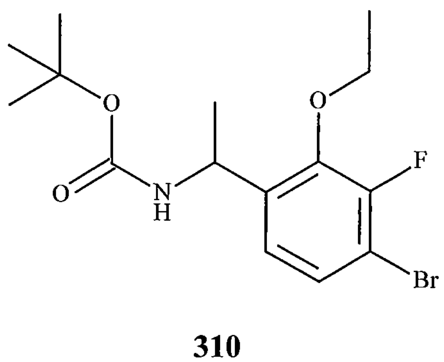

**310**

Intermédiaire 540 :

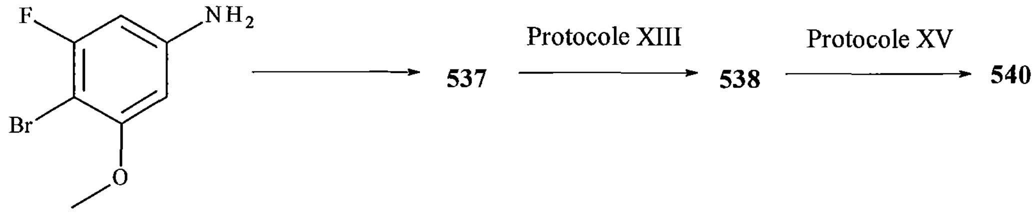


**Intermédiaire 537 :**

Obtenu à partir de la 4-bromo-3-fluoro-5-méthoxyaniline, préparée par bromation de la 3-fluoro-5-méthoxyaniline, selon le protocole décrit pour l'intermédiaire **117**
**RMN $^1$H** (300MHz, DMSO-$d_6$): δ 7,55 (dd, 1H), 7,40 (d, 1H), 4,00 (s, 3H), 2,60 (s, 3H)
**IR (cm$^{-1}$)** : 1682, 1229

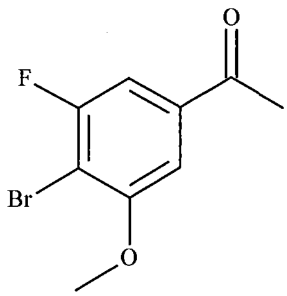

**537**

**Intermédiaire 538 :**

Obtenu par réaction de l'intermédiaire **537** et du (*R*)-(+)-2-méthyl-2-propanesulfinamide selon le **protocole XIII**
**RMN $^1$H** (400 MHz, DMSO-$d_6$): δ 7,05-7,00 (2m, 2H), 5,75 (d, 1H), 4,38 (m, 1H), 3,88 (s, 3H), 1,39 (d, 3H), 1,12 (s, 9H)
**IR (cm$^{-1}$)** : 3265, 1058

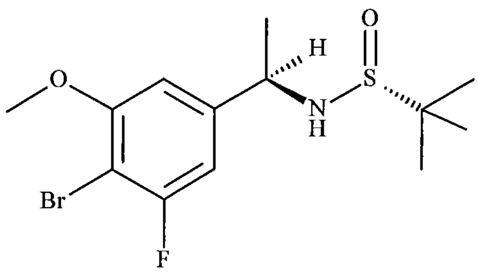

**538**

**Intermédiaire 540 :**

Obtenu à partir de l'intermédiaire **538** selon le **protocole XV**
**RMN $^1$H** (400MHz, DMSO-$d_6$): δ 7,40 (d, 1H), 6,92 (s large, 1H), 6,88 (dd, 1H), 4,6 (m, 1H), 3,87 (s, 3H), 1,35 (s large, 9H), 1,3 (d, 3H)
**Pureté optique:** > 99%

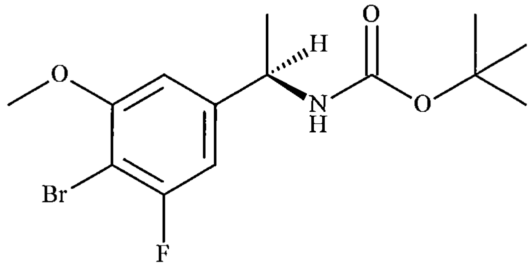

**540**

__Intermédiaire 589 :__

Obtenu par protection de la 1-(4-bromophényl)éthanamine commerciale selon le protocole décrit pour l'intermédaire **158 (protocole VI)**
**RMN $^1$H** (300MHz, DMSO-$d_6$): δ 7,50(d, 2H), 7,40 (d, 1H), 7,25 (d, 2H), 4,60 (m, 1H), 1,40 (s, 9H), 1,30 (d, 3H)
**IR (cm$^{-1}$):** 3373, 1681

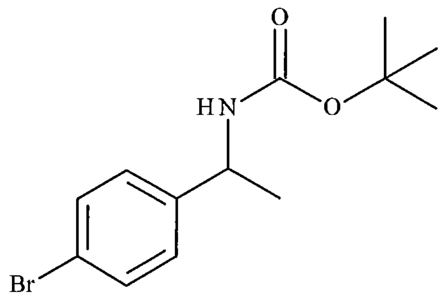

**589**

__Intermédiaire 594 :__

Obtenu par protection de la (1*S*)-1-(4-bromophényl)éthanamine commerciale selon le protocole décrit pour l'intermédaire **158 (protocole VI)**
**RMN $^1$H** (300MHz, DMSO-$d_6$): δ 7,50 (d, 2H), 7,40 (d, 1H), 7,25 (d, 2H), 4,60 (m, 1H), 1,40 (s, 9H), 1,30 (d, 3H)
**IR (cm$^{-1}$):** 3373, 1681

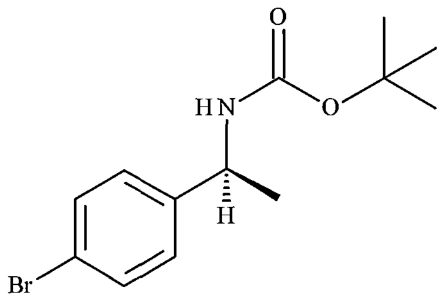

**594**

__Intermédiaire 608 :__

Obtenu à partir du (4-bromophényl)acétonitrile selon le **protocole V** en présence de 1,3-dibromopropane à la première étape
**RMN $^1$H** (300 MHz ; DMSO-$d_6$) : δ 7,65 (m, 1H) ; 7,50-7,30 (dd, 4H) ; 2,35 (m, 4H) ; 2,00-1,75 (m, 2H) ; 1,30 (m, 9H)
**IR (cm$^{-1}$)** : 3346, 1683

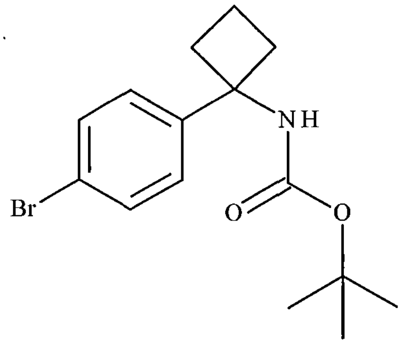

**608**

Intermédiaire 623 :

Obtenu à partir du (4-bromophényl)acétonitrile selon le **protocole V** en présence de 1-bromo-2-(2-bromoéthoxy)éthane à la première étape
**RMN $^1$H** (300 MHz ; DMSO-$d_6$) : δ 7,50 (d, 2H); 7,30 (d, 2H); 3,75-3,55 (m, 4H); 2,20 (m, 2H); 1,85 (m, 2H); 1,30 (s large, 9H)
**IR (cm$^{-1}$)** : 3255-3135, 1692

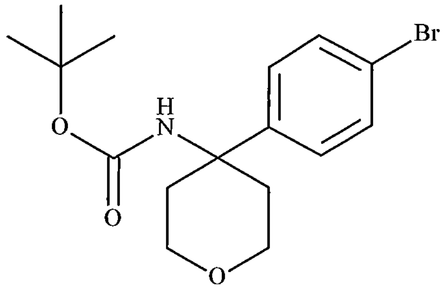

**623**

Intermédiaire 633 :

Obtenu à partir de la 5-bromoindèn-1-one commerciale selon la procédure utilisée pour préparer l'intermédiaire **87**
**RMN $^1$H** (400MHz, $CDCl_3$): δ 7,31 (d et s, 1H), 7,12 (d, 1H), 4,80 (s, 1H), 3,00 (m, 1H), 2,85 (m, 1H), 2,60 (m, 1H), 2,12 (m, 1H), 1,50 (s, 3H), 1,38 (s, 9H)
**IR (cm-1):** 3347, 1694

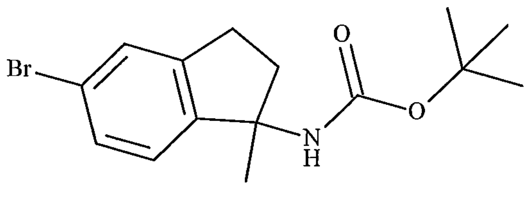

**633**

Intermédiaire 645 :

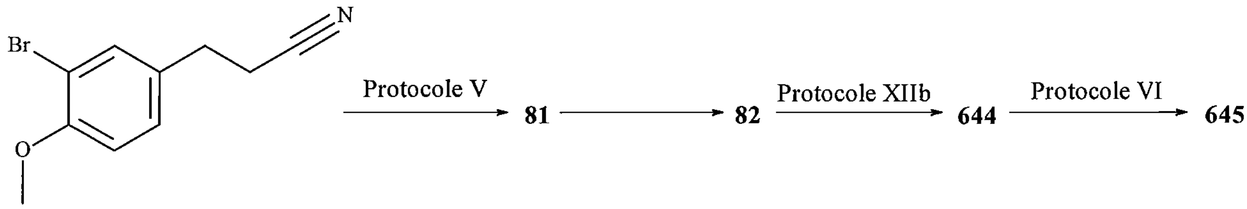


**Intermédiaire 81 :**

Obtenu à partir du 3-(3-bromo-4-méthoxyphényl)propanenitrile commercial selon le protocole décrit pour l'obtention de l'intermédiaire **599 (protocole V)**

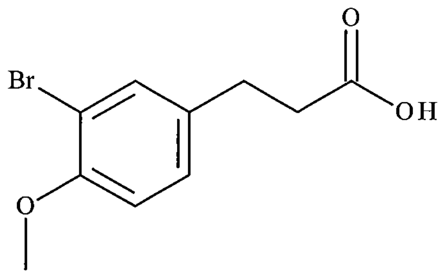

**81**

**Intermédiaire 82 :**

L'intermédiaire **81** est agité en présence de $PCl_5$ (44 g, 211 mmoles) pendant 2 heures 30 minutes, puis le mélange est concentré sous vide avec précaution. Le résidu dilué dans du chlorure de méthylène (960 mL) et refroidi à 0°C est traité par de l'$AlCl_3$ (28 g, 211 mmoles). Le mélange réactionnel est agité 2 heures à 15°C, puis il est versé avec précaution sur de la glace. Le mélange est extrait par de l'AcOEt, la phase organique est lavée successivement par de l'eau, par une solution aqueuse de NaOH 4N, à nouveau de l'eau et une solution d'HCl. La phase organique est séchée sur $MgSO_4$ et concentrée sous vide. Le résidu est concrétisé dans de l'éther isopropylique. L'intermédiaire **82** (39 g) est obtenu sous forme d'un solide.
**RMN $^1$H** (300 MHz ; $CDCl_3$): δ 7,70 (s, 1H); 7,20 (s, 1H); 3,92 (s, 3H); 3,06 (t, 2H); 2,70 (t, 2H).
**IR (cm$^{-1}$)** : 1698

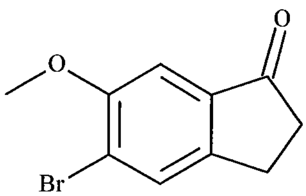

**82**

**Intermédiaire 644 :**

Obtenu à partir de l'intermédiaire **82** selon le **protocole XIIb**
**RMN $^1$H** (300MHz ; $CDCl_3$): 7,35 (s, 1H); 6,90 (s, 1H), 4,30 (t, 1H); 3,90 (s, 3H), 2,85-2,7 (2m, 2H), 2,50 (m, 1H), 1,70 (m, 1H), 1,50 (m, 2H)

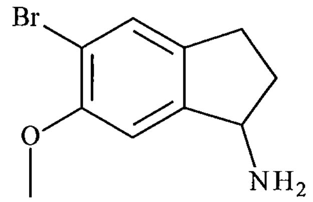

644

**Intermédiaire 645 :**

Obtenu par protection de l'intermédiaire **644** selon le protocole décrit pour l'intermédiaire **158 (protocole VI)**
**RMN $^1$H** (300MHz, DMSO-d6): δ 7,35 (s, 1H), 6,90 (s, 1H), 4,90 (m, 1H), 3,80 (s, 3H), 2,9-2,6 (2m, 2H), 2,35 (m, 1H), 1,80 (m, 1H), 1,45 (s, 9H)
**IR (cm$^{-1}$):** 3309, 1682

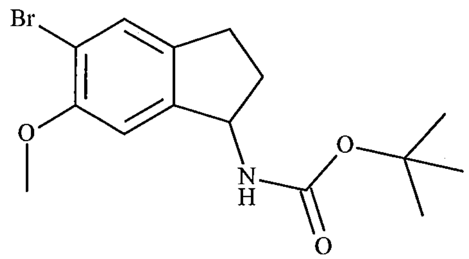

645

**Intermédiaire 684 :**

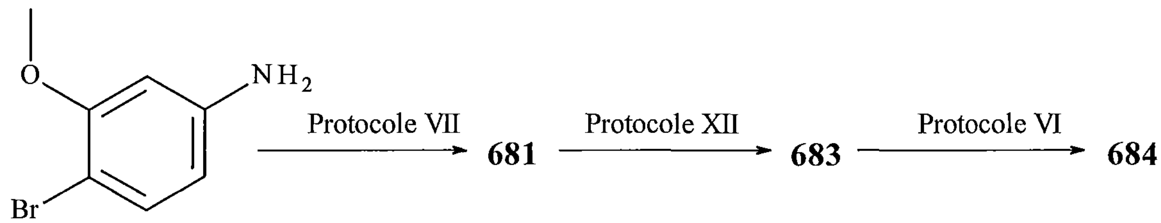


La synthèse de l'intermédiaire **681** est décrite au **protocole VII**

**Intermédiaire 683 :**

Obtenu à partir de l'intermédiaire **681** selon le **protocole XII**
**RMN $^1$H** (300MHz ; $CDCl_3$): 7,40 (d, 1H), 7,15 (d, 1H); 6,90 (dd, 1H), 4,00 (quad, 1H) ; 3,85 (s, 3H), 1,90 (m, 2H), 1,20 (d, 3H)
**IR (cm$^{-1}$):** 3750, 3000

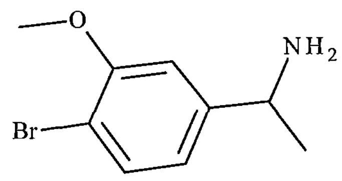

**683**

**Intermédiaire 684 :**

Obtenu par protection de l'intermédiaire **683** selon le protocole décrit pour l'intermédiaire **158 (protocole VI)**
**RMN $^1$H** (400MHz, DMSO-$d_6$): δ 7,50 (d, 1H), 7,40 (d, 1H), 7,05 (d, 1H), 6,80 (dd, 1H), 4,60 (quint, 1H), 3,80 (s, 3H), 1,38 (m, 9H), 1,30 (d, 3H)
**IR (cm$^{-1}$):** 3286, 1690

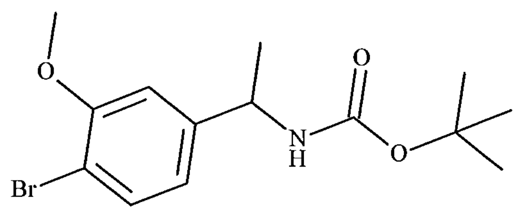

**684**

**Intermédiaire 714 :**

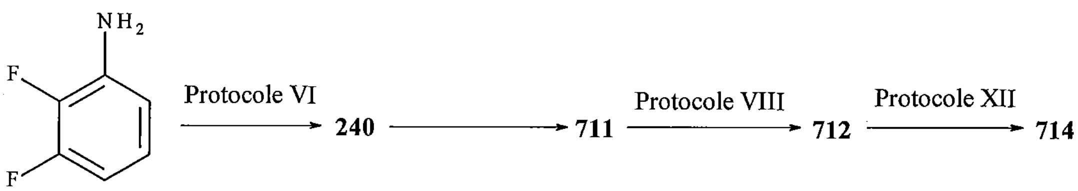


**Intermédiaire 240 :**

**4-bromo-2,3-difluorobenzonitrile**

Obtenu à partir de la 2,3-difluoroaniline commerciale selon le protocole décrit pour la préparation de l'intermédiaire **156 (protocole VI)**

**Intermédiaire 711 :**

**4-bromo-3-fluoro-2-(2-méthylpropoxy)benzonitrile**

Obtenu à partir de l'intermédiaire **240** (5 g, 23 mmoles) et d'isobutanol (2,1 mL, 23mmoles) dans du DMF (100 mL) à 0°C en présence d'hydrure de sodium à 60% dans l'huile (0,92 g).
Le milieu réactionnel est agité 72 heures à température ambiante, puis il est versé sur de l'eau et extrait par de l'éther éthylique. La phase organique est lavée à l'eau, séchée sur $MgSO_4$ puis concentrée. Le résidu est purifié sur gel de silice (éluant cyclohexane/chlorure de méthylène 70/30 à 0/100). L'intermédiaire **711** (4,1 g) est obtenu.
**RMN $^1$H** (400MHz, DMSO-d6): δ 7,60 (s, 1H), 4.10 (d, 2H), 2.05 (m, 1H), 1.00 (d, 6H)
**IR (cm$^{-1}$):** 2240
**GC-EI** (70 eV): M+. = 271

**Intermédiaire 712 :**

Obtenu à partir de l'intermédiaire **711** selon le **protocole VIII**
**RMN $^1$H** (400 MHz ; DMSO-$d_6$) : δ 7,50 (m, 1H), 7,35 (dd, 1H), 3,90 (d, 2H), 2,55 (s, 3H), 2,05 (m, 1H), 1,00 (d, 6H)
**IR (cm$^{-1}$)** : 1686

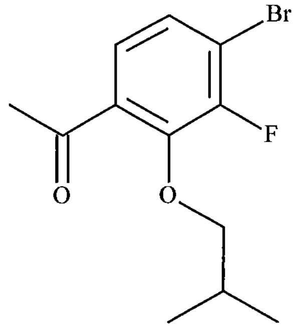

**712**

**Intermédiaire 714 :**

Obtenu à partir de l'intermédiaire **712** selon le **protocole XII**
**RMN $^1$H** (400MHz ; DMSO-$d_6$): 8,70 (s large, 3H), 7,55 (m, 1H), 7,45 (d, 1H), 4,60 (quad, 1H), 4,0-3,8 (2dd, 2H), 2,05 (m, 1H), 1,50 (d, 3H), 1,00 (d, 6H)
**RMN $^{19}$F:** -123 (1F)
**IR (cm$^{-1}$):** 3154, 2000

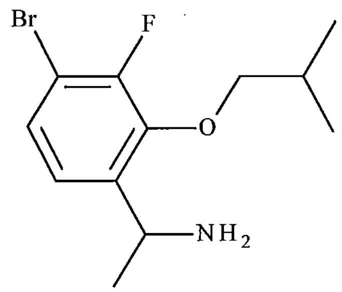

**714**

**Intermédiaire 726 :**

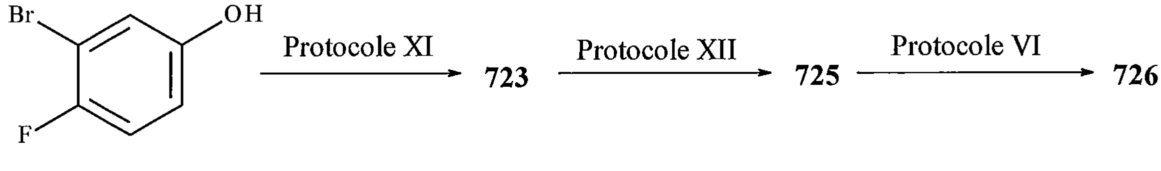


**Intermédiaire 723 :**

Obtenu à partir du 3-bromo-4-fluorophénol commercial selon le **protocole XI RMN $^1$H** (400MHz, DMSO-d6): δ 7,68 (d, 1H), 7,54 (d, 1H), 7,51 (d, 2H), 7,44 (t, 2H), 7,38 (t, 1H), 5,28 (s, 2H), 2,51 (s, 3H)
**RMN$^{19}$F:** -117,49 (1F)
**IR (cm$^{-1}$)** : 1662

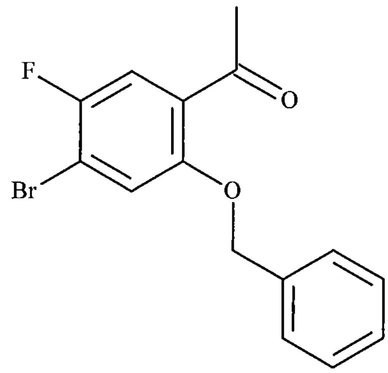

723

**Intermédiaire 725 :**

Obtenu à partir de l'intermédiaire **723** selon le **protocole XII**
**RMN $^1$H** (300/400MHz: DMSO-$d_6$): 7,46 (d, 1H), 7,5-7,35 (m, 5H), 7,31 (d, 1H), 5,15 (2d, 2H), 4,26 (quad, 1H), 1,85 (slarge, 2H), 1,19 (d, 3H)
**RMN $^{19}$F:** -117,8 (dd, 1F)
**IR (cm$^{-1}$):** 3154,2000.

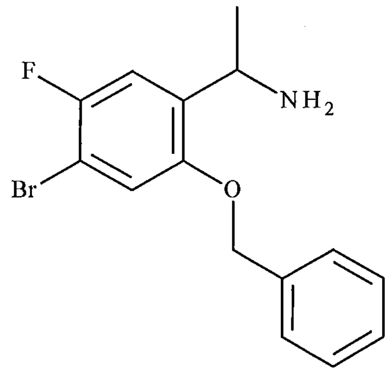

725

**Intermédiaire 726 :**

Obtenu par protection de l'intermédiaire **725** selon le protocole décrit pour l'intermédaire **158** (protocole VI)
**RMN $^1$H** (300MHz, DMSO-$d_6$): δ 7,40 (m et d, 6H), 7,25 (d, 1H), 5,20 (s, 2H), 4,95 (m, 1H), 1,35 (s large, 9H), 1,25 (d, 3H)
**IR (cm$^{-1}$):** 3303, 1675

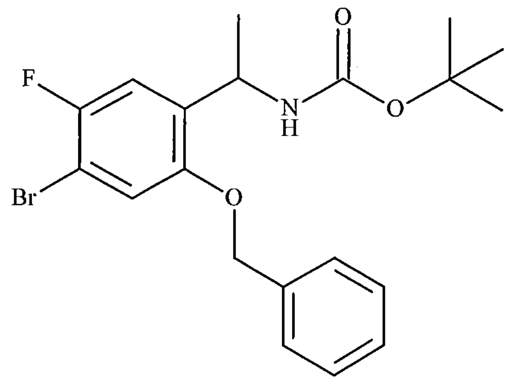

**726**

<u>**Intermédiaire 331b :**</u>

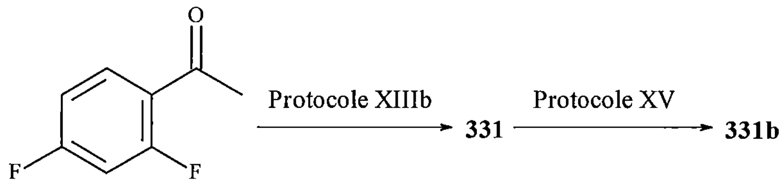


La synthèse de l'intermédiaire 331 est décrite au **protocole XIIIb.**

**Intermédiaire 331b :**

Obtenu à partir de l'intermédiaire **331** selon le **protocole XV**
**RMN $^1$H** (400MHz, DMSO-$d_6$): δ 7,46 (d large, 1H), 7,40 (m, 1H), 7,14 (m, 1H), 7,06 (m, 1H), 4,83 (m, 1H), 1,34 (s large, 9H), 1,27 (d, 3H)
**IR (cm$^{-1}$):** 3373, 1678
**Excès énantiomérique** > 99%

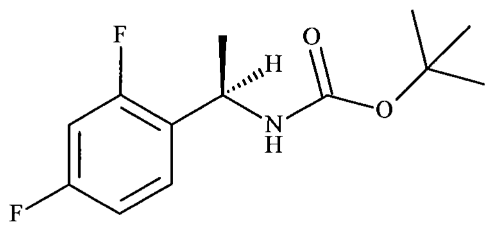

**331b**

<u>**Intermédiaire 234 :**</u>

Obtenu par réaction de la 4,6-difluoro-2,3-dihydro-1*H*-indén-1-one commerciale et du (+/)-2-méthyl-2-propanesulfinamide selon le **protocole XIII**
**RMN $^1$H** (400/500MHz, DMSO-$d_6$): δ 7,28 (d, 1H), 7,04 (t, 1H), 5,90 (d, 1H), 4,78 (quad, 1H), 2,9-2,7 (2m, 2H), 2,45-1,98 (2m, 2H), 1,16 (s, 9H)
**IR (cm$^{-1}$) :** 3246

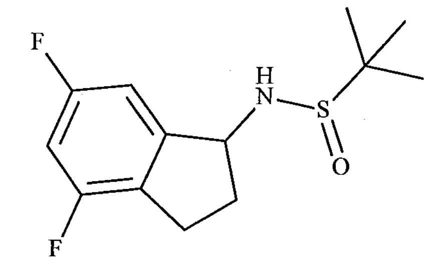

**234**

__Intermédiaire 260 :__

Obtenu par réaction de la 5,7-difluoro-2,3-dihydro-1*H*-indén-1-one commerciale et du (+/)-2-méthyl-2-propanesulfinamide selon le **protocole XIII**
**RMN $^1$H** (400MHz, DMSO-$d_6$): δ 6,98 (m, 2H), 5,64 (d, 1H), 4,89 (m, 1H), 3,13 (m, 1H), 2,8 (m, 1H), 2,34 (m, 1H), 2,13 (m, 1H), 1,07 (s, 9H)
**IR (cm$^{-1}$) :** 3209, 1048

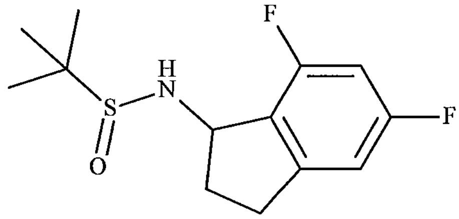

**260**

__Intermédiaires 343 et 346 :__

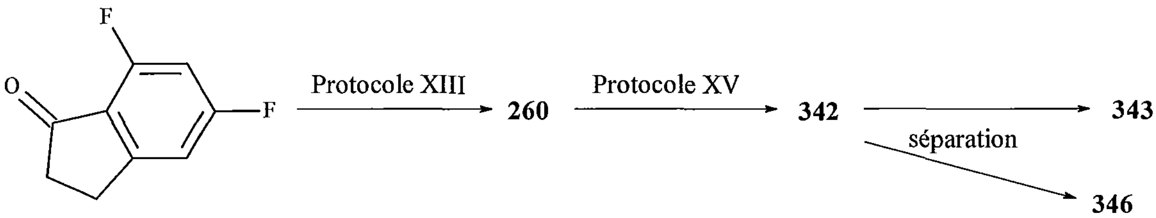


**Intermédiaire 342 :**

Obtenu à partir de l'intermédiaire **260** selon le **protocole XV**
**RMN $^1$H** (400MHz, DMSO-$d_6$): δ 7,26 (d, 1H), 6,95 (m, 2H), 5,16 (quad, 1H), 3,00 (m, 1H), 2,77 (m, 1H), 2,38 (m, 1H), 1,87 (m, 1H), 1,41 (s, 9H)
**IR (cm$^{-1}$):** 3241, 1708-1680

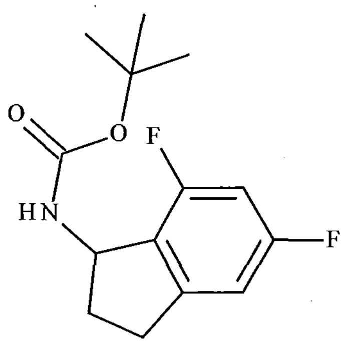

**342**

L'intermédiaire **342** (7,9 g) a été purifié par chromatographie haute pression sur support chiral (Colonne ChiralPak IC, éluant éthanol/n-heptane 10/90, détection: 260 nm) pour donner les énantiomères **343** (3,7 g) et **346** (3,7 g).

**Intermédiaire 343 :**

**pureté optique** (Colonne ChiralPak IC3: 3μm, 4,6x250mm, éluant éthanol/n-heptane 10/90, détection: 210 nm): >99%, intermédiaire **346**< à 1%
**IR ($cm^{-1}$):** 3355, 1680
$\alpha_D$ (589nM) = + 60,7 (c = 0,013 g/mL, EtOH) à 20°C

**Intermédiaire 346 :**

**Pureté optique** (Colonne ChiralPak IC3: 3μm, 4,6x250mm, éluant éthanol/n-heptane 10/90, détection: 210 nm): >99%, intermédiaire **343**< à 1%
**IR ($cm^{-1}$):** 3354, 1678
$\alpha_D$ (589nM) = - 60,7 (c = 0,013 g/mL, EtOH) à 20°C

**Intermédiaire 269 :**

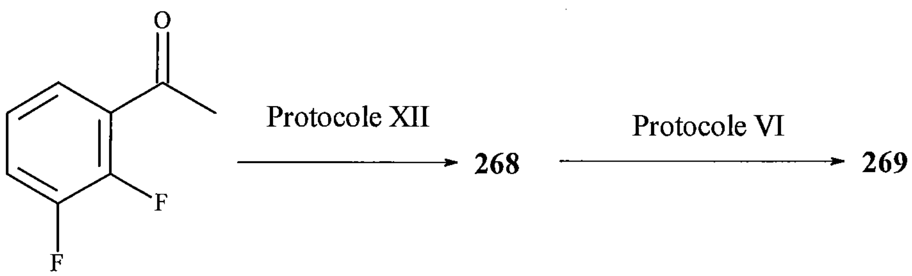


**Intermédiaire 268 :**

Obtenu à partir de la 1-(2,3-difluorophényl)éthanone commerciale selon le **protocole XII**
**RMN $^1$H** (400MHz ; DMSO-$d_6$): 7,40 (ddd, 1H), 7,25 (m, 1H), 7,20 (m, 1H), 4,30 (quad, 1H), 1,95 (m, 2H), 1,25 (d, 3H)
**IR ($cm^{-1}$):** 3750, 3000.

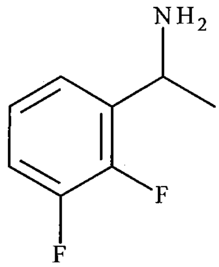

**268**

**Intermédiaire 269 :**

Obtenu par protection de l'intermédiaire **268** selon le protocole décrit pour l'intermédaire **158 (protocole VI)** **RMN $^1$H** (400MHz, DMSO-$d_6$): δ 7,53 (d, 1H), 7,20 (m, 3H), 4,90 (m, 1H), 1,35 (m, 9H), 1,30 (d, 3H), **IR ($cm^{-1}$):** 3377, 1681 **RMN 19F :** -140, -146
GC-EI (70 eV): 257,1

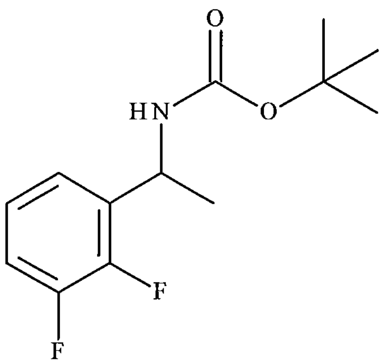

**269**

**Intermédiaire 288 :**

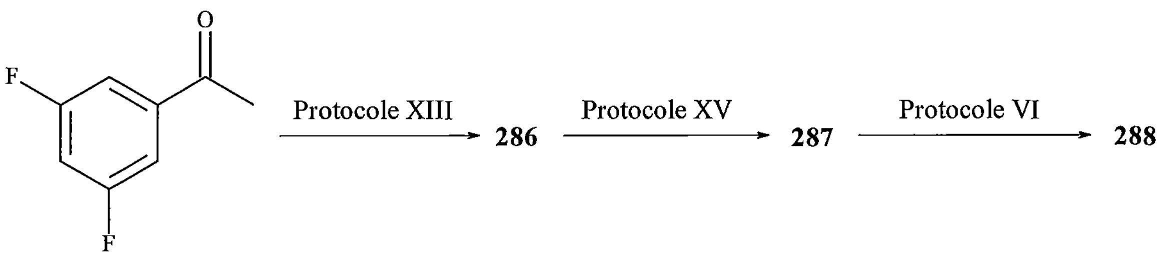


Les préparations respectives des intermédiaires **286** et **287** sont décrites dans les **protocoles XIII** et **XV.**

**Intermédiaire 288 :**

Obtenu par protection de l'intermédiaire **287** selon le protocole décrit pour l'intermédaire **158 (protocole VI)** **RMN $^1$H** (300MHz, DMSO-$d_6$): δ 7,42 (d, 1H), 7,03 (m, 1H), 7,00 (m, 2H), 4,61 (m, 1H), 1,37 (s, 9H), 1,30 (d, 3H) **IR ($cm^{-1}$):** 3369, 1682
$\alpha_D$ (589nM) = 58,22 (c = 0,0087 g/mL, méthanol) à 20°C

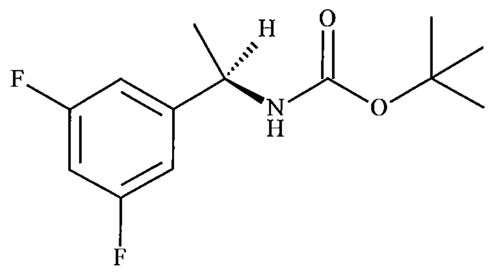

288

Intermédiaire 317 :

Obtenu par réaction de la 4,6-difluoro-2,3-dihydro-1*H*-indén-1-one commerciale et du (*R*)-(+)-2-méthyl-2-propanesulfinamide selon le **protocole XIIIb**
**RMN $^1$H** (400MHz, DMSO-$d_6$): δ 7,29 (d, 1H), 7,05 (t, 1H), 5,91 (d, 1H), 4,79 (m, 1H), 2,9-2,7 (m, 2H), 2,45-1,99 (m, 2H), 1,15 (s, 9H)
**IR (cm$^{-1}$) :** 3207

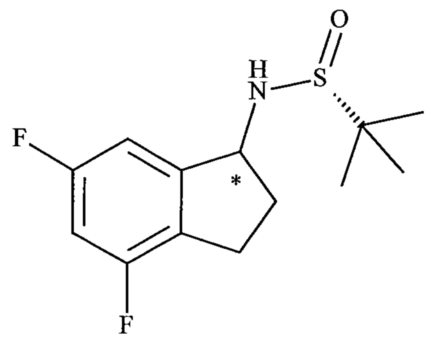

317

Intermédiaire 324 :

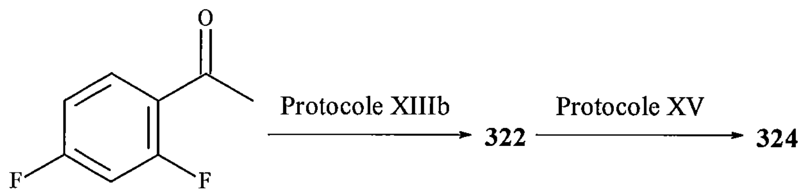


Intermédiaire 322 :

Obtenu par réaction de la 3,5-difluoroacetophénone commerciale et du (*S*)-(-)-2-méthyl-2-propanesulimamide selon le **protocole XIIIb**
**RMN $^1$H** (300 MHz, DMSO-$d_6$): δ 7,14 (d, 2H), 7,05 (tt, 1H), 4,40 (m, 1H), 3,80 (d, 1H), 1,38 (d, 3H), 1,13 (s, 9H)
**IR (cm$^{-1}$) :** 3125, 1624, 1598, 1117, 853, 699

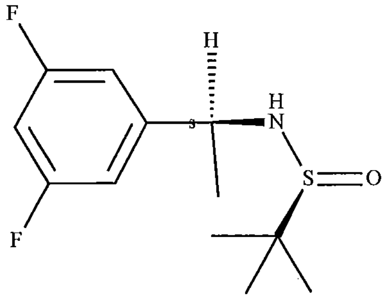

**322**

**Intermédiaire 324 :**

Obtenu à partir de l'intermédiaire **322** selon le **protocole XV**
**RMN $^1$H** (400MHz, DMSO-$d_6$): δ 7,40 (d, 1H), 7,05 (m, 1H), 7,00 (m, 2H), 4,65 (m, 1H), 1,35 (s large, 9H), 1,30 (d, 3H)
**IR (cm$^{-1}$):** 3364, 1683
**Excès énantiomérique** > 99%

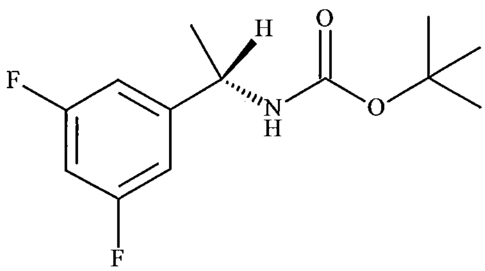

**324**

**Intermédiaire 334 :**

Obtenu par réaction de la 1-(2,4-difluorophényl)éthanone commerciale et du (R)-(+)-2-méthyl-2-propane sulfinamide puis réduction au L-sélectride (1M dans le THF) selon le **protocole XIII**
**RMN $^1$H** (300MHz, DMSO-$d_6$): δ 7,6 (m, 1H), 7,20 (m, 1H), 7,10 (m, 1H), 5,45 (d, 1H), 4,65 (quint, 1H), 1,5 (d, 3H), 1,10 (s, 9H)
**IR (cm$^{-1}$) :** 3214
**RMN $^{19}$F**: -111, -114 (2m)

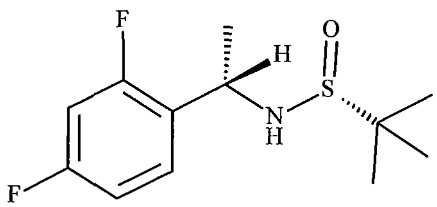

**334**

**Intermédiaire 337 :**

Obtenu par réaction de la 4,6-difluoro-2,3-dihydro-1*H*-indén-1-one commerciale et du (*S*)-(-)-2-méthyl-2-propanesulfinamide selon le **protocole XIIIb**
**RMN $^1$H** (400MHz, DMSO-$d_6$): δ 7,3 (dd, 1H), 7,05 (td, 1H), 5,95 (d, 1H), 4,80 (quad, 1H), 2,9-2,7 (m, 2H), 2,45-2.0 (m, 2H), 1,15 (s, 9H)
**IR (cm$^{-1}$) :** 3207

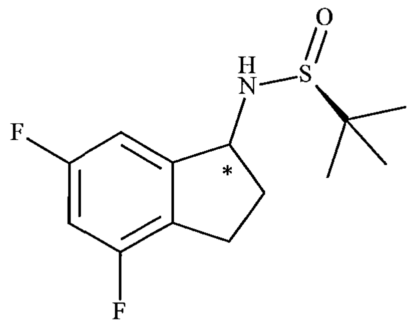

**337**

**Intermédiaire 478 :**

Obtenu à partir du *N*-[(2,4-difluorophényl)méthylidène]-2-méthylpropane-2-sulfinamide (précurseur de l'intermédiaire **334** avant réduction) et de EtMgCl selon le **protocole XIV**
**RMN $^1$H** (300MHz, DMSO-$d_6$): δ 7,49 (m, 1H), 7,09 (m,1H), 5,44 (d, 1H), 4,39 (m, 1H), 4,37 (m, 1H), 1,9 (m, 1H), 1,71 (m, 1H), 1,07 (s, 9H), 0,81 (s, 3H)
**RMN $^{19}$F**: -112,2, -115,4
**IR (cm$^{-1}$)**: 3205, 1049

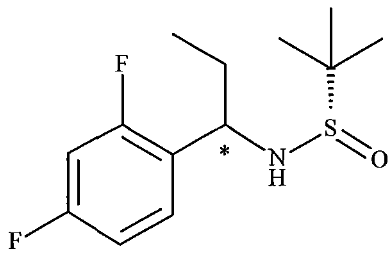

**478**

**Intermédiaire 367 :**

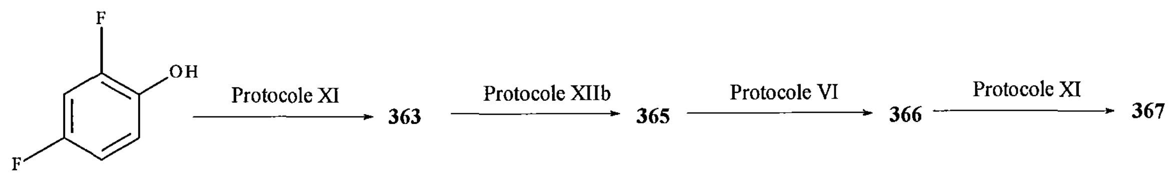


La préparation de l'intermédiaire **363** est décrite au **protocole XI.**

**Intermédiaire 365 :**

Obtenu à partir de l'intermédiaire **363** selon le **protocole XIIIb**
**RMN $^1$H** (400MHz ; DMSO-$d_6$): 7,00 (m, 1H), 6,80 (m, 1H), 7,00-5,0 (m, 3H), 4,20 (quad, 1H), 1,30 (d, 3H)
**IR (cm$^{-1}$):** 3300-2000

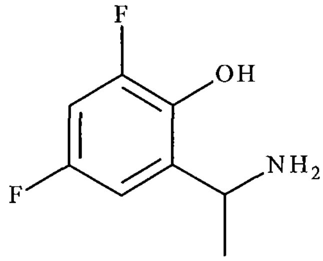

**365**

**Intermédiaire 366 :**

Obtenu par protection de l'intermédiaire **365** selon le protocole décrit pour l'intermédaire **158 (protocole VI)**
**RMN $^1$H** (400MHz, DMSO-$d_6$): δ 9,40 (m, 1H), 7,35 (d 1, 1H), 7,05 (m, 1H), 6,90 (dd, 1H), 4,95 (quint, 1H), 2,39 (s, 3H), 1,35 (d, 9H), 1,20 (d, 3H)
**IR (cm$^{-1}$):** 3500, 2600, 1690, 1672.

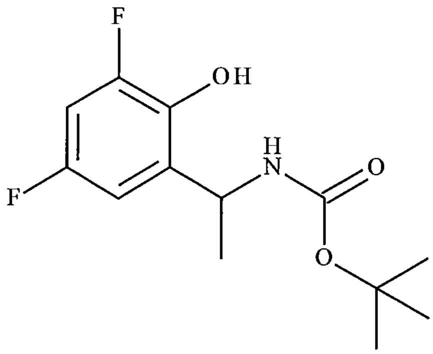

**366**

**Intermédiaire 367 :**

Obtenu à partir de l'intermédiaire **366** selon le protocole décrit pour l'intermédiaire **384 (Protocole XI)**
**RMN $^1$H** (400MHz, DMSO-$d_6$): δ 7,43 (d, 1H), 7,15 (m, 1H), 7,00 (m, 1H), 4,93 (m, 1H), 3,82 (s, 3H), 1,36 (s, 9H), 1,21 (d, 3H)
**IR (cm$^{-1}$):** 3368, 1681

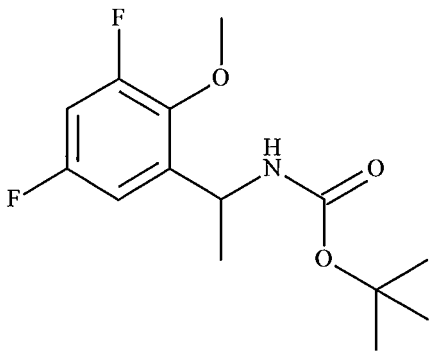

**367**

Intermédiaire 430 :

Obtenu à partir de l'intermédiaire **366** selon le protocole décrit pour l'intermédiaire **562 (Protocole XXI)**
**RMN $^1$H** (400MHz, DMSO-$d_6$): δ 7,50 (d, 1H), 7,05 (dd, 1H), 7,00 (td, 1H), 4,85 (quint, 1H), 2,70 (m, 1H), 2,60 (m, 1H), 1,35 (m, 9H), 1,30 (d, 3H), 1,15 (t, 3H)
**IR (cm$^{-1}$):** 3380, 1671

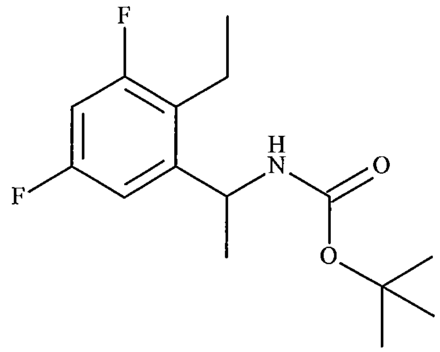

**430**

Intermédiaire 378:

Obtenu à partir du 3,5-difluorobenzonitrile commercial selon le **protocole VI**
**RMN $^1$H** (400 MHz ; DMSO-$d_6$) : δ 7,28 (m, 1H) ; 7,00 (m, 1H) ; 6,98 (m, 2H) ; 1,48 (s, 6H) ; 1,30 (m, 9H)
**IR (cm$^{-1}$):** 3314 ; 1685 ; 1523

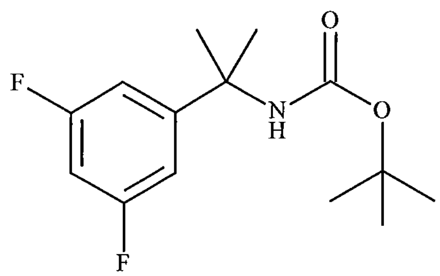

**378**

Intermédiaire 387:

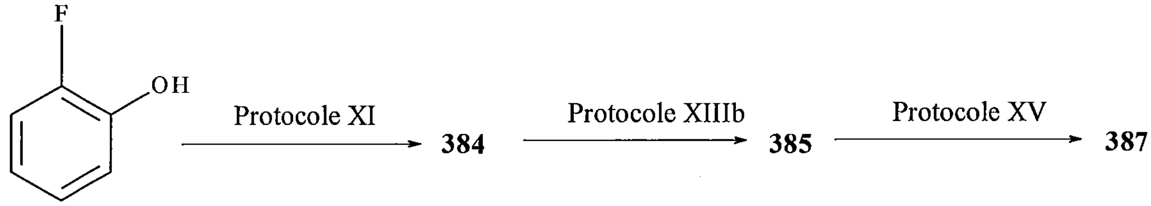


**Intermédiaire 384 :**

Obtenu à partir du 2-fluorophénol commercial selon le **protocole XI**, en présence d'iodure de méthyle à la dernière étape
**RMN $^1$H** (400MHz, DMSO-$d_6$): δ 7,50 (dd, 1H), 7,40 (dd, 1H), 7,20 (m, 1H), 3,90 (s, 3H), 2,55 (s, 3H)
**IR (cm$^{-1}$) :** 1685

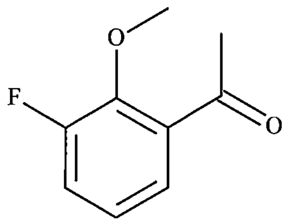

**384**

**Intermédiaire 385 :**

Obtenu par réaction de l'intermédiaire **384** et du (*R*)-(+)-2-méthyl-2-propanesulfinamide selon le **protocole XIIIb**
**RMN $^1$H** (400 MHz, DMSO-$d_6$): δ 7,30 (d, 1H), 7,12 (m, 2H), 5,63 (d, 1H), 4,72 (m, 1H),3,89 (d, 3H), 1,36 (d, 3H), 1,11 (s, 9H)
**RMN $^{19}$F**: -130,0
**IR (cm$^{-1}$) :** 3500, 3000, 1056

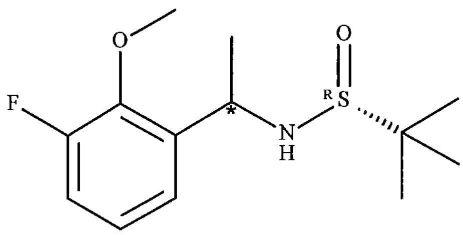

**385**

**Intermédiaire 387 :**

Obtenu à partir de l'intermédiaire **385** selon le **protocole XV**
**RMN $^1$H** (400MHz, DMSO-$d_6$): δ 7,42 (d large, 1H), 7,16 (d large, 1H), 7,10 (m, 2H), 4,95 (m, 1H), 3,87 (s, 3H), 1,36 (s, 9H), 1,25 (d, 3H)
**IR (cm$^{-1}$):** 3346, 1695
**Excès énantiomérique** > 99%

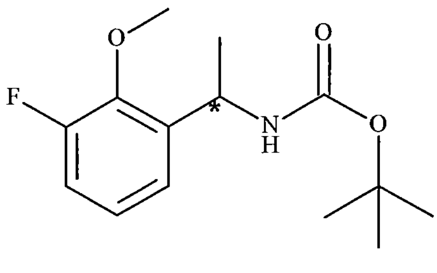

**387**

<u>**Intermédiaire 739 :**</u>

**Intermédiaire 737 :**

Obtenu par réaction de l'intermédiaire **384** et du (*S*)-(-)-2-méthyl-2-propanesulfinamide selon le **protocole XIIIb**
**RMN $^1$H** (400 MHz, DMSO-$d_6$): δ 7,30 (d, 1H), 7,12 (m, 2H), 5,63 (d, 1H), 4,72 (m, 1H), 3,89 (d, 3H), 1,36 (d, 3H), 1,11 (s, 9H)
**RMN $^{19}$F**: -130,02
**IR (cm$^{-1}$):** 3219, 1050.

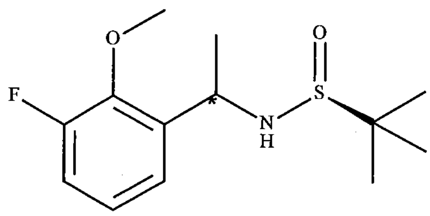

**737**

**Intermédiaire 739 :**

Obtenu à partir de l'intermédiaire **737** selon le **protocole XV**
**RMN $^1$H** (300MHz, DMSO-$d_6$): δ 7,2-7,0 (m, 3H), 6,95 (m, 1H), 4,98 (quint, 1H), 3,90 (s, 3H), 1,35 (s, 9H), 1,30 (d, 3H)
**IR (cm$^{-1}$):** 3353, 1697

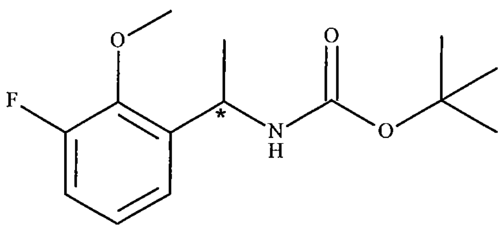

**739**

**Intermédiaire 401:**

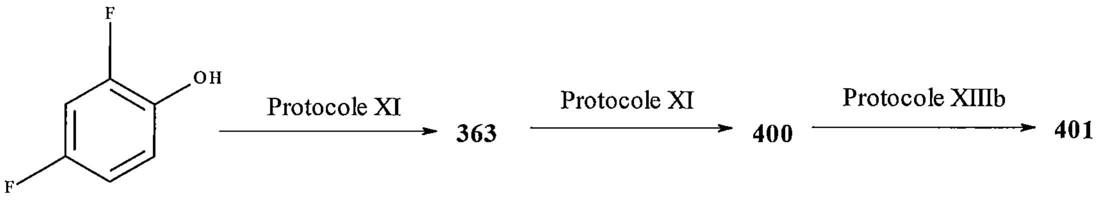


La préparation de l'intermédiaire **363** est décrite au **protocole XI.**

**Intermédiaire 400 :**

Obtenu à partir de l'intermédiaire **363** selon le **protocole XI**, en présence d'iodure de méthyle à la dernière étape
**RMN $^1$H** (400MHz, $CDCl_3$): δ 7,20 (ddd, 1H), 7,00 (ddd, 1H), 4,00 (s, 3H), 2,65 (s, 3H)
**IR (cm$^{-1}$) :** 1674

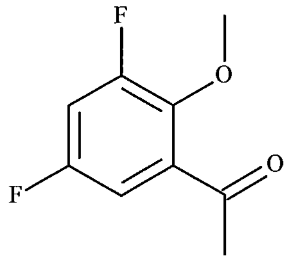

**400**

**Intermédiaire 401 :**

Obtenu par réaction de l'intermédiaire **400** et du (*R*)-(+)-2-méthyl-2-propanesulfinamide selon le **protocole XIIIb**
**RMN $^1$H** (400MHz, DMSO-$d_6$): δ 7,2 (m, 2H), 5,70 (s, 1H), 4,70 (quint, 1H), 3,85 (d, 3H), 1,35 (d, 3H), 1,10 (s, 9H)
**IR (cm$^{-1}$):** 3150
Pureté diastéréoisomérique : de>99%

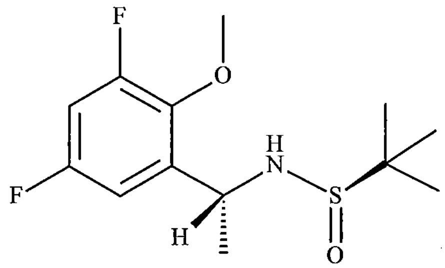

**401**

**Intermédiaire 404:**

Obtenu par réaction de l'intermédiaire **400** et du (*S*)-(-)-2-méthyl-2-propanesulfinamide puis réduction au L-sélectride (1M dans le THF) selon le **protocole XIII**
**RMN $^1$H** (400MHz, DMSO-$d_6$): δ 7,2 (m, 1H), 7,1 (ddd, 1H), 5,40 (d, 1H), 4,75 (quint, 1H), 3,85 (s, 3H), 1,4 (d, 3H), 1,10 (s, 9H)
**IR (cm$^{-1}$):** 3260

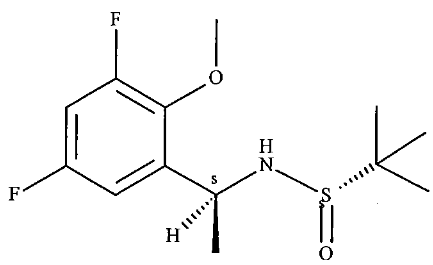

**404**

**Intermédiaires 423 et 415:**

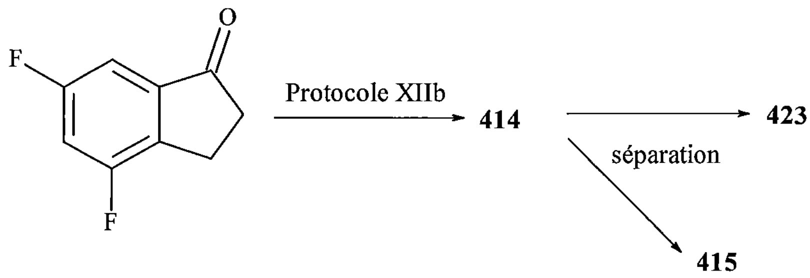


**Intermédiaire 414 :**

Obtenu à partir de la 4,6-difluoro-2,3-dihydro-1*H*-indén-1-one commerciale selon le **protocole XIIIb**
**RMN $^1$H** (400MHz ; DMSO-$d_6$): 8,85 (m, 3H), 7,50 (dd, 1H), 7,20 (td, 1H), 4,75 (t, 1H), 3,05 (m, 1H), 2,85 (m, 1H), 2,55 (m, 1H), 2,10 (m, 1H)
**IR ($cm^{-1}$):** 3450-2440

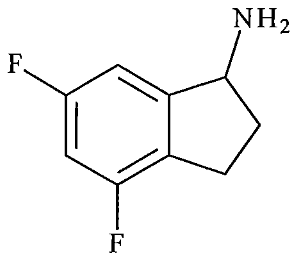

**414**

L'intermédiaire **414** (11 g) a été purifié par chromatographie haute pression sur support chiral (Colonne ChiralPak T304, éluant Acétonitrile : 100 : détection 260nm) pour donner les énantiomères **415** (5,2 g) et **423** (5,5 g).

**Intermédiaire 415 :**

**RMN $^1$H** (400MHz, DMSO-$d_6$): 7,00 (td, 1H), 6,80 (dd, 1H), 5,00 (m, 1H), 2,90 (m, 1H), 2,70 (m, 1H), 2,40 (m, 1H), 1,90 (m, 1H), 1,40 (s, 9H)
**Pureté optique** (SFC: Colonne Kromasil-3-amy coat 3µM 4,6x250 mm; $CO_2$ / (éthanol/diéthylamine:100/0,5): 80/20; Détection: 260nm):> 99%, intermédiaire **423<** à 1%

**Intermédiaire 423:**

**Pureté optique** (SFC: Colonne Kromasil-3-amy coat 3µM 4,6x250 mm; $CO_2$ / (éthanol/diéthylamine:100/0,5) : 80 / 20; Détection: 260nm) : > 99%, intermédiaire **415**< à 1%.

**Intermédiaire 444 :**

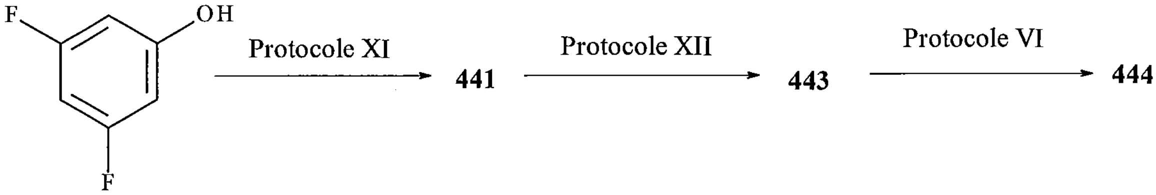

**Intermédiaire 441 :**

Obtenu à partir du 3,5-difluorophénol commercial selon le **protocole XI**
**RMN $^1$H** (400MHz, DMSO-d6): δ 7,50-7,30 (m, 6H), 7,05 (d, 1H), 6,95 (t, 1H), 5,20 (s, 2H), 2,45 (s, 3H)
**IR** ($cm^{-1}$) : 1699

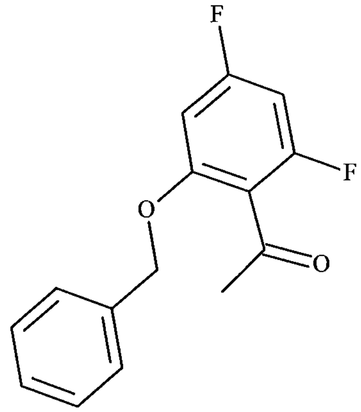

**441**

**Intermédiaire 443 :**

Obtenu à partir de l'intermédiaire **441** selon le **protocole XII**
**RMN $^1$H** (400MHz ; DMSO-$d_6$): 8,30 (m, 3H), 7,50 (d, 2H), 7,40 (t, td, 3H), 7,00 (dd, 1H), 6,95 (td, 1H), 5,30 (s, 2H), 4,65 (quad, 1H), 1,50 (d, 3H)
**IR ($cm^{-1}$):** 3500-2450

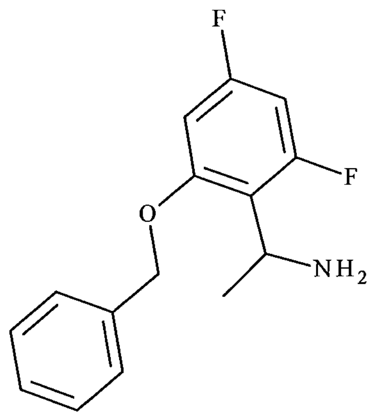

**443**

**Intermédiaire 444 :**

Obtenu par protection de l'intermédiaire **443** selon le protocole décrit pour l'intermédaire **158 (protocole VI)**
**RMN $^1$H** (400MHz, DMSO-$d_6$): δ 7,50 (d, 2H), 7,40 (t, 2H), 7,35 (td, 1H), 6,85 (dd, 1H), 6,80 (d, 1H), 6,75 (td, 1H), 5,20 (s, 2H), 5,10 (quint, 1H), 1,45-1,15 (m, 12H)
**IR ($cm^{-1}$):** 3475, 1709

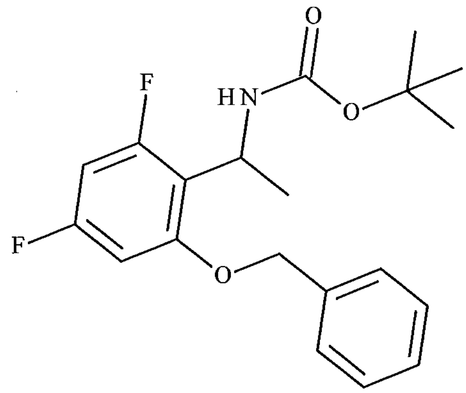

**444**

__Intermédiaire 558 :__

Obtenu à partir de l'intemédiaire **444** selon le mode opératoire suivant :

6,3g de l'intermédiaire **444** sont engagés dans une réaction de débenzylation en présence de 10% en masse de Pd/C 10% dans l'acétate d'éthyle pour obtenir 4,5g de l'intermédiaire phénolique **556.** Les 4,5g de l'intermédiaire **556** ont conduit à l'intermédiaire **557** (5.2g de triflate) (Chromatographie flash sur $SiO_2$, gradient Cyclohexane/chlorure de méthylène 10/90 à 100% de chlorure de méthylène). L'intermédiaire **557** (4,3g) a été transformé en intermédiaire **558** selon le mode opératoire suivant :

Un mélange de **557** (1g, 2.47 mmoles), de triméthyl-boroxine (0,62 g, 5 mmoles), de $K_2CO_3$ (1.36 g, 9.8 mmoles) dans le 1,4-dioxane (10 mL) dégazé par $N_2$ pendant 15minutes est traité avec du $Pd(PPh_3)_4$ (0,57 g, 0,5 mmoles). Le milieu est chauffé au reflux 1 heure. Après retour à température ambiante le solide est filtré, le filtrat est concentré sous vide. L'intermédiaire **558** (0.55g) est obtenu après purification sur silice. (obtention de 2,4g de méthyle après chromatographie flash sur $SiO_2$, gradient chlorure de méthylène 100% à chlorure de méthylène/AcOEt 90/10 ).
**RMN $^1$H** (400MHz, DMSO-$d_6$): δ 7,24-6,95 (d large et m, 1H), 6,95 (ddd, 1H), 6,86 (d large, 1H), 4,85 (quint, 1H), 2,39 (s, 3H), 1,35 (d, 3H), 1,33-1,19 (2s large, 9H)
**IR (cm$^{-1}$):** 3468, 1705

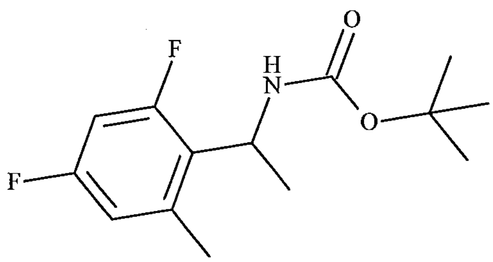

**558**

__Intermédiaire 465 :__

Obtenu à partir du 2,4-difluorobenzonitrile commercial selon le **protocole VI**
**RMN $^1$H** (300 MHz; DMSO-$d_6$): δ 7,30 (m, 1H); 7,20 (m, 1H); 7,10 (m, 1H); 7,00 (m, 1H); 1,50 (s, 6H); 1,30 (m, 9H)
**IR (cm$^{-1}$) :** 3410; 1697; 1613; 1160; 848-700.

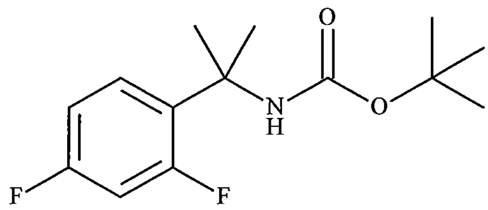

**465**

<u>**Intermédiaire 484 :**</u>

***N*-[(2,4-difluorophényl)méthylidène]-(2*S*)-2-méthylpropane-2-sulfinamide**

Obtenu par réaction du 2,4-difluorobenzaldéhyde avec le (*S*)-(-)-2-méthylpropane-2-sulfinamide selon le **protocole XIV**

**Intermédiaire 482 :**

A une solution de *N*-[(2,4-difluorophényl)méthylidène]-(2*S*)-2-méthylpropane-2-sulfinamide (5,1 g, 20 mmoles) dans le THF (30 mL), refroidie à -60°C est ajouté une solution de 2-(1,3-dioxolan-2-yl)éthyl-MgBr dans le chlorure de méthylène (32 mL) préparée par réaction de Magnesium (0,42 g) et de 2-(2-bromoéthyl)-1,3-dioxolane. Le mélange réactionnel est agité à -60°C pendant 20 minutes, puis il est hydrolysé à -40°C par une solution aqueuse saturée de $NH_4Cl$. Le milieu est décanté en présence d'éther éthylique, la phase organique est lavée par une solution saturée en NaCl, séchée sur $MgSO_4$ puis concentrée. Une chromatographie sur silice (éluant $CH_2Cl_2$/THF 95/5) conduit à l'obtention de 1,7 g de l'intermédiaire **482** sous forme d'une huile.
**RMN $^1$H** (300MHz, DMSO-$d_6$): δ 7,58 (m, 1H), 7,15 (m, 1H), 7,09 (m, 1H), 5,71 (d,NH), 4,78 (t, 1H), 4,45 (m, 1H), 3,85-3,73 (2m, 4H), 1,95-1,4 (m, 4H), 1,09 (s, 9H)
**IR (cm$^{-1}$):** 3230, 1048
**Pureté optique** (SFC: Colonne AD 5μM 4,6x250 mm; $CO_2$ / MeOH : 90 / 10; Détection: 260nm) : > 98,6%

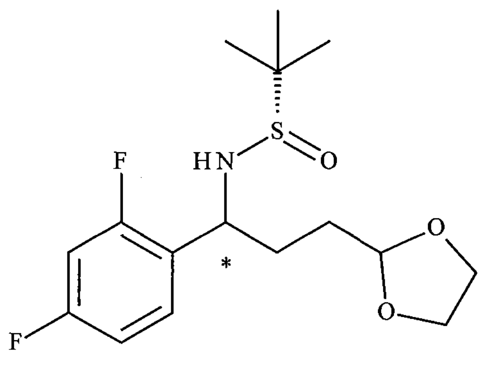

**482**

**Intermédiaire 483 :**

A une solution de l'intermédiaire **482** (5,2 g, 15mmoles) dans un mélange EtOH/$H_2O$ (50 mL/50 mL) est ajouté de l'acide trifluoro acétique (10 mL) et du $PtO_2$ (0,5 g). Le mélange est hydrogéné à pression atmosphérique et à température ambiante pendant 22 heures. Le catalyseur est filtré et le filtrat concentré. Le résidu repris par de l'eau est extrait par de l'éther éthylique. La phase aqueuse est amenée à pH basique par une solution de NaOH 10N. Après extraction à l'éther éthylique, lavage par une solution aqueuse saturée en NaCl séchage sur $MgSO_4$, l'évaporation sous pression réduite conduit à l'obtention de l'intermédiaire **483** (2,2 g).
**RMN $^1$H** (300MHz, DMSO-$d_6$): δ 7,59 (m, 1H), 7,11 (m, 1H), 7,0 (m, 1H), 4,25 (m, 1H), 2,9 (m, 2H), 2,75 (s large, NH), 1,72 (quint, 2H), 1,4 (m, 1H), 1,12 (m, 1H)
**IR (cm$^{-1}$):** 3286, 1097, 846

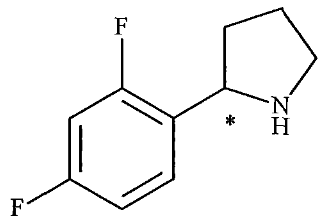

**483**

**Intermédiaire 484 :**

Obtenu par protection de l'intermédiaire **483** selon le protocole décrit pour l'intermédaire **158 (protocole VI)**
**RMN $^1$H** (300MHz, DMSO-$d_6$): δ 7,21 (dt, 1H), 7,09 (t, 1H), 7,00 (t, 1H), 4,94 (dd, 1H), 3,50 (t, 2H), 2,32 (m, 1H), 1,85 (quint, 2H), 1,73 (m, 1H), 1,23 (s, 9H)
**IR (cm$^{-1}$):** 1687, 1117
**Excès énantiomérique** > 99%

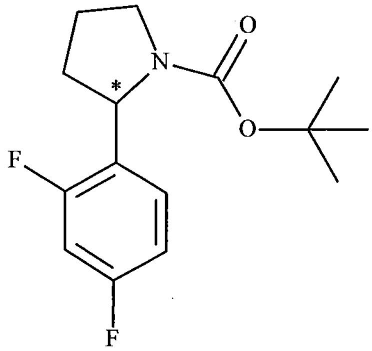

**484**

**Intermédiaire 521 :**

***N*-[(2,4-difluorophényl)méthylidène]-(2*R*)-2-méthylpropane-2-sulfinamide**

Obtenu par réaction du 2,4-difluorobenzaldéhyde avec le (*R*)-(+)-2-méthylpropane-2-sulfinamide selon le **protocole XIV.**

L'intermédiaire **520,** inverse optique de l'intermédiaire **483,** a été obtenu selon le protocole décrit pour l'intermédiaire **483** à partir du *N*-[(2,4-difluorophényl)méthylidène]-(2*R*)-2-méthylpropane-2-sulfinamide, via l'intermédiaire **519.**

**Intermédiaire 519 :**

**RMN $^1$H** (400MHz, DMSO-$d_6$): δ 7,60 (m, 1H), 7,15 (m, 1H), 7,1 (m, 1H), 5,7 (d, 2H), 4,8 (m, 1H), 4,45 (m, 1H), 3,85-3,75 (2m, 4H), 1,95-1,6 (m, 4H), 1,1 (s, 9H)
**IR (cm$^{-1}$):** 3230, 1047
**Pureté optique** (SFC: Colonne AD 5μM 4,6x250 mm; $CO_2$/MeOH: 90/10; Détection: 260nm): > 99%.

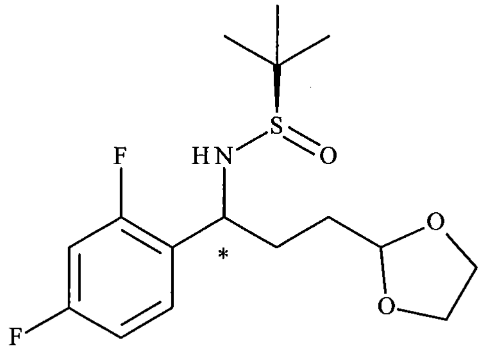

**519**

**Intermédiaire 520 :**

**RMN $^1$H** (300MHz, DMSO-$d_6$): δ 7,59 (m, 1H), 7,11 (m, 1H), 7,0 (m, 1H), 4,25 (m, 1H), 2,9 (m, 2H), 2,75 (s large, NH), 1,72 (quint, 2H), 1,4 (m, 1H), 1,12 (m, 1H).
**IR (cm$^{-1}$):** 3286, 1097, 846.

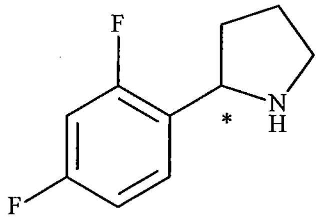

**520**

**Intermédiaire 521 :**

Obtenu par protection de l'intermédiaire **520** selon le protocole décrit pour l'intermédaire **158**
**RMN $^1$H** (400MHz, DMSO-$d_6$): δ 7,3-7,1 (2m, 1H), 7,05 (m, 1H), 5,0-4,85 (m, 1H), 3,6-3,35 (m, 2H), 2,32-1,7 (2m, 2H), 1,85 (m, 2H), 1,35-1,1 (2s, 9H)
**IR (cm$^{-1}$):** 1687

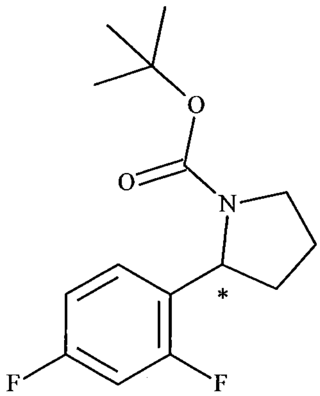

**521**

**Intermédiaire 492 :**

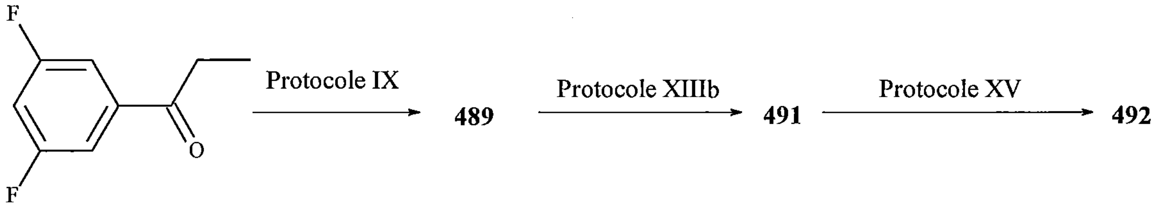


**Intermédiaire 489 :**

Obtenu à partir de l'acide 3,5-difluorobenzoïque commercial et du bromure d'éthylmagnésium selon le **protocole IX**
**GC-EI** (70 eV): $M^{+\cdot}$ = 170.

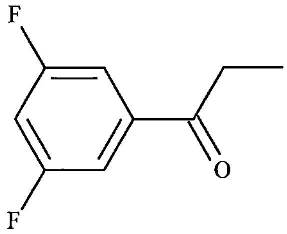

**489**

**Intermédiaire 491 :**

Obtenu par réaction de l'intermédiaire **489** et du (*R*)-(+)-2-méthyl-2-propanesulfinamide selon le **protocole XIIIb**
**RMN $^1$H** (400 MHz, DMSO-d6): δ 7,15 (dd, 2H), 7,05 (td, 1H), 5,70 (d, 1H), 4,10 (dd, 1H), 1,85-1,65 (m, 2H), 1,15 (s, 9H), 0,85 (t, 3H)
**IR ($cm^{-1}$) :** 3151, 1040

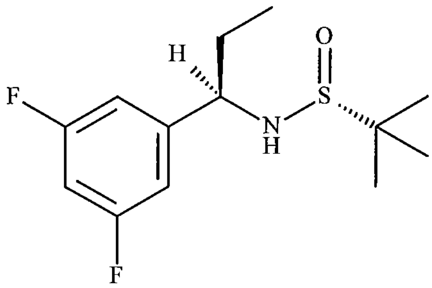

**491**

**Intermédiaire 492 :**

Obtenu à partir de l'intermédiaire **491** selon le **protocole XV**
**RMN $^1$H** (400MHz, DMSO-$d_6$): δ 7,05 (td, 1H), 7,00 (d large, 2H), 4,40 (m, 1H), 1,60 (quint, 2H), 1,35 (s, 9H), 0,80 (t, 3H)
**IR ($cm^{-1}$):** 3371, 1679
**Excès énantiomérique** > 99%

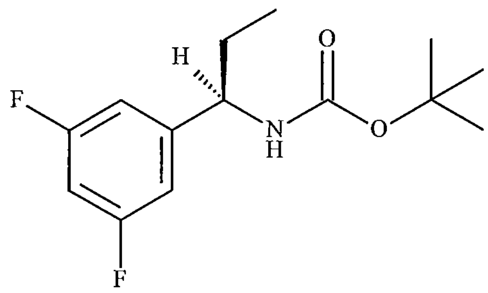

**492**

**Intermédiaire 499 :**

La préparation de l'intermédiaire **497** est décrite au **protocole XIV.**

**Intermédiaire 499 :**

Obtenu à partir de l'intermédiaire **497** selon le **protocole XV**
**RMN $^1$H** (400MHz, DMSO-$d_6$): δ 7,05 (m, 3H), 7,40 (d, 1H), 4,25 (t, 1H), 1,85 (m, 1H), 1,40-1,2 (s, 9H), 0,9-0,7 (2d, 6H)
**IR (cm$^{-1}$):** 3365, 1678, 1161

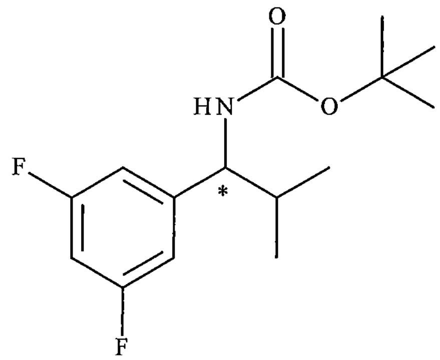

**499**

**Intermédiaire 505 :**

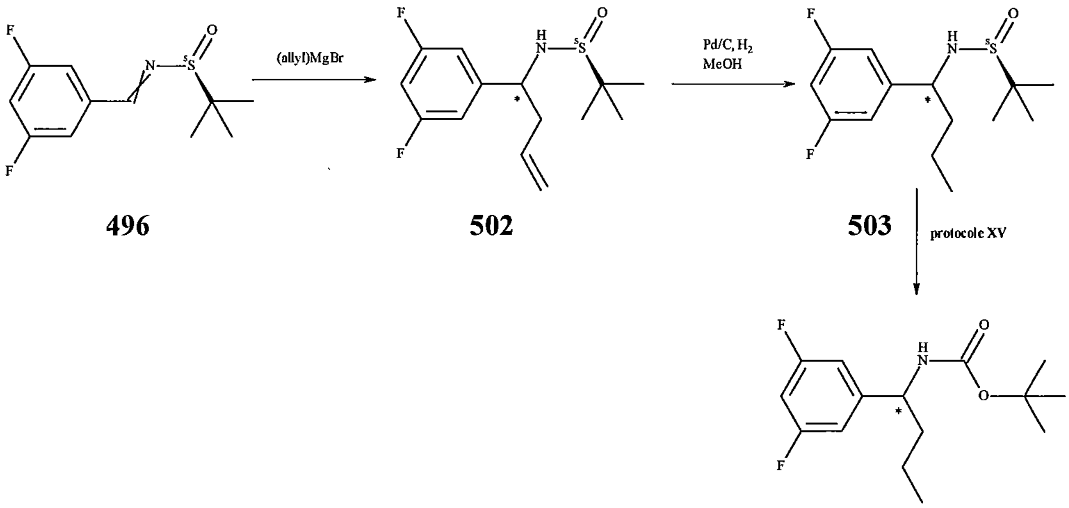


496 502 503

505

La préparation de l'intermédiaire **496** est décrite au protocole **XIV.**

**Intermédiaire 502 :**

Obtenu à partir de l'intermédiaire **496** et de (allyl)MgBr selon le **protocole XIV** (voir préparation de l'intermédiaire **497**)
**RMN $^1$H** (400MHz, DMSO-$d_6$): δ 7,1 (m, 3H), 5,7 (m, 1H), 5,05 (m, 2H), 4,35 (quad, 1H), 2,65-2,45 (m, 2H), 1,1 (s, 9H)
**IR (cm$^{-1}$):** 3205, 1053.

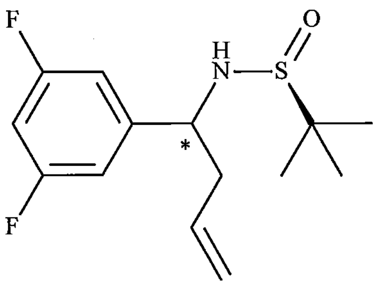

502

**Intermédiaire 503 :**

Obtenu à partir de l'intermédiaire **502** en présence de Pd/C 10% dans le méthanol sous hydrogène pendant 2 jours (2,9 g de l'intermédiaire **502** engagés, 2,9g de l'intermédiaire **503** obtenus).
**RMN $^1$H** (400MHz, DMSO-d6): δ 7,1 (d, 3H), 5,45 (d, 1H), 4,25 (quad, 1H), 1,85-1,6 (m, 2H), 1,35-1,15 (m, 2H), 1,1 (s, 9H), 1,85 (t, 3H)
**IR (cm$^{-1}$):** 3208, 1052

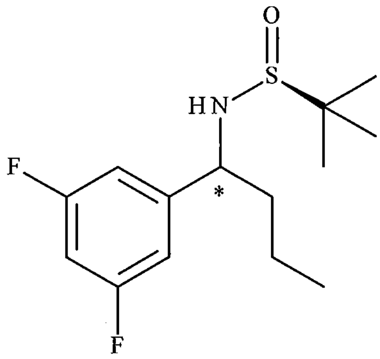

**503**

**Intermédiaire 505 :**

Obtenu à partir de l'intermédiaire **503** selon le **protocole XV**
**RMN $^1$H** (400MHz, DMSO-$d_6$): δ 7,38 (d, 1H), 7,1-7,0 (m, 3H), 4,45 (quad, 1H), 1,55 (m, 2H), 1,35 (s, 9H), 1,4-1,3 (m, 2H), 0,85 (t, 3H)
**IR (cm$^{-1}$):** 3372, 1681

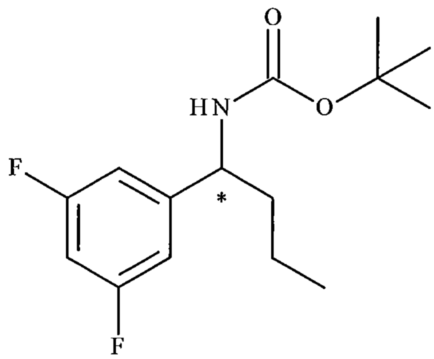

**505**

<u>**Intermédiaire 510 :**</u>

La préparation de l'intermédiaire **496** est décrite au **protocole XIV.**

**Intermédiaire 508 :**

Obtenu à partir de l'intermédiaire **496** et de *i*-BuMgCl selon le **protocole XIV**
**RMN $^1$H** (400MHz, DMSO-$d_6$): δ 7,1 (m, 3H), 5,45 (d, 1H), 4,3 (quad, 1H), 1,75-1,5 (m, 2H), 1,5 (m, 1H), 1,1 (s, 9H), 0,9 (2d, 6H)
**IR (cm$^{-1}$):** 3200, 1725, 1057

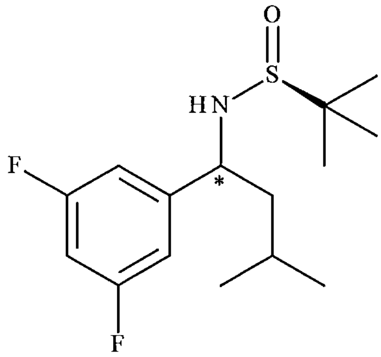

**508**

**Intermédiaire 510 :**

Obtenu à partir de l'intermédiaire **508** selon le **protocole XV**
**RMN $^1$H** (400MHz, DMSO-$d_6$): δ 7,35 (d large, 1H), 7,0 (m, 3H), 4,55 (m, 1H), 1,55 (m, 2H), 1,40 (s, 9H), 1,35 (m, 1H), 0,95 (d, 6H)
**IR ($cm^{-1}$):** 3367, 1681, 1253

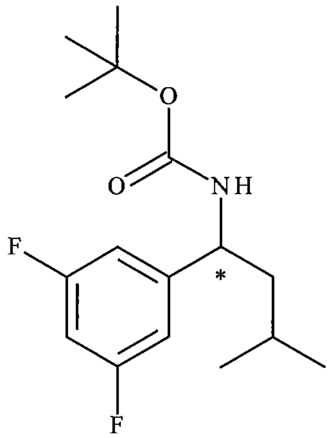

**510**

__**Intermédiaire 526 :**__

**Intermédiaire 525 :**

Obtenu par réaction du (*S*)-2-méthylpropane-2-sulfinamide avec le 3-bromo-2-fluoro-benzaldéhyde commercial selon le **protocole XIV**

**Intermédiaire 526 :**

Obtenu par traitement au MeMgBr (3M/éther) de l'intermédiaire **525** selon le **protocole XIV**
**RMN $^1$H** (400MHz, DMSO-$d_6$): δ 7,60-7,5 (m, 1H), 7,5 (m, 1H), 7,18 (m, 1H), 5,51 (d, 1H), 4,7 (m, 1H), 1,5 (d, 3H), 1,1 (s, 9H)
**IR ($cm^{-1}$):** 3206, 1048

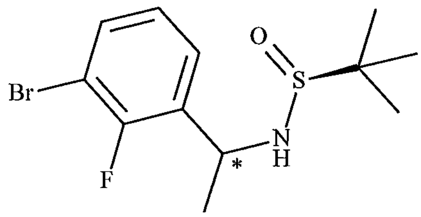

**526**

<u>**Intermédiaire 550 :**</u>

La préparation de l'intermédiaire **547** est décrite au **protocole XI.**

**Intermédiaire 548 :**

Obtenu par réaction de l'intermédiaire **547** et du (*R*)-(+)-2-méthyl-2-propanesulfinamide selon le **protocole XIII**
**RMN $^{1}$H** (400 MHz, DMSO-$d_6$): δ 7,50-7,35 (m, 5H), 7,22 (td, 1H), 7,10 (dd, 1H), 5,42 (d, 1H), 5,05 (s, 2H), 4,75 (m, 1H), 1,35 (d, 3H), 1,10 (s, 9H)
**IR (cm$^{-1}$):** 3206

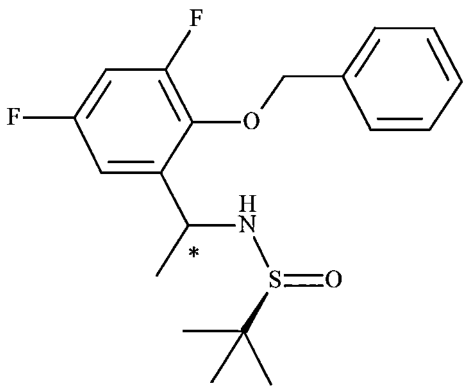

**548**

**Intermédiaire 550 :**

Obtenu à partir de l'intermédiaire **548** selon le **protocole XV**
**RMN $^{1}$H** (400MHz, DMSO-$d_6$): δ 7,49 (m, 3H), 7,42 (t, 2H), 7,37 (t, 1H), 7,20 (m, 1H), 7,05 (m, 1H), 5,04 (m, 3H), 1,36 (s, 9H), 1,19 (d, 3H)
**IR (cm$^{-1}$):** 3329, 1699

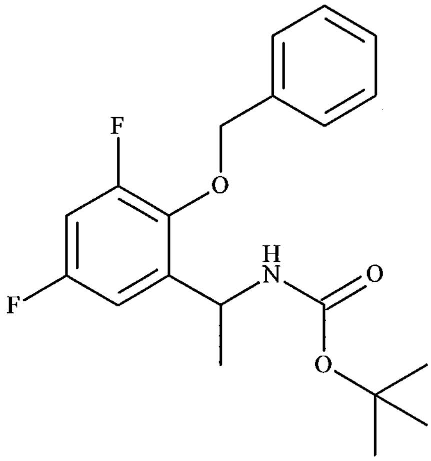

**550**

<u>**Intermédiaire**</u> **696 :**

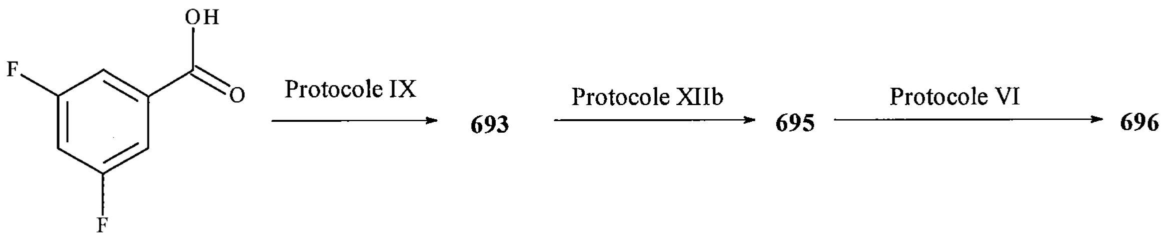


**Intermédiaire 693 :**

Obtenu à partir de l'acide 3,5-difluorobenzoïque commercial et du bromure de 3-méthoxyphénylmagnésium selon le **protocole IX**
**RMN $^1$H** (400 MHz ; DMSO-$d_6$): δ 7,60 (m, 1H), 7,50 (t, 1H), 7,40 (m, 2H), 7,30 (m, 3H), 3,85 (s, 1H)
**IR (cm$^{-1}$) :** 1665

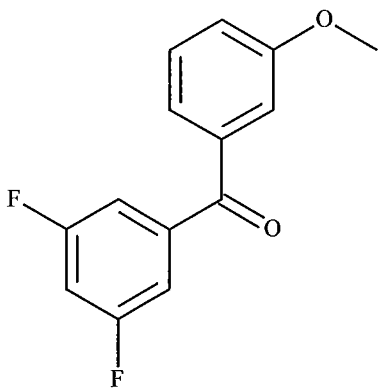

**693**

**Intermédiaire 695 :**

Obtenu à partir de l'intermédiaire **693** selon le **protocole XIIIb**

**RMN $^1$H** (300MHz; DMSO-$d_6$): 7,20 (t, 1H), 7,10 (m, 2H); 7,00 (m, 2H), 6,95 (d, 1H), 6,75 (dd, 1H), 5,05 (s, 1H), 3,75 (s, 3H), 2,35 (s large, 2H)
**IR (cm$^{-1}$):** 3385-3309, 1594, 1254

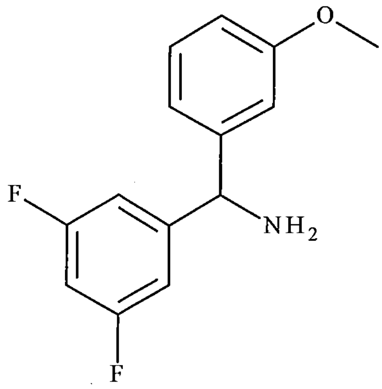

**695**

**Intermédiaire 696 :**

Obtenu par protection de l'intermédiaire **695** selon le protocole décrit pour l'intermédaire **158 (protocole VI)**
**RMN $^1$H** (300MHz, DMSO-$d_6$): δ 8,00 (d large, 1H), 7,25 (t, 1H), 7,10 (m,3H), 6,95 (m, 2H), 6,80 (dd, 1H), 5,70 (d large, 1H), 3,75 (s, 3H), 1,40 (slarge, 9H)
**IR (cm$^{-1}$):** 3357, 1684, 1162

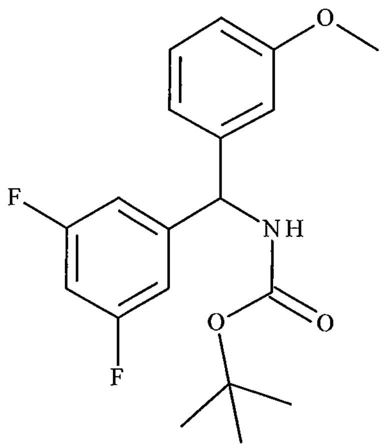

**696**

**Intermédiaire 702 :**

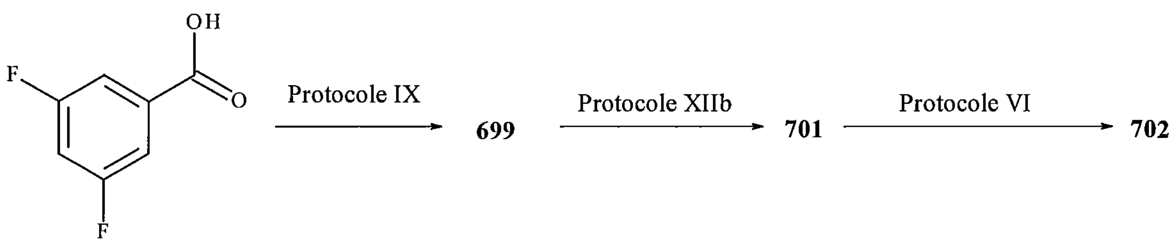

**Intermédiaire 699 :**

Obtenu à partir de l'acide 3,5-difluorobenzoïque commercial et du bromure de méthylcyclohexylmagnésium selon le **protocole IX**
**RMN $^1$H** (400 MHz ; DMSO-$d_6$): δ 7,64 (m, 2H), 7,56 (tt, 1H), 2,90 (d, 2H), 1,83 (m, 1H), 1,6-1,1-0,99 (3m, 10H)
**IR (cm$^{-1}$):** 1688

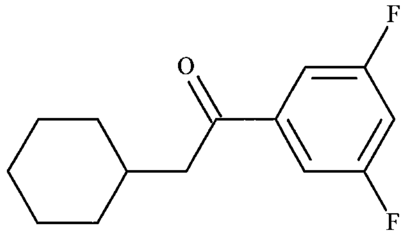

**699**

**Intermédiaire 701 :**

Obtenu à partir de l'intermédiaire **699** selon le **protocole XIIb**
**RMN $^1$H** (400MHz ; DMSO-$d_6$): 8,57 (s large, 3H), 7,35 (m, 2H); 7,28 (tt, 1H), 4,35 (dd, 1H), 1,85-1,67 (m, 3H), 1,6-0,88 (3m, 10H)
**IR (cm$^{-1}$):** 3200-2500, 1126.

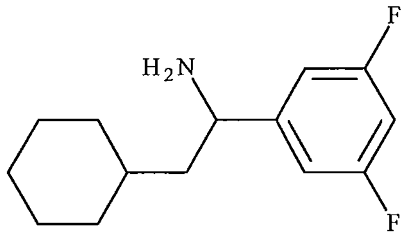

**701**

**Intermédiaire 702 :**

Obtenu par protection de l'intermédiaire **701** selon le protocole décrit pour l'intermédaire **158 (protocole VI)**
**RMN $^1$H** (400MHz, DMSO-$d_6$): δ 7,00 (m, 3H), 7,38 (d, 1H), 4,60 (m,1H), 1,78-1,55 (2m, 2H), 1,7-0,8 (m, 11H), 1,40 (s, 9H)
**IR (cm$^{-1}$):** 3280, 1677

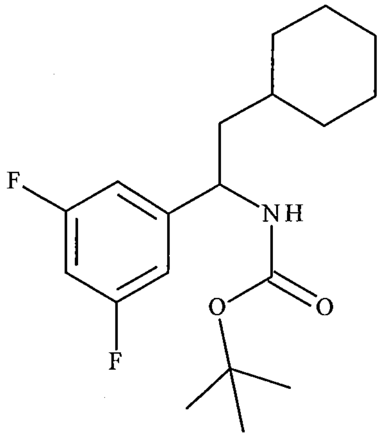

**702**

**Intermédiaire 706 :**

**Intermédiaire 705 :**

Une solution de 1-(3,5-difluorophényl)éthanone commerciale (5 g, 32 mmoles) et de cyclohexaneméthylamine (3,6 g, 32 mmoles) dans du toluène (50 mL) est chauffé 40 heures au reflux dans un montage azéotropique. Le toluène est évaporé et le résidu est mis en solution dans de l'éthanol (40 mL). La solution est refroidie à 10°C, puis du $NaBH_4$ (1,2 g, 32 mmoles) est ajouté par portions; le mélange réactionnel est agité 2 heures, du $NaBH_4$ (0,12 g) est ajouté de nouveau. Après 30 minutes d'agitation, une solution aqueuse d'HCl 3N est ajoutée avec précaution et l'éthanol est évaporé sous vide. Le résidu repris par du toluène (200 mL) est lavé par une solution aqueuse de NaOH 40%. La phase organique est séchée sur $MgSO_4$, l'évaporation sous pression réduite conduit à l'obtention de l'intermédiaire **705** (4,4 g) utilisé sans traitement supplémentaire dans l'étape suivante.
**RMN $^1$H** (300MHz, DMSO-$d_6$): $\delta$ 7,00 (m, 3H), 3,70 (quad, 1H), 2,20 (dd,1H), 2,05 (dd, 1H), 1,80-1,70 (3m, 10H), 1,30 (m, 1H), 1,20 (d, 3H)
**IR (cm$^{-1}$):** 2922-2850, 1114

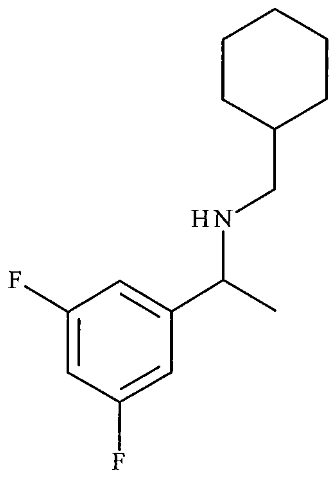

**705**

**Intermédiaire 706 :**

Obtenu par protection de l'intermédiaire **705** selon le protocole décrit pour l'intermédaire **158 (protocole VI)**
**RMN $^1$H** (300MHz, DMSO-$d_6$): $\delta$ 7,10 (tt, 1H), 6,95 (d, 2H), 4,90 (s large,1H), 3,00 (s large, 2H), 1,50 (d, 3H), 1,30 (s large, 9H), 1,70-0,75 (2m, 11H). **RMN 19F :** -110,7

**IR ($cm^{-1}$):** 1686, 1147

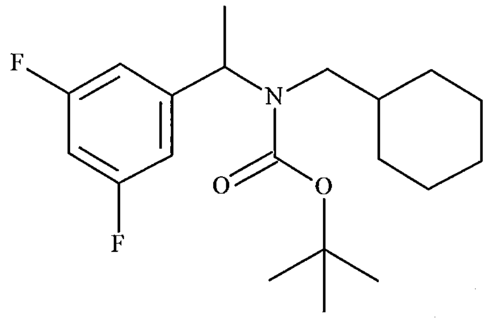

**706**

<u>**Intermédiaire 278 :**</u>

Obtenu à partir de l'intermédiaire **489** selon le **protocole XII**
**RMN $^1$H** (400MHz ; $CDCl_3$): 6,88 (d, 2H); 6,65 (tt, 1H), 4,10 (quad, 2H) ; 1,53 (slarge, 2H), 1,35 (d, 3H)
**IR ($cm^{-1}$):** 3371, 3298

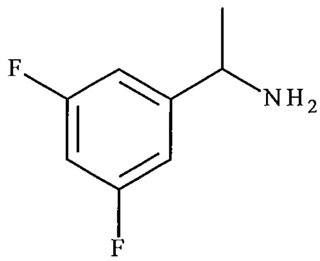

**278**

<u>**Intermédiaire 104 :**</u>

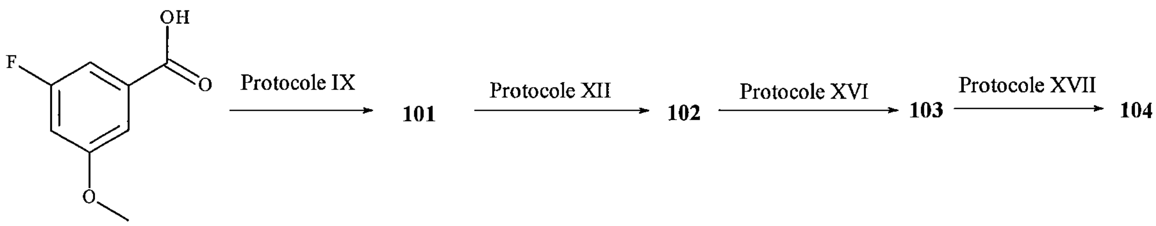


**Intermédiaire 101:**

Obtenu à partir de l'acide 3-fluoro-5-méthoxybenzoïque commercial et du bromure de méthylmagnésium selon le **protocole IX**
**RMN $^1$H** ($CDCl_3$): $\delta$ 7,20 (s, 1H), 7,14 (d, 1H), 6,73 (d, 1H), 3,77 (s, 3H), 2,50 (s, 3H)

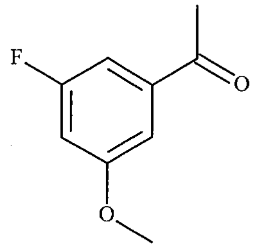

**101**

**Intermédiaire 102 :**

Obtenu à partir de l'intermédiaire **101** selon le **protocole XII**
**RMN $^1$H** (300MHz ; DMSO-$d_6$): 6,75 (t et d, 2H), 6,60 (d et t, 1H), 3,90 (quad, 1H), 3,75 (s, 3H), 1,80 (m, 2H), 1,20 (d, 6H)
**IR (cm$^{-1}$):** 3750-2750

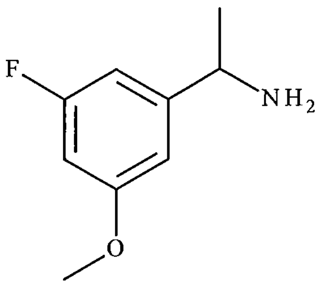

**102**

**Intermédiaire 103 :**

Obtenu à partir de l'intermédiaire **102** selon le **protocole XVI**
**RMN $^1$H** (300 MHz, $CDCl_3$) : δ 6,60 (2m, 2H), 6,55 (t, 1H), 6,40 (m, 1H), 5,10 (quint, 1H),3,80 (s, 3H), 1,55 (d, 3H)
**IR (cm$^{-1}$) :** 3240, 1694, 1627, 1595, 1558

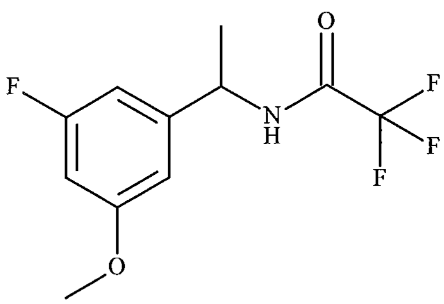

**103**

**Intermédiaire 104 :**

Obtenu à partir de l'intermédiaire **103** selon la procédure utilisée pour la transformation de l'intermédiaire **93a** en intermédiaire **94 (Protocole XVII)**
**RMN $^1$H** (500MHz, DMSO-$d_6$): 10,30 (s, 1H), 9,90 (1, 1H), 7,08 (d, 1H), 6,87 (d, 1H), 5,05 (m, 1H), 3,92 (s, 3H), 1,48 (dd, 3H)
**IR (cm$^{-1}$):** 3294, 1688

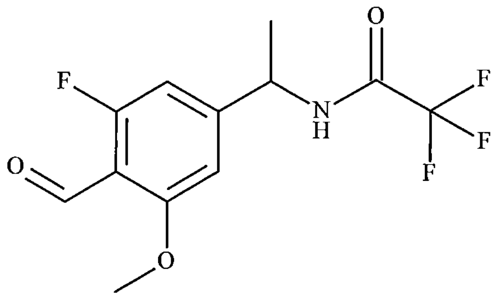

**104**

<u>**Intermédiaire 18 :**</u>

La préparation de l'intermédiaire **17** est décrite au **protocole XVI**.

**Intermédiaire 18 :**

Obtenu à partir de l'intermédiaire **17** selon la procédure utilisée pour la transformation de l'intermédiaire **93a** en intermédiaire **94 (Protocole XVII)**
**RMN $^1$H** (400MHz, DMSO-$d_6$): δ 10,31 (s, 1H), 9,94 (m, 1), 7,68 (d, 1H), 7,22 (s large, 1H) 7,05 (d large, 1H), 5,06 (quad, 1H), 3,93 (s, 3H), 1,48 (d, 3H)
**IR (cm$^{-1}$):** 3299, 1703, 1672

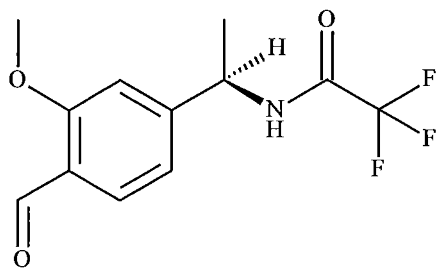

**18**

<u>**Intermédiaire 357 :**</u>

**2,2,2-trifluoro-*N*-[(1*S*)-1-(8-formyl-3,4-dihydro-2*H*-chromén-5-yl)éthyl]acétamide**

Obtenu à partir de la (*S*)-(-)-1-(3-méthoxyphényl)éthylamine selon les **protocoles XVI** et **XVII**
**RMN $^1$H** (400MHz, DMSO-$d_6$): δ 10,30 (s, 1H), 10,00 (s, 1H), 7,55 (d, 1H), 7,05 (d, 1H), 5,12 (quad, 1H), 4,25 (t, 2H) 2,9-2,78 (m, 2H), 2,05 (m, 2H), 1,40 (d, 3H)
**RMN $^{19}$F:** -72 (dd, 1F)
**IR (cm$^{-1}$):** 3298, 1701, 1673.

<u>**Intermédiaire 453:**</u>

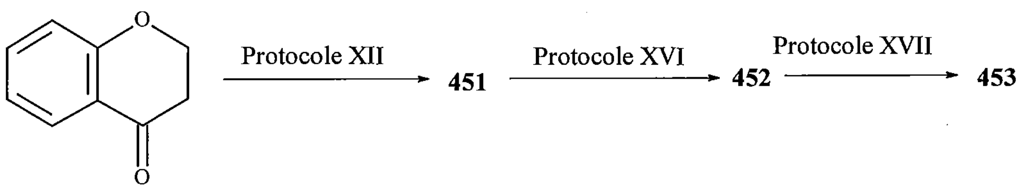

**Intermédiaire 451 :**

Obtenu à partir de la 2,3-dihydro-4*H*-chromén-4-one commerciale selon le **protocole XII**
**RMN $^1$H** (300MHz ; DMSO-$d_6$): 8,70 (m, 3H), 7,60 (d, 1H), 7,25 (t, 1H), 6,95 (t, 1H), 6,85 (d, 1H), 4,50 (m, 1H), 4,25 (m, 2H), 2,20 (m, 2H)
**IR (cm$^{-1}$):** 3400-2250

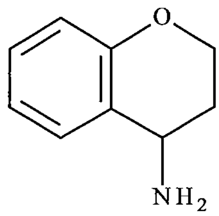

**451**

**Intermédiaire 452 :**

Obtenu à partir de l'intermédiaire **451** selon le **protocole XVI**
**RMN $^1$H** (300 MHz, DMSO-$d_6$) : δ 9,90 (d, 1H), 7,20 (td, 1H), 7,10 (dd, 1H), 6,90 (td, 1H), 6,80 (d, 1H), 5,10 (m, 1H), 4,20 (m, 2H), 2,10 (2m, 2H)
**IR (cm$^{-1}$) :** 3266, 1699, 1546, 754, 711

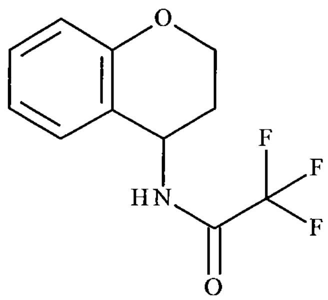

**452**

**Intermédiaire 453 :**

Obtenu à partir de l'intermédiaire **452** selon la procédure utilisée pour la transformation de l'intermédiaire **93a** en intermédiaire **94 (Protocole XVII)**
**RMN $^1$H** (500MHz, $CDCl_3$): 10,35 (s, 1H), 9,95 (s, 1H), 7,65 (dd, 1H), 7,45 (dd, 1H), 7,05 (t, 1H), 5,20 (m, 1H), 4,4-4,35 (2m, 2H), 2,20 (2m, 2H)

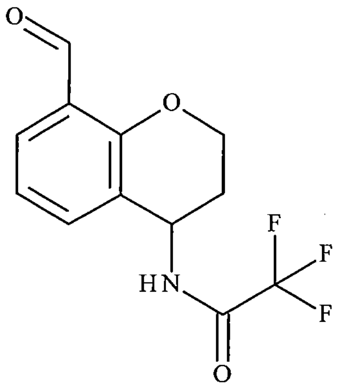

**453**

<u>**Intermédiaire 639:**</u>

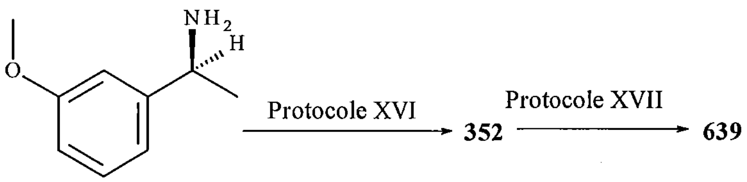


**Intermédiaire 352 :**

Obtenu par protection de la (1*S*)-1-(3-méthoxyphényl)éthanamine commerciale selon le **protocole XVI**
**RMN $^1$H** (400 MHz, $CDCl_3$) : δ 7,30 (m, 1H), 6,90 (d, 1H), 6,85 (m, 2H), 6,68 (m, 1H), 5,10 (quint, 1H), 3,80 (s, 3H), 1,58 (d, 3H)
**IR ($cm^{-1}$) :** 3294, 1697, 1151

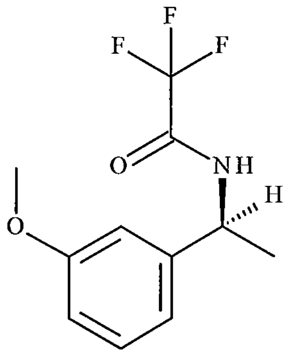

**352**

**Intermédiaire 639 :**

Obtenu à partir de l'intermédiaire **352** selon la procédure utilisée pour la transformation de l'intermédiaire **93a** en intermédiaire **94 (Protocole XVII)**
**RMN $^1$H** (500MHz, DMSO-$d_6$): 10,50 (s, 1H), 7,80 (d, 1H), 6,95 (dd, 1H), 6,90 (d, 1H), 6,60 (s large, 1H), 5,18 (m, 1H), 3,95 (s, 3H), 1,60 (dd, 3H)
**IR ($cm^{-1}$):** 3324, 1692-1660

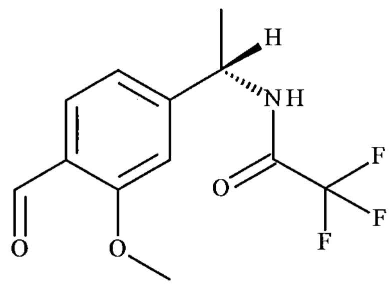

**639**

<u>**Intermédiaire 53:**</u>

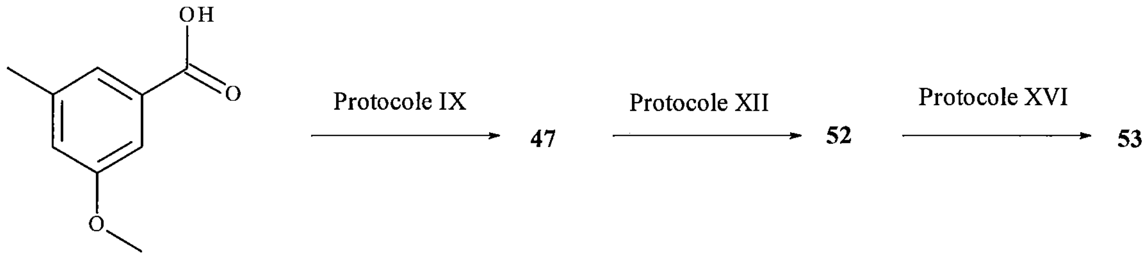


**Intermédiaire 47:**

Obtenu à partir de l'acide 3-méthoxy-5-méthylbenzoïque commercial et du bromure de méthylmagnésiumn selon le **protocole IX**
**RMN $^1$H** ($CDCl_3$): δ 7,26 (s, 1H), 7,20 (s, 1H), 6,84 (s, 1H), 3,74 (s, 3H), 2,49 (s, 3H), 2,29 (s, 3H)

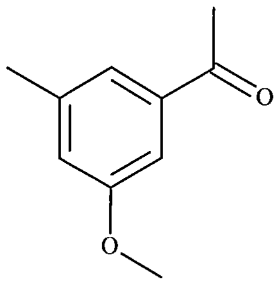

**47**

**Intermédiaire 52 :**

Obtenu à partir de l'intermédiaire **47** selon le **protocole XII**
**RMN $^1$H** (400/500MHz ; DMSO-$d_6$): 6,72 (s, 1H), 6,56 (s, 1H), 3,90 (quad, 1H), 3,70 (s, 3H), 2,25 (s, 3H), 1,70 s, 2H), 1,20 (s, 3H)
**IR ($cm^{-1}$):** 3750-2750

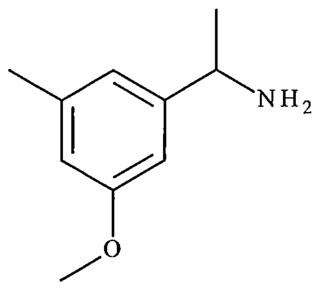

**52**

**Intermédiaire 53 :**

Obtenu à partir de l'intermédiaire **52** selon le **protocole XVI**
**RMN $^1$H** (400 MHz, DMSO-$d_6$) : δ 9,80 (d, 1H), 6,70-6,65 (3s large, 3H), 4,95 (quint, 1H), 3,70 (s, 3H), 2,25 (s, 3H), 1,40 (d, 3H)
**IR ($cm^{-1}$) :** 3295, 1694, 1596, 1558, 1185, 1152, 847-685

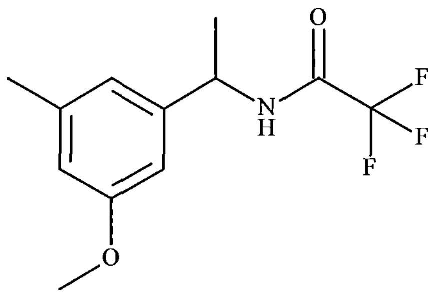

**53**

**Intermédiaire 651 :**

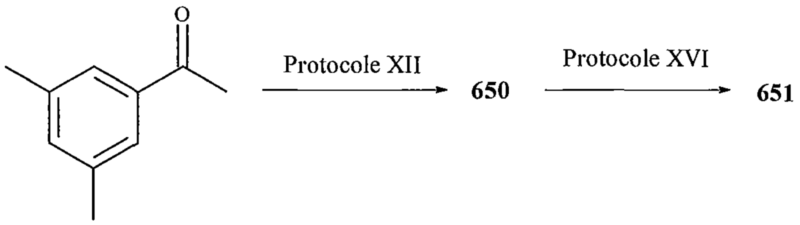


**Intermédiaire 650 :**

Obtenu à partir de la 1-(3,5-diméthylphényl)éthanone commerciale selon le **protocole XII**
**RMN $^1$H** (300MHz ; $CDCl_3$): 7,00 (m, 2H); 6,90 (m, 1H), 4,05 (quad, 1H) ; 2,30 (s, 6H), 1,50 (m, 2H), 1,35 (d, 3H)
**IR ($cm^{-1}$):** 3364, 3290

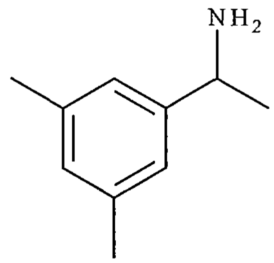

**650**

**Intermédiaire 651 :**

Obtenu à partir de l'intermédiaire **650** selon le **protocole XVI**
**RMN $^1$H** (400 MHz, $CDCl_3$) : δ 6,98 (s, 1H), 6,92 (s, 2H), 6,40 (s large, 1H), 5,05 (quint, 1H), 2,30 (s, 6H), 1,55 (d, 3H)
**IR ($cm^{-1}$) :** 3298, 1694, 1161

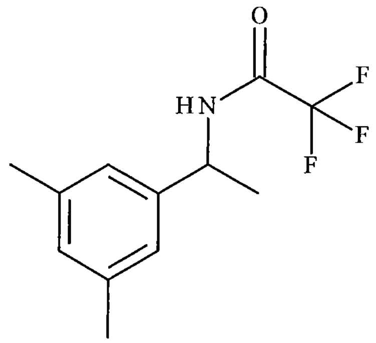

**651**

**Intermédiaire 664:**

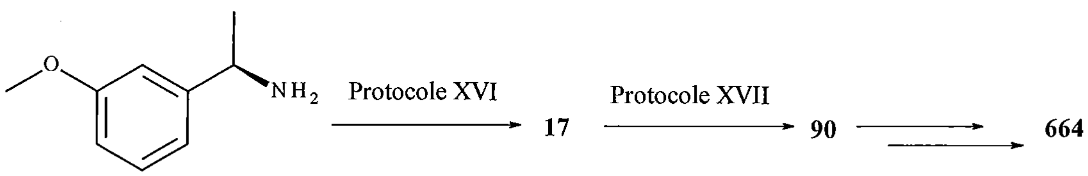


L'intermédiaire **664** a été préparé à partir de l'intermédiaire **90** (lui-même préparée selon le **Protocole XVII,** à partir de l'intermédiaire 17, préparé selon le **protocole XVI)** selon la séquence suivante:

**Intermédiaire 660 :**

A une solution de l'intermédiaire **90** (1 g, 4,3 mmoles) dans le DMF (25 mL) sont ajouté du carbonate de potassium (0,7 g) puis du bromure d'allyle (0,4 mL, 4,5 mmoles). Le mélange est agité à température ambiante pendant 5 heures, puis versé sur un mélange glace-eau. Après décantation en présence d'éther éthylique, la phase organique est séchée sur $MgSO_4$, puis concentrée. Le résidu obtenu est chromatographié sur silice (éluant : $CH_2Cl_2$ 100%), l'intermédiaire **660** (0,7 g) est obtenu sous forme d'un solide.
**RMN $^1$H** (300MHz, DMSO-$d_6$): 9,70 (s large, NH), 7,25 (t, 1H), 6,95-6,80 (m, 3H), 6,10-5,95 (m, 1H), 5,30 (2dd, 2H), 4,95 (quad, 1H), 4,55 (d, 2H), 1,45 (d, 3H)
**IR ($cm^{-1}$):** 3319, 1693, 1662, 1157

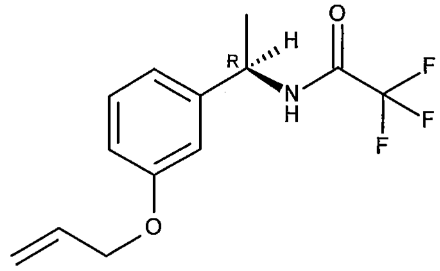

**660**

**Intermédiaire 662 :**

Un mélange de l'intermédiaire **660** (0,7 g) dans de la diéthylaniline (7 mL) est chauffé à 210°C pendant 20 heures. Le mélange réactionnel est lavé par une solution d'HCl 1N, puis extrait par du chlorure de méthylène. La phase organique est séchée sur $MgSO_4$, puis concentrée. Le mélange des isoméres **661** et **662** est chromatographié sur silice (éluant : $CH_2Cl_2$ 100%), l'isomère **662** (0,15 g) est obtenu.
**RMN $^1$H** (300MHz, DMSO-$d_6$): 10,0-9,0 (2 s large, NH-OH), 7,05 (t, 1H), 6,90 (d, 1H), 6,75 (d, 1H), 5,95 (m, 1H), 5,18 (quad, 1H), 4,90 (m, 2H), 3,45 (d, 2H), 1,40 (d, 3H)
**IR ($cm^{-1}$):** 3461, 3293, 1698, 1156

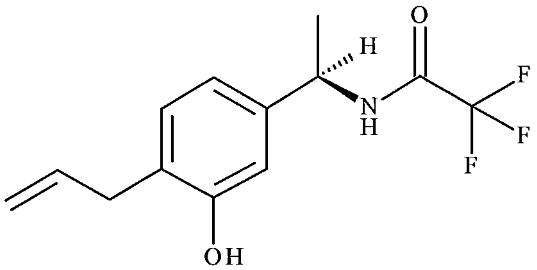

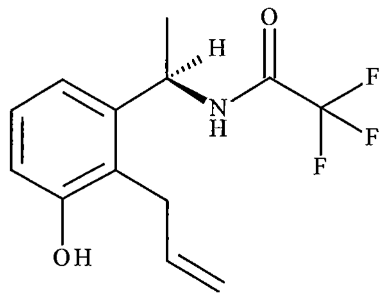

**661** **662**

**Intermédiaire 663 :**

A une solution de l'intermédiaire **662** (2 g) dans un mélange de 1,4-dioxane (90 mL) et d'eau (30 mL), sont ajouté successivement de l'$OsO_4$ (2,5% dans *t*-BuOH) (1,48 g), de la 2,6-lutidine (1,68 mL), et du $NaIO_4$ (6 g). Le mélange réactionnel est agité à température ambiante pendant 20 heures. Après décantation par de l'AcOEt, la phase organique est lavée par de l'eau, par une solution aqueuse d'HCl 1N et une solution aqueuse saturée en NaCl, la phase organique est séchée sur $MgSO_4$, puis concentrée. Le résidu (1 g) repris par du toluène (100 mL) a été traité par de l'acide para-toluènesulfonique (0,5 g). Le mélange réactionnel est chauffé 1 heure au reflux. Le mélange est concentré sous vide et le résidu est chromatograghié sur silice (éluant 100%$CH_2Cl_2$). L'intermédiaire 663 (1 g) est obtenu sous forme d'un solide blanc.
**RMN $^1$H** (300MHz, DMSO-$d_6$): 9,80 (d, 1H), 7,95 (d, 1H), 7,50 (d large, 1H), 7,30 (t, 1H), 7,20 (d large, 1H), 7,10 (d large, 1H), 5,30 (quint, 1H), 1,55 (d, 3H)
**IR ($cm^{-1}$):** 3276, 1695

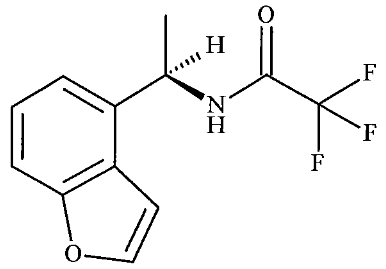

**663**

**Intermédiaire 664 :**

L'intermédiaire **663** (0,9 g, 3.5 mmoles) en solution dans de l'éthanol (90 mL) est hydrogéné à pression atmosphérique et température ambiante en présence de $Pd(OH)_2$ (0,25 g). Le catalyseur est filtré, et la concentration du filtrat conduit à l'obtention de l'intermediaire **664** (0,8 g) qui est utilisé dans l'étape suivante sans purification supplémentaire.
**RMN $^1$H** (300MHz, DMSO-$d_6$): 9,90 (s large, NH), 7,10 (t, 1H), 6,82 (d, 1H), 6,66 (d, 1H), 4,93 (m, 1H), 4,52 (t, 1H), 3,20 (t, 2H), 1,45 (d, 3H).
**IR (cm$^{-1}$):** 3272, 1696

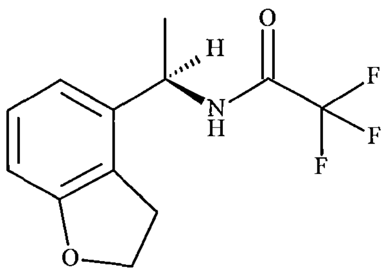

**664**

<u>**Intermédiaire 672**</u>

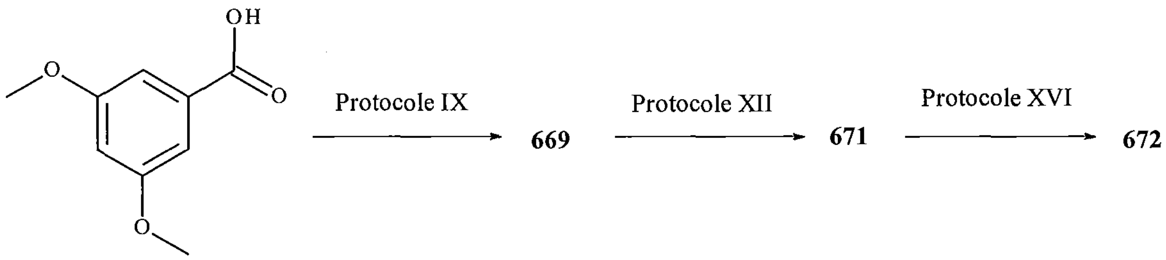


**Intermédiaire 669 :**

Obtenu à partir de l'acide 3,5-diméthoxybenzoïque commercial et du bromure de méthylmagnésium selon le **protocole IX**
**RMN $^1$H** (300 MHz ; DMSO-$d_6$): δ 7,05 (d, 2H), 6,75 (m, 1H), 3,80 (s, 6H), 2,55 (s, 1H)
**IR (cm$^{-1}$):** 1681

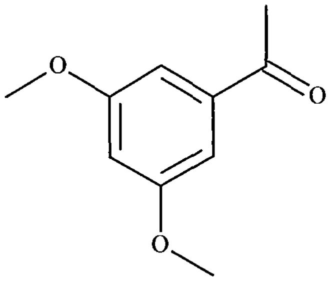

**669**

**Intermédiaire 671 :**

Obtenu à partir de lintermédiaire **669** selon le **protocole XII**
**RMN $^1$H** (300MHz ; $CDCl_3$): 6,50 (d, 2H); 6,32 (t, 1H), 4,02 (quad, 1H) ; 3,80 (s, 6H), 1,50 (s, 2H), 1,35 (d, 3H)
**IR ($cm^{-1}$):** 3359, 3295

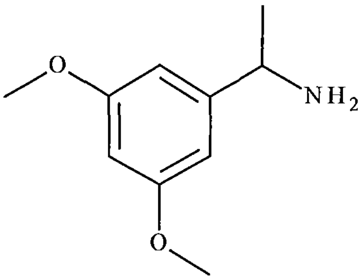

**671**

**Intermédiaire 672 :**

Obtenu à partir de l'intermédiaire **671** selon le **protocole XVI**
**RMN $^1$H** (300 MHz, DMSO-$d_6$) : δ 9,60 (d, 1H), 6,50 (d, 2H), 6,40 (t, 1H), 4,92 (m, 1H), 3,74 (s, 6H), 1,45 (d, 3H)
**IR ($cm^{-1}$) :** 3321, 1698, 1608, 1553, 1182-1144

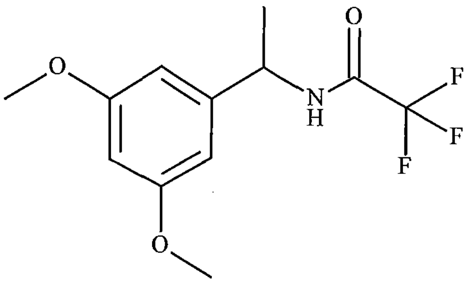

**672**

**Intermédiaire 566:**

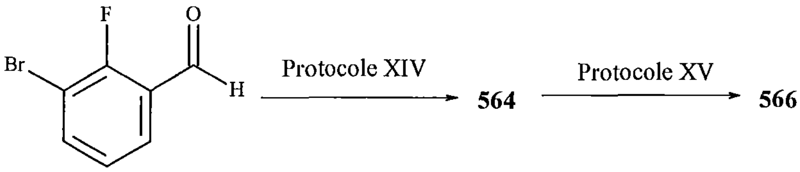


**Intermédiaire 563:**

Obtenu par réaction du (*R*)-2-méthylpropane-2-sulfinamide avec le 3-bromo-2-fluoro-benzaldéhyde commercial selon le **protocole XIV**

**Intermédiaire 564 :**

Obtenu par traitement au MeMgBr (3M/éther) de l'intermédiaire **563** selon le **protocole XIV**
**RMN $^1$H** (400MHz, DMSO-$d_6$): δ 7,60-7,50 (2m, 2H), 7,16 (t, 1H), 5,53 (d, 1H), 4,7 (m, 1H), 1,48 (d, 3H), 1,09 (s, 9H)
**IR (cm$^{-1}$):** 3189, 1040

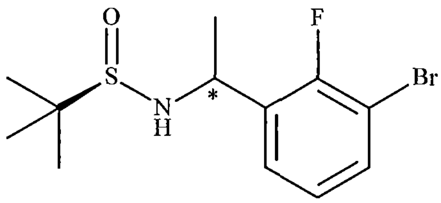

**564**

**Intermédiaire 566 :**

Obtenu à partir de l'intermédiaire **564** selon le **protocole XV**
**RMN $^1$H** (400MHz, DMSO-$d_6$): δ 7,55 (2m, 2H), 7,36 (t, 1H), 7,15 (t, 1H), 4,85 (m, 1H), 1,35 (s large, 9H), 1,3 (d, 3H)
**IR (cm$^{-1}$):** 3360, 1676
$\alpha_D$ (589nM) = 35,09 (0,005 g/mL/MeOH) à 20°C

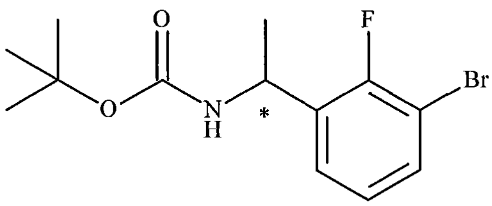

**566**

**Intermédiaire 585:**

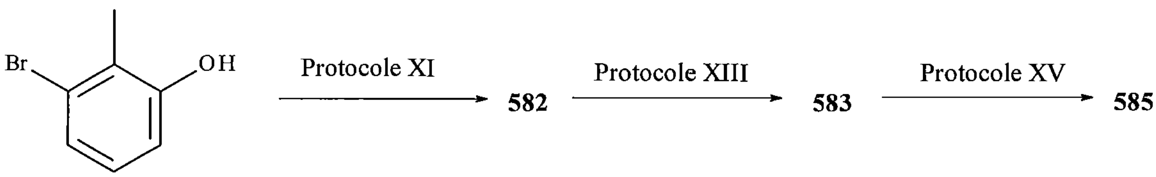

**Intermédiaire 582 :**

Obtenu à partir du 3-bromo-2-méthylphénol commercial selon le **protocole XI**, en présence d'iodure de méthyle à la dernière étape
**RMN $^1$H** (400MHz, DMSO-$d_6$): δ 7,45 (d, 1H), 7,35 (d, 1H), 3,72 (s, 3H), 5,10 (s, 2H), 2,58 (s, 3H), 2,35 (s, 3H)
**IR (cm$^{-1}$) :** 1681

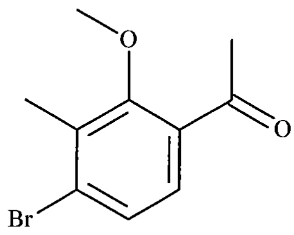

**582**

**Intermédiaire 583 :**

Obtenu par réaction de l'intermédiaire **582** et du (*R*)-(+)-2-méthyl-2-propanesulfinamide selon le **protocole XIII**
**RMN $^1$H** (400 MHz, DMSO-d6): δ 7,40 (d, 1H), 7,31 (d, 1H), 5,62 (d, 1H), 4,64 (m, 1H), 3,71 (s, 3H), 2,29 (s, 3H), 1,33 (d, 3H), 1,10 (s, 9H)
**IR (cm$^{-1}$):** 3215, 1009

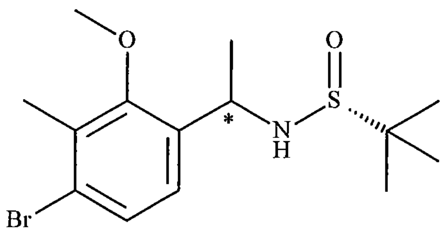

**583**

**Intermédiaire 585 :**

Obtenu à partir de l'intermédiaire **583** selon le **protocole XV**
**RMN $^1$H** (400MHz, DMSO-$d_6$): δ 7,43 (d, 1H), 7,37 (d, 1H), 7,15 (d, 1H), 4,88 (m, 1H), 3,75 (s, 3H), 2,28 (s, 3H), 1,35 (s large, 9H), 1,21 (d, 3H)
**IR (cm$^{-1}$):** 3353, 1695

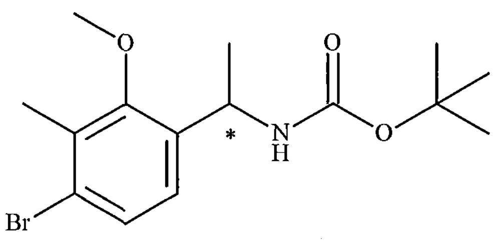

**585**

**Intermédiaire 748 :**

**Intermédiaire 743 :**

A une solution de la 2,3-dihydro-4*H*-chromén-4-one commerciale (20 g, 135 mmoles) dans le chlorure de méthylène (400 mL) sont ajouté du $ZnI_2$ (0,8 g) et du TMSCN (20 g, 201 mmoles). Le mélange réactionnel est agité à température ambiante 20 heures puis la phase organique est lavée par une solution aqueuse de $NaHCO_3$ (400 mL), séchée sur $MgSO_4$, filtrée et concentrée sous vide. L'intermédiaire **743** est obtenu (32 g) sous forme d'une huile qui est utilisée dans l'étape suivante.
**RMN $^1$H** (400MHz, $CDCl_3$): 7,47 (dd, 1H), 7,18 (dd, 1H), 6,88 (td, 1H), 6,75 (dd, 1H), 4,25 (m, 2H), 2,30 (m, 2H), 0,08 (s, 9H)

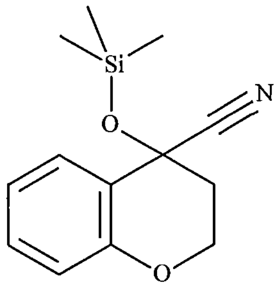

**743**

Intermédiaire 745 et intermédiaire 746:

A une solution de l'intermédiaire 743 (2,7 g, 11 mmoles) dans du THF (20 mL) est ajouté une solution de $LiAlH_4$ (1M/THF) (23 mmoles) diluée dans du THF (10 mL) et préalablement refroidie à 0°C. Le mélange réactionnel est agité 90 minutes à 5°C, puis il est traité successivement par de l'eau, une solution aqueuse de soude à 20% et de l'eau. Les sels sont filtrés, le filtrat est lavé par une solution aqueuse saturée en NaCl, la phase organique est séchée sur $MgSO_4$ puis concentrée. Le residu (2,4 g) obtenu mis en solution dans du chlorure de méthylène (20 mL) est traité par une solution d'anhydride trifluoroacétique (2 mL) dans le chlorure de méthylène (15 mL). Le mélange réactionnel est agité 20 heures à température ambiante, puis il est concentré sous vide. Le résidu obtenu est chromatographié sur silice (éluant : cyclohexane/$CH_2Cl_2$ 30/70 à 0/100). Un mélange des intermédiaires **745** (exo) et **746** (endo) est obtenu, mélange qui est utilisé dans l'étape suivante.

**Intermédiaire 745 :**

**RMN $^1$H** (400MHz, DMSO-$d_6$): 10,8 (s, 1H), 7,61 (d, 1H), 7,15 (m, 2H), 6,89 (t, 1H), 6,72 (d, 1H), 4,15 (t, 2H), 2,75 (t, 2H)
**IR ($cm^{-1}$):** 3350, 1693

**Intermédiaire 746 :**

**RMN $^1$H** (400MHz, DMSO-$d_6$): 9,80 (s, 1), 7,22 (t, 1H), 7,15 (d, 1H), 6,92 (t, 1H), 6,80 (d, 1H), 5,75 (t, 1H), 4,75 (s large, 2H), 4,2 (s large, 2H)
**IR ($cm^{-1}$):** 3300, 1702, 1150

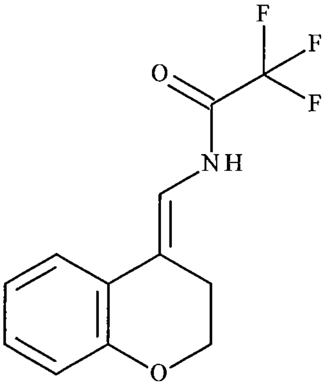 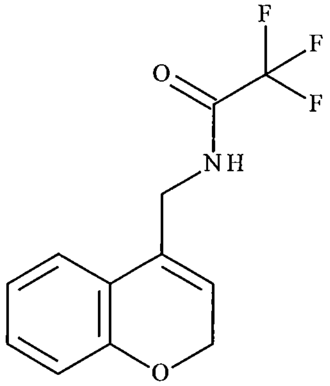

**745** **746**

**Intermédiaire 747 :**

Le mélange d'intermédiaires **745** et **746** (12 g, 46 mmoles) en solution dans de l'éthanol (600 mL) est hydrogéné à pression atmosphérique et température ambiante en présence de Pd/C 10% (1,2 g). Le catalyseur est filtré, et la concentration du filtrat conduit à l'obtention du composé **747** (10 g) qui est utilisé dans le **Protocole XVII** sans purification supplémentaire.
**RMN $^1$H** (400MHz, DMSO-$d_6$): 9,70 (t, 1), 7,15 (d, 1H), 7,10 (t, 1H), 6,85 (t, 1H), 6,75 (d, 1H), 4,15-4,1 (m, 2H), 3,55-3,3 (m, 2H), 3,00 (m, 1H), 1,9-1,8 (m, 2H)
**IR (cm$^{-1}$):** 3300, 1702, 1150

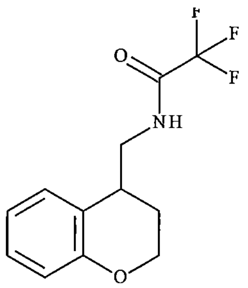

**747**

**Intermédiaire 748 :**

Obtenu à partir de l'intermédiaire **747** selon la procédure utilisée pour la transformation de l'intermédiaire **93a** en intermédiaire **94** (**Protocole XVII**)
**RMN $^1$H** (300MHz, DMSO-$d_6$): 10,30 (s, 1H), 9,70 (m, 1), 7,57 (dd, 1H), 7,48 (m, 1H), 7,00 (t, 1H), 4,39 (m, 1H), 4,28 (m, 1H), 3,55 (m, 1H), 3,40 (m, 1H), 3,09 (m, 1H), 2,02 (m, 1H), 1,85 (m, 1H), 1,48 (d, 3H)
**IR (cm$^{-1}$):** 3300, 1700, 1672

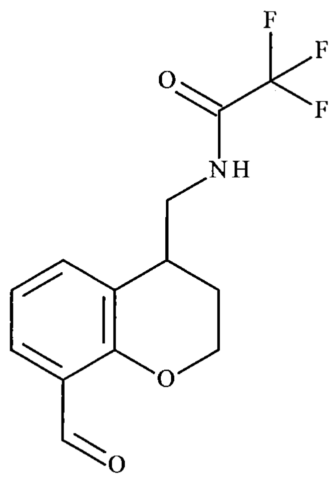

**748**

*Réactions de couplage conduisant aux composés de l'invention*

**Protocole XX :Préparation des composés de formule (I) pour lesquels X représente-C(=O)**

Les composés de formule (I) pour lesquels X représente -C(=O) peuvent être préparés par réaction de couplage *via* un échange halogène metal selon l'exemple de la synthèse de l'intermédiaire **127 :**

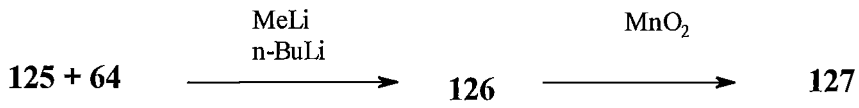


**Intermédiaire 126:**

**(1-{4-[(1-éthoxyisoquinoléin-5-yl)(hydroxy)méthyl]-3,5-difluorophényl}éthyl) carbamate de *tert*-butyle**

A une solution de l'intermédiaire **64** (3 g, 8,92 mmoles) dans 36 mL de THF préalablement refroidie à -78°C sont ajoutés 5,5 mL (8,8 mmoles) d'une solution de MeLi 1,6N dans le pentane, en maintenant une température interne inférieure à -75°C. Après un contact de 15 minutes, 3,54 mL (8,8 mmoles) d'une solution de *n*-BuLi 2,5N dans l'hexane sont ajoutés en maintenant la température interne inférieure à -75°C. Après un contact d'une heure, une solution de l'intermédiaire **125** (1,9 g, 9,83 mmoles) dans 80 mL de THF est ajoutée en maintenant la température inférieure à -75°C. Le mélange est agité à -78°C pendant 1 heure, puis une solution de THF avec 20% d'eau est ajoutée au milieu. Après retour à température ambiante, le milieu est décanté en présence de d'acétate d'éthyle et d'une solution de NaCl saturée. La phase organique est séchée sur $MgSO_4$ L'évaporation sous pression réduite conduit à l'obtention d'une huile, qui est purifiée sur gel de silice (éluant AcOEt/chlorure de méthylène 10/90). L'intermédiaire **126** (2,05 g) est obtenu sous forme d'un solide amorphe incolore.
**RMN $^1$H** (400 MHz, DMSO-$d_6$) : $\delta$ 8,17-8,14 (m, 2H), 7,94 (m, 1H), 7,67 (dd, 1H), 7,38 (d large, 1H), 7,18 (d large, 1H), 6,95 (m, 2H), 6,52 (s large, 1H), 6,34 (s large, 1H), 4,57 (m, 1H), 1,41 (t, 3H), 1,24 (d, 3H), 4,49 (quad, 2H), 1,34-1,15 (2 s large, 9H)
**IR ($cm^{-1}$) :** 3312, 1685, 1161, 1009, 855-804-757

**Intermédiaire 127 :**

**(1-{4-[(1-éthoxyisoquinoléin-5-yl)carbonyl]-3,5-difluorophényl}éthyl)carbamate de *tert*-butyle**

Une solution de l'intermédiaire **126** (2,05 g, 4,47 mmoles) dans 490 mL de chlorure de méthylène est traitée par 7,4 g (85 mmoles) de $MnO_2$, le mélange est agité pendant 20 heures. Le mélange est filtré sur célite®, la célite® est rincée par du $CH_2Cl_2$ puis de l'AcOEt. L'évaporation sous pression réduite conduit à l'obtention de 1,85 g de l'intermédiaire **127** sous la forme d'un solide blanc qui peut être utilisé sans traitement supplémentaire dans l'étape suivante

ou être purifié sur silice (éluant AcOEt/chlorure de méthylène 5/95).
**RMN $^1$H** (300 MHz, DMSO-$d_6$) : δ 8,55 (d, 1H), 8,1-8,2 (dd, 2H), 8,05 (d, 1H), 7,7 (m, 1H), 7,55 (d, 1H), 7,20 (d, 2H), 4,75 (m, 1H), 4,55 (quad, 2H), 1,45 (t, 3H), 1,3-1,45 (d et s, 12H)
**IR (cm$^{-1}$) :** 3362-3309, 1675, 1523, 1255-1160, 856-811-788-752

Cette séquence a été utilisée pour préparer les intermédiaires suivants :

**Intermédiaire 7 :**

**{1-[4-(isoquinoléin-5-ylcarbonyl)phényl]éthyl}carbamate de *tert*-butyle**

Obtenu par oxydation de l'intermédiaire résultant du couplage de **5** et **3**
**RMN $^1$H** (300 MHz, DMSO-$d_6$) : δ 9,45 (s, 1H), 8,55 (d, 1H), 8,35 (d, 1H), 7,95 (d, 1H), 7,75 (m, 4H), 7,50 (d, 1H), 7,45 (d, 2H), 4,70 (m, 1H), 1,35 (s, 9H), 1,30 (d, 3H)
**IR (cm$^{-1}$) :** 1697, 1654

**Intermédiaire 15 :**

**{(1*R*)-1-[4-(isoquinoléin-5-ylcarbonyl)-3-méthylphényl]éthyl}carbamate de *tert*-butyle**

Obtenu par oxydation de l'intermédiaire résultant du couplage de **13** et **3 :**

> **RMN $^1$H** (400 MHz, DMSO-$d_6$) : δ 9,46 (s, 1H), 8,61 (d, 1H), 8,39 (d, 1H), 8,17 (d, 1H), 7,82 (d, 1H), 7,75 (t, 1H), 7,47 (d, 1H), 7,32 (s large, 1H), 7,28 (d, 1H), 7,20 (dl, 1H), 4,65 (m, 1H), 2,39 (s, 3H), 1,38 (s, 9H), 1,33 (d, 3H)
> **IR (cm$^{-1}$) :** 3359, 1680, 1656

**Intermédiaire 34 :**

**[5-(isoquinoléin-5-ylcarbonyl)-6-méthyl-2,3-dihydro-1H-indén-1-yl]carbamate de *tert*-butyle**

Obtenu par oxydation de l'intermédiaire résultant du couplage de **32** et **3**
**RMN $^1$H** (300 MHz, DMSO-$d_6$) : δ 9,45 (s, 1H), 8,60 (d, 1H), 8,35 (d, 1H), 8,20 (d, 1H), 7,80 (m, 1H), 7,70 (m, 1H), 7,20 (s, 1H), 7,15 (s, 1H), 7,10-6,90 (m, 1H), 5,00 (m, 1H), 2,90-2,60 (2m, 2H), 2,35 (m, 1H), 2,30 (s, 3H), 1,85 (m, 1H), 1,40 (s, 9H)

**Intermédiaire 42 :**

**[5-(isoquinoléin-5-ylcarbonyl)-6-méthyl-2,3-dihydro-1*H*-indén-1-yl]carbamate de** *tert*-**butyle**

Obtenu par oxydation de l'intermédiaire résultant du couplage de **40** et **3:**

> **RMN $^1$H** (300 MHz, DMSO-$d_6$) : δ 9,45 (s, 1H), 8,62 (d, 1H), 8,42 (d, 1H), 8,20 (d, 1H), 7,95 (d, 1H), 7,78 (t, 1H), 7,68 (t, 1H), 7,52 (d large, 1H), 7,32 (d, 1H), 7,25 (d, 1H), 4,71 (m, 1H), 1,35 (s, 9H), 1,35 (t, 3H)

**Intermédiaire 51 :**

**{1-[3,5-difluoro-4-(isoquinoléin-5-ylcarbonyl)phényl]éthyl}carbamate de *tert*-butyle**

Obtenu par oxydation de l'intermédiaire résultant du couplage de **64** et **3:**
**RMN $^1$H** (300 MHz, DMSO-$d_6$) : δ 9,50 (s, 1H), 8,70 (d, 1H), 8,63 (d, 1H), 8,50 (d, 1H), 8,10 (d, 1H), 7,80 (t, 1H), 7,52 (d, 1H), 7,25 (d, 2H), 4,70 (m, 1H), 1,40 (s, 9H), 1,35 (d, 3H)
**IR (cm$^{-1}$) :** 3360, 1684, 1667, 1634, 1248-1167, 862-824-761

**Intermédiaire 60c :**

**{1-[3-fluoro-4-(isoquinoléin-5-ylcarbonyl)-5-méthylphényl]éthyl}carbamate de *tert*-butyle**

Obtenu par oxydation de l'intermédiaire résultant du couplage du carbamate de l'intermédiaire **60b** et **3:**

**RMN $^1$H** (300 MHz, DMSO-$d_6$) : δ 9,40 (s, 1H), 8,70 (m, 2H), 8,45 (d, 1H), 7,95 (dd, 1H), 7,80 (t, 1H), 7,45 (m, 1H), 7,20 (d, 1H), 7,10 (dd, 1H), 4,70 (m, 1H), 2,10 (s, 3H), 1,40 (s large, 9H), 1,35 (d, 3H)
**IR (cm$^{-1}$) :** 3500-3080, 1695, 1664, 1619, 1516, 1164, 837-680

**Intermédiaire 75 :**

**{1-[3-chloro-4-(isoquinoléin-5-ylcarbonyl)phényl]éthyl}carbamate de *tert*-butyle**

Obtenu par oxydation de l'intermédiaire résultant du couplage de **73** et **3**
**RMN $^1$H** (300 MHz, DMSO-$d_6$) : δ 9,40 (s, 1H), 8,70 (d, 1H), 8,50 (d, 1H), 8,45 (d, 1H), 7,90 (dd, 1H), 7,75 (t, 1H), 7,60 (d, 1H), 7,50 (s large, 1H), 7,40 (dd, 1H), 4,70 (quint, 1H), 1,40 (m, 12H)
**IR (cm$^{-1}$) :** 3500-3060, 1695, 1664, 1600, 1510, 1163, 834-647

**Intermédiaire 89 :**

**[5-(isoquinoléin-5-ylcarbonyl)-6-méthoxy-1-méthyl-2,3-dihydro-1*H*-indén-1-yl]carbamate de *tert*-butyle**

Obtenu par oxydation de l'intermédiaire résultant du couplage de **87** et **3**
**RMN $^1$H** (300 MHz, DMSO-$d_6$) : δ 9,40 (s, 1H), 8,55 (d, 1H), 8,30 (d large, 1H), 8,20 (d, 1H), 7,80 (d large, 1H), 7,70 (t, 1H), 7,30 (s, 1H), 7,00 (s, 1H), 6,85 (m, 1H), 3,40 (s, 3H), 3,00-2,70 (m, 2H), 2,55 (m, 1H), 2,05 (m, 1H), 1,45 (s, 3H), 1,30 (s, 9H)
**IR (cm$^{-1}$) :** 3390-3240, 1702, 1654

**Intermédiaire 114 :**

**{1-[3-fluoro-4-(isoquinoléin-5-ylcarbonyl)-2-méthylphényl]éthyl}carbamate de *tert*-butyle**

Obtenu par oxydation de l'intermédiaire résultant du couplage de **112** et **3**
**RMN $^1$H** (300 MHz, DMSO-$d_6$) : δ 9,50 (s, 1H), 8,60 (d, 1H), 8,40 (d, 1H), 8,20 (d, 1H), 7,95 (d, 1H), 7,80 (t, 1H), 7,62 (d, 1H), 7,50 (t, 1H), 7,35 (d, 1H), 4,90 (m, 1H), 2,22 (s, 3H), 1,40 (s, 9H), 1,30 (d, 3H)
**IR (cm$^{-1}$) :** 3360, 1680, 1655, 1615, 1525

**Intermédiaire 122 :**

**{1-[8-(isoquinoléin-5-ylcarbonyl)-2,3-dihydro-1,4-benzodioxin-5-yl]éthyl}carbamate de *tert*-butyle**

Obtenu par oxydation de l'intermédiaire résultant du couplage de **120** et **3**
**RMN $^1$H** (400/500 MHz, DMSO-$d_6$) : δ 9,40 (1H), 8,60 (1H), 8,35 (1H), 8,30 (1H), 7,92 (1H), 7,75 (1H), 7,45 (1H), 7,05-7,00 (2H), 4,95 (1H), 4,28-4,00 (4H), 1,40 (9H), 1,30 (3H)
**IR (cm$^{-1}$) :** 3358, 1677, 1246-1161-1058, 833-787-760

**Intermédiaire 137 :**

**{1-[3-chloro-5-fluoro-4-(isoquinoléin-5-ylcarbonyl)phényl]éthyl}carbamate de *tert*-butyle**

Obtenu par oxydation de l'intermédiaire résultant du couplage de **135** et **3**
**RMN $^1$H** (300 MHz, DMSO-$d_6$) : δ 9,50 (s, 1H), 8,80 (d, 1H), 8,50 (d, 1H), 8,02 (d, 1H), 7,80 (t, 1H), 7,53 (d, 1H), 7,45 (s, 1H), 7,38 (d, 1H), 4,72 (m, 1H), 1,40 (s large, 9H), 1,38 (d, 3H), 1,40 (s, 9H), 1,38 (d, 3H)
**IR (cm$^{-1}$) :** 3400, 1675, 1615-1569, 1245-1164, 837-761

**Intermédiaire 160 :**

**{2-[3,5-difluoro-4-(isoquinoléin-5-ylcarbonyl)phényl]propan-2-yl}carbamate de *tert*-butyle**

Obtenu par oxydation de l'intermédiaire résultant du couplage de **158** et **3**
**RMN $^1$H** (400 MHz, DMSO-$d_6$) : δ 9,50 (s, 1H), 8,70 (d, 1H), 8,65 (d, 1H), 8,05 (d large, 1H), 7,80 (t, 1H), 7,50 (d, 1H), 7,35 (s large, 1H), 7,20 (d, 2H), 1,55 (s, 6H), 1,35 (s large, 9H)
**IR (cm$^{-1}$) :** 3256, 3206, 1708, 1663, 1633

**Intermédiaire 165 :**

**[(1*S*)-1-{4-[(1-éthoxyisoquinoléin-5-yl)carbonyl]-3,5-difluorophényl}éthyl]carbamate de *tert*-butyle**

Obtenu par oxydation de l'intermédiaire résultant du couplage de **146** et **125**
**RMN $^1$H** (400/500 MHz, DMSO-$d_6$) : δ 8,55 (d, 1H), 8,20 (d, 1H), 8,15 (d, 1H), 8,05 (d large, 1H), 7,70 (t, 1H), 7,55 (d large, 1H), 7,25 (d, 2H), 4,75 (m, 1H), 4,55 (quad, 2H), 1,45 (t, 3H), 1,40 (s, 9H), 1,35 (d, 3H)
**IR (cm$^{-1}$) :** 3300, 1677, 1260

**Intermédiaire 170 :**

**[(1*R*)-1-{4-[(1-éthoxyisoquinoléin-5-yl)carbonyl]-3,5-difluorophényl}éthyl]carbamate de *tert*-butyle**

Obtenu par oxydation de l'intermédiaire résultant du couplage de **145** et **125**
**RMN $^1$H** (400 MHz, DMSO-$d_6$) : δ 8,55 (d, 1H), 8,20 (d, 1H), 8,15 (d, 1H), 8,05 (d, 1H), 7,75 (m, 1H), 7,55 (d, 1H), 7,25 (m, 2H), 4,75 (m, 1H), 4,55 (quad, 2H), 1,45 (t, 3H), 1,40 (s, 9H), 1,35 (d, 3H)
**IR (cm$^{-1}$) :** 3363, 1681

**Intermédiaire 172 :**

**(2-{4-[(1-éthoxyisoquinoléin-5-yl)carbonyl]-3,5-difluorophényl}propan-2-yl)carbamate de *tert*-butyle**

Obtenu par oxydation de l'intermédiaire résultant du couplage de **158** et **125**
**RMN $^1$H** (400 MHz, DMSO-$d_6$) : δ 8,55 (d, 1H), 8,20-8,10 (dd, 2H), 8,00 (d, 1H), 7,75 (t, 1H), 7,35 (m, 1H), 7,20 (d, 2H), 4,55 (q, 2H), 1,50 (s, 6H), 1,45 (t, 3H), 1,35 (s large, 9H) **IR (cm$^{-1}$) :** 1712, 1682, 1670

**Intermédiaire 176 :**

**{4-[(1-éthoxyisoquinoléin-5-yl)carbonyl]-3,5-difluorobenzyl}carbamate de *tert*-butyle**

Obtenu par oxydation de l'intermédiaire résultant du couplage de **174** et **125**
**RMN $^1$H** (400 MHz, DMSO-$d_6$) : δ 8,54 (dt, 1H), 8,19 (d, 1H), 8,14 (d large, 1H), 8,05 (d large, 1H), 7,71 (dd, 1H), 7,56 (t, 1H), 7,14 (m, 2H), 4,57 (quad, 2H), 4,24 (d, 2H), 1,46 (t, 3H), 1,41 (s large, 9H)
**IR (cm$^{-1}$) :** 3327, 1673, 1251-1160, 1040

**Intermédiaire 178 :**

**(1-{4-[(1-éthoxyisoquinoléin-5-yl)carbonyl]-3-fluoro-2-méthylphényl}éthyl)carbamate de *tert*-butyle**

Obtenu par oxydation de l'intermédiaire résultant du couplage de **112** et **125**
**RMN $^1$H** (400 MHz, DMSO-d6) : δ 8,45 (dt, 1H), 8,10 (d, 1H), 7,90 (d, 1H), 7,70 (m, 1H), 7,65 (m, 1H), 7,60 (d, 1H), 7,50 (t, 1H), 7,30 (d, 1H), 4,85 (m, 1H), 4,55 (q, 2H), 2,20 (s, 3H), 1,45 (t, 3H), 1,35 (s, 9H), 1,30 (d, 3H)
**IR (cm$^{-1}$) :** 3340, 1703, 1660.

**Intermédiaire 180 :**

**(1-{4-[(1-éthoxyisoquinoléin-5-yl)carbonyl]-3-fluorophényl}éthyl)carbamate de *tert*-butyle**

Obtenu par oxydation de l'intermédiaire résultant du couplage de **40** et **125**
**RMN $^1$H** (400 MHz, DMSO-$d_6$) : δ 8,47 (d, 1H), 8,10 (d, 1H), 7,90 (d, 1H), 7,70 (t et m, 2H), 7,65 (d, 1H), 7,55 (d large, 1H), 7,31 (d, 1H), 7,27 (d, 1H), 4,71 (quint, 1H), 4,55 (quad, 2H), 1,48 (t, 3H), 1,45 (d, 3H), 1,40 (s, 9H)
**IR (cm$^{-1}$) :** 3344, 1681, 1655

**Intermédiaire 189 :**

**{2-[3-chloro-4-(isoquinoléin-5-ylcarbonyl)phényl]propan-2-yl}carbamate de *tert*-butyle**

Obtenu par oxydation de l'intermédiaire résultant du couplage de **187** et **3**

**RMN $^1$H** (400 MHz, DMSO-$d_6$) : δ 9.48 (s, 1H), 8,68 (d, 1H), 8.49 (d, 1H), 8.44 (d large, 1H), 7,83 (d large, 1H), 7,76 (t, 1H), 7,57 (d, 1H), 7,48 (s large, 1H), 7.46 (d large, 1H), 7.37 (s large, 1H), 1.55 (s, 6H), 1,35 (s large, 9H)
**IR (cm$^{-1}$) :** 3265, 1707-1657

**Intermédiaire 202 :**

**[(1*R*)-1-{4-[(1-éthoxyisoquinoléin-5-yl)carbonyl]-3-fluoro-2-méthylphényl}éthyl] carbamate de *tert*-butyle**

Obtenu par oxydation de l'intermédiaire résultant du couplage de **200** et **125**
**RMN $^1$H** (400 MHz, DMSO-$d_6$) : δ 8,44 (d, 1H), 8,10 (d, 1H), 7,85 (d, 1H), 7,70 (t, 1H), 7,66 (m, 1H), 7,60 (d, 1H), 7,50 (t, 1H), 7,35 (d, 1H), 4,85 (m, 1H), 4,55 (quad, 2H), 2,20 (s, 3H), 1,47 (t, 3H), 1,38 (s, 9H), 1,30 (d, 3H)
**IR (cm$^{-1}$) :** 3366, 1681, 1670, 1615-1572, 1291-1265-1251-1167, 808-781-755

**Intermédiaire 213 :**

**(1-{4-[(1-éthoxyisoquinoléin-5-yl)carbonyl]-3,5-difluorophényl}éthyl)méthyl carbamate de *tert*-butyle**

Obtenu par oxydation de l'intermédiaire résultant du couplage de **211** et **125**
**RMN $^1$H** (400 MHz, DMSO-$d_6$) : δ 8,55 (d, 1H), 8,20 (d, 1H), 8,20 (d, 1H), 8,05 (d, 1H), 7,71 (t, 1H), 7,15 (d, 2H), 5,30 (m, 1H), 4,59 (quad, 2H), 2,70 (s, 3H), 1,52 (d, 3H), 1,49 (t, 3H), 1,43 (s, 9H)
**IR (cm$^{-1}$) :** 1673, 1632, 1615

**Intermédiaire 219 :**

**[(1*S*)-1-{4-[(1-éthoxyisoquinoléin-5-yl)carbonyl]-3-fluoro-2-méthylphényl}éthyl] carbamate de *tert*-butyle**

Obtenu par oxydation de l'intermédiaire résultant du couplage de **217** et **125**
**RMN $^1$H** (400 MHz, DMSO-$d_6$) : δ 8,44 (d, 1H), 8,09 (d, 1H), 7,87 (dl, 1H), 7,69 (t, 1H), 7,67 (d, 1H), 7,62 (dl, 1H), 7,51 (t, 1H), 7,34 (d, 1H), 4,87 (m, 1H), 4,56 (quad, 2H), 2,21 (s, 3H), 1,46 (t, 3H), 1,37 (s large, 9H), 1,29 (d, 3H)
**IR (cm$^{-1}$) :** 3362, 1738, 1681-1666, 1527, 1291-1165

**Intermédiaire 227 :**

**{(1*S*)-5-[(1-éthoxyisoquinoléin-5-yl)carbonyl]-2,3-dihydro-1*H*-indén-1-yl}carbamate de *tert*-butyle**

Obtenu par oxydation de l'intermédiaire résultant du couplage de **615b** et **125**
**RMN $^1$H** (300 MHz, DMSO-$d_6$) : δ 8,40 (d, 1H), 8,00 (d, 1H), 7,82 (d, 1H), 7,72 (t, 1H), 7,60 (s, 1H), 7,58 (d, 1H), 7,34 (d, 1H), 7,32 (d, 1H), 7,23 (d, 1H), 5,03 (m, 1H), 4,55 (quad, 2H), 2,90 (dd, 1H), 2,80 (dd, 1H), 2,38 (m, 1H), 1,85 (m, 1H), 1,42 (s, 9H) **IR (cm$^{-1}$) :** 3330, 1708-1694, 1656, 1266-1244, 810-756

**Intermédiaire 247 :**

**(1-{4-[(1-éthoxyisoquinoléin-5-yl)carbonyl]-3-fluoro-2-méthoxyphényl}éthyl) carbamate de *tert*-butyle**

Obtenu par oxydation de l'intermédiaire résultant du couplage de **245** et **125**
**RMN $^1$H** (400 MHz, DMSO-$d_6$) : δ 8,45 (d, 1H), 8,10 (d, 1H), 7,95 (d, 1H), 7,70 (m, 2H), 7,55 (d, 1H), 7,40 (m, 1H), 7,30 (d, 1H), 5,00 (m, 1H), 4,55 (quad, 2H), 3,85 (s, 3H), 1,45 (t, 3H), 1,35 (s, 9H), 1,30 (d, 3H)
**IR (cm$^{-1}$) :** 3341, 1702, 1666

**Intermédiaire 257 :**

**(1-{4-[(1-éthoxyisoquinoléin-5-yl)carbonyl]-2,3-diméthylphényl}éthyl)carbamate de *tert*-butyle**

Obtenu par oxydation de l'intermédiaire résultant du couplage de **255** et **125**
**RMN $^1$H** (400 MHz, DMSO-$d_6$) : δ 8,45 (d, 1H), 8,10 (d, 1H), 7,90 (d, 1H), 7,75 (d, 1H), 7,65 (m, 1H), 7,50 (d, 1H), 7,30 (d, 1H), 7,10 (d, 1H), 4,95 (m, 1H), 4,55 (quad, 2H), 2,30 (2s, 6H), 1,45 (t, 3H), 1,35 (s, 9H), 1,25 (d, 3H)
**IR (cm$^{-1}$) :** 3346, 1701, 1659

**Intermédiaire 273 :**

**[(1*R*)-1-{4-[(1-éthoxyisoquinoléin-5-yl)carbonyl]-3-méthylphényl}éthyl]carbamate de *tert*-butyle**

Obtenu par oxydation de l'intermédiaire résultant du couplage de **13** et **125**
**RMN $^1$H** (300/400 MHz, DMSO-$d_6$) : δ 8,42 (d, 1H), 8,06 (d, 1H), 7,75 (d, 1H), 7,70 (t, 1H), 7,60 (d, 1H), 7,45 (dl, 1H), 7,30 (s large, 1H), 7,25 (d, 1H), 7,18 (d large, 1H), 4,65 (quint, 1H), 4,55 (quad, 2H), 2,40 (s, 3H), 1,45 (t, 3H), 1,40 (s large, 9H), 1,32 (d, 3H) **IR (cm$^{-1}$) :** 3336, 1697, 1657

**Intermédiaire 312 :**

**(1-{2-éthoxy-4-[(1-éthoxyisoquinoléin-5-yl)carbonyl]-3-fluorophényl}éthyl)carbamate de *tert*-butyle**

Obtenu par oxydation de l'intermédiaire résultant du couplage de **310** et **125**
**RMN $^1$H** (400 MHz, DMSO-$d_6$) : δ 8,45 (d, 1H), 8,10 (d, 1H), 7,90 (d, 1H), 7,70 (m, 2H), 7,55 (d, 1H), 7,35 (m, 1H), 7,30 (d, 1H), 5,00 (m, 1H), 4,55 (quad, 2H), 4,05 (m, 2H), 1,45 (t, 3H), 1,40 (s, 9H), 1,35 (d, 3H), 1,30 (d, 3H)
**IR (cm$^{-1}$) :** 3348, 1710, 1661

**Intermédiaire 315 :**

**[(1*S*)-1-{4-[(1-éthoxyisoquinoléin-5-yl)carbonyl]-3-méthylphényl}éthyl]carbamate de *tert*-butyle**

Obtenu par oxydation de l'intermédiaire résultant du couplage de **313** et **125**
**RMN $^1$H** (400 MHz, DMSO-$d_6$) : δ 8,45 (d, 1H), 8,05 (d, 1H), 7,80 (d, 1H), 7,70 (m, 1H), 7,60 (d, 1H), 7,45 (d, 1H), 7,30 (s, 1H), 7,25 (d, 1H), 7,20 (dd, 1H), 4,65 (m, 1H), 4,55 (quad, 2H), 2,40 (s, 3H), 1,45 (t, 3H), 1,35 (s, 9H), 1,30 (d, 3H)
**IR (cm$^{-1}$) :** 3392-3355, 1685, 1649

**Intermédiaire 351 :**

**[(1*R*)-1-{8-[(1-éthoxyisoquinoléin-5-yl)carbonyl]-2,3-dihydro-1,4-benzodioxin-5-yl}éthyl]carbamate de *tert*-butyle**

Obtenu par oxydation de l'intermédiaire résultant du couplage de **349b** et **125**
**RMN $^1$H** (400 MHz, DMSO-$d_6$) : δ 8,41 (d, 1H), 8,07 (d, 1H), 7,85 (d, 1H), 7,77 (d, 1H), 7,65 (dd, 1H), 7,42 (d, 1H), 7,03 (d, 1H), 6,98 (d, 1H), 4,93 (m, 1H), 4,55 (quad, 2H), 4,25 (m, 2H), 4,02 (m, 2H), 1,45 (t, 3H), 1,38 (s, 9H), 1,26 (d, 3H)
**IR (cm$^{-1}$) :** 3346, 1703, 1656, 1614

**Intermédiaire 373 :**

**[(1*S*)-1-{8-[(1-éthoxyisoquinoléin-5-yl)carbonyl]-2,3-dihydro-1,4-benzodioxin-5-yl}éthyl]carbamate de *tert*-butyle**

Obtenu par oxydation de l'intermédiaire résultant du couplage de **371** et **125**
**RMN $^1$H** (400 MHz, DMSO-$d_6$) : δ 8,41 (d, 1H), 8,07 (d, 1H), 7,85 (d, 1H), 7,76 (d, 1H), 7,66 (dd, 1H), 7,43 (d, 1H), 7,03 (d, 1H), 6,98 (d, 1H), 4,93 (m, 1H), 4,55 (quad, 2H), 4,25 (m, 2H), 4,02 (m, 2H), 1,45 (t, 3H), 1,38 (s, 9H), 1,26 (d, 3H)
**IR (cm$^{-1}$) :** 3346, 1703, 1656, 1614

**Intermédiaire 391 :**

**[(1S)-1-{8-[(1-éthoxyisoquinoléin-5-yl)carbonyl]-2,3-dihydro-1,4-benzodioxin-5-yl}éthyl]carbamate de *tert*-butyle**

Obtenu par oxydation de l'intermédiaire résultant du couplage de **13** et **294**
**RMN $^1$H** (400 MHz, DMSO-$d_6$) : δ 8,36 (d, 1H), 7,69 (d, 1H), 7,56 (t, 1H), 7,51 (s, 1H), 7,44 (d large, 1H), 7,30 (s, 1H), 7,24 (d, 1H), 7,18 (d, 2H), 4,64 (m, 1H), 4,54 (quad, 2H), 2,46 (s, 3H), 2,38 (s, 3H), 1,45 (t, 3H), 1,37 (s large, 9H), 1,32 (d, 3H)
**IR (cm$^{-1}$) :** 3350, 1677, 1658

**Intermédiaire 408 :**

**[(1*S*)-1-{4-[(1-éthoxy-4-méthylisoquinoléin-5-yl)carbonyl]-3-méthylphényl}éthyl] carbamate de *tert*-butyle**

Obtenu par oxydation de l'intermédiaire résultant du couplage de **313** et **301**
**RMN $^1$H** (400 MHz, DMSO-$d_6$) : δ 8,40 (d, 1H), 7,85 (s, 1H), 7,70 (t, 1H), 7,60 (dd, 1H), 7,45 (d, 1H), 7,35 (s, 1H), 7,25 (d, 1H), 7,15 (dd, 1H), 4,65 (m, 1H), 4,50 (quad, 2H), 2,60 (s, 3H), 2,10 (s, 3H), 1,50 (t, 3H), 1,35 (m, 9H), 1,30 (d, 3H)
**IR (cm$^{-1}$) :** 3410, 1700, 1662

**Intermédiaire 462 :**

**[(1*R*)-1-{4-[(1-éthoxy-4-méthylisoquinoléin-5-yl)carbonyl]-3-méthylphényl}éthyl] carbamate de *tert*-butyle**

Obtenu par oxydation de l'intermédiaire résultant du couplage de **13** et **302**
**RMN $^1$H** (400 MHz, DMSO-$d_6$) : δ 8,40 (d, 1H), 7,85 (s, 1H), 7,70 (t, 1H), 7,60 (dd, 1H), 7,45 (d, 1H), 7,35 (s, 1H), 7,25 (d, 1H), 7,15 (dd, 1H), 4,65 (m, 1H), 4,50 (quad, 2H), 2,60 (s, 3H), 2,10 (s, 3H), 1,50 (t, 3H), 1,35 (m, 9H), 1,30 (d, 3H)
**IR (cm$^{-1}$) :** 3335, 1698, 1662

**Intermédiaire 542 :**

**[(1*R*)-1-{4-[(1-éthoxyisoquinoléin-5-yl)carbonyl]-3-fluoro-5-méthoxyphényl}éthyl] carbamate de *tert*-butyle**

Obtenu par oxydation de l'intermédiaire résultant du couplage de **540** et **125**
**RMN $^1$H** (400 MHz, DMSO-$d_6$) : δ 8,5 (d, 1H), 8,25 (d, 1H), 8,2 (d, 1H), 7,9 (d, 1H), 7,7 (t, 1H), 7,5 (d, 1H), 7,0 (s, 1H), 6,9 (d, 1H), 4,7 (m, 1H), 4,55 (quad, 2H), 3,7 (s, 3H), 1,45 (t, 3H), 1,4 (s, 9H), 1,35 (d, 3H)
**IR (cm$^{-1}$) :** 3389, 1680, 1168

**Intermédiaire 591 :**

**{1-[4-(isoquinoléin-5-ylcarbonyl)phényl]éthyl}carbamate de *tert*-butyle**

Obtenu par oxydation de l'intermédiaire résultant du couplage de **589** et **3**
**RMN $^1$H** (300 MHz, DMSO-$d_6$) : δ 9,45 (s, 1H), 8,55 (d, 1H), 8,35 (d, 1H), 7,95 (d, 1H), 7,75 (m, 4H), 7,50 (d, 1H), 7,45 (d, 2H), 4,70 (m, 1H), 1,35 (s, 9H), 1,30 (d, 3H)
**IR (cm$^{-1}$) :** 1697, 1654

**Intermédiaire 596 :**

**{(1*S*)-1-[4-(isoquinoléin-5-ylcarbonyl)phényl]éthyl}carbamate de *tert*-butyle**

Obtenu par oxydation de l'intermédiaire résultant du couplage de **594** et **3**
**RMN $^1$H** (300 MHz, DMSO-$d_6$) : δ 9,45 (s, 1H), 8,55 (d, 1H), 8,35 (d, 1H), 7,95 (d, 1H), 7,75 (m, 4H), 7,50 (d, 1H), 7,45 (d, 2H), 4,70 (m, 1H), 1,35 (s, 9H), 1,30 (d, 3H)
**IR (cm$^{-1}$) :** 1697, 1654

**Intermédiaire 603 :**

**{2-[4-(isoquinoléin-5-ylcarbonyl)phényl]propan-2-yl}carbamate de *tert*-butyle**

Obtenu par oxydation de l'intermédiaire résultant du couplage de **601** et **3**
**RMN $^1$H** (300 MHz, DMSO-$d_6$) : δ 9,50 (s, 1H), 8,55 (d, 1H), 8,35 (d, 1H), 7,90 (d large, 1H), 7,80 (m, 2H), 7,75-7,5 (dd, 4H), 7,30 (m, 1H), 1,55 (s, 6H), 1,30 (m, 9H)
**IR (cm$^{-1}$) :** 1697, 1654
**LCMS [M+H]**+= 391

**Intermédiaire 610 :**

**{1-[4-(isoquinoléin-5-ylcarbonyl)phényl]cyclobutyl)carbamate de *tert*-butyle**

Obtenu par oxydation de l'intermédiaire résultant du couplage de **608** et **3**
**RMN $^1$H** (300 MHz, DMSO-$d_6$) : δ 9,50 (s, 1H), 8,55 (d, 1H), 8,40 (d, 1H), 7,9-7,7 (m, 4H), 7,8-7,5 (dd, 4H), 2,40 (m, 4H), 2,05-1,8 (m, 2H), 1,30 (m, 9H)
**IR (cm$^{-1}$) :** 1697, 1654
**LCMS [M+H]**+= 403

**Intermédiaire 617 :**

**[(1*R*)-5-(isoquinoléin-5-ylcarbonyl)-2,3-dihydro-1*H*-indén-1-yl]carbamate de *tert*-butyle**

Obtenu par oxydation de l'intermédiaire résultant du couplage de **615a** et **3**
**RMN $^1$H** (300 MHz, DMSO-$d_6$) : δ 9,50 (s, 1H), 8,55 (d, 1H), 8,35 (d, 1H), 7,90 (d, 1H), 7,80 (dd, 1H), 7,80 (d, 1H), 7,65 (d et s, 2H), 7,35 (t large, 1H), 5,05 (m, 1H), 2,9-2,8 (m, 2H), 2,4-1,85 (m, 2H), 1,45 (s, 9H)
**IR (cm$^{-1}$) :** 3300, 1692-1654
**Pureté optique** (Colonne OJ-H, éluant : n-propylalcool/heptane/ diéthylamine : 10/90/0,1, détection 270nm) : 99%.

**Intermédiaire 619 :**

**[(1*S*)-5-(isoquinoléin-5-ylcarbonyl)-2,3-dihydro-1*H*-indén-1-yl]carbamate de *tert*-butyle**

Obtenu par oxydation de l'intermédiaire résultant du couplage de **615b** et **3**
**RMN $^1$H** (300 MHz, DMSO-$d_6$) : δ 9,40 (s, 1H), 8,55 (d, 1H), 8,35 (d, 1H), 7,90 (d, 1H), 7,85 (d, 1H), 7,80 (d, 1H), 7,65 (m, 2H), 7,35 (d, 1H), 5,05 (m, 1H), 3,0-2,7 (2m, 2H), 2,41-1,9 (2m, 2H), 1,45 (s, 9H)
**IR (cm$^{-1}$) :** 3300, 1692-1654
Pureté optique (Colonne OJ-H, éluant : n-propylalcool/ heptane/diéthylamine 10/90/0,1, détection 270nm) : > 98%

Intermédiaire 625 :

**{4-[4-(isoquinoléin-5-ylcarbonyl)phényl]tétrahydro-2*H*-pyran-4-yl}carbamate** de *tert*-butyle

Obtenu par oxydation de l'intermédiaire résultant du couplage de 623 et 3
RMN $^1$H (400 MHz, DMSO-$d_6$) : δ 9,45 (s, 1H), 8,54 (d, 1H), 8,38 (d, 1H), 7,92 (d, 1H), 7,80 (d et t, 2H), 7,76/7,55 (2d, 4H), 7,40 (s large, 1H, NH), 3,70 (m, 4H), 2,2/1,9 (2m, 4H), 1,35 (s large, 9H)
**IR (cm$^{-1}$) :** 3300, 1708-1695, 1655

**Intermédiaire 635 :**

**[5-(isoquinoléin-5-ylcarbonyl)-1-méthyl-2,3-dihydro-1*H*-indén-1-yl]carbamate de *tert*-butyle**

Obtenu par oxydation de l'intermédiaire résultant du couplage de **633** et **3**
**RMN $^1$H** (300 MHz, DMSO-$d_6$) : δ 9,40 (s, 1H), 8,55 (d, 1H), 8,35 (d, 1H), 7,90 (d, 1H), 7,80 (m, 2H), 7,60 (m, 2H), 7,35 (d, 1H), 7,20 (s large, 1H, NH), 3,00-2,8 (m, 2H), 2,55-1,95 (2m, 2H), 1,40 (s, 3H), 1,30 (s large, 9H)
**IR (cm$^{-1}$) :** 3340-3230, 1700, 1653, 1616

**Intermédiaire 637 :**

**{(1*S*)-1-[4-(isoquinoléin-5-ylcarbonyl)-3-méthylphényl]éthyl}carbamate de *tert*-butyle**

Obtenu par oxydation de l'intermédiaire résultant du couplage de 313 et 3
**RMN $^1$H** (400 MHz, DMSO-$d_6$): δ 9,46 (s, 1H), 8,61 (d, 1H), 8,39 (d, 1H), 8,17 (d, 1H), 7,82 (d, 1H), 7,75 (t, 1H), 7,47 (d, 1H), 7,32 (s large, 1H), 7,28 (d, 1H), 7,20 (dl, 1H), 4,65 (m, 1H), 2,39 (s, 3H), 1,38 (s, 9H), 1,33 (d, 3H)

**Intermédiaire 647 :**

**[5-(isoquinoléin-5-ylcarbonyl)-6-méthoxy-2,3-dihydro-1*H*-indén-1-yl]carbamate de *tert*-butyle**

Obtenu par oxydation de l'intermédiaire résultant du couplage de **645** et **3**
**RMN $^1$H** (300 MHz, DMSO-$d_6$) : δ 9,30 (d, 1H), 8,55 (d, 1H), 8,30 (d, 1H), 8,25 (d, 1H), 7,80 (d, 1H), 7,70 (t, 1H), 7,35 (s, 1H), 7,10 (m, 1H, NH), 6,95 (s, 1H), 5,00 (m, 1H), 3,45 (s, 3H), 2,75-2,90 (2m, 2H), 2,40 (m, 1H), 1,90 (m, 1H), 1,45 (s, 9H)
**IR (cm$^{-1}$) :** 3400-3200, 1700, 1651

**Intermédiaire 686 :**

**{1-[4-(isoquinoléin-5-ylcarbonyl)-3-méthoxyphényl]éthyl}carbamate** de ***tert*-butyle**

Obtenu par oxydation de l'intermédiaire résultant du couplage de **684** et **3**
**RMN $^1$H** (400 MHz, DMSO-$d_6$) : δ 9,40 (s, 1H), 8,60 (d, 1H), 8,35 (d, 1H), 8,25 (d, 1H), 7,80 (d, 1H), 7,70 (t, 1H), 7,45 (d, 2H), 7,10 (s, 1H), 7,05 (d, 1H), 4,70 (tl, 1H), 3,50 (s, 3H), 1,40 (s, 9H), 1,35 (d, 3H)
**IR (cm$^{-1}$) :** 3400-3150, 1702-1656, 1607, 1243, 1164, 1032, 832-760

**Intermédiaire 718 :**

**(1-{4-[(1-éthoxyisoquinoléin-5-yl)carbonyl]-3-fluoro-2-(2-méthylpropoxy)phényl} éthyl)carbamate de *tert*-butyle**

Obtenu par oxydation de l'intermédiaire résultant du couplage de l'intermédiaire tert-butylcarbamate de **714** et **125**
**RMN $^1$H** (300 MHz, DMSO-$d_6$) : δ 8,45 (d, 1H), 8,10 (d, 1H), 7,90 (d, 1H), 7,70 (t et m, 2H), 7,53 (d, 1H, NH), 7,34 (dd, 1H), 7,32 (d, 1H), 5,02 (m, 1H), 4,68 (quad,, 2H), 3,78 (m, 2H), 2,03 (m, 1H), 1,46 (t, 3H), 1,36 (s large, 9H), 1,30 (d, 3H), 0,98 (d, 6H)
**IR (cm$^{-1}$) :** 3354, 1704, 1662, 1264, 1161, 813, 759

**Intermédiaire 728 :**

**(1-{2-(benzyloxy)-4-[(1-éthoxyisoquinoléin-5-yl)carbonyl]-5-fluorophényl}éthyl) carbamate de *tert*-butyle**

Obtenu par oxydation de l'intermédiaire résultant du couplage de **726** et **125**
**RMN $^1$H** (400 MHz, DMSO-$d_6$) : δ 8,46 (d, 1H), 8,09 (d, 1H), 7,90 (dl, 1H), 7,70 (dd, 1H), 7,66 (dl, 1H), 7,51 (dl, 1H, NH), 7,45-7,30 (m, 6H), 5,21 (dd, 2H), 5,08 (m, 1H), 4,58 (quad, 2H), 1,48 (t, 3H), 1,39 (s, 9H), 1,30 (d, 3H)
**IR (cm$^{-1}$)** : 3350, 1679, 1657, 1619

**Intermédiaire 757 :**

**[(1*R*)-1-{4-[(8-chloroisoquinoléin-5-yl)carbonyl]phényl)éthyl]carbamate de *tert*-butyle**

Obtenu par oxydation de l'intermédiaire résultant du couplage de **5** et **755**
**RMN $^1$H** (300 MHz, DMSO-$d_6$) : δ 9,70 (s, 1H), 8,68 (d, 1H), 7,95 (d, 1H), 7,90 (d, 1H), 7,88 (d, 1H), 7,77 (d, 2H), 7,53 (d, 1H, NH), 7,47 (d, 2H), 4,70 (quint,, 1H), 1,36 (s, 9H), 1,32 (d, 3H)
**IR (cm$^{-1}$) :** 3378, 1674, 1669, 1245, 1168, 1062, 836

**Intermédiaire 182 :**

Obtenu en deux étapes à partir de l'intermédiaire **75**

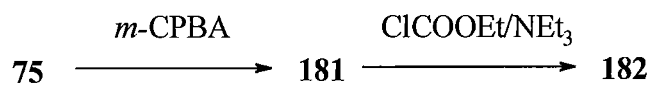

**Intermédiaire 181 :**

**(1-{3-chloro-4-[(2-oxydoisoquinoléin-5-yl)carbonyl]phényl}éthyl)carbamate de *tert*-butyle**

L'intermédiaire **75,** traité selon le protocole décrit pour l'intermédiaire **123,** a été transformé en **181.**

**MS (DEI 70 eV) = 426,1**

**Intermédiaire 182 :**

**(1-{3-chloro-4-[(1-éthoxyisoquinoléin-5-yl)carbonyl]phényl}éthyl)carbamate de *tert*-butyle**

A une solution de l'intermédiaire **181** (1,1 g, 2,57 mmoles) dans l'éthanol (680 mL), à température ambiante sont ajoutés successivement du chloroformiate d'éthyle (0,9 mL) puis après 5 minutes de la $NEt_3$ (1,9 mL), Le milieu réactionnel est agité 10minutes à température ambiante, puis du chloroformiate d'éthyle (0,9 mL) est ajouté à nouveau. Après 10 minutes d'agitation à température ambiante le mélange réactionnel est concentré sous vide. Le résidu repris par de l'eau est extrait en présence de chlorure de méthylène. La phase organique est séchée sur $MgSO_4$ et concentrée. Par chromatographie sur silice (éluant $CH_2Cl_2$/AcOEt 100/0 à 90/10) l'intermédiaire **182** est obtenu (0,5 g)
**RMN $^1$H** (400 MHz, DMSO-$d_6$) : δ 8,50 (d, 1H), 8,15 (d, 1H), 8,00 (d, 1H), 7,80 (d, 1H), 7,70 (m, 1H), 7,55 (d, 1H), 7,55 (s large, 1H), 7,50 (s, 1H), 7,40 (d, 1H), 4,70 (m, 1H), 4,55 (quad, 2H), 1,45 (t, 3H), 1,40 (s, 9H), 1,35 (d, 3H)
**IR ($cm^{-1}$) :** 3336, 1694, 1668
Cette procédure a été appliquée pour préparé l'intermédiaire **191** issu de l'intermédiaire **189 :**

**Intermédiaire 191 :**

**(2-{3-chloro-4-[(1-éthoxyisoquinoléin-5-yl)carbonyl]phényl}propan-2-yl)carbamate de *tert*-butyle**

**RMN $^1$H** (400 MHz, DMSO-$d_6$) : δ 8,48 (d, 1H), 8,15 (d, 1H), 7,96 (d, 1H), 7,78 (d large, 1H), 7,69 (t, 1H), 7,54 (d, 1H), 7,46 (s large, 1H), 7,45 (d large, 1H), 7,36 (s large, 1H), 4,57 (quad, 2H), 1,54 (s, 6H), 1,46 (t, 3H), 1,35 (s large, 9H)
**IR ($cm^{-1}$) :** 3350, 1698, 1666, 1243-1159

**Intermédiaire 395 :**

Obtenu en trois étapes à partir de l'intermédiaire **127**

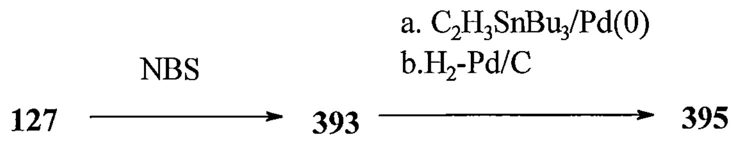


**Intermédiaire 393 :**

**(1-{4-[(4-bromo-1-éthoxyisoquinoléin-5-yl)carbonyl]-3,5-difluorophényl}éthyl) carbamate de *tert*-butyle**

A une solution de l'intermédiaire **127** (2,7 g, 5,9 mmoles) dans le DMF (65 mL) est ajouté de du-*N*-bromosuccinimide (1 g, 6 mmoles). Le mélange réactionnel est agité à température ambiante pendant 24 heures. Le mélange est repris par de l'AcOEt et de l'eau, la phase organique est lavée par une aqueuse saturée en NaCl, séchée sur $MgSO_4$ et concentrée. Par chromatographie sur silice (éluant $CH_2Cl_2$/AcOEt 100/0 à 95/5) l'intermédiaire **393** est obtenu sous forme d'un solide amorphe (1,35 g).
**RMN $^1$H** (400 MHz, DMSO-$d_6$) : δ 8,43 (d, 1H), 8,31 (s, 1H), 7,86 (d, 1H), 7,74 (t, 1H), 7,52 (d large, 1H), 7,16 (d, 2H), 4,69 (m, 1H), 4,56 (quad, 2H), 1,46 (t, 3H), 1,37 (s large, 9H), 1,30 (d, 3H)
**IR ($cm^{-1}$) :** 3346, 1708, 1678

**Intermédiaire 395 :**

**(1-{4-[(1-éthoxy-4-éthylisoquinoléin-5-yl)carbonyl]-3,5-difluorophényl}éthyl) carbamate de *tert*-butyle**

Etape 1 :

A une solution dégazée par $N_2$, de l'intermédiaire **393** (1,6 g) dans le DMF (33 mL) sont ajoutés du vinyl-tributylétain (0,89 mL) et du $Pd(PPh_3)_4$ (56 mg). Le mélange est chauffé à 100°C pendant 5 heures. Après retour à température ambiante, le milieu est traité par une solution de aqueuse KF à 10%, les sels sont filtrés et le filtrat est extrait par de l'AcOEt. La phase organique est séchée sur $MgSO_4$ et concentrée sous vide. Le produit attendu est obtenu par chromatographie sur silice (éluant $CH_2Cl_2$/AcOEt 100/0 à 90/10) sous forme d'un solide amorphe (0,9 g).
**RMN $^1H$** (400 MHz, DMSO-$d_6$) : δ 8,45 (d, 1H), 8,09 (s, 1H), 7,85 (d, 1H), 7,70 (t, 1H), 7,51 (d, 1H), 7,13 (d, 2H), 6,61 (dd, 1H), 5,43 (d, 1H), 5,08 (d, 1H), 4,67 (m, 1H), 4,57 (quad, 2H), 1,46 (t, 3H), 1,37 (s large, 9H), 1,30 (d, 3H)
**IR ($cm^{-1}$) :** 3344, 1710, 1673, 1631

Etape 2 :

Une solution de l'intermédiaire obtenu à l'étape 1 ci-dessus (0,94 g, 1,95 mmoles) dans de l'éthanol (90 mL) est hydrogénée à pression atmosphérique d'$H_2$ et à température ambiante en présence de Pd/C 10% (0,2 g) pendant 24 heures. Une nouvelle charge de Pd/C à 10% est ajoutée et le mélange est hydrogéné 24 heures supplementaires, le catalyseur est alors filtré et le filtrat concentré sous vide. Par chromatographie sur silice (éluant $CH_2Cl_2$/cyclohexane 20/80 à 100/0 puis $CH_2Cl_2$/AcOEt 95/5) l'intermédiaire **395** est obtenu sous forme d'un solide amorphe (0,5 g).
**RMN $^1H$** (300 MHz, DMSO-$d_6$) : δ 8,45 (d, 1H), 8,00 (s, 1H), 7,80 (d, 1H), 7,65 (t, 1H), 7,50 (d large, 1H), 7,20 (d, 2H), 4,70 (m, 1H), 4,55 (quad, 2H), 2,65 (quad, 2H), 1,50 (t, 3H), 1,35 (s large, 9H), 1,32 (d, 3H), 1,20 t, 3H)
**IR ($cm^{-1}$) :** 3340, 1678

L'intermédiaire **433** a été obtenu par chloration (au *N*-chlorosuccinimide) de l'intermédiaire **170** ou (1-{4-[(4-chloro-1-éthoxyisoquinoléin-5-yl)carbonyl]-3,5-difluorophényl}éthyl)carbamate de *tert*-butyle.

**Intermédiaire 433 :**

**(1-{4-[(4-chloro-1-éthoxyisoquinoléin-5-yl)carbonyl]-3,5-difluorophényl}éthyl) carbamate de *tert*-butyle**

A une solution de **170** (1 g, 2,19 mmoles) dans $CH_3CN$ (30 mL) est ajouté du *N*-chlorosuccinimide (0,3 g, 2,3 mmoles). Le mélange réactionnel est chauffé au reflux pendant 24 heures, puis le solvant est évaporé sous vide. Le résidu repris par de l'AcOEt est lavé par de l'eau, la phase organique est séchée sur $MgSO_4$ et concentrée. Par chromatographie sur silice (éluant $CH_2Cl_2$/AcOEt 98/2) l'intermédiaire **433** est obtenu sous forme d'un solide amorphe (0,6 g).
**RMN $^1H$** (400 MHz, DMSO-$d_6$) : δ 8,43 (dd, 1H), 8,18 (s, 1H), 7,88 (d, 1H), 7,77 (t, 1H), 7,52 (d, 1H), 7,16 (d, 2H), 4,69 (m, 1H), 4,57 (quad, 2H), 1,47 (t, 3H), 1,38 (s, 9H), 1,31 (d, 3H)
**IR ($cm^{-1}$) :** 3350, 1712-1680

**Intermédiaire 434 :**

**[(1*S*)-1-{4-[(4-chloro-1-éthoxyisoquinoléin-5-yl)carbonyl]-3,5-difluorophényl}éthyl] carbamate de *tert*-butyle**

Obtenu à partir de **165** selon le protocole décrit pour la préparation de l'intermédiaire **433.**
**RMN $^1H$** (400 MHz, DMSO-$d_6$) : δ 8,43 (dd, 1H), 8,18 (s, 1H), 7,88 (d, 1H), 7,77 (t, 1H), 7,52 (d, 1H), 7,16 (d, 2H), 4,69 (m, 1H), 4,57 (quad, 2H), 1,47 (t, 3H), 1,38 (s, 9H), 1,31 (d, 3H)
**IR ($cm^{-1}$) :** 3500-3400, 1679-1632

**Intermédiaire 688 :**

**{1-[3-hydroxy-4-(isoquinoléin-5-ylcarbonyl)phényl]éthyl}carbamate de *tert*-butyle**

Obtenu en deux étapes, par traitement de l'intermédiaire **686** avec du $BBr_3$ dans le chlorure de méthylène, et un traitement de l'intermédiaire phénol formé **687** en présence de $Boc_2O$ et de $NEt_3$
**RMN $^1H$** (400 MHz, DMSO-$d_6$) : δ 11,30, 9,50, 8,60, 8,40, 7,95, 7,90, 7,82, 7,50, 7,32, 6,97, 6,88, 4,62,1,40, 1,35
**IR ($cm^{-1}$)** : 3330,1699
**LCMS [M+H]**+= 392

**Intermédiaire 230 :**

**(4-bromo-2,5-difluorophényl)(isoquinoléin-5-yl)méthanone**

Obtenu par oxydation de l'intermédiaire **229** résultant du couplage de **228** et **125** selon le protocole suivant:

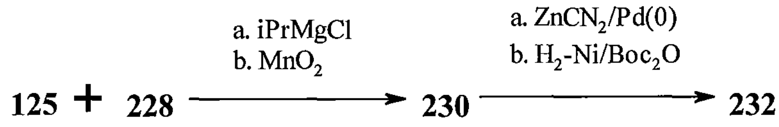


**125** + **228** **230** **232**

Etape 1 :

On solubilise 500 mg (1,83 mmoles) de 1,4-dibromo-2,5-difluorobenzène **228** dans 10 mL de THF anhydre. On refroidit à -50°C sous argon et on ajoute goutte à goutte 1 mL (2 mmoles, 1,1 éq.) d'une solution 2M dans le THF d'isopropyl chlorure de magnésium. La température est maintenue entre -50°C et -40°C. On laisse agiter 30 minutes à -50°C et on contrôle l'avancement de la réaction par HPLC (environ 75%). On ajoute alors, toujours à - 50°C, une solution de 0,37 g (1,83 mmoles, 1 éq.) de **125** dans 3 mL de THF anhydre. On laisse remonter à -10°C. Un contrôle de la réaction par HPLC montre la formation de 68% de l'alcool désiré. On hydrolyse avec 15 mL d'HCl 1N. On extrait avec 3 fois 25 mL d'éther, la phase organique est lavée avec une solution aqueuse saturée de NaCl, sèchée sur $MgSO_4$, filtrée et évaporée à sec. Le résidu obtenu cristallise dans du dichlorométhane. Le solide est alors filtré et séché pour donner 280 mg de produit attendu sous la forme d'un solide. Les jus de cristallisations sont évaporés à sec et purifiés par flash-chromatographie sur 40 g de silice, éluant : gradient $CH_2Cl_2$-AcOEt : 99-1 à 90-10 pour donner 200 mg de l'intermédiaire purifié.
**RMN $^1$H** (400/500 MHz, DMSO-$d_6$) : δ 8,18 (d, 1H), 8,01 (d, 1H), 7,73 (d, 1H), 7,61 (t, 1H), 7,67 (dd, 1H), 7,53 (d, 1H), 7,47 (dd, 1H), 6,51 (d, 1H), 6,41 (d, 1H), 4,52 (quad, 2H), 1,45 (t, 3H).
**IR ($cm^{-1}$) :** 1621

Etape 2 :

L'oxydation au $MnO_2$ de l'intermédiaire obtenu ci-dessus conduit à l'intermédiaire **230:**
**RMN $^1$H** (400 MHz, DMSO-$d_6$) : δ 8,50 (d, 1H), 8,13 (d, 1H), 8,01 (d, 1H), 7,93 (dd, 1H), 7,82 (d, 1H), 7,73 (dd, 1H), 7,70 (t, 1H), 4,58 (quad, 2H), 1,46 (t, 3H)
**IR ($cm^{-1}$) :** 3211, 1652

**Intermédiaire 232 :**

**[3-fluoro-4-(isoquinoléin-5-ylcarbonyl)benzyl]carbamate de *tert*-butyle**

Obtenu à partir de **230** selon le protocole suivant:

Etape 1 :

On met en solution dans 2 mL de DMF anhydre dégazée à l'argon 220 mg (0,558 mmoles) de l'intermédiaire **230.** On ajoute à cette solution, sous argon, 11 mg (0,055 mmoles, 0,1 éq.) de CuI, 92 mg (0,558 mmoles, 1 éq.) d'iodure de potassium et 20 mg (0,11 mmole, 0,2 éq.) de phénantroline. On chauffe à 110°C pendant 18h. Un contrôle HPLC montre une conversion à 90% en dérivé iodé. On ajoute alors 36 mg (0,0558 mmoles, 1 éq.) de cyanure de potassium et on continue de chauffer à 110°C pendant 3 heures. La conversion est incomplète et il reste 20% de dérivé iodé. On rajoute 5 mg (0,077 mmoles, 0,13 éq.) de KCN. On chauffe encore 3 heures à 110°C. On refroidit le milieu réactionnel et on hydrolyse sur 10 mL d'eau. Le mélange est filtré, le solide est rincé 3 fois à l'eau. Puis on ajoute au filtrat une solution aqueuse ammoniacale à 5% et de l'acétate d'éthyle. Un solide précipite. Il est filtré, lavé avec de l'acétate d'éthyle et de l'eau. La phase acétate d'éthyle est décantée, lavée à l'eau puis par une solution aqueuse saturée de NaCl. La phase organique est ensuite séchée sur $MgSO_4$, filtrée et évaporée pour donner 170 mg d'un résidu qui est purifié par flash-chromatographie sur 12 g de silice, éluant = $CH_2Cl_2$ 100% pour donner 100 mg de l'intermédiaire attendu.
**RMN $^1$H** (400 MHz, DMSO-$d_6$) : δ 8,55 (d, 1H), 8,20 (dd, 1H), 8,20 (d, 1H), 8,05 (m, 3H), 7,95 (dd, 1H), 7,70 (m, 1H), 4,60 (quad, 2H), 1,45 (t, 3H)
**IR ($cm^{-1}$) :** 2244, 1663

Etape 2 :

La réduction de l'intermédiaire obtenu à l'étape 1 selon le protocole décrit pour l'intermédiaire **223** conduit à l'intermédiaire **232**
**RMN $^1$H** (400 MHz, DMSO-$d_6$): δ 8,49 (d, 1H), 8,10 (d, 1H), 7,95 (d, 1H), 7,72 (d, 1H), 7,70 (t, 1H), 7,52 (m, 1H), 7,51 (m, 1H), 7,20 (m, 1H), 4,55 (quad, 2H), 4,22 (m, 2H), 1,45 (t, 3H), 1,40 (s, 9H)
**RMN $^{19}$F :** -123, -117
**IR (cm$^{-1}$) :** 3351, 1676-1662.

**Intermédiaire 730 :**

**(1-{2-[2-(diméthylamino)éthoxy]-4-[(1-éthoxyisoquinoléin-5-yl)carbonyl]-5-fluorophényl}éthyl)carbamate de *tert*-butyle**

L'intermédiaire **730** a été obtenu en deux étapes à partir de **728 :**

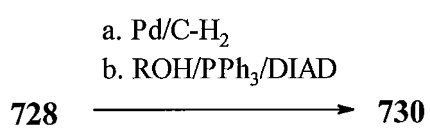


Etape 1 :

L'intermédiaire **728** ou (1-{2-(benzyloxy)-4-[(1-éthoxyisoquinoléin-5-yl)carbonyl]-5-fluorophényl}éthyl)carbamate de *tert*-butyle a été transformé en dérivé phénolique correspondant selon les conditions décrites pour l'obtention du composé **553** (intermédiaire de **555**).

Etape 2 :

L'intermédiaire phénolique obtenu ci-dessus (1 g, 2,2 mmoles) est mis en solution dans le THF et en présence de diméthyl-éthanol-amine commerciale (0,22 mL) est traité par de la tri-phénylphosphine (0,87 g, 3,3 mmoles) et du diisopropylazodicarboxylate (0,65 mL, 3,3 mmoles). le mélange réactionnel a été agité à température ambiante pendant 3 jours. Après concentration sous vide, le résidu est chromatographié sur silice (éluant : $CH_2Cl_2$/EtOH 90/10), le solide obtenu est recristallisé dans du méthanol. L'intermédiaire **730** est obtenu sous forme d'un solide (0,36 g).
**RMN $^1$H** (300 MHz, DMSO-$d_6$) : δ 8,45 (d, 1H), 8,10 (d, 1H), 7,90 (d, 1H), 7,70 (m, 2H), 7,55 (d, 1H, NH), 7,30 (d, 1H), 7,20 (d, 1H), 5,00 (quint, 1H), 4,60 (quad, 2H), 4,10 (t, 2H), 2,65 (m, 2H), 2,20 (s, 6H), 1,50 (t, 3H), 1,40 (s large, 9H), 1,30 (d, 3H)
**IR (cm$^{-1}$) :** 3410, 1679, 1630, 1533, 1166
Les intermédiaires cétones obtenus par le protocole **XX** ont été déprotégés en milieu acide pour conduire aux produits finaux, tel que dans l'exemple du produit **P110:**

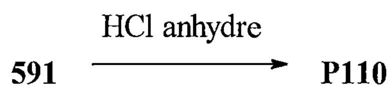


**Produit P110 :**

**Chlorhydrate de [4-(1-aminoéthyl)phényl](isoquinoléin-5-yl)méthanone**

A une solution de l'intermédiaire **591** (2,9 g, 7,7 mmoles) dans de l'$Et_2O$ (60 mL) est ajouté une solution d'éther chlorhydrique 2N (30 mL). La suspension résultante est agitée à température ambiante pendant 4 jours. Le précipité collecté sur fritté est séché sous vide, le produit **P110** est obtenu sous forme d'un solide blanc (2,05 g).
Les intermédiaires cétones 1-éthoxyisoquinoléin-5-yl ont été déprotégés ensuite selon l'un des deux exemples décrits ci-dessous pour **P17** et **P68:**

**Produit P17 :**

**Chlorhydrate de [4-(1-aminoéthyl)-2,6-difluorophényl](1-hydroxyisoquinoléin-5-yl)méthanone**

Un mélange de 11,86 g (36 mmoles) de l'intermédiaire **127** dans 710 mL d'HCl 4N est chauffé à 80°C pendant 48h. Après contrôle HPLC, le milieu est refroidit à 0°C et le solide est filtré sur fritté ce qui conduit à l'obtention de 8,2 g de produit **P17** sous forme d'un solide blanc (1,1% de produit de déamination).

**Produit P68 :**

**Méthanesulfonate de 5-{4-[(1*R*)-1-aminoéthyl]-2-méthylbenzoyl}-3-méthylisoquinoléin-1(2*H*)-one**

Une solution de l'intermédiaire **391** (0,7g, 1,58 mmoles) dans un mélange 1,4-dioxane/eau (14 mL/4 mL) et de l'acide méthane sulfonique (0,51 mmoles) est chauffé à 80°C pendant 24 heures. Le mélange réactionnel est concentré sous vide et le résidu est repris par du $CH_3CN$. Le solide résultant est filtré et séché sous vide. Le produit **P68** est obtenu sous forme d'un solide.

| Produit | Issu de | Nomenclature<br>Description analytique |
|---|---|---|
| **P1** | **7** | **Dichlorhydrate de {4-[(1*R*)-1-aminoéthyl]phényl)(isoquinoléin-5-yl)méthanone**<br>**RMN $^1$H** (400 MHz, DMSO-$d_6$) : $\delta$ 9,80 (s, 1H), 8,75 (m, 3H), 8,65 (2d, 2H), 8,20 (d, 1H), 8,15 (d, 1H), 8,00 (t, 1H), 7,88 (t, 2H), 7,75 (d, 2H), 4,50 (m, 1H), 1,55 (d, 3H)<br>**IR (cm$^{-1}$) :** 3000-2000, 1650<br>**HRMS (ESI) :** m/z théorique pour $C_{18}H_{17}N_2O$ $[M+H]^+$ 277,1341, mesuré 277,1357<br>**Pureté optique :** (Colonne ADH $\mu$m 4,6x250mm, éluant : EtOH/triéthylamine 1000/1, détection : 265nm) : > 99%. (absence de **P111**) |
| **P2** | **15** | **Chlorhydrate de {4-[(1*R*)-1-aminoéthyl]-2-méthylphényl}(isoquinoléin-5-yl)méthanone**<br>**RMN $^1$H** (400 MHz, DMSO-$d_6$) : $\delta$ 9,88 (s, 1H), 8,74 (m, 4H), 8,68 (d, 1H), 8,59 (d, 1H), 8,04 (d, 1H), 7,98 (t, 1H), 7,62 (s large, 1H), 7,48 (d large, 1H), 7,42 (d, 1H), 4,46 (m, 1H), 2,43 (s, 3H), 1,56 (d, 3H)<br>**IR (cm$^{-1}$) :** 3000-2000, 1654<br>**HRMS (ESI) :** m/z théorique pour $C_{19}H_{19}N_2O$ $[M+H]^+$ 291,1497, mesuré 291,1525<br>**Pureté optique :** (Colonne ADH 5$\mu$m 4,6x250mm, éluant : EtOH/diéthylamine 100/0,1, détection : 270nm) : > 99%.<br>(absence de **P121**)<br>$\alpha_D$ (589nM) = 7 (c = 0,004 g/mL, MeOH) à 20°C |
| **P4** | **34** | **Dichlorhydrate de (1-amino-6-méthyl-2,3-dihydro-1*H*-indén-5-yl)(isoquinoléin-5-yl) méthanone**<br>**RMN $^1$H** (300 MHz, DMSO-$d_6$) : $\delta$ 9,85 (s, 1H), 8,88-8,55 (3d, 3H), 8,75 (m, 3H), 8,00 (d et t, 2H), 7,70 (s, 1H), 7,30 (s, 1H), 5,05 (m, 1H), 4,75 (m, 1H), 3,00-2,80 (2m, 2H), 2,40 (s, 3H), 2,50-2,02 (2m, 2H)<br>IR **(cm$^{-1}$) :** 3100-2200, 1657<br>**HRMS (ESI) :** m/z théorique pour $C_{20}H_{19}N_2O$ $[M+H]^+$ 303,1497, mesuré 303,1511 |
| **P5** | **42** | **Dichlorhydrate de [4-(1-aminoéthyl)-2-fluorophényl](isoquinoléin-5-yl)méthanone**<br>**RMN $^1$H** (300 MHz, DMSO-$d_6$) : $\delta$ 9,95 (s, 1H), 9,00 (s large, 3H), 8,80 (2dd, 2H), 8,62 (d, 1H), 8,21 (dd, 1H), 8,05 (t, 1H), 7,80 (m, 1H), 7,72-7,58 (m, 2H), 7-6,50 (m, 1H), 4,60 (m, 1H), 1,60 (d, 3H)<br>**IR (cm$^{-1}$) :** 3000-2000, 1663<br>**HRMS (ESI) :** m/z théorique pour $C_{18}H_{16}F_1N_2O$ $[M+H]^+$ 295,1247, mesuré 295,1249 |

(suite)

| Produit | Issu de | Nomenclature<br>Description analytique |
|---|---|---|
| **P6** | **51** | **Dichlorhydrate de [4-(1-aminoéthyl)-2,6-difluorophényl](isoquinoléin-5-yl)méthanone**<br>**RMN $^1$H** (300 MHz, DMSO-$d_6$) : δ 9,70 (s, 1H) 8,90 (d, 1H) 8,80 (d etm, 4H) 8,70 (d, 1H) 8,25 (d, 1H) 7,95 (t, 1H) 7,60 (d, 2H) 4,60 (sept, 1H) 1,60 (d, 3H)<br>**IR (cm$^{-1}$) :** 3280-2000, 1673<br>**HRMS (ESI) :** m/z théorique pour $C_{18}H_{15}F_2N_2O$ [M+H]$^+$ 313,1152, mesuré 313,1167 |
| **P8** | **60c** | **Dichlorhydrate de [4-(1-aminoéthyl)-2-fluoro-6-méthylphényl](isoquinoléin-5-yl) méthanone**<br>**RMN $^1$H** (300 MHz, DMSO-$d_6$) : δ 9,90 (m, 1H), 9,10 (m, 1H), 8,80 (m, 4H), 8,70 (d, 2H), 8,60 (d, 1H), 8,00 (t, 1H), 7,50 (s et d, 2H), 4,50 (m, 1H), 2,30 (s, 3H), 1,60 (d, 3H)<br>**IR (cm$^{-1}$) :** 3385-1900, 1660<br>**HRMS (ESI) :** m/z théorique pour $C_{19}H_{18}F_1N_2O$ [M+H]$^+$ 309,1403, mesuré 309,1403 |
| **P11** | **75** | **Dichlorhydrate de [4-(1-aminoéthyl)-2-chlorophényl](isoquinoléin-5-yl)méthanone**<br>**RMN $^1$H** (300 MHz, DMSO-$d_6$) : δ 9,90 (s et d et m, 5H), 8,80 (d, 1H), 8,70 (d, 2H), 8,10 (d,1H), 8,00 (t, 1H), 7,90 (s large, 1H), 7,70 (m, 2H), 4,55 (m, 1H), 1,60 (d, 3H)<br>**IR (cm$^{-1}$) :** 3290-1910, 1663<br>**HRMS (ESI) :** m/z théorique pour $C_{18}H_{16}Cl_1N_2O$ [M+H]$^+$ 310,0873, mesuré 310,0859 |
| **P12** | **89** | **Dichlorhydrate de (1-amino-6-méthoxy-1-méthyl-2,3-dihydro-1*H*-indén-5-yl)(isoquinoléin-5-yl)méthanone**<br>**RMN $^1$H** (300 MHz, DMSO-$d_6$) : δ 9,60 (s, 1H), 8,70 (m, 3H), 8,65 (d, 1H), 8,44 (d, 2H), 7,90 (d, 1H), 7,82 (t, 1H), 7,60 (s, 1H), 7,45 (s, 1H), 3,10-2,85 (m, 4H), 2,40-2,20 (m, 2H), 1,65 (s, 3H)<br>**IR (cm$^{-1}$) :** 2800-2056, 1654<br>**HRMS (ESI) :** m/z théorique pour $C_{21}H_{21}N_2O_2$ [M+H]$^+$ 333,1603, mesuré 333,1616 |
| **P15** | **114** | **Dichlorhydrate de [4-(1-aminoéthyl)-2-fluoro-3-méthylphényl](isoquinoléin-5-yl) méthanone**<br>**RMN $^1$H** (300 MHz, DMSO-$d_6$) : δ 9,95 (s, 1H), 8,80 (m, 4H), 8,25 (d, 1H), 8,52 (d, 1H), 8,10 (d, 1H), 7,95 (t, 1H), 7,65 (m, 2H), 4,70 (m, 1H), 2,78 (s, 3H), 1,55 (d, 3H)<br>**IR (cm$^{-1}$) :** 3400-2200, 1668<br>**HRMS (ESI) :** m/z théorique pour $C_{19}H_{18}FN_2O$ [M+H]$^+$ 309,1403, mesuré 309,1391 |
| **P16** | **122** | **Dichlorhydrate de [8-(1-aminoéthyl)-2,3-dihydro-1,4-benzodioxin-5-yl](isoquinoléin-5-yl) méthanone**<br>**RMN $^1$H** (300 MHz, DMSO-$d_6$) : δ 9,90 (s, 1H), 8,75 (d, 1H), 8,70 (m, 6H), 8,15 (d, 1H), 8,00 (t, 1H), 7,25 (d, 1H), 7,20 (d, 1H), 4,60 (d, 1H), 4,30 (m, 2H), 4,05 (m, 2H), 1,55 (d, 3 H)<br>**IR** (**cm$^{-1}$**) : 3200-2400, 165<br>**HRMS (ESI) :** m/z théorique pour $C_{20}H_{19}N_2O_3$ [M+H]$^+$ 335,1396, mesuré 335,1392 |
| **P17** | **127** | **Chlorhydrate de** [**4**-(1-**aminoéthyl**)-**2**,**6**-**difluorophényl**](1-**hydroxyisoquinoléin**-**5**-**yl**) **méthanone**<br>**RMN $^1$H** (300 MHz, DMSO-$d_6$) : δ 11,60 (s large, 1H), 8,52 (dd, 1H), 8,50 (m, 3H), 7,90 (dd, 1H), 7,58 (t, 1H), 7,40 (s, 2H), 7,50 (d, 2H), 4,53 (quad, 1H), 1,55 (d, 3H)<br>**IR (cm$^{-1}$) :** 3000-2500, 1674<br>**HRMS (ESI) :** m/z théorique pour $C_{18}H_{15}F_2N_2O_2$ [M+H]$^+$ 329,1102, mesuré 329,1102 |
| **P18** | **137** | **Dichlorhydrate de [4-(1-aminoéthyl)-2-chloro-6-fluorophényl](isoquinoléin-5-yl) méthanone**<br>**RMN $^1$H** (300 MHz, DMSO-$d_6$) : δ 10,80 (s, 1H), 9,05 (d, 1H), 8,70 (d, 1H), 8,85 (d large, 4H), 8,20 (d, 1H), 7,95 (t, 1H), 7,80 (s, 1H), 7,75 (d, 1H), 4,55 (t, 1H), 1,60 (d, 3H)<br>**IR (cm$^{-1}$) :** 3100-2400, 1675<br>**HRMS (ESI) :** m/z théorique pour $C_{18}H_{15}ClFN_2O$ [M+H]$^+$ 329,0857, mesuré 329,0846 |

(suite)

| Produit | Issu de | Nomenclature<br>Description analytique |
|---|---|---|
| P24 | 160 | **Dichlorhydrate de [4-(2-aminopropan-2-yl)-2,6-difluorophényl**](**isoquinoléin-5-yl**) **méthanone**<br>**RMN $^1$H** (400 MHz, DMSO-$d_6$) : δ 9,84 (s, 1H), 9,10 (m, 4H), 8,94 (d, 1H), 8,82 (d, 1H), 7,73 (d, 1H), 8,32 (d, 1H), 7,99 (t, 1H), 7,64 (d, 2H), 1,71 (s, 6H)<br>**IR (cm$^{-1}$) :** 3200-2300, 1674.<br>**HRMS (ESI) :** m/z théorique pour $C_{19}R_{17}F_2N_2O$ $[M+H]^+$ 327,1309, mesuré 327,129 |
| P25 | 165 | **Chlorhydrate de {4-[(1*S*)-1-aminoéthyl]-2,6-difluorophényl)(1-hydroxyisoquinoléin-5-yl) méthanone**<br>**RMN $^1$H** (400 MHz, DMSO-$d_6$) : δ 11,70 (s, 1H), 8,90 (s large, 3H), 8,55 (d, 1H), 7,90 (d, 1H), 7,60 (m, 3H), 7,40 (s, 2H), 4,55 (quad, 1H), 1,60 (d, 3H)<br>**IR (cm$^{-1}$) :** 3200-2300, 1673, 1631<br>**HRMS (ESI) :** m/z théorique pour $C_{18}H_{15}F_2N_2O_2$ $[M+H]^+$ 329,1102, mesuré 329,1112<br>**Pureté optique** (SFC: Chiralpak ID 3µM 4,6x250 mm ; éluant: $CO_2$/ (isopropanol/diéthylamine: 100/0,5) : 65 / 35 ; détection:255nm) :>99%.<br>(absence de **P26**) |
| P26 | 170 | **Chlorhydrate de {4-[(1*R*)-1-aminoéthyl]-2,6-difluorophényl}(1-hydroxyisoquinoléin-5-yl) méthanone**<br>**RMN $^1$H** (400 MHz, DMSO-$d_6$) : δ 11,70 (m, 1H), 8,80 (m, 3H), 8,55 (d, 1H), 7,90 (d, 1H), 7,55 (t et m, 3H), 7,40 (m, 2H), 4,55 (quad, 1H), 1,60 (d, 3H)<br>**IR (cm$^{-1}$) :** 3500-3000, 2863, 1673<br>**HRMS (ESI) :** m/z théorique pour $C_{18}H_{15}F_2N_2O_2$ $[M+H]^+$ 329,1102, mesuré 329,1119<br>**Pureté optique** (SFC: Chiralpak ID 3µM 4,6x250 mm; éluant: $CO_2$/ (isopropanol/diéthylamine: 100/0,5): 65 / 35 ; détection: 255nm) :>99%.<br>(absence de **P25**)<br>**[P26** sous forme de méthane sulfonate $\alpha_D$ (589nM) = +2,48 (c = 0,01 g/mL, MeOH) à 20°C |
| P27 | 172 | **Chlorhydrate de 5-[4-(2-aminopropan-2-yl)-2,6-difluorobenzoyl]isoquinoléin-1(2*H*)-one**<br>**RMN $^1$H** (300 MHz, DMSO-$d_6$) : δ 11,70 (m, 1H), 9,00 (m, 3H), 8,55 (dd, 1H), 7,90 (dd, 1H), 7,60 (t et d, 3H), 7,40 (m, 2H), 1,70 (s large, 6H)<br>**IR (cm$^{-1}$) :** 3400-2400, 1677, 1631<br>**HRMS (ESI) :** m/z théorique pour $C_{19}H_{17}F_2N_2O_2$ $[M+H]^+$ 343,1258, mesuré 343,1271 |
| P28 | 176 | **Chlorhydrate de [4-(aminométhyl)-2,6-difluorophényl](1-hydroxyisoquinoléin-5-yl) méthanone**<br>**RMN $^1$H** (400 MHz, DMSO-$d_6$): δ 11,67 (s large, 1H), 8,55 (s large, 3H), 8,54 (dd, 1H), 7,88 (d large, 1H), 7,58 (t, 1H), 7,50 (m, 2H), 7,40 (m, 2H), 4,16 (s, 2H)<br>**IR (cm$^{-1}$) :** 3250-1950, 1671<br>**HRMS (ESI) :** m/z théorique pour $C_{17}H_{13}F_2N_2O_2$ $[M+H]^+$ 314,0867, mesuré 314,0865 |
| P29 | 178 | **Chlorhydrate de 5-[4-(1-aminoéthyl)-2-fluoro-3-méthylbenzoyl]isoquinoléin-1(2*H*)-one**<br>**RMN $^1$H** (400 MHz, DMSO-$d_6$) : δ 11,60 (m, 1H), 9,00-8,50 (m, 3H), 8,45 (dd, 1H), 7,75 (dd, 1H), 7,70-7,55 (2m, 3H), 7,30 (m, 1H), 6,95 (d, 1H), 4,65 (quad, 1H), 2,25 (s, 3H), 1,55 (d, 3H)<br>**IR (cm$^{-1}$) :** 3300-2000, 1668, 1629<br>MS **(DEI 70 eV) :** m/z mesuré pour $C_{19}H_{18}FN_2O_2$ 324,10 |
| P30 | 180 | **Chlorhydrate de 5-[4-(1-aminoéthyl)-2-fluorobenzoyl]isoquinoléin-1(2*H*)-one**<br>**RMN $^1$H** (400 MHz, DMSO-$d_6$) : δ 11,56 (s large, 1H), 8,60 (s large, 3H), 8,46 (d, 1H), 7,76 (d, 1H), 7,73 (t, 1H), 7,60-7,50 (m, 3H), 7,30 (dd, 1H), 6,91 (d, 1H), 4,53 (quad, 1H), 1,55 (d, 3H)<br>**IR (cm$^{-1}$) :** 3200-2400, 1684-1659, 1625<br>**HRMS (ESI) :** m/z théorique pour $C_{18}H_{16}FN_2O_2$ $[M+H]^+$ 311,1196, mesuré 311,1205 |

(suite)

| Produit | Issu de | Nomenclature<br>Description analytique |
|---|---|---|
| **P31** | **182** | **Chlorhydrate de 5-[4-(1-aminoéthyl)-2-chlorobenzoyl]isoquinoléin-1(2*H*)-one**<br>**RMN $^1$H** (400 MHz, DMSO-$d_6$) : δ 11,62 (s large, 1H), 8,58 (s large, 3H), 8,49 (d, 1H), 7,80 (s, 1H), 7,66 (m, 3H), 7,55 (t, 1H), 7,37 (d large 1H), 7,28 (d, 1H), 4,53 (quad, 1H), 1,56 (d, 3H)<br>**IR (cm$^{-1}$) :** 3300-2500, 1661, 1631<br>**HRMS (ESI) :** m/z théorique pour $C_{18}H_{16}ClN_2O_2$ [M+H]$^+$ 327,0900, mesuré 327,0902 |
| **P32** | **191** | **Chlorhydrate de 5-[4-(2-aminopropan-2-yl)-2-chlorobenzoyl]isoquinoléin-1(2*H*)-one**<br>**RMN $^1$H** (400 MHz, DMSO-$d_6$) : δ 11,63 (d, 1H), 8,82 (s large, 3H), 8,49 (d, 1H), 7,81 (s large, 1H), 7,68 (m, 3H), 7,55 (t, 1H), 7,38 (dd, 1H), 7,29 (d, 1H), 1,69 (s, 6H)<br>**IR (cm$^{-1}$) :** 3500-2400, 1662-1651, 1623<br>**HRMS (ESI) :** m/z théorique pour $C_{19}H_{18}ClN_2O_2$ [M+H]$^+$ 341,1057, mesuré 341,1062 |
| **P33** | **202** | **Chlorhydrate de 5-{4-[(1*R*)-1-aminoéthyl]-2-fluoro-3-méthylbenzoyl}isoquinoléin-1 (2*H*)-one**<br>**RMN $^1$H** (400 MHz, DMSO-$d_6$) : δ 11,60 (d, 1H), 8,80-8,60 (m, 3H), 8,45 (d, 1H), 7,75 (d, 1H), 7,65-7,55 (m, 3H), 7,30 (m, 1H), 6,95 (d, 1H), 4,65 (m, 1H), 2,25 (s, 3H), 1,50 (d, 3H)<br>**IR (cm$^{-1}$) :** 3500-2000, 1650-1630<br>**HRMS (ESI) :** m/z théorique pour $C_{19}H_{18}FN_2O_2$ [M+H]$^+$ 325,1352, mesuré 325,1354.<br>**Pureté optique** (Colonne ASH 5μM 4,6x250 mm ; éluant: EtOH/ heptane/diéthylamine: 70/30/0,1; détection: 620nm) : >99%. (absence **P36**) |
| **P34** | **189** | **Dichlorhydrate de [4-(2-aminopropan-2-yl)-2-chlorophényl](isoquinoléin-5-yl)méthanone**<br>**RMN $^1$H** (400 MHz, DMSO-$d_6$) : δ 9,90 (s, 1H), 9,40-8,90 (m, 3H), 8,90 (d, 1H), 8,80 (d, 1H), 8,70 (d large, 1H), 8,05 (dd, 1H), 7,95 (t, 1H), 7,90 (s, 1H), 7,75 (m, 2H), 1,70 (s 6H)<br>**IR (cm$^{-1}$) :** 3200-2000, 1662<br>**HRMS (ESI) :** m/z théorique pour $C_{19}H_{18}ClN_2O$ [M+H]$^+$ 325,1108, mesuré 325,1116 |
| **P35** | **213** | **Chlorhydrate de 5-{2,6-difluoro-4-[1-(méthylamino)éthyl]benzoyl}isoquinoléin-1(2*H*)-one**<br>**RMN $^1$H** (400 MHz, DMSO-$d_6$) : δ 11,68 (s, 1H), 9,68 (s large, 2H), 8,54 (dd, 1H), 7,98 (d large, 1H), 7,59 (m, 2H), 7,58 (t, 1H), 7,42 (m, 2H), 4,44 (quad, 1H), 2,47 (s, 3H), 1,61 (d, 3H)<br>**IR (cm$^{-1}$) :** 3250-3200, 1672-1668, 1632<br>**HRMS (ESI) :** m/z théorique pour $C_{19}H_{17}F_2N_2O_2$ [M+H]$^+$ 343,1258, mesuré 343,1258 |
| **P36** | **219** | **Chlorhydrate de 5-{4-[(1*S*)-1-aminoéthyl]-2-fluoro-3-méthylbenzoyl)isoquinoléin-1 (2*H*)-one**<br>**RMN $^1$H** (400 MHz, DMSO-$d_6$) : δ 11,50 (d, 1H), 8,57 (s large, 3H), 8,46 (d, 1H), 7,73 (d, 1H), 7,59 (m, 2H), 7,58 (t, 1H), 7,30 (m, 1H), 6,92 (d, 1H), 4,65 (quad, 1H), 2,27 (s, 3H), 1,50 (d, 3H)<br>**IR (cm$^{-1}$) :** 3500-2000, 1650-1630<br>**HRMS (ESI) :** m/z théorique pour $C_{19}H_{18}FN_2O_2$ [M+H]$^+$ 325,1352, mesuré 325,1363.<br>**Pureté optique** (Colonne ASH5μM 4,6x250 mm; éluant: EtOH/ heptane/ diéthylamine:70/30/0,1; détection: 620nm): >99%. (absence **P36**) |
| **P37** | **223** | **Chlorhydrate de 5-[4-(aminométhyl)-2-fluorobenzoyl]isoquinoléin-1(2*H*)-one**<br>**RMN $^1$H** (400 MHz, DMSO-$d_6$) : δ 11,55 (d, 1H), 8,60 (m, 3H), 8,47 (d, 1H), 7,77 (d, 1H), 7,70 (t, 1H), 7,60-7,49 (m, 2H), 7,58 (t, 1H), 7,30 (m, 1H), 6,90 (d, 1H), 4,15 (s, 2H)<br>**IR (cm$^{-1}$) :** 2970, 1680-1655, 1622<br>**HRMS (ESI) :** m/z théorique pour $C_{17}H_{14}FN_2O_2$ [M+H]$^+$ 297,1093, mesuré 297,1024 |

(suite)

| Produit | Issu de | Nomenclature<br>Description analytique |
|---|---|---|
| **P39** | **227** | **Chlorhydrate de 5-{[(1*S*)-1-amino-2,3-dihydro-1*H*-indén-5-yl]carbonyl}isoquinoléin-1 (2*H*)-one**<br>**RMN $^1$H** (400 MHz, DMSO-$d_6$) : δ 11,50 (s, 1H), 8,55 (s large, 3H), 8,40 (d, 1H), 7,78 (d, 1H), 7,70 (d, 1H), 7,65 (m, 2H), 7,60 (t, 1H), 7,20 (d, 1H), 6,40 (m, 1H), 4,78 (t, 1H), 3,10-2,90 (m, 2H), 2,50-2,00 (m, 2H)<br>**IR (cm$^{-1}$) :** 3300-2400, 1687-1665<br>**HRMS (ESI) :** m/z théorique pour $C_{19}H_{17}N_2O_2$ [M+H]$^+$ 305,1290, mesuré 305,1311.<br>**Pureté optique** (Colonne ASH 5μM 4,6x250 mm ; éluant: heptane/propanol/diéthylamine: 70/30/0,1; détection: 270nm) : >99%. |
| **P40** | **232** | **Chlorhydrate de 5-[4-(aminométhyl)-2,5-difluorobenzoyl]isoquinoléin-1(2*H*)-one**<br>**RMN $^1$H** (400 MHz, DMSO-$d_6$) : δ 11,60 (s, 1H), 8,60 (s large, 3H), 8,49 (d, 1H), 7,80 (d, 1H), 7,70-7,60 (m, 2H), 7,59 (t, 1H), 7,30 (m, 1H), 7,00 (d, 1H), 4,15 (s, 2H)<br>**IR (cm$^{-1}$) :** 3200-2400, 1689-1658<br>**HRMS (ESI) :** m/z théorique pour $C_{17}H_{13}F_2N_2O_2$ [M+H]$^+$ 315,0945, mesuré 315,0934 |
| **P42** | **247** | **Chlorhydrate de 5-[4-(1-aminoéthyl)-2-fluoro-3-méthoxybenzoyl]isoquinoléin-1(2R)-one**<br>**RMN $^1$H** (400 MHz, DMSO-$d_6$) : δ 12,60 (s, 1H), 8,65 (s large, 3H), 8,50 (d, 1H), 7,80 (d, 1H), 7,55 (m, 2H), 7,45 (m, 1H), 7,30 (d, 1H), 7,00 (d, 1H), 4,65 (q, 1H), 3,95 (s, 3H), 1,50 (d, 3H)<br>**IR (cm$^{-1}$) :** 3359-2437, 1690, 1656<br>**HRMS (ESI) :** m/z théorique pour $C_{19}H_{18}FN_2O_3$ [M+H]$^+$ 341,1031, mesuré 341,1318 |
| **P43** | **257** | **Chlorhydrate de 5-[4-(1-aminoéthyl)-2,3-diméthylbenzoyl]isoquinoléin-1(2*H*)-one**<br>**RMN $^1$H** (400 MHz, DMSO-d6) : δ 11,58 (d large, 1H), 8,57 (m, 3H), 8,46 (d, 1H), 7,63 (d, 1H), 7,52 (m, 2H), 7,34 (dd, 1H), 7,22 (d, 1H), 7,18 (d, 1H), 4,71 (m, 1H), 2,31 (s, 3H), 2,20 (s, 3H), 1,51 (d, 3H)<br>**IR (cm$^{-1}$) :** 3300-2400, 1642, 1626<br>**HRMS (ESI) :** m/z théorique pour $C_{20}H_{21}N_2O_2$ [M+H]$^+$ 321,1603, mesuré 321,1594 |
| **P47** | **273** | **Chlorhydrate de 5-{4-[(1*R*)-1-aminoéthyl]-2-méthylbenzoyl}isoquinoléin-1(2*H*)-one**<br>**RMN $^1$H** (400 MHz, DMSO-$d_6$) : δ 11,60-11,50 (m, 1H), 8,60-8,40 (m, 3H), 8,45 (m, 1H), 7,65 (dd, 1H), 7,60 (m, 1H), 7,50 (m, 1H), 7,40 (dd, 1H), 7,35 (d, 1H), 7,30 (m, 1H), 6,90 (d, 1H), 4,45 (quad, 1H), 2,35 (s, 3H), 1,55 (d, 3H)<br>**IR (cm$^{-1}$) :** 3500, 3300-1950, 1685, 1653<br>**HRMS (ESI) :** m/z théorique pour $C_{19}H_{19}N_2O_2$ [M+H]$^+$ 307,1441, mesuré 307,1455<br>m/z théorique pour $C_{19}H_{16}NO_2$ [M+H-$NH_3$]$^+$ 290,1176, mesuré 290,1175<br>**Pureté optique :** (Colonne AD 5μm 4,6x250mm, éluant : EtOH/Heptane /Diéthylamine 40/60/0,1, détection : 255nm) : > 99%, (absence de **P52**)<br>$\alpha_D$ (589nM) = 5,7 (c = 0,01 g/mL, MeOH) à 20°C |
| **P51** | **312** | **Chlorhydrate de 5-(4-(1-aminoéthyl)-3-éthoxy-2-fluorobenzoyl]isoquinoléin-1(2H)-one**<br>**RMN $^1$H** (400 MHz, DMSO-$d_6$) : δ 11,60 (m, 1H), 8,70-8,30 (m, 3H), 8,50 (d, 1H), 7,80 (d, 1H), 7,65 (m, 1H), 7,50 (m, 1H), 7,40 (m, 1H), 7,30 (d, 1H), 6,95 (d, 1H), 4,70 (quad, 1H), 4,15 (q, 2H), 1,50 (d, 3H), 1,30 (t, 3H)<br>**IR (cm$^{-1}$) :** 3200-2300, 1667, 1626<br>**HRMS (ESI) :** m/z théorique pour $C_{20}H_{20}FN_2O_3$ [M+H]$^+$ 355,1489, mesuré 355,1489 |
| **P52** | **315** | **Chlorhydrate de 5-{4-[(1*S*)-1-aminoéthyl]-2-méthylbenzoyl}isoquinoléin-1(2*H*)-one**<br>**RMN $^1$H** (400 MHz, DMSO-$d_6$) : δ 11,60-11,50 (m, 1H), 8,60-8,40 (m, 3H), 8,45 (m, 1H), 7,65 (dd, 1H), 7,60-7,50 (2m, 2H), 7,40 (dd, 1H), 7,35 (d, 1H), 7,30 (m, 1H), 6,90 (d, 1H), 4,45 (quad, 1H), 2,35 (s, 3H), 1,55 (d, 3H) |

(suite)

| Produit | Issu de | Nomenclature<br>Description analytique |
|---|---|---|
| | | **IR ($cm^{-1}$) :** 3200-2340, 1652, 1615<br>**HRMS (ESI) :** m/z théorique pour $C_{19}H_{19}N_2O_2$ $[M+H]^+$ 307,1447, mesuré 307,1455.<br>**Pureté optique :** (Colonne AD 5µm 4,6x250mm, éluant : EtOH/Heptane /Diéthylamine 40/60/0,1, détection : 255nm) : > 99%. (absence de **P47**) |
| **P61** | **351** | **Chlorhydrate de 5-({8-[(1*R*)-1-aminoéthyl]-2,3-dihydro-1,4-benzodioxin-5-yl}carbonyl) isoquinoléin-1(2*H*)-one**<br>**RMN $^1$H** (300 MHz, DMSO-$d_6$) : δ 11,50 (s large, 1H), 8,45 (d, 1H), 8,38 (s large, 3H), 7,72 (d, 1H), 7,52 (t, 1H), 7,29 (m, 1H), 7,18 (d, 1H), 7,09 (d, 1H), 7,05 (d, 1H), 4,59 (quad, 1H), 4,30 (m, 2H), 4,10 (m, 2H), 1,51 (d, 3H)<br>**IR ($cm^{-1}$) :** 3211, 3100-2500, 1669, 1219, 1076, 777<br>**HRMS (ESI) :** m/z théorique pour $C_{20}H_{19}N_2O_4$ $[M+H]^+$ 351,1345, mesuré 351,1341<br>**Pureté optique** (SFC: Chiralpak IA 3µM 4,6x250 mm ; éluant: $CO_2$/ (éthanol/butylamine: 100/0,5) : 70 / 30 ; détection: 260nm) :>99%.<br>(absence de **P64**)<br>$\alpha_D$ (589nM) = -15,1 (c = 0,009 g/mL, MeOH) à 20°C |
| **P64** | **373** | **Chlorhydrate de 5-({8-[(1*S*)-1-aminoéthyl]-2,3-dihydro-1,4-benzodioxin-5-yl}carbonyl) isoquinoléin-1(2*H*)-one**<br>**RMN $^1$H** (400 MHz, DMSO-$d_6$) : δ 11,52 (m, 1H), 8,43 (d, 1H), 8,36 (m, 3H), 7,73 (d, 1H), 7,53 (t, 1H), 7,29 (d large 1H), 7,16 (d, 1H), 7,08 (d, 1H), 7,05 (d, 1H), 4,58 (quad, 1H), 4,31 (m, 2H), 4,10 (m, 2H), 1,51 (d, 3H)<br>**IR ($cm^{-1}$) :** 3221, 3200-2300, 1669<br>**HRMS (ESI) :** m/z théorique pour $C_{20}H_{19}N_2O_4$ $[M+H]^+$ 351,1345, mesuré 351,1353. |
| | | m/z théorique pour $C_{20}H_{19}N_2O_4$ $[M+H]^+$ 351,1345, mesuré 351,1353.<br>**Pureté optique** (SFC: Chiralpak IA 3µM 4,6x250 mm; éluant: $CO_2$/ (éthanol/butylamine: 100/0,5) . 70 / 30; détection: 260nm) :>99%.<br>(absence de **P61**)<br>$\alpha_D$ (589nM) = 15,41 (c = 1, DMSO) à 20°C |
| **P68** | **391** | **Méthanesulfonate de 5-{4-[(1R)-1-aminoéthyl]-2-méthylbenzoyl}-3-méthylisoquinoléin-1 (2*H*)-one**<br>**RMN $^1$H** (400 MHz, DMSO-$d_6$) : δ 8,40 (d, 1H), 7,60 (d, 1H), 7,50 (s large, 1H), 7,45 (t, 1H), 7,40 (m, 2H), 6,80 (s large, 1H), 4,50 (m, 1H), 2,40-2,20 (3s, 9H), 1,50 (d, 3H)<br>**IR ($cm^{-1}$) :** 3400-2450, 1684, 1637, 1600, 1550, 1315, 1239, 1149,825-681<br>**HRMS (ESI) :** m/z théorique pour $C_{20}H_{21}N_2O_2$ $[M+H]^+$ 321,1603, mesuré 321,1591<br>m/z théorique pour $C_{40}H_{41}N_4O_4$ $[2M+H]^+$ 641,3128, mesuré 641,3080<br>m/z théorique pour $C_{40}H_{40}N_4NaO_4$ $[2M+Na]^+$ 663,2947, mesuré 663,2919<br>**Pureté optique** (électrophorèse capillaire : CE standard, tampon phosphate/Cyclodextrine HS α, détection 210nm) : >99%. |
| **P74** | **408** | **Chlorhydrate de 5-{4-[(1*S*)-1-aminoéthyl]-2-méthylbenzoyl}-4-méthylisoquinoléin-1 (2*H*)-one**<br>**RMN $^1$H** (500 MHz, DMSO-$d_6$) : δ 11,62 (d, 1H), 8,61 (s large, 3H), 8,44 (dd, 1H), 7,61 (d, 1H), 7,55 (t, 1H), 7,50 (dd, 1H), 7,42 (dd, 1H), 7,39 (d, 1H), 7,06 (d, 1H), 4,43 (quad, 1H), 2,63 (s, 3H), 1,89 (s, 3H), 1,52 (d, 3H)<br>**IR ($cm^{-1}$) :** 3200-2000, 1677, 1645<br>**HRMS (ESI) :** m/z théorique pour $C_{20}H_{21}N_2O_2$ [M+H]+ 321,1603, mesuré 321,1579. |
| | | m/z mesuré pour $C_{20}H_{20}N_2O_2$ [M]+ 320,20.<br>**Pureté optique** (SFC: Whelk (S,S 5µM 4,6x250 mm; éluant: $CO_2$/ (éthanol/butylamine:100/0,5) : 75 / 25 ; détection: 255nm):>99%.<br>(absence de **P87**) |

(suite)

| Produit | Issu de | Nomenclature<br>Description analytique |
|---|---|---|
| **P87** | **462** | **Chlorhydrate de 5-{4-[(1*R*)-1-aminoéthyl]-2-méthylbenzoyl}-4-méthylisoquinoléin-1 (2*H*)-one**<br>**RMN $^1$H** (400 MHz, DMSO-$d_6$) : δ 11,40 (s large, 1H), 8,45 (s large, 3H), 8,45 (dd, 1H), 7,60 (s, 1H), 7,56 (t, 1H), 7,50 (dd, 1H), 7,40 (2m, 2H), 7,06 (s large, 1H), 4,42 (quad, 1H), 2,63 (s, 3H), 1,90 (s, 3H), 1,50 (d, 3H)<br>**IR (cm$^{-1}$) :** 3000-2500, 1677, 1645<br>**HRMS (ESI) :** m/z théorique pour $C_{20}H_{21}N_2O_2$ [M+H]$^+$ 321,1603, mesuré 321,1589,<br>**Pureté optique** (SFC: Whelk (S,S) 5μM 4,6x250 mm; éluant: $CO_2$/ (éthanol/butylamine:100/0,5) : 75 / 25 ; détection: 255nm) :>99%.<br>(absence de **P74**) |
| **P102** | **542** | **Chlorhydrate de 5-{4-[(1*R*)-1-aminoéthyl]-2-fluoro-6-méthoxybenzoyl}isoquinoléin-1 (2*H*)-one**<br>**RMN $^1$H** (400 MHz, DMSO-$d_6$) : δ 11,63 (m, 1H), 8,80-8,50 (m, 3H), 8,50 (d, 1H), 7,78 (d, 1H), 7,55 (t, 1H), 7,52 (d, 1H), 7,38 (dd, 1H), 7,35 (s, 1H), 7,18 (d, 1H), 4,49 (quad, 1H), 3,74 (s, 3H), 1,57 (d, 3H)<br>**IR (cm$^{-1}$) :** 3300-2000, 1674, 1648, 1617<br>**HRMS (ESI) :** m/z théorique pour $C_{19}H_{18}FN_2O_3$ [M+H]$^+$ 341,1301, mesuré 341,1292. |
| **P110** | **591** | **Dichlorhydrate de [4-(1-aminoéthyl)phényl](isoquinoléin-5-yl)méthanone**<br>**RMN $^1$H** (300 MHz, DMSO-$d_6$) : δ 9,80 (s, 1H), 8,75 (m, 3H), 8,65 (2d, 2H), 8,20 (d, 1H), 8,15 (d, 1H), 8,00 (t, 1H), 7,88 (d, 2H), 7,75 (d, 2H), 4,50 (m, 1H), 1,55 (d, 3H) |
| | | **IR (cm$^{-1}$) :** 3000-2500, 2051, 1665-1645, 1604, 819-746<br>**HRMS (ESI) :** m/z théorique pour $C_{18}H_{16}N_2O$ [M+H]$^+$ 277,1341, mesuré 277,1356<br>m/z théorique pour $C_{18}H_{17}N_2O$ [M+H-$NH_3$]$^+$ 260,1075, mesuré 260,1078 |
| **P111** | **596** | **Dichlorhydrate de {4-[(1*S*)-1-aminoéthyl]phényl}(isoquinoléin-5-yl)méthanone**<br>**RMN $^1$H** (300 MHz, DMSO-$d_6$) : δ 9,80 (s, 1H), 8,75 (m, 3H), 8,65 (2d, 2H), 7,20 (d, 1H), 8,15 (d, 1H), 8,00 (t, 1H), 7,88 (d, 2H), 7,75 (d, 2H), 4,50 (m, 1H), 1,55 (d, 3H)<br>**IR (cm$^{-1}$) :** 3000-2000, 1650<br>**HRMS (ESI) :** m/z théorique pour $C_{18}H_{17}N_2O$ [M+H]$^+$ 277,1341, mesuré 277,1353<br>**Pureté optique :** (Colonne ADH 5μm 4,6x250mm, éluant : EtOH/ triéthylamine 1000/1, détection : 265nm) : > 99%. (absence de **P1)** |
| **P112** | **603** | **Dichlorhydrate de [4-(2-aminopropan-2-yl)phényl](isoquinoléin-5-yl)méthanone**<br>**RMN $^1$H** (300 MHz, DMSO-$d_6$) : δ 10,00 (s, 1H), 9,00 (m, 3H), 8,70 (m, 2H), 8,30-8,20 (2d, 2H), 8,05 (t, 1H), 7,85 (dd, 4H), 1,70 (s, 6H)<br>**IR (cm$^{-1}$) :** 2800, 1651<br>**HRMS (ESI) :** m/z théorique pour $C_{19}H_{19}N_2O$ [M+H]$^+$ 291,1497, mesuré 291,1514 |
| **P113** | **610** | **Dichlorhydrate de [4-(1-aminocyclobutyl)phényl](isoquinoléin-5-yl)méthanone**<br>**RMN $^1$H** (300 MHz, DMSO-$d_6$) : δ 9,95 (s, 1H), 9,00 (m, 3H), 8,70 (m, 2H), 8,25 (d, 1H), 8,20 (d, 1H), 8,05 (t, 1H), 7,85-7,75 (dd, 4H), 2,65 (m, 4H), 2,25-1,80 (m, 2H)<br>**IR (cm$^{-1}$) :** 3000-2500, 1650<br>**HRMS (ESI) :** m/z théorique pour $C_{20}H_{19}N_2O$ [M+H]$^+$ 303,1497, mesuré 303,1528 |

(suite)

| Produit | Issu de | Nomenclature<br>Description analytique |
|---|---|---|
| P114 | 617 | **Dichlorhydrate de [(1*R*)-1-amino-2,3-dihydro-1*H*-indén-5-yl](isoquinoléin-5-yl)méthanone**<br>**RMN $^1$H** (300 MHz, DMSO-$d_6$) : δ 9,90 (s, 1H), 8,90 (s large, 3H), 8,68 (2d, 2H), 8,25 (d, 1H), 8,18 (d, 1H), 8,05 (t, 1H), 7,90 (d, 1H), 7,72 (m, 2H), 4,78 (m, 1H), 3,10 (m, 1H), 2,90 (m, 1H), 2,50 (m, 1H), 2,10 (m, 1H)<br>**IR (cm$^{-1}$) :** 2484-2077, 2077-1955-1866, 1662-1651, 1607, 820-754<br>**HRMS (ESI) :** m/z théorique pour $C_{19}H_{17}N_2O$ [M+H]$^+$ 289,1341, mesuré 289,1348<br>**Pureté optique** (électrophorèse capillaire : CE standard, tampon phosphate/Cyclodextrine HS α, détection 210nm) : >99%. (absence de **P115**) |
| P115 | 619 | **Dichlorhydrate de [(1*S*)-1-amino-2,3-dihydro-1*H*-indén-5-yl](isoquinoléin-5-yl)méthanone**<br>**RMN $^1$H** (300 MHz, DMSO-$d_6$) : δ 9,85 (s, 1H), 8,80 (s large, 3H), 8,65 (m, 2H), 8,20 (d, 1H), 8,15 (d, 1H), 8,05 (m, 1H), 7,85 (d, 1H), 7,75 (m, 2H), 4,80 (m, 1H), 3,15-2,90 (2m, 2H), 2,55-2,05 (2m, 2H)<br>**IR (cm$^{-1}$) :** 2725-2150, 2076, 1957, 1866, 1664, 1651, 1606-1590<br>**HRMS (ESI) :** m/z théorique pour $C_{19}H_{17}N_2O$ [M+H]$^+$ 289,1341, mesuré 289,1364<br>**Pureté optique** (électrophorèse capillaire : CE standard, tampon phosphate/Cyclodextrine HS α, détection 210nm) : >99%. (absence de **P114**)<br>$\alpha_D$ (589nM) = -12,83 (c = 0,013 g/mL, MeOH) à 20°C |
| P116 | 625 | **Dichlorhydrate de [4-(4-aminotétrahydro-2*H*-pyran-4-yl)phényl](isoquinoléin-5-yl) méthanone**<br>**RMN $^1$H** (400 MHz, DMSO-$d_6$) : δ 9,80 (s, 1H), 8,95-8,75 (m large, 3H), 8,67 (d, 1H), 8,63 (d, 1H), 8,21 (d, 1H), 8,15 (d, 1H), 8,00 (t, 1H), 7,88 (m, 4H), 3,92-3,40 (2m, 4H), 2,45-2,20 (2m, 4H)<br>**IR (cm$^{-1}$) :** 3700-3200, 3300-1800, 1659<br>**HRMS (ESI) :** m/z théorique pour $C_{21}H_{21}N_2O_2$ [M+H]$^+$ 333.1603, mesuré 333,2 |
| P117 | 635 | **Dichlorhydrate de (1-amino-1-méthyl-2,3-dihydro-1*H*-indén-5-yl)(isoquinoléin-5-yl) méthanone**<br>**RMN $^1$H** (300 MHz, DMSO-$d_6$) : δ 9,90 (s, 1H), 8,88 (s large, 3H), 8,68 (m, 2H), 8,20 (d, 1H), 8,15 (d, 1H), 8,01 (t, 1H), 7,85 (d, 1H), 7,75 (m, 2H), 3,13 (m, 1H), 2,99 (m, 1H), 2,35 (m, 1H), 2,25 (m, 1H), 1,63 (s, 3H)<br>**IR (cm$^{-1}$) :** 3200-2000, 1659<br>**HRMS (ESI) :** m/z théorique pour $C_{20}H_{19}N_2O$ [M+H]$^+$ 303,1497, mesuré 303,1511 |
| P118 | 637 | **Chlorhydrate de {4-[(1*S*)-1-aminoéthyl]-2-méthylphényl}(isoquinoléin-5-yl)méthanone**<br>**RMN $^1$H** (400 MHz, DMSO-$d_6$) : δ 9,88 (s, 1H), 8,74 (m, 4H), 8,68 (d, 1H), 8,59 (d, 1H), 8,04 (d, 1H), 7,98 (t, 1H), 7,62 (s large, 1H), 7,48 (d large, 1H), 7,42 (d, 1H), 4,46 (m, 1H), 2,43 (s, 3H), 1,56 (d, 3H)<br>**IR (cm$^{-1}$) :** 3468, 3000-2000, 1657<br>**HRMS (ESI) :** m/z théorique pour $C_{19}H_{19}N_2O$ [M+H]$^+$ 291,1497, mesuré 291,1521<br>**Pureté optique :** (Colonne ADH 5μm 4,6x250mm, éluant : EtOH/diéthylamine 100/0,1, détection : 270nm) : > 99%. (absence de **P2**) |
| P120 | 647 | **Dichlorhydrate de (1-amino-6-méthoxy-2,3-dihydro-1*H* indén-5-yl)(isoquinoléin-5-yl) méthanone**<br>**RMN $^1$H** (300 MHz, DMSO-$d_6$) : δ 9,70 (s large, 1H), 8,90-8,60 (m, 3H), 8,65 (d, 1H), 8,60-8,50 (2m, 2H), 7,95 (d, 1H), 7,85 (t, 1H), 7,70 (s, 1H), 7,50 (s, 1H), 4,75 (m, 1H), 3,50 (s, 3H), 3,10 (m, 1H), 2, 85 (m, 1H), 2,50 (m, 1H), 2,10 (m, 1H)<br>**IR (cm$^{-1}$) :** 3600-3300, 3100-2000, 1647<br>**HRMS (ESI) :** m/z théorique pour $C_{20}H_{19}N_2O_2$ [M+H]$^+$ 319,1447, mesuré 319,1432 |

(suite)

| Produit | Issu de | Nomenclature<br>Description analytique |
|---|---|---|
| P126 | 688 | **Dichlorhydrate de [4-(1-aminoéthyl)-2-hydroxyphényl](isoquinoléin-5-yl)méthanone**<br>**RMN $^1$H** (400 MHz, DMSO-$d_6$) : δ 11,03 (m, 1H), 9,87 (s, 1H), 8,72 (m, 3H), 8,69 (d, 1H), 8,64 (d, 1H), 8,38 (d, 1H), 8,14 (d, 1H), 8,00 (t, 1H), 7,52 (d, 1H), 7,18 (d(fin), 1H), 7,14 (dd(fin), 1H), 4,41 (m, 1H), 1,53 (d, 3H)<br>**IR (cm$^{-1}$) :** 3600-2000, 1630<br>**HRMS (ESI) :** m/z théorique pour $C_{18}H_{17}N_2O_2$ [M+H]$^+$ 293,1290, mesuré 293,1284 |
| P132 | 718 | **Chlorhydrate de 5-[4-(1-aminoéthyl)-2-fluoro-3-(2-méthylpropoxy)benzoyl]isoquinoléin-1(*2H*)-one**<br>**RMN $^1$H** (400 MHz, DMSO-$d_6$) : δ 12,60 (s, 1H), 8,50 (s, 3H), 8,50 (d, 1H), 7,80 (d, 1H), 7,60 (d, 1H), 7,55 (m, 1H), 7,45 (m, 1H), 7,35 (m, 1H), 7,00 (d, 1H), 4,70 (quad, 1H), 3,95-3,80 (m, 2H), 2,05 (m, 1H), 1,55 (d, 3H), 1,00 (d, 6H)<br>**IR (cm$^{-1}$) :** 3220-2450, 1664, 1628<br>**HRMS (ESI) :** m/z théorique pour $C_{22}H_{24}FN_2O_3$ [M+H]$^+$ 383,1771, mesuré 383,1760 |
| P141 | 757 | **Dichlorhydrate de {4-[(1R)-1-aminoéthyl]phényl}(8-chloroisoquinoléin-5-yl)-1(*2H*)-que**<br>**RMN $^1$H** (400 MHz, DMSO-$d_6$) : δ 9.76 (s, 1H), 8,72 (d, 1H), 8,68 (s large, 1H), 8.02 (d, 1H), 7,98 (d, 1H), 7,92 (d, 1H), 7,87 (d, 1H), 7,71 (d, 2H), 4.53 (m, 1H), 1.54 (d, 3H)<br>**IR (cm$^{-1}$) :** 3200-2000, 1655-1643<br>**HRMS (ESI) :** m/z théorique pour $C_{18}H_{15}ClN_2O$ [M+H]$^+$ 311.095, mesuré 311.093 |

Les produits **P9, P10, P22** et **P23** ont été obtenus par séparation des produits racémiques correspondants :

Le produit **P6** sous forme de base libre (3,4 g) a été chromatographié par chromatographie haute pression sur support chiral (Colonne OD, éluant $CH_3CN$, détection : 255nm) pour donner après salification les produits **P9** et **P10.**

| Produit | Issu de | Nomenclature<br>Description analytique |
|---|---|---|
| P9 | P6 | **Dichlorhydrate de {4-[(1*R*)-1-aminoéthyl]-2,6-difluorophényl}(isoquinoléin-5-yl) méthanone**<br>**RMN $^1$H** (300MHz ; DMSO-$d_6$) : δ 9,79 (s, 1H); 8,90 (d, 1H); 8,85 (s large, 3H); 8,80 (d, 1H); 8,70 (d, 1H); 8,27 (d, 1H); 7,96 (t, 1H); 7,61 (d, 2H); 4,57 (m, 1H); 1,59 (d, 3H)<br>**IR (cm$^{-1}$) :** 3200-2200, 1668<br>**HRMS (ESI) :** m/z calculé pour $C_{18}H_{14}F_2N_2O$ [M+H]$^+$; 313,1152 trouvé 313,1140<br>**Pureté optique** : (Colonne Kromasil Cellucoat 4,6x250mm, éluant : Heptane/ EtOH/diéthylamine 70/30/0,1, détection : 252nm) : > 99%. (absence de **P10**)<br>$\alpha_D$ (589nM) = 2,49 (c = 0,008 g/mL, MeOH) à 20°C |
| P10 | P6 | **Dichlorhydrate de {4-[(1*S*)-1-aminoéthyl]-2,6-difluorophényl}(isoquinoléin-5-yl) méthanone**<br>**RMN $^1$H** (300MHz ; DMSO-$d_6$) : δ 9,79 (s, 1H); 8,90 (d, 1H); 8,85 (s large, 3H); 8,80 (d, 1H); 8,70 (d, 1H); 8,27 (d, 1H); 7,96 (t, 1H); 7,61 (d, 2H); 4,57 (m, 1H); 1,59 (d, 3H)<br>**IR (cm$^{-1}$) :** 3200-2200, 1670<br>**HRMS (ESI) :** m/z calculé pour $C_{18}H_{14}F_2N_2O$ [M+H]$^+$; 313,1152 trouvé 313,1141<br>**Pureté optique** : (Colonne Kromasil Cellucoat 4,6x250mm, éluant : Heptane/ EtOH/diéthylamine 70/30/0,1, détection : 252nm) : > 99%. (absence de P9)<br>$\alpha_D$ (589nM) = -2,2 (c = 0,008 g/mL, $CHCl_3$) à 20°C |

Le produit **P11** sous forme de base libre (1,8 g) a été chromatographié par chromatographie haute pression sur support chiral (Colonne AD, éluant MeOH, détection : 295nm) pour donner après salification les produits **P22** et **P23.**

| Produit | Issu de | Nomenclature<br>Description analytique |
|---|---|---|
| **P22** | **P11** | **Dichlorhydrate de {4-[(1*S*)-1-aminoéthyl]-2-chlorophényl}(isoquinoléin-5-yl) méthanone**<br>**RMN $^1$H** (400MHz ; DMSO-$d_6$): δ 9,90 (s, 1H); 9,90 (d et m, 4H); 8,80 (d, 1H); 8,70 (d, 2H); 8,10 (d, 1H); 8,00 (t, 1H); 7,90 (s large, 1H); 7,70 (m, 2H); 4,55 (m, 1H); 1,60 (d, 3H)<br>**IR (cm$^{-1}$) :** 3000-2500, 1661<br>**MS (DEI 70 eV) :** m/z mesuré pour $C_{18}H_{15}Cl_1F_1N_2O$ 310,1<br>**Pureté optique :** (Colonne ADH 5μm 4,6x250mm, éluant : EtOH/Heptane /Diéthylamine 70/30/0,1, détection : 235nm) : > 99%. (absence de **P23**) |
| **P23** | **P11** | **Dichlorhydrate de {4-[(1*R*)-1-aminoéthyl]-2-chlorophényl}(isoquinoléin-5-yl) méthanone**<br>**RMN $^1$H** (400MHz ; DMSO-d6): δ 9,90 (s, 1H); 9,90 (d et m, 4H); 8,80 (d, 1H); 8,70 (d, 2H); 8,10 (d, 1H); 8,00 (t, 1H); 7,90 (s large, 1H); 7,70 (m, 2H); 4,55 (m, 1H); 1,60 (d, 3H)<br>**IR (cm$^{-1}$) :** 3000-2500, 1660<br>**MS (DEI 70 eV) :** m/z mesuré pour $C_{18}H_{15}Cl_1F_1N_2O$ 310,1<br>**Pureté optique :** (Colonne ADH 5μm 4,6x250mm, éluant : EtOH/Heptane /Diéthylamine 70/30/0,1, détection : 235nm) : > 99%. (absence de **P22**) |

**Protocole XXI: Méthode alternative de préparation des composés de formule (I) pour lesquels X représente -C(=O)**

Les composés de formule (I) pour lesquels X représente -C(=O)- peuvent aussi être préparés par réaction de couplage *via* ortho-métallation dirigée selon l'exemple de la synthèse de l'intermédiaire **333** ci dessous :

$$\mathbf{125} + \mathbf{331b} \xrightarrow{\text{LDA}} \mathbf{332} \xrightarrow{MnO_2} \mathbf{333}$$

**Intermédiaire 332 :**

**[(1*R*)-1-{3-[(1-éthoxyisoquinoléin-5-yl)(hydroxy)méthyl]-2,4-difluorophényl}éthyl] carbamate de *tert*-butyle**

A une solution de l'intermédiaire **331b** (35 g, 137 mmoles) dans le THF (700 mL) refroidie à -78°C sous atmosphère d'azote, est ajouté une solution de LDA 2N dans l'heptane/THF/éthylbenzène (170 mL, 342 mmoles) en maintenant une température inférieure à -75°C. Le mélange réactionnel est agité à -78°C pendant 30 minutes puis une solution de l'intermédiaire **125** (28,8 g, 143 mmoles) dans du THF (330 mL) est ajoutée en 1 heure en maintenant une température inférieure à -75°C. Le milieu réactionnel est agité pendant 30 minutes. Le milieu réactionnel est hydrolysé avec de l'eau puis laissé remonter à température ambiante. Le THF est éliminé sous pression réduite. La phase aqueuse est extraite avec 2 x 350 mL d'AcOEt puis les phases organiques sont rassemblées et évaporées sous vide. Le résidu est purifié par flash-chromatographie sur silice (éluant = $CH_2Cl_2$/AcOEt : 70/30). L'intermédiaire **332** (56,3 g) est obtenu sous la forme d'un solide jaunâtre.
**RMN $^1$H** (400 MHz, DMSO-$d_6$) : δ 8,15 (m, 2H), 7,92 (d, 1H), 7,64 (t, 1H), 7,35 (m, 1H), 7,26 (m, 1H), 6,98 (m, 1H), 6,60 (s, 1H), 6,11 (s large, 1H), 4,81 (m, 1H), 4,53 (quad, 2H), 1,42 (t, 3H), 1,38-1,22 (m, 12H), 1,35 (d, 3H).
**RMN 19F:** - 119, -115
**IR (cm$^{-1}$) :** 3331, 1689, 1572, 1161

**Intermédiaire 333 :**

**[(1*R*)-1-{3-[(1-éthoxyisoquinoléin-5-yl)carbonyl]-2,4-difluorophényl}éthyl]carbamate de *tert*-butyle**

L'intermédiaire **332** est transformé en intermédiaire **333** selon le protocole décrit pour l'intermédiaire **127 (Protocole XX).**
**RMN $^1$H** (300/400 MHz, DMSO-$d_6$) : δ 8,55 (d, 1H), 8,05 (d, 1H), 8,20 (m, 2H), 7,75 (t, 1H), 7,65 (quad, 1H), 7,55 (d large, 1H), 7,30 (t, 1H), 4,85 (m, 1H), 4,55 (quad, 2H), 1,45 (t, 3H), 1,40 (m, 9H), 1,3 (d, 3H)

**IR (cm$^{-1}$)** : 3300, 1676, 1250

Cette séquence a été utilisée pour préparer les intermédiaires suivants :

**Intermédiaire 236 :**

***N*-{5-[(1-éthoxyisoquinoléin-5-yl)carbonyl]-4,6-difluoro-2,3-dihydro-1H-indén-1-yl}-2-méthylpropane-2-sulfinamide**

Obtenu par oxydation de l'intermédiaire résultant du couplage de **234** et **125**
RMN **$^1$H** (400 MHz, DMSO-$d_6$) : δ 8,53 (d, 1H), 8,19 (2d, 2H), 8,00 (d, 1H), 7,71 (t, 1H), 7,48 (d, 1H), 6,01 (d, 1H), 4,89 (m, 1H), 4,58 (quad, 1H), 2,96-2,78 (m, 2H), 2,50-2,05 (m, 2H), 1,47 (t, 3H), 1,19 (s, 9H)
IR **(cm$^{-1}$)** : 3200, 1668

Intermédiaire 262 :

***N*-{6-[(1-éthoxyisoquinoléin-5-yl)carbonyl]-5,7-difluoro-2,3-dihydro-1*H*-indén-1-yl}-2-méthylpropane-2-sulfinamide**

Obtenu par oxydation de l'intermédiaire résultant du couplage de **260** et **125**
**RMN $^1$H** (400 MHz, DMSO-$d_6$) : δ 8,55 (d, 1H), 8,20 (d, 1H), 8,15 (d, 1H), 8,00 (d, 1H), 7,70 (m, 1H), 7,25 (d, 1H), 5,75 (d, 1H), 4,95 (m, 1H), 4,55 (quad, 2H), 3,20-2,90 (2m, 2H), 2,40-2,20 (2m, 2H), 1,45 (t, 3H), 1,05 (s, 9H)
**IR (cm$^{-1}$) :** 3215, 1738, 1670.

**Intermédiaire 271 :**

**(1-{4-[(1-éthoxyisoquinoléin-5-yl)carbonyl]-2,3-difluorophényl}éthyl)carbamate de *tert*-butyle**

Obtenu par oxydation de l'intermédiaire résultant du couplage de **269** et **125**
**RMN $^1$H** (300/400 MHz, DMSO-$d_6$) : δ 8,47 (d, 1H), 8,11 (d, 1H), 7,95 (d, 1H), 7,70 (t, 1H), 7,70 (d, 1H), 7,70 (d large, 1H), 7,49 (t, 1H), 7,32 (t, 1H), 4,95 (quint, 1H), 4,55 (quad, 2H), 1,45 (t, 3H), 1,40 (s large, 9H), 1,35 (d, 3H)
**IR (cm$^{-1}$) :** 3345, 1671, 1523

**Intermédiaire 296 :**

**[(1*R*)-1-{4-[(1-éthoxy-3-méthylisoquinoléin-5-yl)carbonyl]-3,5-difluorophényl}éthyl] carbamate de *tert*-butyle**

Obtenu par oxydation de l'intermédiaire résultant du couplage de **288** et **294**
**RMN $^1$H** (400 MHz, DMSO-$d_6$) : δ 8,50 (d, 1H), 8,05 (s, 1H), 8,00 (d, 1H), 7,65 (t, 1H), 7,55 (d large, 1H), 7,15 (d large, 2H), 4,75 (m, 1H), 4,55 (quad, 2H), 2,55 (s, 3H), 1,45 (t, 3H), 1,40 (s, 9H), 1,40 (d, 6H)
**IR (cm$^{-1}$) :** 3400, 1679, 1260

**Intermédiaire 305 :**

**[(1*R*)-1-{4-[(1-éthoxy-4-méthylisoquinoléin-5-yl)carbonyl]-3,5-difluorophényl}éthyl] carbamate de *tert*-butyle**

Obtenu par oxydation de l'intermédiaire résultant du couplage de **288** et **303**
**RMN $^1$H** (400 MHz, DMSO-$d_6$) : δ 8,45 (d, 1H), 8,00 (s, 1H), 7,80 (d, 1H), 7,65 (m, 1H), 7,55 (d, 1H), 7,20 (d, 2H), 4,75 (m, 1H), 4,55 (quad, 2H), 2,20 (s, 3H), 1,45 (t, 3H), 1,40 (s, 9H), 1,30 (t, 3H)
**IR (cm$^{-1}$) :** 3387, 1686, 1668

**Intermédiaire 319 :**

**N-{5-[(1-éthoxyisoquinoléin-5-yl)carbonyl]-4,6-difluoro-2,3-dihydro-1*H*-indén-1-yl}-2-méthylpropane-2-sulfinamide**

Obtenu par oxydation de l'intermédiaire résultant du couplage de **317** et **125**
**RMN $^1$H** (400 MHz, DMSO-$d_6$) : δ 8,53 (d, 1H), 8,19 (2d, 2H), 8,00 (d, 1H), 7,71 (t, 1H), 7,48 (d, 1H), 6,01 (d, 1H), 4,89 (m, 1H), 4,58 (quad, 1H), 2,96-2,78 (m, 2H), 2,50-2,05 (m, 2H), 1,47 (t, 3H), 1,19 (s, 9H)

**IR ($cm^{-1}$) :** 3200, 1734, 1668, 1033

**Intermédiaire 326 :**

**[(1*S*)-1-{3,5-difluoro-4-[(4-méthylisoquinoléin-5-yl)carbonyl]phényl}éthyl]carbamate de *tert*-butyle**

Obtenu par oxydation de l'intermédiaire résultant du couplage de **324** et **321**
**RMN $^1$H** (400 MHz, DMSO-$d_6$) : δ 9,30 (s, 1H), 8,50 (s, 1H), 8,35 (d, 1H), 7,90 (d, 1H), 7,70 (m, 1H), 7,60 (d, 1H), 7,25 (d, 2H), 4,75 (m, 1H), 2,35 (s, 3H), 1,40 (s, 9H), 1,35 (d, 3H)
**IR ($cm^{-1}$) :** 3392, 1685, 1635

**Intermédiaire 328 :**

**[(1*S*)-1-{4-[(1-éthoxy-4-méthylisoquinoléin-5-yl)carbonyl]-3,5-difluorophényl}éthyl] carbamate de *tert*-butyle**

Obtenu à partir de **326** selon le protocole décrit pour **182**
**RMN $^1$H** (400 MHz, DMSO-$d_6$) : δ 8,53 (d, 1H), 8,05 (s, 1H), 7,90 (d large, 1H), 7,75 (t, 1H), 7,62 (d large, 1H), 7,30 (m, 2H), 4,81 (quint, 1H), 4,64 (quad, 2H), 2,29 (s, 3H), 1,55 (t, 3H), 1,48 (s large, 9H), 1,41 (d, 3H)
**LCMS [M+H]**+= 471.

**Intermédiaire 336 :**

***N*-[(1*S*)-1-{3-[(1-éthoxyisoquinoléin-5-yl)carbonyl]-2,4-difluorophényl}éthyl]-2-méthylpropane-2-sulfinamide**

Obtenu par oxydation de l'intermédiaire résultant du couplage de **334** et **125**
**RMN $^1$H** (300/400 MHz, DMSO-$d_6$) : δ 8,55 (d, 1H), 8,02 (dd, 2H), 8,00 (d, 1H), 7,75 (t, 2H), 7,35 (t, 1H), 5,55 (d, 1H), 4,7 (m, 1H), 4,55 (quad, 2H), 1,55 (d, 3H), 1,48 (t, 3H), 1,12 (s, 9H)
**IR ($cm^{-1}$) :** 1669, 1048

**Intermédiaire 339 :**

***N*-{5-[(1-éthoxyisoquinoléin-5-yl)carbonyl]-4,6-difluoro-2,3-dihydro-1*H*-indén-1-yl}-2-méthylpropane-2-sulfinamide**

Obtenu par oxydation de l'intermédiaire résultant du couplage de **337** et **125**
**RMN $^1$H** (400 MHz, DMSO-$d_6$) : δ 8,60 (ddd, 1H), 8,20 (d, 1H), 8,20 (dd, 1H), 8,00 (d, 1H), 7,75 (t, 1H), 7,50 (d, 1H), 6,05 (d, 1H), 4,90 (quad, 1H), 4,60 (quad, 2H), 3,00 (m, 1H), 2,80 (m, 1H), 2,50 (m, 1H), 2,05 (m, 1H), 1,50 (t, 3H), 1,20 (s, 9H)
**IR ($cm^{-1}$) :** 3240, 1667, 1633, 1605, 1568, 815, 758

**Intermédiaire 345 :**

**{(1R)-6-[(1-éthoxyisoquinoléin-5-yl)carbonyl]-5,7-difluoro-2,3-dihydro-1*H*-indén-1-yl}carbamate de *tert*-butyle**

Obtenu par oxydation de l'intermédiaire résultant du couplage de **343** et **125**
**RMN $^1$H** (400 MHz, DMSO-$d_6$) : δ 8,55 (d, 1H), 8,20 (2d, 2H), 8,05 (d, 1H), 7,70 (t, 1H), 7,35 (d, 1H), 7,20 (d, 1H), 5,20 (quad, 1H), 4,60 (quad, 2H), 3,10 (m, 1H), 2,90 (m, 1H), 2,45 (m, 1H), 1,95 (m, 1H), 1,50 (t, 3H), 1,35 (s, 9H)
**IR ($cm^{-1}$) :** 3430, 1672, 1635, 1575, 1517, 1162

**Intermédiaire 348 :**

**{(1*S*)-6-[(1-éthoxyisoquinoléin-5-yl)carbonyl]-5,7-difluoro-2,3-dihydro-1*H*-indén-1-yl}carbamate de *tert*-butyle**

Obtenu par oxydation de l'intermédiaire résultant du couplage de **346** et **125**
**RMN $^1$H** (400 MHz, DMSO-$d_6$) : δ 8,55 (d, 1H), 8,20 (2d, 2H), 8,05 (d, 1H), 7,70 (t, 1H), 7,35 (d, 1H), 7,20 (d, 1H), 5,20 (quad, 1H), 4,60 (quad, 2H), 3,10 (m, 1H), 2,90 (m, 1H), 2,45 (m, 1H), 1,95 (m, 1H), 1,50 (t, 3H), 1,35 (m, 9H)
**IR ($cm^{-1}$) :** 3430, 1673, 1636, 1575, 1517, 1162

**Intermédiaire 369 :**

**(1-{4-[(1-éthoxyisoquinoléin-5-yl)carbonyl]-3,5-difluoro-2-méthoxyphényl}éthyl) carbamate de *tert*-butyle**

Obtenu par oxydation de l'intermédiaire résultant du couplage de **367** et **125**
**RMN $^{1}$H** (300 MHz, DMSO-$d_6$) : δ 8,55 (d, 1H), 8,20 (2d, 2H), 8,10 (d, 1H), 7,75 (t, 1H), 7,50 (d, 1H), 7,20 (d, 1H), 5,00 (m, 1H), 4,60 (quad, 2H), 3,90 (s, 3H), 1,45 (t, 3H), 1,40 (m, 9H), 1,30 (d, 3H)
**RMN $^{19}$F** : -118, -129
**IR (cm$^{-1}$) :** 3390, 1682, 1675, 1517, 1162, 851-738

**Intermédiaire 375 :**

**(1-{4-[(1-éthoxy-3-méthylisoquinoléin-5-yl)carbonyl]-3,5-difluoro-2-méthoxyphényl}éthyl)carbamate de *tert*-butyle**

Obtenu par oxydation de l'intermédiaire résultant du couplage de **367** et **294**
**RMN $^{1}$H** (300 MHz, DMSO-$d_6$) : δ 8,50 (d, 1H), 8,05 (s, 1H), 8,05 (d, 1H), 7,65 (t, 1H), 7,50 (d, 1H), 7,20 (d, 1H), 5,00 (m, 1H), 4,55 (quad, 2H), 3,90 (s, 3H), 2,55 (s, 3H), 1,50 (t, 3H), 1,40 (m, 9H), 1,30 (d, 3H)
**RMN $^{19}$F** : -118, -129
**IR (cm$^{-1}$) :** 3400, 1679, 1665, 1616, 1571, 1520, 1148, 860-691

**Intermédiaire 380 :**

**(2-{4-[(1-éthoxy-4-méthylisoquinoléin-5-yl)carbonyl]-3,5-difluorophényl}propan-2-yl)carbamate de *tert*-butyle**

Obtenu par oxydation de l'intermédiaire résultant du couplage de **378** et **303**
**RMN $^{1}$H** (400 MHz, DMSO-$d_6$) : δ 8,45 (m, 1H), 7,95 (s, 1H), 7,80 (m, 1H), 7,65 (t, 1H), 7,35 (m, 1H), 7,15 (d, 2H), 4,55 (quad, 2H), 2,20 (s, 3H), 1,50 (s, 6H), 1,45 (t, 3H), 1,35 (s large, 9H)
**IR (cm$^{-1}$) :** 3310, 1718, 1688, 1627

**Intermédiaire 389 :**

**[1-{4-[(1-éthoxyisoquinoléin-5-yl)carbonyl]-3-fluoro-2-méthoxyphényl}éthyl] carbamate de *tert*-butyle**

Obtenu par oxydation de l'intermédiaire résultant du couplage de **387** et **125**
**RMN $^{1}$H** (400 MHz, DMSO-$d_6$) : δ 8,46 (d, 1H), 8,10 (d, 1H), 7,93 (d, 1H), 7,70 (m, 2H), 7,56 (d, 1H), 7,37 (dd, 1H), 7,30 (d, 1H), 4,99 (m, 1H), 4,57 (quad, 2H), 3,86 (s, 3H), 1,46 (t, 3H), 1,37 (s, 9H), 1,29 (d, 3H)
**IR (cm$^{-1}$) :** 3354, 1703, 1660

**Intermédiaire 403 :**

**N-[(1R)-1-{4-[(1-éthoxyisoquinoléin-5-yl)carbonyl]-3,5-difluoro-2-méthoxyphényl] éthyl]-2-méthylpropane-2-sulfinamide**

Obtenu par oxydation de l'intermédiaire résultant du couplage de **401** et **125**
**RMN $^{1}$H** (400 MHz, DMSO-$d_6$) : δ 8,55 (d, 1H), 8,20 (2d, 2H), 8,10 (d, 1H), 7,75 (t, 1H), 7,40 (dd, 1H), 5,80 (d, 1H), 4,75 (quint, 1H), 4,60 (quad, 2H), 3,90 (s, 3H), 1,50 (t, 3H), 1,40 (d, 3H), 1,15 (s, 9H)
**IR (cm$^{-1}$) :** 3245, 1668, 1617, 1569, 816, 757

**Intermédiaire 406 :**

***N*-[(1*S*)-1-{4-[(1-éthoxyisoquinoléin-5-yl)carbonyl]-3,5-difluoro-2-méthoxyphényl} éthyl]-2-méthylpropane-2-sulfinamide**

Obtenu par oxydation de l'intermédiaire résultant du couplage de **404** et **125**
**RMN $^{1}$H** (400 MHz, DMSO-$d_6$) : δ 8,55 (d, 1H), 8,20 (2d, 2H), 8,10 (d, 1H), 7,75 (t, 1H), 7,30 (dd, 1H), 5,55 (d, 1H), 4,80 (quint, 1H), 4,60 (quad, 2H), 3,90 (s, 3H), 1,50 (t, 3H), 1,50 (d, 3H), 1,15 (s, 9H)
**IR (cm$^{-1}$) :** 3230, 1668, 1617, 1569, 815, 757

**Intermédiaire 417 :**

**{5-[(1-éthoxyisoquinoléin-5-yl)carbonyl]-4,6-difluoro-2,3-dihydro-1*H*-indén-1-yl}carbamate de *tert*-butyle**

Obtenu par oxydation de l'intermédiaire résultant du couplage de **415** et **125**
**RMN $^1$H** (400 MHz, DMSO-$d_6$) : δ 8,55 (dd, 1H), 8,20 (2d, 2H), 8,00 (dd, 1H), 7,70 (t, 1H), 7,50 (d, 1H), 7,00 (d, 1H), 5,10 (m, 1H), 4,60 (quad, 2H), 3,00 (m, 1H), 2,80 (m, 1H), 2,45 (m, 1H), 1,95 (m, 1H), 1,45 (t, 3H), 1,45 (s, 9H)
**IR (cm$^{-1}$) :** 3385, 1680, 1662, 1569, 1511, 1162

**Intermédiaire 425 :**

**{5-[(1-éthoxyisoquinoléin-5-yl)carbonyl]-4,6-difluoro-2,3-dihydro-1*H*-indén-1-yl}carbamate de *tert*-butyle**

Obtenu par oxydation de l'intermédiaire résultant du couplage de **423** et **125**
**RMN $^1$H** (400 MHz, DMSO-$d_6$) : δ 8,55 (dd, 1H), 8,20 (2d, 2H), 8,00 (dd, 1H), 7,70 (t, 1H), 7,50 (d, 1H), 7,00 (d, 1H), 5,10 (m, 1H), 4,60 (quad, 2H), 3,00 (m, 1H), 2,80 (m, 1H), 2,45 (m, 1H), 1,95 (m, 1H), 1,45 (t, 3H), 1,45 (s, 9H)
**IR (cm$^{-1}$) :** 3385, 1680, 1662, 1569, 1510

**Intermédiaire 432 :**

**(1-{4-[(1-éthoxyisoquinoléin-5-yl)carbonyl]-2-éthyl-3,5-difluorophényl}éthyl) carbamate de *tert*-butyle**

Obtenu par oxydation de l'intermédiaire résultant du couplage de **430** et **125**
**RMN $^1$H** (400 MHz, DMSO-$d_6$) : δ 8,54 (d, 1H), 8,19 (d, 1H), 8,15 (d, 1H), 8,03 (d, 1H), 7,72 (dd, 1H), 7,60 (d, 1H), 7,25 (d, 1H), 4,94 (m, 1H), 4,57 (quad, 2H), 2,74 (m, 1H), 2,64 (m, 1H), 1,46 (t, 3H), 1,38 (s, 9H), 1,33 (d, 3H), 1,16 (t, 3H)
**IR (cm$^{-1}$) :** 3358, 1677, 1631

**Intermédiaire 446 :**

**(1-{6-(benzyloxy)-3-[(1-éthoxyisoquinoléin-5-yl)carbonyl]-2,4-difluorophényl}éthyl) carbamate de *tert*-butyle**

Obtenu par oxydation de l'intermédiaire résultant du couplage de **444** et **125**
**RMN $^1$H** (300 MHz, DMSO-$d_6$) : δ 8,50 (d, 1H), 8,15 (d, 1H), 8,0 (m, 2H), 7,70 (t, 1H), 7,6-7,3 (m, 5H), 7,05 (d, 1H), 6,95 (m, 1H), 5,30 (s, 2H), 5,10 (m, 1H), 4,55 (quad, 2H), 1,45 (t, 3H), 1,30 (m, 12H)
**RMN $^{19}$F** : -112,5, -115,5
**IR (cm$^{-1}$)** 3490, 1709

**Intermédiaire 467 :**

**(2-{3-[(1-éthoxyisoquinoléin-5-yl)carbonyl]-2,4-difluorophényl}propan-2-yl) carbamate de *tert*-butyle**

Obtenu par oxydation de l'intermédiaire résultant du couplage de **465** et **125**
**RMN $^1$H** (400 MHz, DMSO-$d_6$) : δ 8,54 (d, 1H), 8,20 (s, 2H), 7,92 (d, 1H), 7,69 (t, 1H), 7,50 (m, 1H), 7,30 (s large, 1H), 7,22 (t, 1H), 4,58 (quad, 2H), 1,55 (s, 6H), 1,45 (t, 3H), 1,30 (m, 9H)
**RMN $^{19}$F** :-117,2, -116,2
**IR (cm$^{-1}$) :** 3345, 1697, 1672

**Intermédiaire 480 :**

***N*-(1-{3-[(1-éthoxyisoquinoléin-5-yl)carbonyl]-2,4-difluorophényl}propyl)-2-méthylpropane-2-sulfinamide**

Obtenu par oxydation de l'intermédiaire résultant du couplage de **478** et **125**
**RMN $^1$H** (300 MHz, DMSO-$d_6$) : δ 8,56 (d, 1H), 8,19 (2d, 2H), 7,99 (d, 1H), 7,72 (2m, 2H), 7,31 (t, 1H), 5,50 (d, 1H), 4,59 (quad., 2H), 4,40 (m, 1H), 1,92 (m, 1H), 1,78 (m, 1H), 1,48 (t, 3H), 1,08 (s, 9H), 0.87 (t, 3H)
**IR (cm$^{-1}$) :** 3197, 1670, 1264, 1050, 1018

**Intermédiaire 486 :**

**2-{3-[(1-éthoxyisoquinoléin-5-yl)carbonyl]-2,4-difluorophényl}pyrrolidine-1-carboxylate de *tert*-butyle**

Obtenu par oxydation de l'intermédiaire résultant du couplage de **484** et **125**
**RMN $^1$H** (300 MHz, DMSO-$d_6$) : δ 8,45 (d, 1H), 8,17 (2d, 2H), 7,98 (d, 1H), 7,80 (t, 1H), 7,45 (m, 1H), 7,21 (t, 1H), 4,95 (m, 1H), 4,61 (quad, 2H), 3,52 (m, 2H), 2,33 (m, 1H), 1,89 (m, 2H), 1,80 (m, 1H), 1,48 (t, 3H), 1,29 (s, 9H)
**IR ($cm^{-1}$) :** 1694, 1674, 1159
**Pureté optique** (SFC: Colonne ID 3μM 4,6x250 mm; $CO_2$ / (isopropanol/n-butylamine:100/0,5) : 75 / 25; Détection: 254nm) : > 99%.

**Intermédiaire 494 :**

**[(1*R*)-1-{4-[(1-éthoxyisoquinoléin-5-yl)carbonyl]-3,5-difluorophényl}propyl] carbamate de *tert*-butyle**

Obtenu par oxydation de l'intermédiaire résultant du couplage de **492** et **125**
**RMN $^1$H** (400 MHz, DMSO-$d_6$) : δ 8,55 (d, 1H), 8,20 (d, 1H), 8,15 (d, 1H), 8,05 (d large, 1H), 7,25 (d, 2H), 4,55 (m et quad, 3H), 1,7 (m, 2H), 1,45 (t, 3H), 1,4 (s, 9H), 0.9 (t, 3H)

**Intermédiaire 501 :**

**(1-{4-[(1-éthoxyisoquinoléin-5-yl)carbonyl]-3,5-difluorophényl}-2-méthylpropyl) carbamate de *tert*-butyle**

Obtenu par oxydation de l'intermédiaire résultant du couplage de **499** et **125**
**RMN $^1$H** (400 MHz, DMSO-$d_6$) : δ 8,54 (d, 1H), 8,19 (d, 1H), 8,11 (d, 1H), 8,05 (d, 1H), 7,71 (t, 1H), 7,22 (m, 2H), 4,58 (quad, 2H), 4,39 (t, 1H), 1,95 (m, 1H), 1,48 (t, 3H), 1,38 (s large, 9H), 0.9-0,79 (2d, 6H)
**RMN $^{19}$F** :-113,1
**IR ($cm^{-1}$) :** 3354, 1678, 1633

**Intermédiaire 507 :**

**(1-{4-[(1-éthoxyisoquinoléin-5-yl)carbonyl]-3,5-difluorophényl}butyl)carbamate de *tert*-butyle**

Obtenu par oxydation de l'intermédiaire résultant du couplage de **505** et **125**
**RMN $^1$H** (400 MHz, DMSO-$d_6$) : δ 8,55 (d, 1H), 8,15 (d, 1H), 8,2 (d, 1H), 8,05 (d, 1H), 7,7 (t, 1H), 7,45 (d large, 1H), 7,2 (d large, 2H), 4,6 (m, 1H), 4,58 (quad, 2H), 1,65 (m, 2H), 1,45 (t, 3H), 1,4 (s large, 9H), 1,3 (m, 2H), 0.9 (t, 3H)
**IR ($cm^{-1}$) :** 3350, 1676, 1160

**Intermédiaire 512 :**

**(1-{4-[(1-éthoxyisoquinoléin-5-yl)carbonyl]-3,5-difluorophényl}-3-méthylbutyl) carbamate de *tert*-butyle**

Obtenu par oxydation de l'intermédiaire résultant du couplage de **510** et **125**
**RMN $^1$H** (400 MHz, DMSO-$d_6$) : δ 8,55 (d, 1H), 8,15 (dd, 1H), 8,05 (d, 1H), 7,7 (t, 1H), 7,25 (d, 2H), 4,65 (m, 1H), 4,55 (quad, 2H), 1,65-1,45 (m, 3H), 1,45 (t, 3H), 1,4 (s, 9H), 0.95 (2d, 6H)
**IR ($cm^{-1}$) :** 3380, 1681, 1673, 1663

**Intermédiaire 523 :**

**2-{3-[(1-éthoxyisoquinoléin-5-yl)carbonyl]-2,4-difluorophényl}pyrrolidine-1-carboxylate de *tert*-butyle**

Obtenu par oxydation de l'intermédiaire résultant du couplage de **521** et **125**
**RMN $^1$H** (400 MHz, DMSO-$d_6$) : δ 8,56 (d, 1H), 8,18 (2d, 2H), 7,98 (d, 1H), 7,7 (t, 1H), 7,46 (m, 1H), 7,22 (m, 1H), 4,96 (m, 1H), 4,62 (quad, 2H), 3,52 (m, 2H), 2,35-1,8 (2m, 2H), 1,90 (m, 2H), 1,5 (t, 3H), 1,29 (s, 9H)
**IR ($cm^{-1}$) :** 1682, 1667
**Pureté optique** (SFC: Colonne ID 3μM 4,6x250 mm; $CO_2$ / (isopropanol/n-butylamine:100/0,5) : 75 / 25; Détection: 254nm) : > 99%.

**Intermédiaire 528 :**

***N*-(1-{3-[(1-éthoxyisoquinoléin-5-yl)carbonyl]-2-fluorophényl}éthyl)-2-méthylpropane-2-sulfinamide**

Obtenu par oxydation de l'intermédiaire résultant du couplage de **526** et **125**
**RMN $^1$H** (300/400 MHz, DMSO-$d_6$) : δ 8,50 (d large, 1H), 8,10 (d, 1H), 7,87 (d large, 1H), 7,69 (dd, 1H), 7,71 (dd, 1H), 7,77 (td, 1H), 7,57 (td, 1H), 7,38 (t, 1H), 4,64 (quint., 1H), 4,56 (quad., 2H), 1,47 (d, 3H), 1,46 (t, 3H), 1,07 (s, 9H)
**RMN $^{19}$F**: -118
**IR (cm$^{-1}$) :** 1663, 1051,3203

**Intermédiaire 552 :**

**(1-{2-(benzyloxy)-4-[(1-éthoxyisoquinoléin-5-yl)carbonyl]-3,5-difluorophényl}éthyl) carbamate de *tert*-butyle**

Obtenu par oxydation de l'intermédiaire résultant du couplage de **550** et **125**
**RMN $^1$H** (400 MHz, DMSO-$d_6$) : δ 8,56 (d, 1H), 8,2-8,16 (2d, 2H), 8,06 (d, 1H), 7,74 (t, 1H), 7,54 (d, 1H), 7,48 (d, 2H), 7,41 (t, 2H), 7,37 (t, 1H), 7,24 (d, 1H), 5,11 (AB, 2H), 5,12 (m, 1H), 4,58 (quad, 2H), 1,46 (t, 3H), 1,39 (s large, 12H), 1,27 (d, 3H)
**IR (cm$^{-1}$) :** 3358, 1679

**Intermédiaire 560 :**

**(1-{3-[(1-éthoxyisoquinoléin-5-yl)carbonyl]-2,4-difluoro-6-méthylphényl}éthyl) carbamate de *tert*-butyle**

Obtenu par oxydation de l'intermédiaire résultant du couplage de **558** et **125**
**RMN $^1$H** (400 MHz, DMSO-$d_6$) : δ 8,53 (d, 1H), 8,19-8,12 (2d, 2H), 7,96 (d, 1H), 7,69 (t, 1H), 7,33 (d, 1H), 7,11 (d, 1H), 4,88 (quint, 1H), 4,58 (quad, 2H), 2,49 (s, 3H), 1,46 (t, 3H), 1,36 (d, 3H), 1,33-1,25 (2s larges, 9H)
**IR (cm$^{-1}$) :** 3340, 1705, 1670, 1258, 1162

**Intermédiaire 698 :**

**[{4-[(1-éthoxyisoquinoléin-5-yl)carbonyl]-3,5-difluorophényl}(3-méthoxyphényl) méthyl]carbamate de *tert*-butyle**

Obtenu par oxydation de l'intermédiaire résultant du couplage de **696** et **125**
**RMN $^1$H** (300 MHz, DMSO-$d_6$) : δ 8,55 (d, 1H), 8,20 (d, 1H), 8,15 (d, 1H), 8,05 (d, 1H), 7,70 (t, 1H), 7,35 (d, 2H), 7,25 (t, 1H), 7,00 (m, 2H), 6,85 (dd, 1H), 5,95 (m, 1H), 4,55 (quad., 2H), 3,75 (s, 3H), 1,45 (t, 3H), 1,40 (s large, 9H)
**IR (cm$^{-1}$) :** 3342, 2980, 1675, 1632-1612, 1568, 1250, 1159

**Intermédiaire 704 :**

**(2-cyclohexyl-1-{4-[(1-éthoxyisoquinoléin-5-yl)carbonyl]-3,5-difluorophényl}éthyl) carbamate de *tert*-butyle**

Obtenu par oxydation de l'intermédiaire résultant du couplage de **702** et **125**
**RMN $^1$H** (300 MHz, DMSO-$d_6$) : δ 8,55 (d, 1H), 8,20 (d, 1H), 8,15 (d, 1H), 8,05 (d, 1H), 7,80 (d, 1H), 7,45 (dl, 1H, NH), 7,20 (d, 2H), 4,70 (m, 1H), 4,55 (quad., 2H), 1,85-0.90 (m, 13H), 1,45 (t, 3H), 1,40 (s, 9H)
**IR (cm$^{-1}$) :** 3359, 2924-2854, 1680-1634, 1616, 1526, 1252, 1164

**Intermédiaire 708 :**

**(cyclohexylméthyl)(1-{4-[(1-éthoxyisoquinoléin-5-yl)carbonyl]-3,5-difluorophényl}éthyl)carbamate de *tert*-butyle**

Obtenu par oxydation de l'intermédiaire résultant du couplage de **706** et **125**
**RMN $^1$H** (300 MHz, DMSO-$d_6$) : δ 8,53 (d, 1H), 8,18 (d, 1H), 8,13 (d, 1H), 8,00 (d, 1H), 7,70 (t, 1H), 7,15 (m, 2H), 4,92 (m, 1H), 4,56 (quad., 2H), 3,10 (m, 2H), 1,75-075 (m, 11H), 1,58 (d, 3H), 1,45 (t, 3H), 1,30 (s large, 9H)
**IR (cm$^{-1}$) :** 1674, 1632, 1254-1150, 957, 816, 757

**Intermédiaire 710 :**

**(1-{4-[(1-éthoxy-3-méthylisoquinoléin-5-yl)carbonyl]-3,5-difluorophényl}éthyl) carbamate de *tert*-butyle**

Obtenu par oxydation de l'intermédiaire résultant du couplage de l'intermédiaire **278** et **294**
**RMN $^1$H** (400 MHz, DMSO-$d_6$) : δ 8,50 (m, 1H), 8,05 (s, 1H), 8,00 (d, 1H), 7,60 (t, 1H), 7,50 (d, 1H), 7,20 (d, 2H), 4,75 (m, 1H), 4,55 (quad., 2H), 2,55 (s, 3H), 1,45 (t, 3H), 1,40 (s large, 9H), 1,35 (d, 3H)
**IR ($cm^{-1}$):** 3349, 1672

**Intermédiaire 732 :**

**(2-{4-[(1-éthoxy-3-méthylisoquinoléin-5-yl)carbonyl]-3,5-difluorophényl}propan-2-yl)carbamate de *tert*-butyle**

Obtenu par oxydation de l'intermédiaire résultant du couplage de **378** et **294**
**RMN $^1$H** (400 MHz, DMSO-$d_6$) : δ 8,45 (d, 1H), 8,00 (s, 1H), 7,95 (d, 1H), 7,65 (t, 1H), 7,35 (m, 1H, NH), 7,20 (d, 2H), 4,55 (quad., 2H), 2,55 (s, 3H), 1,50 (s, 6H), 1,45 (t, 3H), 1,35 (s large, 9H)
**IR ($cm^{-1}$) :** 3331, 1687, 1669

**Intermédiaire 734 :**

**(1-{4-[(1-éthoxy-3-méthylisoquinoléin-5-yl)carbonyl]-3,5-difluoro-2-méthoxyphényl} éthyl)carbamate de *tert*-butyle**

Obtenu par oxydation de l'intermédiaire résultant du couplage de **367** et **294**
**RMN $^1$H** (300 MHz, DMSO-$d_6$) : δ 8,50 (d, 1H), 8,05 (s et d, 2H), 7,65 (t, 1H), 7,50 (d, 1H), 7,20 (d, 1H), 5,0 (m, 1H), 4,55 (quad, 2H), 3,90 (s, 3H), 2,55 (s, 3H), 1,50 (t, 3H), 1,40 (m, 9H), 1,30 (d, 3H)
**RMN 19F:** -118, -129
**IR ($cm^{-1}$) :** 3259, 1697-1663

**Intermédiaire 741 :**

**[1-{4-[(1-éthoxyisoquinoléin-5-yl)carbonyl]-3-fluoro-2-méthoxyphényl}éthyl] carbamate de *tert*-butyle**

Obtenu par oxydation de l'intermédiaire résultant du couplage de **739** et **125**
**RMN $^1$H** (400 MHz, DMSO-$d_6$) : δ 8,50 (d, 1H), 8,10 (d, 1H), 7,95 (d, 1H), 7,70 (m, 2H), 7,55 (d, 1H, NH), 7,40 (d, 1H), 7,30 (dd, 1H), 5,00 (quint., 1H), 4,60 (quad., 2H), 3,85 (s, 3H), 1,50 (t, 3H), 1,35 (m, 9H), 1,30 (d, 3H)
**IR ($cm^{-1}$) :** 3410, 1709, 1665, 1616, 1570, 1265, 1160, 813, 757

Les intermédiaires **448, 546, 555, 562** et **730** ont été obtenus à partir d'intermédiaires préparés en utilisant le protocole de couplage **XXI** et décrit précedement.

**Intermédiaire 546 :**

**(1-{4-[(1-éthoxyisoquinoléin-5-yl)carbonyl]-2-fluoro-3-méthoxyphényl}éthyl) carbamate de *tert*-butyle**

L'intermédiaire **546** été obtenu par réaction de substitution nucléophile (MeONa/DMF) à partir de de l'intermédiaire **271:** On met en solution 4,8 g (10,5 mmoles) de l'intermédiaire **271** dans 150 mL de DMF sous courant d'azote. On ajoute en une seule fois 1,5 g (27,75 mmoles, 2,6 éq.) de méthylate de sodium en poudre. On agite à température ambiante la nuit. Un contrôle HPLC après 16h à température ambiante ne montre que 40% de produit formé. On rajoute 1,5 g (27,75 mmoles, 2,6 éq.) de méthylate de sodium en poudre et on laisse agiter 6 heures supplémentaires. On hydrolyse par ajout d'eau glacée. On extrait avec 3 fois 250 mL d'acétate d'éthyle, la phase organique est séchée sur $MgSO_4$, puis filtrée et évaporée à sec. On obtient 18 g d'une huile contenant encore de la DMF. Le résidu est repris dans 200 mL d'eau, extrait par 5 fois 200 mL d'éther. La phase organique est séchée sur $MgSO_4$, filtrée et évaporée à sec. On obtient 4,89 g d'une huile orangée qui est purifiée par flash-chromatographie sur silice, (éluant : gradient $CH_2Cl_2$-AcOEt : 99-1 à 90-10) pour donner 1,55 g de l'intermédiaire **546** sous la forme d'une meringue jaune et 1,6 g de fractions impures.
**RMN $^1$H** (400 MHz, DMSO-$d_6$) : δ 8,44 (d, 1H), 8,11 (d, 1H), 7,84 (d, 1H), 7,80 (d, 1H), 7,68 (t, 1H), 7,35 (d, 1H), 7,25 (t, 1H), 4,94 (quint, 1H), 4,56 (quad, 2H), 3,49 (s, 3H), 1,45 (t, 3H), 1,38 (s, 9H), 1,36 (d, 3H)
**RMN $^{19}$F** :-137,1

**IR ($cm^{-1}$) :** 3342, 1701, 1664

L'intermédiaire **554** a été obtenu à partir de **552** selon la séquence suivante :

**Intermédiaire 553 :**

**(1-{4-[(1-éthoxyisoquinoléin-5-yl)carbonyl]-3,5-difluoro-2-hydroxyphényl}éthyl) carbamate de *tert*-butyle**

Une solution de **552** (6,7 g, 12 mmoles) dans un mélange éthanol/AcOEt (500 mL, 1/1) est hydrogénée à pression atmosphérique d'$H_2$ et à 60°C en présence de Pd/C 10% (0,2 g) pendant 7 heures. Le catalyseur est filtré, la concentration du filtrat conduit à l'intermédiaire **553** sous forme d'un solide (5,7 g).

**RMN $^1H$** (400 MHz, DMSO-$d_6$) : δ 9,88 (s large, 1H), 8,53 (d, 1H), 8,19-8,13 (2d, 2H), 8,06 (d, 1H), 7,72 (t, 1H), 7,47 (d large, 1H), 7,07 (d, 1H), 5,03 (quint, 1H), 4,57 (quad, 2H), 1,46 (t, 3H), 1,39 (s large et d, 12H), 1,29 (d, 3H)

**IR ($cm^{-1}$) :** 3350, 3500-2600, 1675

**Intermédiaire 554 :**

**Trifluorométhanesulfonate de 6-{1-[(*tert*-butoxycarbonyl)amino]éthyl}-3-[(1-éthoxyisoquinoléin-5-yl)carbonyl]-2,4-difluorophényle**

Une solution à 0°C de **553** (5,6 g, 11 mmoles) dans la pyridine (45 mL) est traitée par de l'anhydride triflique (3,36 g, 11 mmoles). Le mélange réactionnel est agité à température ambiante pendant 2 heures puis à nouveau refroidi à 0°C et traité avec de l'anhydride triflique (0,2 éq.) jusqu'à conversion totale. La pyridine est évaporée sous vide, le résidu est repris par de l'eau et de l'AcOEt. La phase organique est lavée par une solution saturée de NaCl, séchée sur $MgSO_4$ puis concentrée. Par chromatographie sur silice (éluant $CH_2Cl_2$/AcOEt 100/0 à 95/5) l'intermédiaire **554** est obtenu sous forme d'un solide (6,2 g).

**RMN $^1H$** (300 MHz, DMSO-$d_6$) : δ 8,59 (d, 1H), 8,23-8,19 (2d, 2H), 8,17 (d, 1H), 7,74 (t, 1H), 7,71 (d large, 1H), 7,5 (d, 1H), 4,97 (quint, 1H), 4,57 (quad, 2H), 1,47 (t, 3H), 1,4 (d, 3H), 1,38 (s large, 9H)

**IR ($cm^{-1}$) :** 3375, 1675

**LCMS [M+H]+:** 604

L'intermédiaire **448** a été obtenu en deux étapes à partir de l'intermédiaire **446 :** l'intermédiaire **446** a été transformé en phénol **447** selon le protocole décrit pour l'obtention de **553.** Le phénol **447** a été traité par de l'iodure de méthyle selon le protocole décrit pour l'intermédiaire **93.**

**Intermédiaire 448:**

**(1-{3-[(1-éthoxyisoquinoléin-5-yl)carbonyl]-2,4-difluoro-6-méthoxyphényl}éthyl) carbamate de *tert*-butyle**

**RMN $^1H$** (400 MHz, DMSO-$d_6$) : δ 8,50 (d, 1H), 8,15 (d, 1H), 7,97 (2m, 2H), 7,69 (tt, 1H), 6,99 (d, 1H), 7,0-6,7 (s large, 1H), 5,1-4,9 (m, 1H), 4,57 (quad, 2H), 3,92 (s, 3H), 1,45 (t, 3H), 1,4-1,3 (s large, 12H)

**IR ($cm^{-1}$) :** 3455, 1707, 1163

L'intermédiaire **554** a été utilisé pour préparer les intermédiaires **555** et **562:**

**Intermédiaire 555 :**

**(1-{4-[(1-éthoxyisoquinoléin-5-yl)carbonyl]-3,5-difluoro-2-méthylphényl}éthyl) carbamate de *tert*-butyle**

Un mélange de **554** (1 g, 1,65 mmoles), de triméthyl-boroxine (0,42 g, 3,3 mmoles), de $K_2CO_3$ (0.91 g, 6 mmoles) dans le 1,4-dioxane (15 mL) dégazé par $N_2$ pendant 15minutes est traité avec du $Pd(PPh_3)_4$ (0,38 g, 0,3 mmoles). Le milieu est chauffé au reflux 1 heure. Après retour à température ambiante le solide est filtré, le filtrat est concentré sous vide. Par chromatographie sur silice (éluant $CH_2Cl_2$/AcOEt 100/0 à 50/50) 0,7 g de l'intermédiaire **555** sont obtenus.

**RMN $^1H$** (400 MHz, DMSO-$d_6$) : δ 8,54 (d, 1H), 8,2-8,15 (2d, 2H), 8,03 (d, 1H), 7,72 (t, 1H), 7,59 (d large, 1H), 7,21 (d, 1H), 4,9 (quint, 1H), 4,57 (quad, 2H), 2,22 (s large, 3H), 1,46 (t, 3H), 1,38-1,26 (2s larges, 9H), 1,3 (d, 3H)

**IR ($cm^{-1}$):** 3369, 1682-1672

**LCMS $[M+H]^+$:** 470

L'intermédiaire **562** a été obtenu à partir de l'intermédiaire **554** selon la séquence :

**Intermédiaire 561 :**

**(1-{2-éthényl-4-[(1-éthoxyisoquinoléin-5-yl)carbonyl]-3,5-difluorophényl}éthyl) carbamate de *tert*-butyle**

A une solution dégazée par $N_2$, de l'intermédiaire **554** (1 g) dans du 1,4-dioxane (20 mL) sont ajoutés du vinyl-tributyl-étain (0,58 g) et du $Pd(PPh_3)_4$ (50 mg) et du LiCl (0,2 g,). Le mélange est chauffé à 100°C pendant 2 heures. Après retour à température ambiante, le milieu est traité par une solution aqueuse de KF à10%, les sels sont filtrés et le filtrat est extrait par de l'AcOEt. La phase organique est séchée sur MgSO4 et concentrée sous vide. Par chromatographie sur silice (éluant $CH_2Cl_2$/AcOEt 100/0 à 90/5) l'intermédiaire **561** est obtenu sous forme d'un solide amorphe (0,7 g).
**RMN $^1$H** (400 MHz, DMSO-$d_6$) : δ 8,55 (d, 1H), 8,21-8,18 (2d, 2H), 8,09 (d, 1H), 7,72 (t, 1H), 7,62 (d large, 1H), 7,28 (d, 1H), 6,73 (dd, 1H), 5,69 (d, 1H), 5,66 (d, 1H), 4,98 (quint, 1H), 4,57 (quad, 2H), 1,46 (t, 3H), 1,38-1,26 (2s larges, 9H), 1,3 (d, 3H)
**IR ($cm^{-1}$) :** 3367, 1683-1670

Le traitement de l'intermédiaire **561** selon le protocole décrit pour l'intermédiaire **395** a conduit à l'obtention de **562.**

**Intermédiaire 562:**

**(1-{4-[(1-éthoxyisoquinoléin-5-yl)carbonyl]-2-éthyl-3,5-difluorophényl}éthyl) carbamate de *tert*-butyle**

**RMN $^1$H** (400 MHz, DMSO-$d_6$) : δ 8,54 (d, 1H), 8,2-8,15 (2d, 2H), 8,03 (d, 1H), 7,72 (t, 1H), 7,6 (d large, 1H), 7,25 (d, 1H), 4,94 (quint, 1H), 4,57 (quad, 2H), 2,74-2,64 (2m, 2H), 1,46 (t, 3H), 1,38 (s large, 9H), 1,33 (d, 3H), 1,16 t, 3H)
**LCMS [M+H]+:** 484

L'intermédiaire **412** a été obtenu selon le protocole suivant:

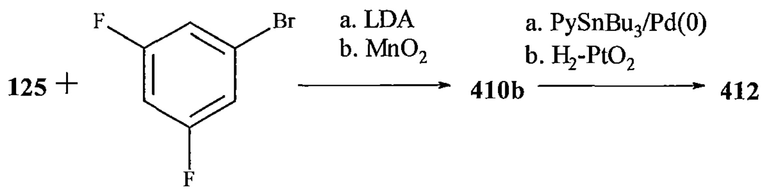


**Intermédiaire 410b :**

**(4-bromo-2,6-difluorophényl)(1-éthoxyisoquinoléin-5-yl)méthanone**

Obtenu par oxydation de l'intermédiaire résultant du couplage du 1-bromo-3,5-difluorobenzène et **125**
**RMN $^1$H** (400 MHz, DMSO-$d_6$) : δ 8,55 (d, 1H), 8,20 (m, 2H), 8,15 (d, 1H), 7,70 (m, 3H), 4,60 (quad, 2H), 1,45 (t, 3H)
**IR ($cm^{-1}$) :** 1668.

**Intermédiaire 412 :**

**[2,6-difluoro-4-(pipéridin-2-yl)phényl](1-éthoxyisoquinoléin-5-yl)méthanone**

<u>Etape 1 :</u>

A une solution dégazée à l'azote de l'intermédiaire **410b** (1,1 g) dans du DMF anhydre (20 mL) est ajouté de la 2-(tributylstannyl)-pyridine (1 g, 2,7 mmoles) et du $Pd(PPh_3)_4$ (500 mg, 0,43 mmoles). Le milieu réactionnel est chauffé à 100°C pendant 20 heures, puis il est dilué par de l'acétate d'éthyle et de l'eau. La phase organique est décantée, lavée à l'eau, séchée sur $MgSO_4$, filtrée et évaporée sous vide. Le résidu est purifié par flash-chromatographie sur silice (éluant : gradient $CR_2Cl_2$-AcOEt : 99-1 à 85-15). L'intermédiaire attendu (700 mg) est obtenu sous la forme d'un solide blanc.
**RMN $^1$H** (400 MHz, DMSO-$d_6$) : δ 8,75 (dd, 1H), 8,56 (d, 1H), 8,21 (dd, 2H), 8,18 (m, 2H), 8,04 (d, 2H), 7,99 (td, 1H), 7,73 (t, 1H), 7,50 (dd, 1H), 4,58 (quad, 2H), 1,46 (t, 3H) **IR ($cm^{-1}$) :** 1668

Etape 2 :

A une solution de l'intermédiaire obtenu ci-dessus (700 mg, 1,79 mmoles) dans 45 mL de méthanol sont ajoutés 0,4 mL d'une solution concentrée d'HCl à 37% et 140 mg de $PtO_2$. Le mélange réactionnel est hydrogèné à température ambiante et sous pression atmosphérique d'$H_2$ pendant 15 heures. Le catalyseur est filtré, et le filtrat est concentré sous vide. Le résidu mis en suspension dans l'eau est traité par une solution aqueuse de soude 10N et extrait à l'acétate d'éthyle. La phase organique est lavée à l'eau, séchée sur $MgSO_4$, filtrée et évaporée sous vide. Le produit est purifié sur Phase Strategy RP 15 μm, éluant : eau-acétonitrile-acide trifluoroacétique. Après évaporation de l'acétonitrile, la phase aqueuse est basifiée par ajout de soude 10N puis extraite à l'acétate d'éthyle. La phase organique est lavée à l'eau, séchée sur $MgSO_4$, filtrée et évaporée sous vide. L'intermédiaire **412** (180 mg) est obtenu sous la forme d'une huile qui cristallise à température ambiante.
**RMN $^1$H** (400 MHz, DMSO-$d_6$) : δ 8,54 (d, 1H), 8,20 (d, 1H), 8,14 (d, 1H), 8,01 (d, 1H), 7,72 (t, 1H), 7,30 (d, 2H), 4,57 (quad., 2H), 3,78 (m, 1H), 3,12 (m, 1H), 2,72 (m, 1H), 1,84 (m, 2H), 1,62 (m, 1H), 1,46 (t, 3H), 1,60-1,30 (m, 3H)
**IR (cm$^{-1}$) :** 1667

Les produits **P66** et **P134** ont été obtenus respectivement à partir des intermédiaires **380** et **732** selon la procédure décrite pour le produit **P68.**

Les autres intermédiaires cétones obtenus par le protocole **XXI** ont été déprotégés en milieu acide (HCl 4N ou éther chlorhydrique 2N) pour conduire aux produits finaux, selon les procédures décrites pour les produits **P17** et **P110.**

| Produit | Issu de | Nomenclature Description analytique |
|---|---|---|
| P41 | 236 | **Chlorhydrate de 5-[(1-amino-4,6-difluoro-2,3-dihydro-1*H*-indén-5-yl)carbonyl] isoquinoléin-1(2*H*)-one**<br>**RMN $^1$H** (400 MHz, DMSO-$d_6$): δ 11,60 (s, 1H), 8,65 (m, 3H), 8,52 (d, 1H), 7,90 (d, 1H), 7,60 (d et t, 2H), 7,40 (s, 2H), 4,85 (m, 1H), 3,10 (m, 1H), 2,92 (m, 1H), 2,60 (m, 1H), 2,12 (m, 1H)<br>**IR (cm$^{-1}$) :** 1687-1671, 1629<br>**HRMS (ESI):** m/z théorique pour $C_{19}H_{15}F_2N_2O_2$ $[M+H]^+$ 341,1102, mesuré 341,1107 |
| P44 | 262 | **Chlorhydrate de 5-[(3-amino-4,6-difluoro-2,3-dihydro-1*H*-indén-5-yl)carbonyl] isoquinoléin-1(2*H*)-one**<br>**RMN $^1$H** (400 MHz, $CDCl_3$): δ 11,70 (s, 1H), 8,65 (s large, 3H), 8,55 (d, 1H), 7,95 (d, 1H), 7,60 (m, 1H), 7,40 (2d, 2H), 7,30 (d, 1H), 4,90 (dd, 1H), 3,35-3,00 (2m, 2H), 2,55-2,20 (2m, 2H)<br>**IR (cm$^{-1}$):** 3250-2480, 1687-1672<br>**HRMS (ESI):** m/z théorique pour $C_{19}H_{15}F_2N_2O_2$ $[M+H]^+$ 341,1102, mesuré 341,1107 |
| P46 | 271 | **Chlorhydrate de 5-[4-(1-aminoéthyl)-2,3-difluorobenzoyl]isoquinoléin-1(2*H*)-one**<br>**RMN $^1$H** (400 MHz, DMSO-$d_6$): δ 11,60 (m, 1H), 9,00-8,50 (m, 3H), 8,50 (d large, 1H), 7,85 (d large, 1H), 7,65 (t large, 1H), 7,60-7,50 (m, 2H), 7,30 (m, 1H), 7,00 (d, 1H), 4,70 (quad, 1H), 1,6 (d, 3H)<br>**IR (cm$^{-1}$) :** 3200-1950, 1671, 1632<br>**HRMS (ESI):** m/z théorique pour $C_{18}H_{15}F_2N_2O_2$ $[M+H]^+$ 329,1101, mesuré 329,1102 |
| P49 | 296 | **Chlorhydrate de 5-{4-[(1*R*)-1-aminoéthyl]-2,6-difluorobenzoyl}-3-méthylisoquinoléin-1 (2*H*)-one**<br>**RMN $^1$H** (400 MHz, DMSO-$d_6$) : δ 11,80 (s, 1H), 8,75 (s, 3H), 8,50 (d, 1H), 7,85 (d, 1H), 7,55 (d, 2H), 7,50 (m, 1H), 7,30 (s, 1H), 4,55 (quad, 1H), 2,30 (s, 3H), 1,55 (d, 3H)<br>**IR (cm$^{-1}$) :** 3237-2450, 1687-1672, 1622<br>**HRMS (ESI):** m/z théorique pour $C_{19}R_{17}F_2N_2O_2$ $[M+H]^+$ 343,1258, mesuré 343,1256 |
| P50 | 305 | **Chlorhydrate de 5-{4-[(1*R*)-1-aminoéthyl]-2,6-difluorobenzoyl}-4-méthylisoquinoléin-1 (2*H*)-one**<br>**RMN $^1$H** (400 MHz, DMSO-$d_6$): δ 11,60 (s, 1H), 8,75 (s, 3H), 8,50 (d, 1H), 7,70 (d, 1H), 7,55 (m, 1H), 7,55 (m, 2H), 7,20 (s, 1H), 4,55 (quad, 1H), 1,95 (s, 3H), 1,55 (d, 3H)<br>**IR (cm$^{-1}$) :** 3158, 3120-2432, 1676, 1633<br>**HRMS (ESI):** m/z théorique pour $C_{19}R_{17}F_2N_2O_2$ $[M+H]^+$ 343,1258, mesuré 343,1274 |
| P53 | 319 | **Chlorhydrate de 5-[(1-amino-4,6-difluoro-2,3-dihydro-1*H*-indén-5-yl)carbonyl] isoquinoléin-1(2*H*)-one** |

(suite)

| Produit | Issu de | Nomenclature Description analytique |
|---|---|---|
| | | **RMN $^1$H** (400 MHz, DMSO-$d_6$): δ 11,60 (s, 1H), 8,65 (m, 3H), 8,52 (d, 1H), 7,90 (d, 1H), 7,60 (d, 1H), 7,60 (t, 1H), 7,40 (s, 2H), 4,85 (m, 1H), 3,10 (m, 1H), 2,92 (m, 1H), 2,60 (m, 1H), 2,12 (m, 1H)<br>**IR (cm$^{-1}$) :** 3600-2600, 1687, 1633<br>**HRMS (ESI) :** m/z théorique pour $C_{19}H_{15}F_2N_2O_2$ [M+H]$^+$ 341,1101, mesuré 341,1101<br>**Pureté optique** (SFC:colonne AD 3μM 4,6x250 mm ; éluant: $CO_2$ / (isopropanol/diéthylamine: 100/0,5) : 70 / 30 ; détection:255nm) : >99%.<br>(absence de **P58**) |
| **P55** | **328** | **Chlorhydrate de 5-{4-[(1*S*)-1-aminoéthyl]-2,6-difluorobenzoyl}-4-méthylisoquinoléin-1 (2*H*)-one**<br>**RMN $^1$H** (400 MHz, DMSO-$d_6$) : δ 11,50 (s, 1H), 8,65 (m, 3H), 8,48 (dd, 1H), 7,70 (d, 1H), 7,50 (t, 1H), 7,50 (d, 2H), 7,15 (d, 1H), 4,55 (s large, 1H), 1,99 (s, 3H), 1,55 (d, 3H)<br>**RMN$^{19}$F** : -108,2<br>**IR (cm$^{-1}$) :** 3500-2500, 1680, 1632<br>**MS (DEI 70 eV) :** m/z 342,1 |
| **P56** | **333** | **Chlorhydrate de 5-{3-[(1*R*)-1-aminoéthyl]-2,6-difluorobenzoyl}isoquinoléin-1(2*H*)-one**<br>**RMN $^1$H** (400 MHz, DMSO-$d_6$) : δ 11,80 (s large, 1H), 8,80 (s large, 3H), 8,55 (d, 1H), 8,00 (d et dd, 2H), 7,60 (t, 1H), 7,40 (t et d, 3H), 4,62 (quad, 1H), 1,60 (d, 3H)<br>**RMN $^{19}$F :** -112, -115<br>**IR (cm$^{-1}$) :** 3450-2480, 1668, 1619, 1589, 1273-1237<br>**HRMS (ESI):** m/z théorique pour $C_{18}H_{15}F_2N_2O_2$ [M+H]$^+$ 329,1102, mesuré 329,1115<br>**Pureté optique** (SFC:colonne ID 5μM 4,6x250 mm ; éluant: $CO_2$ / (isopropanol/n-butylamine: 100/0,5): 75 / 25 ; détection: 254nm) : >99%. (absence de **P57**)<br>$\alpha_D$ (589nM) = -3,09 (c = 0,0097 g/mL, DMSO) à 20°C |
| **P57** | **336** | **Chlorhydrate de 5-{3-[(1*S*)-1-aminoéthyl]-2,6-difluorobenzoyl}isoquinoléin-1(2*H*)-one**<br>**RMN $^1$H** (400 MHz, DMSO-$d_6$): δ 11,80 (s large, 1H), 8,80 (s large, 3H), 8,60 (d, 1H), 8,00 (d et dd, 2H), 7,65 (t, 1H), 7,50 (t et d, 3H), 4,68 (quad, 1H), 1,62 (d, 3H)<br>**RMN 19F :** -111, -115<br>**IR (cm$^{-1}$):** 3450-2480, 1674, 1612, 1589, 1262-1222<br>**HRMS (ESI) :** m/z théorique pour $C_{18}F_2H_{15}N_2O_2$ [M+H]$^+$ 329,1102, mesuré 329,1105<br>**Pureté optique** (SFC: colonne ID 5μM 4,6x250 mm ; éluant: $CO_2$ / (isopropanol/n-butylamine: 100/0,5) : 75 / 25 ; détection:254nm) : >99%.<br>(absence de **P56**)<br>$\alpha_D$ (589nM) = 3,43 (c = 1, DMSO) à 20°C |
| **P58** | **339** | **Chlorhydrate de 5-[(1-amino-4,6-difluoro-2,3-dihydro-1*H*-indén-5-yl)carbonyl] isoquinoléin-1(2*H*)-one**<br>**RMN $^1$H** (400 MHz, DMSO-$d_6$) : δ 11,60 (s, 1H), 8,65 (m, 3H), 8,52 (d, 1H), 7,90 (d, 1H), 7,60 (d, 1H), 7,60 (t, 1H), 7,40 (s, 2H), 4,85 (m, 1H), 3,10 (m, 1H), 2,92 (m, 1H), 2,60 (m, 1H), 2,12 (m, 1H)<br>**IR (cm$^{-1}$) :** 3410-2390, 1675, 1632, 1600, 879-740<br>**HRMS (ESI) :** m/z théorique pour $C_{19}H_{15}F_2N_2O_2$ [M+H]$^+$ 341,1101, mesuré 341,1104<br>**Pureté optique** (SFC:colonne AD 3μM 4,6x250 mm; éluant: $CO_2$ / (isopropanol/ diéthylamine: 100/0,5) : 70/30; détection:255nm): >99%.(absence de **P53**) |
| **P59** | **345** | **Chlorhydrate de 5-{[(3*R*)-3-amino-4,6-difluoro-2,3-dihydro-1*H*-indén-5-yl]carbonyl} isoquinoléin-1(2*H*)-one**<br>**RMN $^1$H** (400 MHz, DMSO-$d_6$) : δ 11,70 (m, 1H), 8,65 (m, 3H), 8,50 (d, 1H), 7,95 (d, 1H), 7,60 (t, 1H), 7,40 (2d, 2H), 7,30 (d, 1H), 4,90 (m, 1H), 3,30 (m, 1H), 3,00 (m, 1H), 2,55 (m, 1H), 2,20 (m, 1H)<br>**IR (cm$^{-1}$) :** 3200-2300, 1657, 1628 |

(suite)

| Produit | Issu de | Nomenclature Description analytique |
|---|---|---|
| | | **HRMS (ESI) :** m/z théorique pour $C_{19}H_{15}F_2N_2O_2$ $[M+H]^+$ 341,1102, mesuré 341,1104<br>**Pureté optique** (SFC:colonne AD-H 3μM 4,6x250 mm ; éluant: $CO_2$ / (méthanol/butylamine: 100/0,5) : 65 / 35 ; détection: 308nm) : >99%.<br>(absence de **P60**)<br>$\alpha_D$ (589nM) = -27,7 (c = 0,009 g/mL, MeOH) à 20°CD |
| **P60** | **348** | **Chlorhydrate de 5-{[(3*S*)-3-amino-4,6-difluoro-2,3-dihydro-1*H*-indén-5-yl]carbonyl} isoquinoléin-1(2*H*)-one**<br>**RMN $^1$H** (400 MHz, DMSO-$d_6$) : δ 11,70 (m, 1H), 8,65 (m, 3H), 8,50 (d, 1H), 7,95 (d, 1H), 7,60 (t, 1H), 7,40 (2d, 2H), 7,30 (d, 1H), 4,90 (m, 1H), 3,30 (m, 1H), 3,00 (m, 1H), 2,55 (m, 1H), 2,2 (m, 1 H)<br>**IR ($cm^{-1}$) :** 3200-2300, 1657, 1627<br>**HRMS (ESI) :** m/z théorique pour $C_{19}H_{15}F_2N_2O_2$ $[M+H]^+$ 341,1102, mesuré 341,1101<br>**Pureté optique** (SFC: colonne AD-H 3μM 4,6x250 mm ; éluant: $CO_2$ / (méthanol/butylamine: 100/0,5) 65 / 35; détection: 308nm) : >99%.<br>(absence de **P59**) |
| **P63** | **369** | **Chlorhydrate de 5-[4-(1-aminoéthyl)-2,6-difluoro-3-méthoxybenzoyl]isoquinoléin-1 (2*H*)-one**<br>**RMN $^1$H** (300 MHz, DMSO-$d_6$) : δ 11,70 (m, 1H), 8,80 (m, 3H), 8,55 (d, 1H), 7,95 (d, 1H), 7,60 (dd, 1H), 7,60 (t, 1H), 7,40 (s large, 2H), 4,70 (quad, 1H), 3,95 (s, 3H), 1,55 (d, 3H)<br>**IR ($cm^{-1}$) :** 3410-2080, 1666, 1629, 1610, 1589, 832-702<br>**HRMS (ESI) :** m/z théorique pour $C_{19}H_{17}F_2N_2O_3$ $[M+H]^+$ 359,1207, mesuré 359,1232 |
| **P65** | **375** | **Chlorhydrate de 5-[4-(1-aminoéthyl)-2,6-difluoro-3-méthoxybenzoyl]-3-méthylisoquinoléin-1(2*H*)-one**<br>**RMN $^1$H** (300 MHz, DMSO-$d_6$) : δ 11,70 (m, 1H), 8,70 (m, 3H), 8,50 (d, 1H), 7,95 (d, 1H), 7,65 (dd, 1H), 7,50 (t, 1H), 7,30 (s, 1H), 4,70 (quad, 1H), 3,90 (s, 3H), 2,30 (s, 3H), 1,55 (d, 3H)<br>**IR ($cm^{-1}$) :** 3430-2250, 1666, 1632, 1595, 838-687<br>**HRMS (ESI) :** m/z théorique pour $C_{20}H_{19}F_2N_2O_3$ $[M+H]^+$ 373,1364, mesuré 373,1365 |
| **P66** | **380** | **Méthanesulfonate de 5-[4-(2-aminopropan-2-yl)-2,6-difluorobenzoyl]-4-méthylisoquinoléin-1(2*H*)-one**<br>**RMN $^1$H** (400 MHz, DMSO-$d_6$) : δ 11,50 (d, 1H), 8,70-8,50 (m, 3H), 8,50 (d, 1H), 7,70 (d, 1H), 7,55 (t, 1H), 7,50 (d, 2H), 7,15 (d large, 1H), 1,95 (s large, 3H), 1,65 (s, 6H)<br>**IR ($cm^{-1}$) :** 3300-2200, 1682, 1634, 1162, 1035<br>**HRMS (ESI) :** m/z théorique pour $C_{20}F_2H_{19}N_2O_2$ $[M+H]^+$ 357,1415, mesuré 357,1423 m/z théorique pour $C_{20}F_2H_{19}N_2O_2$ $[M+H-NH_3]^+$ 340,1149, mesuré 340,1142 |
| **P67** | **389** | **Chlorhydrate de 5-{4-[-1-aminoéthyl]-2-fluoro-3-méthoxybenzoyl}isoquinoléin-1(2*H*)-one, énantiomère 1**<br>**RMN $^1$H** (400 MHz, DMSO-$d_6$) : δ 11,57 (d, 1H), 8,55 (m, 3H), 8,47 (d, 1H), 7,80 (d, 1H), 7,57 (t, 1H), 7,54 (d, 1H), 7,42 (dd, 1H), 7,31 (dd, 1H), 6,96 (d, 1H), 4,67 (quad, 1H), 3,93 (d, 3H), 1,52 (d, 3H)<br>**IR ($cm^{-1}$) :** 3300-2500, 1665, 1627<br>**HRMS (ESI) :** m/z théorique pour $C_{19}H_{18}FN_2O_3$ $[M+H]^+$ 341,1301, mesuré 341,1297; m/z théorique pour $C_{19}FH_{18}N_2O_3$ $[M+H-NH_3]^+$ 324,1036, mesuré 324,1014<br>**Pureté optique** (colonne AD-H 5μM 4,6x250 mm ; éluant: EtOH/ $CH_3CN$/butylamine:95/5/0,1) ; détection: 260nm) : >99%.<br>$\alpha_D$ (589nM) = -6,99 (c = 0,01 g/mL, MeOH) à 20°C |
| **P70** | **395** | **Chlorhydrate de 5-[4-(1-aminoéthyl)-2,6-difluorobenzoyl]-4-éthylisoquinoléin-1(2*H*)-one**<br>**RMN $^1$H** (400 MHz, DMSO-$d_6$) : δ 11,60 (d, 1H), 8,75 (s large, 3H), 8,47 (d, 1H), 7,70 (dd, 1H), 7,52 (t, 1H), 7,52 (d, 2H), 7,08 (d, 1H), 4,52 (s large, 1H), 2,33 (quad, 2H), 1,55 (d, 3H), 1,10 (t, 3H) |

(suite)

| Produit | Issu de | Nomenclature Description analytique |
|---|---|---|
| | | **RMN $^{19}$F** : -108,7<br>**IR ($cm^{-1}$) :** 3300-2000, 1676, 1631<br>**HRMS (ESI) :** m/z théorique pour $C_{20}H_{19}F_2N_2O_2$ $[M+H]^+$ 357,1415, mesuré 357,1415 |
| **P72** | **403** | **Chlorhydrate de 5-{4-[(1R)-1-aminoéthyl]-2,6-difluoro-3-méthoxybenzoyl}isoquinoléin-1 (2*H*)-one**<br>**RMN $^1$H** (400 MHz, DMSO-$d_6$) : δ 11,70 (m, 1H), 8,55 (d, 1H), 8,55 (m, 3H), 7,95 (dd, 1H), 7,60 (t et dd, 2H), 7,40 (m, 2H), 4,70 (quad, 1H), 3,90 (s, 3H), 1,50 (d, 3H)<br>**IR ($cm^{-1}$) :** 3300-2300, 1687, 1649, 1630, 1589<br>**HRMS (ESI) :** m/z théorique pour $C_{19}H_{17}F_2N_2O_3$ $[M+H]^+$ 359,1207, mesuré 359,1189.<br>**Pureté optique** (SFC: colonne ID 5μM 4,6x250 mm ; éluant: $CO_2$ / (isopropanol/butylamine: 100/0,5) : 70 / 30 ; détection: 256nm) : >99%.<br>(absence de **P73**) |
| **P73** | **406** | **Chlorhydrate** de **5-{4-[(1*S*)-1-aminoéthyl]-2,6-difluoro-3-méthoxybenzoyl}isoquinoléin-1 (2*H*)-one**<br>**RMN $^1$H** (400 MHz, DMSO-$d_6$) : δ 11,70 (m, 1H), 8,60 (m, 3H), 8,55 (d, 1H), 7,95 (dd, 1H), 7,60 (t, 1H), 7,60 (dd, 1H), 7,40 (m, 2H), 4,70 (quad, 1H), 3,90 (s, 3H), 1,50 (d, 3H) **IR ($cm^{-1}$) :** 3300-2300, 1687, 1649, 1630, 1589<br>**HRMS (ESI) :** m/z théorique pour $C_{19}H_{17}F_2N_2O_3$ $[M+H]^+$ 359,1207, mesuré 359,1215.<br>**Pureté optique** (colonne ID 5μM 4,6x250 mm ; éluant: $CO_2$/(isopropanol/ butylamine :100/0,5) : 70 / 30 ; détection:<br>256nm) : >99%.<br>(absence de **P72**) |
| **P75** | **412** | **Chlorhydrate de 5-[2,6-difluoro-4-(pipéridin-2-yl)benzoyl]isoquinoléin-1(2*H*)-one**<br>**RMN $^1$H** (400 MHz, DMSO-$d_6$) : δ 11,67 (s large, 1H), 9,46 (m, 2H), 8,54 (d, 1H), 7,90 (d, 1H), 7,59 (m, 1H), 7,59 (m, 2H), 7,41 (m, 2H), 4,37 (dd, 1H), 3,39 (m, 1H), 3,03 (m, 1H), 2,02 (m, 1H), 2,00-1,50 (m, 5H)<br>**IR ($cm^{-1}$) :** 3200-2400, 1673, 1632<br>**HRMS (ESI) :** m/z théorique pour $C_{21}H_{19}F_2N_2O_2$ $[M+H]^+$ 369,1415, mesuré 369,1415. |
| **P80** | **432** | **Chlorhydrate de 5-[4-(1-aminoéthyl)-3-éthyl-2,6-difluorobenzoyl]isoquinoléin-1(2*H*)-one**<br>**RMN $^1$H** (400 MHz, DMSO-$d_6$) : δ 11,70 (m, 1H), 8,80 (m, 3H), 8,50 (dd, 1H), 7,90 (dd, 1H), 7,75 (d, 1H), 7,60 (t, 1H), 7,40 (m, 1H), 7,40 (s large, 2H), 4,65 (quad, 1H), 2,70 (m, 2H), 1,60 (d, 3H), 1,10 (t, 3H)<br>**IR ($cm^{-1}$) :** 3500-2250, 1673-1624, 1594, 788-697<br>**HRMS (ESI) :** m/z théorique pour $C_{20}H_{19}F_2N_2O_2$ $[M+H]^+$ 357,1415, mesuré 357,1396. |
| **P81** | **433** | **Chlorhydrate de 5-{4-[(1*R*)-1-aminoéthyl]-2,6-difluorobenzoyl}-4-chloroisoquinoléin-1 (2*H*)-one**<br>**RMN $^1$H** (400 MHz, DMSO-$d_6$) : δ 12,10-11,60 (s large, 1H), 8,90-8,40 (s large, 3H), 8,42 (d, 1H), 7,77 (d, 1H), 7,63 (t, 1H), 7,53 (s, 1H), 7,47 (m, 2H), 4,50 (quad, 1H), 1,51 (d, 3H)<br>**IR ($cm^{-1}$) :** 3600-2400, 1671, 1648<br>**HRMS (ESI) :** m/z théorique pour $C_{18}H_{14}ClF_2N_2O_2$ $[M+H]^+$ 363,0712, mesuré 363,0706.<br>$\alpha_D$ (589nM) = 4,39 (c = 1, DMSO) à 20°C |
| **P82** | **434** | **Chlorhydrate de 5-{4-[(1*S*)-1-aminoéthyl]-2,6-difluorobenzoyl}-4-chloroisoquinoléin-1 (2*H*)-one**<br>**RMN $^1$H** (400 MHz, DMSO-$d_6$) : δ 12,10-11,60 (s large, 1H), 8,90-8,40 (s large, 3H), 8,42 (d, 1H), 7,77 (d, 1H), 7,63 (t, 1H), 7,53 (s, 1H), 7,47 (m, 2H), 4,50 (quad, 1H), 1,51 (d, 3H)<br>**RMN $^{19}$F :** -107,2<br>**IR ($cm^{-1}$) :** 3600-2400, 1681, 1634<br>**HRMS (ESI) :** m/z théorique pour $C_{18}H_{14}ClF_2N_2O_2$ $[M+H]^+$ 363,0712, mesuré 363,0701. |

(suite)

| Produit | Issu de | Nomenclature Description analytique |
|---|---|---|
| | | $\alpha_D$ (589nM) = -4,4 (c = 1, DMSO) à 20°C |
| **P85** | **448** | **Chlorhydrate de 5-[3-(1-aminoéthyl)-2,6-difluoro-4-méthoxybenzoyl]isoquinoléin-1 (2*H*)-one**<br>**RMN $^1$H** (400 MHz, DMSO-$d_6$) : $\delta$ 11,60 (s large, 1H), 8,50 (d, 1H), 8,29 (s large, 3H), 7,93 (d, 1H), 7,58 (t, 1H), 7,37 (d, 1H), 7,23 (d, 1H), 7,23 (d, 1H), 4,60 (quad, 2H), 3,97 (s, 3H), 1,52 (d, 6H)<br>**RMN $^{19}$F** : -109, -115<br>**IR (cm$^{-1}$) :** 3200-2600, 1674, 1650, 1625, 1144, 1052, 781<br>**HRMS (ESI) :** m/z théorique pour $C_{19}R_{17}F_2N_2O_3$ [M+H]$^+$ 359,1207, mesuré 359,1212. |
| **P88** | **467** | **Chlorhydrate de 5-[3-(2-aminopropan-2-yl)-2,6-difluorobenzoyl]isoquinoléin-1(2*H*)-one**<br>**RMN $^1$H** (400 MHz, DMSO-$d_6$) : $\delta$ 11,70 (s, 1H), 8,83 (s large, 3H), 8,54 (d, 1H), 7,97 (d, 1H), 7,74 (m, 1H), 7,59 (t, 1H), 7,45 (2d, 2H), 7,40 (m, 1H), 1,70 (s, 6H)<br>**RMN $^{19}$F :** -109,7, -112,6<br>**IR (cm$^{-1}$) :** 3200-2500, 1684, 1625<br>**HRMS (ESI) :** m/z théorique pour $C_{19}R_{17}F_2N_2O_2$ [M+H]$^+$ 343,1258, mesuré 343,1265. |
| **P90** | **480** | **Chlorhydrate de 5-[3-(1-aminopropyl)-2,6-difluorobenzoyl]isoquinoléin-1(2*H*)-one**<br>**RMN $^1$H** (400 MHz, DMSO-$d_6$) : $\delta$ 11,70 (s large, 1H), 8,54 (s large, 3H), 8,53 (d, 1H), 7,93 (2m, 2H), 7,59 (t, 1H), 7,45<br>(t, 1H), 7,42 (s, 2H), 4,39 (dd, 1H), 2,01 (m, 1H), 1,88 (m, 1H), 0.82 (t, 3H)<br>**IR (cm$^{-1}$) :** 3350-2500, 1675, 1630, 1026, 1006<br>**HRMS (ESI) :** m/z théorique pour $C_{19}H_{17}F_2N_2O_2$ [M+H]$^+$ 343,1258, mesuré 343,1248<br>**Pureté optique** (SFC: colonne Whelk (S, S) 5$\mu$M 4,6x250 mm ; éluant: $CO_2$ / (isopropanol/Diéthylamine: 100/0,5) : 70 / 30 ; détection: 254nm) : >99%. |
| **P91** | **486** | **Chlorhydrate de 5-[2,6-difluoro-3-(pyrrolidin-2-yl)benzoyl]isoquinoléin-1(2*H*)-one**<br>**RMN $^1$H** (400 MHz, DMSO-$d_6$) : $\delta$ 11,70 (s large, 1H), 9,50 (s large, 2H), 8,54 (d, 1H), 8,00 (d, 1H), 7,92 (m, 1H), 7,59 (t, 1H), 7,42 (m, 3H), 4,76 (dd, 1H), 3,30 (m, 2H), 2,38-2,13-1,99 (m, 4H)<br>**IR (cm$^{-1}$) :** 3250-2400, 1687, 1667, 787<br>**HRMS (ESI) :** m/z théorique pour $C_{20}H_{17}F_2N_2O_2$ [M+H]$^+$ 355,1258, mesuré 355,1267.<br>**Pureté optique** (SFC: colonne ID 5$\mu$M 4,6x250 mm ; éluant: $CO_2$ / (isopropanol/Diéthylamine: 100/0,5) : 70 / 30 ; détection: 255nm) : >99%. (absence de **P99**) |
| **P92** | **494** | **Chlorhydrate de 5-{4-[(1*R*)-1-aminopropyl]-2,6-difluorobenzoyl}isoquinoléin-1(2*H*)-one**<br>**RMN $^1$H** (400 MHz, DMSO-$d_6$) : $\delta$ 11,80 (s large, 1H), 8,70 (m, 3H), 8,50 (d, 1H), 7,90 (d, 1H), 7,60 (t, 1H), 7,55 (d, 2H), 7,40 (m, 2H), 4,30 (dd, 1H), 2,05-1,85 (m, 2H), 0.85 (t, 3H)<br>**IR (cm$^{-1}$) :** 3300-2100, 1670, 1624<br>**HRMS (ESI) :** m/z théorique pour $C_{19}H_{17}F_2N_2O_2$ [M+H]$^+$ 343,1258, mesuré 343,1248. |
| **P93** | **501** | **Chlorhydrate de 5-[4-(1-amino-2-méthylpropyl)-2,6-difluorobenzoyl]isoquinoléin-1 (2*H*)-one**<br>**RMN $^1$H** (300-500 MHz, DMSO-$d_6$) : $\delta$ 11,67 (s large, 1H),<br>8,61 (s large, 3H), 8,54 (d, 1H), 7,90 (d, 1H), 7,59 (t, 1H), 7,49 (d, 2H), 7,39 (s, 2H), 4,13 (d, 1H), 2,21 (m, 1H), 1,06-0.81 (d, 6H)<br>**IR (cm$^{-1}$) :** 3500-2500, 1626, 1524, 1480<br>**RMN $^{19}$F** : -111,3<br>**HRMS (ESI) :** m/z théorique pour $C_{20}H_{19}F_2N_2O_2$ [M+H]$^+$ 357,1415, mesuré 357,1409. |
| **P94** | **507** | **Chlorhydrate de 5-[4-(1-aminobutyl)-2,6-difluorobenzoyl]isoquinoléin-1(2*H*)-one**<br>**RMN $^1$H** (400 MHz, DMSO-$d_6$) : $\delta$ 11,70 (s, 1H), 8,80 (m, 3H), 8,55 (d, 1H), 7,90 (d, 1H), 7,60 (m, 3H), 7,40 (m, 2H), 4,35 (m, 1H), 1,90 (m, 2H), 1,25 (m, 2H), 0.90 (t, 3H)<br>**IR (cm$^{-1}$) :** 3200, 2800, 1632<br>**HRMS (ESI) :** m/z théorique pour $C_{20}H_{19}F_2N_2O_2$ [M+H]$^+$ 357,1415, mesuré 357,1422. |

(suite)

| Produit | Issu de | Nomenclature Description analytique |
|---|---|---|
| P95 | 512 | **Chlorhydrate de 5-[4-(1-amino-3-méthylbutyl)-2,6-difluorobenzoyl]isoquinoléin-1(2*H*)-one**<br>**RMN $^1$H** (400 MHz, DMSO-$d_6$) : δ 11,70 (m, 1H), 8,75 (m, 3H), 8,55 (d, 1H), 7,90 (d, 1H), 7,60 (m, 3H), 7,40 (m, 2H), 4,40 (t, 1H), 1,85 (dd, 2H), 1,45 (m, 1H), 0,90 (2d, 6H)<br>**IR (cm$^{-1}$) :** 3165, 2870, 1658, 1216<br>**HRMS (ESI):** m/z théorique pour $C_{21}H_{21}F_2N_2O_2$ [M+H]$^+$ 371,1571, mesuré 371,1576. |
| P99 | 523 | **Chlorhydrate de 5-[2,6-difluoro-3-(pyrrolidin-2-yl)benzoyl]isoquinoléin-1(2*H*)-one**<br>**RMN $^1$H** (400 MHz, DMSO-$d_6$) : δ 11,69 (d, 1H), 9,64 (m, 2H), 8,55 (d large, 1H), 8,01 (d, 1H), 7,92 (m, 1H), 7,60 (t, 1H), 7,5-7,35 (m, 3H), 4,8 (dd, 1H), 3,3 (m, 2H), 2,38 (m, 1H), 2,2-2,05 (2m, 2H), 2,00 (m, 1H)<br>**IR (cm$^{-1}$) :** 3300-2000, 1686, 1666<br>**HRMS (ESI) :** m/z théorique pour $C_{20}H_{17}F_2N_2O_2$ [M+H]$^+$ 355,1258, mesuré 355,1266.<br>**Pureté optique** (SFC: colonne ID 5μM 4,6x250 mm; éluant: $CO_2$ / (isopropanol/Diéthylamine: 100/0,5) : 70 / 30 ; détection: 255nm) : >99%. (absence de **P91**) |
| P100 | 528 | **Chlorhydrate de 5-[3-(1-aminoéthyl)-2-fluorobenzoyl]isoquinoléin-1(2*H*)-one**<br>**RMN $^1$H** (400 MHz, DMSO-$d_6$) : δ 11,57 (d, 1H), 8,48 (d, 1H), 7,96 (m, 1H), 7,80 (d, 1H), 7,66 (m, 1H), 7,55 (t, 1H), 7,50 (t, 1H), 7,30 (dd, 1H), 6,98 (d, 1H), 6,65 (m, 3H), 4,62 (quad, 1H), 1,55 (d, 3H)<br>**IR (cm$^{-1}$) :** 3167, 3000-2000, 1664, 1630<br>**HRMS (ESI) :** m/z théorique pour $C_{18}H_{16}FN_2O_2$ [M+H]$^+$ 311,1196, mesuré 311,1195.<br>**Pureté optique** (SFC: colonne ID 3μM 4,6x250 mm ; éluant: $CO_2$ / (isopropanol/Diéthylamine: 100/0,5) : 70 / 30 ; détection: 253nm) : >99%. (absence de **P108**) |
| P104 | 546 | **Chlorhydrate de 5-[4-(1-aminoéthyl)-3-fluoro-2-méthoxybenzoyl]isoquinoléin-1(2*H*)-one**<br>**RMN $^1$H** (400 MHz, DMSO-$d_6$) : δ 11,57 (m, 1H), 8,59 (s large, 3H), 8,46 (d large, 1H), 7,71 (dd, 1H), 7,55 (t, 1H), 7,50 (dd, 1H), 7,42 (d, 1H), 7,32 (dd, 1H), 7,08 (d, 1H), 4,68 (quad, 1H), 3,57 (s, 3H), 1,57 (d, 3H)<br>**IR (cm$^{-1}$) :** 3650-2400, 1678, 1651, 783, 752<br>**HRMS (ESI) :** m/z théorique pour $C_{19}H_{18}FN_2O_3$ [M+H]$^+$ 341,1301, mesuré 341,1292 |
| P105 | 555 | **Chlorhydrate de 5-[4-(1-aminoéthyl)-2,6-difluoro-3-méthylbenzoyl]isoquinoléin-1(2*H*)-one**<br>**RMN $^1$H** (400 MHz, DMSO-$d_6$): δ 11,67 (s large, 1H), 8,62 (s large, 3H), 8,53 (dd, 1H), 7,88 (d large, 1H), 7,59 (d, 1H), 7,58 (t, 1H), 7,41 (m, 2H), 4,69 (quad, 1H), 2,26 (s, 3H), 1,53 (d, 3H).<br>**IR (cm$^{-1}$) :** 3350-2000, 1672, 1626<br>**HRMS (ESI) :** m/z théorique pour $C_{19}H_{17}F_2N_2O_2$ [M+H]$^+$<br>343,1253, mesuré 343,1244 |
| P106 | 560 | **Chlorhydrate de 5-[3-(1-aminoéthyl)-2,6-difluoro-4-méthylbenzoyl]isoquinoléin-1(2*H*)-one**<br>**RMN $^1$H** (400 MHz, DMSO-$d_6$) : δ 11,7 (m, 1H), 8,55 (m, 4H), 8,00 (d, 1H), 7,60 (t, 1H), 7,40 (m, 2H), 7,25 (d, 1H), 4,60 (m, 1H), 1,50 (m, 6H).<br>**IR (cm$^{-1}$) :** 2859, 1622, 789-747-709<br>**HRMS (ESI) :** m/z théorique pour $C_{19}H_{17}F_2N_2O_2$ [M+H]$^+$ 343,1253, mesuré 343,1246 |
| P107 | 562 | **Chlorhydrate de 5-[4-(1-aminoéthyl)-3-éthyl-2,6-difluorobenzoyl]isoquinoléin-1(2*H*)-one**<br>**RMN $^1$H** (400 MHz, DMSO-$d_6$) : δ 11,68 (s large, 1H), 8,59 (s large, 3H), 8,54 (d largel, 1H), 7,88 (d large, 1H), 7,64 (d, 1H), 7,59 (t, 1H), 7,41 (m, 2H), 4,67 (quad, 1H), 2,70 (m, 2H), 1,55 (d, 3H), 1,13 (t, 3H)<br>**IR (cm$^{-1}$) :** 3200-2500, 1674, 789<br>**HRMS (ESI) :** m/z théorique pour $C_{20}H_{19}F_2N_2O_2$ [M+H]$^+$ 357,1409, mesuré 357,1403. |
| P128 | 698 | **Chlorhydrate de 5-{4-[amino(3-méthoxyphényl)méthyl]-2,6-difluorobenzoyl}isoquinoléin-1(2*H*)-one** |

(suite)

| Produit | Issu de | Nomenclature Description analytique |
|---|---|---|
| | | **RMN $^1$H** (300 MHz, DMSO-$d_6$) : δ 11,65 (s large, 1H), 9,30 (s large, 3H), 8,50 (d, 1H), 7,90 (d, 1H), 7,55 (m, 3H), 7,40 (m, 3H), 7,30 (s, 1H), 7,15 (d, 1H), 7,00 (dd, 1H), 5,75 (s, 1H), 3,80 (s, 3H)<br>**IR (cm$^{-1}$) :** 3300-2400, 1661, 1627-1591, 1259, 783<br>**HRMS (ESI) :** m/z théorique pour $C_{24}H_{19}F_2N_2O_3$ [M+H]$^+$ 421,1364, mesuré 421,1346 |
| P129 | 704 | **Chlorhydrate de 5-[4-(1-amino-2-cyclohexyléthyl)-2,6-difluorobenzoyl]isoquinoléin-1 (2*H*)-one**<br>**RMN $^1$H** (300 MHz, DMSO-$d_6$) : δ 11,70 (s large, 1H), 8,75 (m, 3H), 8,55 (d, 1H), 7,90 (d, 1H), 7,60 (m, 3H), 7,40 (m, 2H), 4,45 (m, 1H), 1,95-0.85 (3m, 13H)<br>**IR (cm$^{-1}$) :** 3387, 3150-2600, 2923-2851, 1661-1631, 1591, 1260<br>**HRMS (ESI) :** m/z théorique pour $C_{24}H_{25}F_2N_2O_2$ [M+H]$^+$ 411,1884, mesuré 411,1869 |
| P130 | 708 | **Chlorhydrate de 5-(4-{1-[(cyclohexylméthyl)amino]éthyl}-2,6-difluorobenzoyl) isoquinoléin-1(2*H*)-one**<br>**RMN $^1$H** (300 MHz, DMSO-$d_6$) : δ 8,50 (d, 1H), 7,85 (d, 1H), 7,55 (m, 3H), 7,35 (s, 2H), 4,45 (m, 1H), 2,75 (m, 1H), 2,60 (m, 1H), 1,75-0.90 (3m, 11H), 1,65 (d, 3H)<br>**IR (cm$^{-1}$) :** 3163, 3019, 2921-2854, 2661, 1692-1673, 1634-1592,1437,1237<br>**HRMS (ESI) :** m/z théorique pour $C_{25}H_{27}F_2N_2O_2$ [M+H]$^+$ 425,2041, mesuré 425,2042 |
| P131 | 710 | **Chlorhydrate de 5-[4-(1-aminoéthyl)-2,6-difluorobenzoyl]-3-méthylisoquinoléin-1(2*H*)-one**<br>**RMN $^1$H** (400 MHz, DMSO-$d_6$) : δ 11,80-11,60 (m, 1H), 9,00-8,60 (m, 3H), 8,50 (dd, 1H), 7,85 (dd, 1H), 7,55 (d, 2H), 7,50 (t, 1H), 7,30 (s large, 1H), 4,55 (quad, 1H), 2,30 (s, 3H), 1,55 (d, 3H)<br>**IR (cm$^{-1}$) :** 3200-2300, 1672<br>**HRMS (ESI) :** m/z théorique pour $C_{19}F_2H_{17}N_2O_2$ [M+H]$^+$ 343,1258, mesuré 343,1268; m/z théorique pour $C_{19}H_{17}F_2N_2O_2$ [M+H-NH$_3$]$^+$ 326,0993, trouvé 326,0991 |
| P133 | 730 | **Chlorhydrate de 5-{4-(1-aminoéthyl)-5-[2-(diméthylamino)éthoxy]-2-fluorobenzoyl} isoquinoléin-1(2*H*)-one**<br>**RMN $^1$H** (400 MHz, DMSO-$d_6$) : δ 11,70 (m, 1H), 10.80 (m, 1H), 8,60 (m, 3H), 8,50 (d, 1H), 7,80 (d, 1H), 7,60 (t, 1H), 7,60 (d, 1H), 7,40 (d, 1H), 7,35 (dd, 1H), 7,00 (t, 1H), 5,00 (quad, 1H), 4,45 (m, 2H), 3,60 (m, 2H), 2,85 (s large, 6H), 1,55 (d, 3H)<br>**IR (cm$^{-1}$) :** 3367, 2800-2300, 1655, 1627, 1481<br>**HRMS (ESI) :** m/z théorique pour $C_{22}H_{25}FN_3O_3$ [M+H]$^+$ 398,1880, mesuré 398,1881 |
| P134 | 732 | **Méthanesulfonate de 5-[4-(2-aminopropan-2-yl)-2,6-difluorobenzoyl]-3-méthylisoquinoléin-1(2*H*)-one**<br>**RMN $^1$H** (400 MHz, DMSO-$d_6$): δ 11,80-11,60 (m, 1H), 8,50 (d, 1H), 8,50-8,30 (m, 3H), 7,85 (d, 1H), 7,55-7,45 (m, 3H), 7,30 (s, 1H), 2,30 (s large, 3H), 1,70 (s, 6H)<br>**IR(cm$^{-1}$) :** 3300-2000, 1675, 1632, 1176<br>**HRMS (ESI) :** m/z théorique pour $C_{20}H_{19}F_2N_2O_2$ [M+H]$^+$ 357,1415, mesuré 357,1412 |
| P135 | 734 | **Chlorhydrate de 5-[4-(1-aminoéthyl)-2,6-difluoro-3-méthoxybenzoyl]-3-méthylisoquinoléin-1(2*H*)-one**<br>**RMN $^1$H** (300 MHz, DMSO-$d_6$) : δ 11,70 (m, 1H), 8,70 (m, 3H), 8,50 (d, 1H), 7,95 (d, 1H), 7,65 (dd, 1H), 7,50 (t, 1H), 7,30 (s, 1H), 4,70 (quad, 1H), 3,90 (s, 3H), 2,30 (s, 3H), 1,55 (d, 3H)<br>**IR (cm$^{-1}$) :** 3430-2250, 1666, 1632, 1595, 838-687<br>**HRMS (ESI) :** m/z théorique pour $C_{20}H_{19}F_2N_2O_3$ [M+H]$^+$ 373,1364, mesuré 373,1365 |
| P137 | 741 | **Chlorhydrate de 5-{4-[-1-aminoéthyl]-2-fluoro-3-méthoxybenzoyl}isoquinoléin-1(2*H*)-one, énantiomère 2**<br>**RMN $^1$H** (400 MHz, DMSO-$d_6$) : δ 11,60 (d, 1H), 8,70 (s large, 3H), 8,45 (d, 1H), 7,80 (d, 1H), 7,58 (t, 1H), 7,45 (dd,<br>1H), 7,42 (dd, 1H), 7,30 (dd, 1H), 6,95 (d, 1H), 4,68 (quad, 1H), 3,93 (s, 3H), 1,55 (d, 3H)<br>**IR (cm$^{-1}$) :** 3300-2200, 1664, 1621 |

(suite)

| Produit | Issu de | Nomenclature Description analytique |
|---|---|---|
| | | **HRMS (ESI) :** m/z théorique pour $C_{19}FH_{18}N_2O_3$ $[M+H]^+$ 341,1301, mesuré 341,1298; m/z théorique pour $C_{19}H_{18}FN_2O_3$ $[M+H-NH_3]^+$ 324,1012, trouvé 324,1036<br>**Pureté optique** (colonne AD-H 5µM 4,6x250 mm ; éluant: EtOH/ $CH_3CN$/butylamine:95/5/0,1) ; détection: 260nm) : >99%.<br>(absence de **P67)**<br>$\alpha_{\mathbf{D}}$ (589nM) = 6,85 (c = 0,01 g/mL, MeOH) à 20°C |

**Protocole XXII : Méthode alternative de préparation des composés de formule (I) pour lesquels X représente -C(=O)**

Les composés de formule (I) pour lesquels X représente -C(=O)peuvent également être préparés par réaction de couplage par échange halogène metal d'un dérivé halo-isoquinoléine selon l'exemple de la synthèse de l'intermédiaire **106 :**

$$\mathbf{2} + \mathbf{104} \xrightarrow{\text{n-BuLi}} \mathbf{105} \xrightarrow{MnO_2} \mathbf{106}$$

**Intermédiaire 105 :**

**2,2,2-trifluoro-*N*-(1-{3-fluoro-4-[hydroxy(isoquinoléin-5-yl)méthyl]-5-méthoxyphényl}éthyl)acétamide**

A une solution de l'intermédiaire **2** (1 g, 4,8 mmoles) dans du THF anhydre (15 mL) sous $N_2$ et refroidie à -78°C, est ajouté une solution de *n*-BuLi (2,5N/hexane) (2,1 mL) en maintenant la température inférieure à -70°C. La solution résultante est agitée 20 minutes à -78°C puis une solution de l'intermédiaire **104** (1,3 g, 4,4 mmoles) dans du THF anhydre (15 mL) est ajoutée en maintenant la température inférieure à -70°C. Le mélange réactionnel est agité 1 heure à -78°C puis 1h30 à -50°C puis il est hydrolysé, extrait avec de l'AcOEt. La phase organique est lavée à l'eau, séchée sur $MgSO_4$ et évaporée sous vide.

Le résidu est purifié par flash-chromatographie sur silice (éluant : $CH_2Cl_2$ / AcOEt : gradient : 100/0 à 90/10). L'intermédiaire **105** (1 g) est obtenu.

**RMN $^1$H** (300 MHz, DMSO-$d_6$) : δ 9,80 (s large, 1H), 9,27 (s, 1H), 8,42 (d, 1H), 8,14 (d large, 1H), 8,00 (d, 1H), 7,75 (d, 1H), 7,71 (t, 1H), 6,94 (s large, 1H), 6,70 (d large, 1H), 6,65 (dl, 1H), 5,98 (d, 1H), 4,97 (m, 1H), 3,93 (s, 3H), 1,41 (2d, 3H).

**IR ($cm^{-1}$) :** 3400-3100, 1707, 1209-1183-1157, 733

**Intermédiaire 106 :**

**2,2,2-trifluoro-*N*-{1-[3-fluoro-4-(isoquinoléin-5-ylcarbonyl)-5-méthoxyphényl] éthyl}acétamide**

A une solution de (0,68 g 1,61 mmoles) de l'intermédiaire **105** dans du chlorure de méthylène (100 mL) est ajouté du $MnO_2$ (2,6 g, 30 mmoles). Le mélange est agité 17 heures à température ambiante puis le $MnO_2$ est filtré. L'évaporation du filtrat conduit à l'obtention de l'intermédiaire **106** (450 mg) sous la forme d'un solide.

**RMN $^1$H** (300 MHz, DMSO-$d_6$) : δ 9,90 (m, 1H), 9,50 (s, 1H), 8,75 et 8,70 (2d, 2H), 8,45 (d, 1H), 8,00 (d, 1H), 7,80 (t, 1H), 7,10 (s large, 1H), 7,00 (d large, 1H), 5,10 (q, 1H), 3,70 (s, 3H), 1,50 (d, 3H)

**IR ($cm^{-1}$) :** 3125 (faible), 1717, 1677, 1621 et 1590, 1570, 1208-1095, 833-667

Cette séquence a été utilisée pour préparer les intermédiaires suivants :

**Intermédiaire 20 :**

**2,2,2-trifluoro-*N*-{(1*R*)-1-[4-(isoquinoléin-5-ylcarbonyl)-3-méthoxyphényl] éthyl}acétamide**

Obtenu par oxydation de l'intermédiaire résultant du couplage de **18** et **2**

**RMN $^1$H** (300 MHz, DMSO-$d_6$) : δ 9,90 (s large, 1H), 9,40 (s, 1H), 8,60 (d, 1H), 8,35 (d, 1H), 8,30 (d, 1H), 7,80 (d, 1H), 7,42 (t, 1H), 7,50 (d, 1H), 7,15 (s, 1H), 7,10 (d, 1H), 5,10 (m, 1H), 3,53 (s, 3H), 1,50 (d, 3H)

**IR ($cm^{-1}$):** 3125, 1708, 1653, 1608, 1568, 1204-1181-1149, 831-760-734

**Intermédiaire 96**

**2,2,2-trifluoro-*N*-{(1*R*)-1-[8-(isoquinoléin-5-ylcarbonyl)-3,4-dihydro-2*H*-chromén-5-yl]éthyl}acétamide**

Obtenu par oxydation de l'intermédiaire résultant du couplage de **94** et **2**
**RMN $^1$H** (300 MHz, DMSO-$d_6$) : $\delta$ 10,00 (s large, 1H), 9,50 (s, 1H), 8,60 (d, 1H), 8,35 (m, 2H), 7,85 (d, 1H), 7,70 (t, 1H), 7,35 (d, 1H), 7,10 (d, 1H), 5,15 (quad, 1H), 3,80 (t, 2H), 2,80 (2m, 2H), 1,85 (quint, 2H), 1,45 (d, 3H)
**IR ($cm^{-1}$) :** 3395, 1708, 1652, 1610, 1595, 1572 . 1200-1148, 830-700

**Intermédiaire 264 :**

***N*-[(1*R*)-1-{8-[(1-éthoxyisoquinoléin-5-yl)carbonyl]-3,4-dihydro-2*H*-chromén-5-yl}éthyl]-2,2,2-trifluoroacétamide**

Obtenu par oxydation de l'intermédiaire résultant du couplage de **94** et **124**
**RMN 1H** (300 MHz, DMSO-$d_6$) : $\delta$ 10,0 (m, 1H), 8,40 (d, 1H), 8,10 (d, 1H), 7,9-7,75 (2d, 2H), 7,65 (t, 1H), 7,35 (d, 1H), 7,10 (d, 1H), 5,15 (m, 1H), 4,55 (quad, 2H), 3,80 (m, 2H), 2,9-2,65 (m, 2H), 1,95-1,8 (m, 2H), 1,5-1,35 (d et t, 6H)
**IR (cm-1) :** 3294, 1724, 1700, 1656, 1100-1280
**LCMS [M+H]+** = 472
**Pureté optique** (SFC: Colonne IA 3$\mu$M 4,6x250 mm; $CO_2$ / (éthanol/diéthylamine:100/0,5) : 80 / 20; Détection: 215nm) : > 99%.
$\alpha_D$ (589nM) = +65.93 (c=0.010gmL, MeOH, 20°C).

**Intermédiaire 359 :**

***N*-[(1*S*)-1-{8-[(1-éthoxyisoquinoléin-5-yl)carbonyl]-3,4-dihydro-2*H*-chromén-5-yl}éthyl]-2,2,2-trifluoroacétamide**

Obtenu par oxydation de l'intermédiaire résultant du couplage de **357** et **124**
**RMN $^1$H** (400 MHz, DMSO-$d_6$) : $\delta$ 9,99 (s, 1H), 8,41 (d, 1H), 8,09 (d, 1H), 7,84 (d, 1H), 7,80 (d, 1H), 7,66 (dd, 1H), 7,36 (d, 1H), 7,09 (d, 1H), 5,16 (quad, 1H), 4,56 (quad, 2H), 3,80 (m, 2H), 2,87 (dt, 1H), 2,76 (dt, 1H), 1,88 (m, 2H), 1,47 (m, 6H)
**IR ($cm^{-1}$) :** 3286, 1702, 1651
**Pureté optique** (SFC: Colonne IA 3$\mu$M 4,6x250 mm; $CO_2$ / (éthanol/diéthylamine:100/0,5) : 80 / 20; Détection: 215nm) : > 99%.

**Intermédiaire 399 :**

**(2-{3-chloro-4-[(1-éthoxy-4-méthylisoquinoléin-5-yl)carbonyl]phényl}propan-2-yl)carbamate de *tert*-butyle**

Obtenu par oxydation de l'intermédiaire résultant du couplage de **193** et **301**
**RMN $^1$H** (400 MHz, DMSO-$d_6$) : $\delta$ 8,45 (d, 1H), 7,92 (s, 1H), 7,65 (d et t, 3H), 7,50 (d, 1H), 7,45 (dd, 1H), 7,38 (s large, 1H), 4,52 (quad, 2H), 2,18 (s, 3H), 1,55 (s, 6H), 1,45 (t, 3H), 1,35 (s large, 9H)
**IR ($cm^{-1}$):** 3313, 1684-1658

**Intermédiaire 455 :**

***N*-{8-[(1-éthoxyisoquinoléin-5-yl)carbonyl]-3,4-dihydro-2*H*-chromén-4-yl}-2,2,2-trifluoroacétamide**

Obtenu par oxydation de l'intermédiaire résultant du couplage de **453** et **124**
**RMN $^1$H** (400 MHz, DMSO-$d_6$) : $\delta$ 9,95 (s large, 1H), 8,42 (d, 1H), 8,10 (d, 1H), 7,90 (d, 1H), 7,82 (d, 1H), 7,67 (d, 1H), 7,41 (d, 1H), 7,38 (d, 1H), 7,09 (t, 1H), 5,15 (m, 1H), 4,57 (quad, 2H), 3,98 (t, 2H), 2,05 (m, 1H), 1,92 (m, 1H), 1,46 (t, 3H)
**IR ($cm^{-1}$) :** 3280, 1703, 1655

**Intermédiaire 514 :**

***N*-[(1*R*)-1-{8-[(1-éthoxy-4-méthylisoquinoléin-5-yl)carbonyl]-3,4-dihydro-2*H*-chromén-5-yl}éthyl]-2,2,2-trifluoroacétamide**

Obtenu par oxydation de l'intermédiaire résultant du couplage de **94** et **302**
**RMN $^1$H** (300 MHz, DMSO-$d_6$) : δ 10,00 (m, 1H), 7,85 (s, 1H), 7,60 (m, 2H), 7,50 (dd, 1H), 7,10 (d, 1H), 5,15 (quad, 1H), 4,50 (quad, 2H), 3,70 (t, 2H), 2,80 (m, 2H), 2,10 (s, 3H), 1,80 (m, 2H), 1,45 (t, 6H)
**RMN $^{19}$F** :-73, **IR (cm$^{-1}$) :** 3270,1722

**Intermédiaire 544 :**

***N*-[(1*R*)-1-{4-[(1-éthoxyisoquinoléin-5-yl)carbonyl]-3-méthoxyphényl}éthyl]-2,2,2-trifluoroacétamide**

Obtenu par oxydation de l'intermédiaire résultant du couplage de **18** et **124**
**RMN $^1$H** (400 MHz, DMSO-$d_6$) : δ 10,00 (m, 1H), 8,4 (d, 1H), 8,05 (d, 1H), 7,8-7,75 (d et dd, 2H), 7,65 (dd, 1H), 7,5 (d, 1H), 7,15 (d, 1H), 7,05 (d, 1H), 5,01 (quad, 1H), 4,55 (quad, 2H), 3,5 (s, 3H), 1,5 (d, 3H), 1,45 (t, 3H)
**IR (cm$^{-1}$):** 3290, 1698, 1651

**Intermédiaire 641 :**

**2,2,2-trifluoro-*N*-{(1*S*)-1-[4-(isoquinoléin-5-ylcarbonyl)-3-méthoxyphényl] éthyl}acétamide**

Obtenu par oxydation de l'intermédiaire résultant du couplage de **639** et **2**
**RMN $^1$H** (300 MHz, DMSO-d6) : δ 10,00 (s large, 1H, NH), 9,45 (s, 1H), 8,60 (d, 1H), 8,35 (d, 1H), 8,30 (d, 1H), 7,85 (d, 1H), 7,75 (t, 1H), 7,52 (d, 1H), 7,20 (s, 1H), 7,10 (d, 1H), 5,10 (m, 1H), 3,55 (s, 3H), 1,53 (d, 3H)
**IR (cm$^{-1}$) :** 3222, 1710, 1651, 1608, 1569

**Intermédiaire 736 :**

**(2-{3-chloro-4-[(1-éthoxy-3-méthylisoquinoléin-5-yl)carbonyl]phényl}propan-2-yl)carbamate de *tert*-butyle**

Obtenu par oxydation de l'intermédiaire résultant du couplage de **397** et **293**
**RMN $^1$H** (400 MHz, DMSO-$d_6$) : δ 8,42 (d, 1H), 7,85 (s, 1H), 7,70 (d, 1H), 7,60 (t, 1H), 7,52 (d, 1H), 7,45 (s et d, 2H), 7,35 (s large, 1H, NH), 4,52 (quad, 2H), 2,50 (s, 3H), 1,55 (s, 6H), 1,45 (t, 3H), 1,30 (s large, 9H)
**IR (cm$^{-1}$) :** 3259, 1697-1663

**Intermédiaire 750 :**

***N*-({8-[(1-éthoxyisoquinoléin-5-yl)carbonyl]-3,4-dihydro-2*H*-chromén-4-yl}méthyl)-2,2,2-trifluoroacétamide**

Obtenu par oxydation de l'intermédiaire résultant du couplage de **748** et **125**
**RMN $^1$H** (300 MHz, DMSO-$d_6$) : δ 9,65 (s, 1H, NH), 8,40 (d, 1H), 8,09 (d, 1H), 7,82 (d, 1H), 7,81 (dd, 1H), 7,65 (t, 1H), 7,40-7,37 (2d, 2H), 7,07 (t, 1H), 4,55 (quad., 2H), 3,83 (m, 2H), 3,55 (dd, 1H), 3,38 (dd, 1H), 3,08 (m, 1H), 1,87 (m, 1H), 1,70 (m, 1H), 1,47 (t, 3H)
**IR(cm$^{-1}$) :** 3307, 1709, 1653, 1568, 1277, 1155
L'**intermédiaire 221** a été obtenu par oxydation de **220** résultant du couplage entre le 4-bromo-2-fluorobenzaldéhyde et l'intermédiaire **124.**

124 +

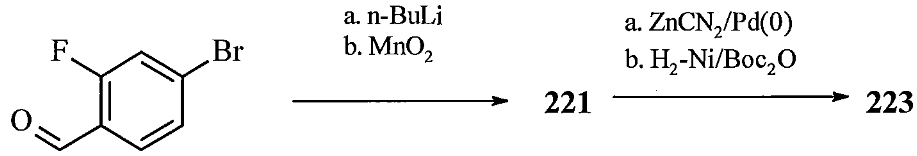

**Intermédiaire 221 :**

**(4-bromo-2-fluorophényl)(1-éthoxyisoquinoléin-5-yl)méthanone**

Etape 1 :

A une solution de l'intermédiaire **124** (2 g, 7,94 mmoles) dans du THF anhydre (40 mL) refroidie à -70°C est ajoutée, en 20 minutes, une solution 2,5N de *n*-BuLi dans l'hexane (3,2 mL, 8 mmoles). La solution résultante est agitée 10 minutes à -70°C puis une solution de 4-bromo-2-fluoro-benzaldéhyde commercial (1,66 g, 8,17 mmoles) dans du THF (15 mL) est ajoutée en 15 minutes. Le mélange réactionnel est agité à -70°C jusqu'à disparition du produit de départ et hydrolysé par une solution aqueuse saturée de chlorure d'ammonium. La phase organique est extraite à l'éther éthylique, lavée par une solution aqueuse saturée en NaCl, séchée sur $MgSO_4$, filtrée et évaporée sous vide. Par recristallisation dans l'acétonitrile l'intermédiaire attendu (1,8 g) est obtenu.
**RMN $^1$H** (400 MHz, DMSO-$d_6$) : $\delta$ 8,16 (d, 1H), 7,98 (d, 1H), 7,80 (d, 1H), 7,62 (t, 1H), 7,50 (d, 1H),7,42 (d, 1H), 7,38 (m, 2H), 6,52 (d, 1H), 6,32 (d, OH), 4,50 (quad., 2H), 1,40 (t, 3H)
**IR (cm$^{-1}$) :** 3252, 1620-1602-1571, 1209-1163, 1038, 867-801-755

Etape 2 :

L'oxydation de l'intermédiaire obtenu ci-dessus en présence de $MnO_2$ conduit à l'intermédiaire **221.**
**RMN $^1$H** (400 MHz, DMSO-$d_6$) : $\delta$ 8,48 (d, 1H), 8,10 (d, 1H), 7,95 (d, 1H), 7,78 à 7,6 (m, 5H), 4,57 (quad, 2H), 1,45 (t, 3H)
**IR (cm$^{-1}$) :** 1650
L'intermédiaire **223** a été obtenu à partir de **221 :**

**Intermédiaire 222 :**

**4-[(1-éthoxyisoquinoléin-5-yl)carbonyl]-3-fluorobenzonitrile**

A une solution de l'intermédiaire **221** (2 g, 5,34 mmoles) dans le DMF (10 mL) dégazée à l'azote, à température ambiante, sont ajoutés du $Zn(CN)_2$ (0,75 g, 6,41 mmoles) et du $Pd(PPh_3)_4$ (0,31 g). Le mélange réactionnel est chauffé à 100°C pendant 45 minutes puis il est hydrolysé avec de l'eau. La phase organique est extraite par de l'acétate d'éthyle, lavée 4 fois par de l'eau et par une solution aqueuse saturée de NaCl, séchée sur $MgSO_4$, filtrée et évaporée sous vide. Le résidu est purifié par flash-chromatographie sur silice (éluant : gradient $CH_2Cl_2$-AcOEt : 100/0 à 95-5. L'intermédiaire **222** attendu (1,6 g) est obtenu sous forme d'un solide.
**RMN $^1$H** (400 MHz, DMSO-$d_6$) : $\delta$ 8,51 (d, 1H), 8,17 (d, 1H), 8,08 (d, 1H), 7,99 (d, 1H), 7,94 (d, 1H), 7,89 (m, 2H), 7,70 (t, 1H), 4,58 (quad, 2H), 1,46 (t, 3H)
**IR (cm$^{-1}$) :** 1651, 2239

**Intermédiaire 223 :**

**{4-[(1-éthoxyisoquinoléin-5-yl)carbonyl]-3-fluorobenzyl}carbamate de *tert*-butyle**

A une solution de l'intermédiaire **222** (250 mg, 0,78 mmoles) dans l'éthanol (5 mL) sont ajoutés du di-tert-butyl dicarbonate (220 mg, 1,04 mmoles) et du Nickel de Raney (200 mg). Le mélange est hydrogéné à pression atmosphérique et température ambiante pendant 18 heures puis chauffé à 70°C pendant 2 heures 30. Après refroidissement, le catalyseur est filtré. Le filtrat est évaporé à sec et purifié par flash-chromatographie sur silice (éluant : gradient $CH_2Cl_2$-AcOEt : 99-1 à 90-10). L'intermédiaire **223** (190 mg) est obtenu.
**RMN $^1$H** (400 MHz, DMSO-$d_6$) : $\delta$ 8,45 (d, 1H), 8,08 (d, 1H), 7,89 (d, 1H), 7,72 à 7,62 (m, 3H), 7,53 (t, 1H), 7,25 (dl, 1H), 7,16 (dl, 1H), 4,56 (quad., 2H), 4,23 (d, 2H), 1,46 (t, 3H), 1,40 (s large, 9H)
**IR (cm$^{-1}$) :** 3360, 1740, 1683-1662, 1525, 1278-1159, 810-783-752
Les intermédiaires cétones protégés sous forme de trifluoro-acetamides ont été déprotégés en milieu basique selon l'exemple de l'intermédiaire **56.**

**Intermédiaire 21:**

**{4-[(1*R*)-1-aminoéthyl]-2-méthoxyphényl}(isoquinoléin-5-yl)méthanone**

**RMN $^1$H** (300 MHz, DMSO-$d_6$) : $\delta$ 9,50 (s, 1H), 8,60 (d, 1H), 8,35 (d, 1H), 8,25 (d, 1H), 7,80 (d, 1H), 7,70 (t,

1H), 7,50 (d, 1H), 7,20 (d, 1H), 7,12 (d, 1H), 4,10 (quad, 1H), 3,52 (s, 3H), 2,10 (m, 2H), 1,35 (d, 3H)
**IR ($cm^{-1}$) :** 3344, 3277, 1646, 1609

**Intermédiaire 265:**

**{5-[(1*R*)-1-aminoéthyl]-3,4-dihydro-2*H*-chromén-8-yl}(1-éthoxyisoquinoléin-5-yl)méthanone**

Obtenu à partir de l'intermédiaire **264**
**RMN $^1$H** (400 MHz, DMSO-$d_6$): δ 8,40 (d, 1H), 8,05 (d, 1H), 7,80 (m, 1H), 7,70 (m, 1H), 7,65 (t, 1H), 7,30 (d, 1H), 7,25 (d, 1H), 4,55 (q, 2H), 4,20 (q, 1H), 3,75 (m, 2H), 2,85-2,70 (2m, 2H), 2,30-1,90 (m, 2H), 1,80 (m, 2H), 1,25 (d, 3H), 1,45 (t, 3H)
**IR ($cm^{-1}$):** 3383, 3269, 1643

**Intermédiaire 360 :**

**{5-[(1*S*)-1-aminoéthyl]-3,4-dihydro-2*H*-chromén-8-yl}(1-éthoxyisoquinoléin-5-yl)méthanone**

**RMN $^1$H** (400 MHz, DMSO-$d_6$) : δ 8,40 (d, 1H), 8,07 (d, 1H), 7,77 (m, 2H), 7,65 (dd, 1H), 7,32 (d, 1H), 7,27 (d, 1H), 4,56 (quad, 2H), 4,19 (quad, 1H), 3,75 (t, 2H), 2,85 (dt, 1H), 2,69 (dt, 1H), 1,83 (m, 4H), 1,47 (t, 3H), 1,24 (d, 3H)
**IR ($cm^{-1}$) :** 3387, 3290, 1643, 1612

**Intermédiaire 456 :**

**(4-amino-3,4-dihydro-2*H*-chromén-8-yl)(1-éthoxyisoquinoléin-5-yl)méthanone**

**RMN $^1$H** (400 MHz, DMSO-$d_6$) : δ 8,40 (d, 1H), 8,08 (d, 1H), 7,81 (d, 1H), 7,79 (d, 1H), 7,64 (t, 1H), 7,62 (dd, 1H), 7,31 (dd, 1H), 7,00 (t, 1H), 4,55 (quad, 2H), 3,94 (m, 1H), 3,90 (m, 1H), 3,83 (m, 1H), 2,04 (s large, 2H), 1,90 (m, 1H), 1,64 (m, 1H), 1,45 (t, 3H)
**IR ($cm^{-1}$):** 3361, 3285, 1651, 1585, 1272, 1235, 807, 756

**Intermédiaire 515 :**

**{5-[(1*R*)-1-aminoéthyl]-3,4-dihydro-2*H*-chromén-8-yl}(1-éthoxy-4-méthylisoquinoléin-5-yl)méthanone**

**RMN $^1$H** (500 MHz, DMSO-$d_6$) : δ 8,31 (d, 1H), 7,86 (s, 1H), 7,57 (t, 1H), 7,52 (d, 1H), 7,52 (d, 1H), 7,28 (d, 1H), 4,51 (quad, 2H), 4,17 (quad, 1H), 3,66 (m, 2H), 2,81 (m, 1H), 2,66 (m, 1H), 2,17 (s, 3H), 1,79 (m, 2H), 1,45 (t, 3H), 1,22 (d, 3H), 1,95 (s large, 2H)
**IR ($cm^{-1}$) :** 3379, 3310, 1653

**Intermédiaire 545 :**

**{4-[(1*R*)-1-aminoéthyl]-2-méthoxyphényl}(1-éthoxyisoquinoléin-5-yl)méthanone**

**RMN $^1$R** (400 MHz, DMSO-$d_6$) : δ 8,39 (dt, 1H), 8,05 (d, 1H), 7,74 (dd, 1H), 7,70 (dd, 1H), 7,64 (dd, 1H), 7,45 (d, 1H), 7,18 (d, 1H), 7,10 (dd, 1H), 4,55 (quad, 2H), 4,05 (quad, 1H), 3,50 (s, 3H), 1,90 (s large, 2H), 1,46 (t, 3H), 1,29 (d, 3H)
**IR ($cm^{-1}$):** 3365, 3300, 1657, 1245, 1033, 810, 788

**Intermédiaire 751 :**

**[4-(aminométhyl)-3,4-dihydro-2*H*-chromén-8-yl](1-éthoxyisoquinoléin-5-yl)méthanone**

**RMN $^1$H** (400 MHz, DMSO-$d_6$): δ 8,40 (d, 1H), 8,05 (d, 1H), 7,78 (d, 1H), 7,78 (d, 1H), 7,62 (t, 1H), 7,45 (d, 1H), 7,30 (d, 1H), 6,98 (t, 1H), 4,53 (quad, 2H), 3,80 (m, 2H), 2,90-2,65 (2dd, 2H), 2,74 (m, 1H), 1,93-1,80 (2m, 2H), 1,50 (s large, 2H), 1,48 (t, 3H)
**IR ($cm^{-1}$) :** 3381, 1654
Les intermédiaires **223, 265, 360, 399, 456, 515, 545** et **751** ont été traités en milieu acide pour conduire aux produits finaux, selon les procédures décrites pour le produit **P17.**

| Produit | Issu de | Nomenclature<br><br>Description analytique |
|---|---|---|
| P3 | 20 | **Dichlorhydrate de {4-[(1*R*)-1-aminoéthyl]-2-méthoxyphényl}(isoquinoléin-5-yl)méthanone**<br>**RMN $^1$H** (400 MHz, DMSO-$d_6$) : δ 9,90 (s, 1H), 8,75 (d, 1H), 8,56 (dl, 2H), 8,05 (dd, 1H), 8,00 (t, 1H), 7,65 (d, 1H), 7,55 (d, 1H), 7,30 (dd, 1H), 4,50 (m, 1H), 3,55 (s, 3H), 1,60 (d, 3H)<br>**IR (cm-1):** 3300-1980, 1656<br>**HRMS (ESI) :** m/z théorique pour C19H19N2O2 [M+H]+ 307,1447, mesuré 307,1472<br>**Pureté optique** (Colonne ADH 3μm 4,6x250 mm ; éluant : $CH_3CN$/isopropanol/triéthylamine) : 85/15/0,1 ; Détection: 325nm : >99%. (absence de **P119)**<br>$\alpha_D$ (589nM) = 1,15 (c = 0,011 g/mL, MeOH) à 20°C |
| P13 | 96 | **Dichlorhydrate de {5-[(1*R*)-1-aminoéthyl]-3,4-dihydro-2*H*-chromén-8-yl}(isoquinoléin-5-yl) méthanone**<br>**RMN $^1$H** (400 MHz, DMSO-$d_6$) : δ 9,80 (s, 1H), 8,70 (d, 1H), 8,60 (m, 5H), 8,05 (d, 1H), 7,90 (t, 1H), 7,50 (d, 1H), 7,30 (d, 1H), 4,55 (m, 1H), 3,80 (t, 2H), 2,80 (dd, 2H), 1,90 (m, 2H), 1,50 (d, 3H)<br>**IR ($cm^{-1}$) :** 3000-2500, 1650<br>**HRMS (ESI) :** m/z théorique pour $C_{21}H_{21}N_2O_2$ $[M+H]^+$ 333,1603, mesuré 333,1593 |
| P14 | 106 | **Dichlorhydrate de [4-(1-aminoéthyl)-2-fluoro-6-méthoxyphényl](isoquinoléin-5-yl) méthanone**<br>**RMN $^1$H** (400 MHz, DMSO-$d_6$) : δ 9,76 (s, 1H), 8,98 (d, 1H), 8,79 (d, 1H), 8,76 (s large, 3H), 8,65 (d, 1H), 8,13 (d, 1H), 7,93 (t, 1H), 7,42 (s, 1H), 7,23 (d, 1H), 4,51 (quint, 1H), 3,72 (s, 3H), 1,59 (d, 1H)<br>**IR ($cm^{-1}$) :** 3000-2500, 1673<br>**HRMS (ESI) :** m/z théorique pour $C_{19}H_{18}FN_2O_2$ $[M+H]^+$ 352,1352, mesuré 325,1352 |
| P45 | 265 | **Chlorhydrate de 5-({5-[(1*R*)-1-aminoéthyl]-3,4-dihydro-2*H*-chromén-8-yl}carbonyl) isoquinoléin-1(2*H*)-one**<br>**RMN $^1$H** (400 MHz, DMSO-$d_6$) : δ 11,60 (s, 1H), 8,80-8,30 (m, 3H), 8,40 (d large, 1H), 7,65 (dd, 1H), 7,50 (t, 1H), 7,40 (d, 1H), 7,30 (2m, 2H), 7,10 (d, 1H), 4,50 (quad, 1H), 3,85 (m, 2H), 2,85-2,75 (2m, 2H), 1,90 (m, 2H), 1,50 (d, 2H)<br>**IR ($cm^{-1}$) :** 3500-1950, 1632<br>**HRMS (ESI) :** m/z théorique pour $C_{21}H_{21}N_2O_3$ $[M+H]^+$ 349,1552, mesuré 349,1543.<br>α**D 589nM** = -19,28 (c = 0,00688 g/mL, MeOH) à 21 °C |
| P62 | 359 | **Chlorhydrate de 5-({5-[(1*S*)-1-aminoéthyl]-3,4-dihydro-2*H*-chromén-8-yl}carbonyl) isoquinoléin-1(2*H*)-one**<br>**RMN $^1$H** (400 MHz, DMSO-$d_6$) : δ 11,50 (d, 1H), 8,48 (m, 3H), 8,41 (d, 1H), 7,66 (d, 1H), 7,51 (t, 1H), 7,38 (d, 1H), 7,27 (m, 2H), 7,09 (d, 1H), 4,54 (quad, 1H), 3,86 (t, 2H), 2,88 (m, 1H), 2,73 (m, 1H), 1,88 (m, 2H), 1,50 (d, 3H)<br>**IR (cm-1) :** 3600-2300, 1632<br>**HRMS (ESI) :** m/z théorique pour C21H21N2O3 [M+H]+ 349,1552, mesuré 349,1564<br>**Pureté optique** (SFC: Colonne AD 3μm 4,6x250 mm ; Composition: $CO_2$ / (éthanol/n butylamine: 100/0,5) : 65/ 35 ; Détection:254nm : >99%. (absence de **P45)** |
| P71 | 399 | **Chlorhydrate de 5-[4-(2-aminopropan-2-yl)-2-chlorobenzoyl]-4-méthylisoquinoléin-1 (2*H*)-one**<br>**RMN $^1$H** (400 MHz, DMSO-$d_6$) : δ 11,50 (d, 1H), 8,82 (m, 3H), 8,46 (m, 1H), 7,85 (d, 1H), 7,78 (d, 1H), 7,68 (dd, 1H), 7,52 (m, 2H), 7,14 (d, 1H), 1,96 (s, 3H), 1,67 (s, 6H).<br>**IR ($cm^{-1}$) :** 3300-2300, 1684, 1662<br>**HRMS (ESI) :** m/z théorique pour $C_{20}H_{20}ClN_2O_2$ [M+H]+ 355,1213, mesuré 355,1212; m/z théorique pour $C_{20}H_{20}ClN_2O_2$ [M+H-NH3]+ 338,0948, mesuré 338,0934 |
| P86 | 455 | **Chlorhydrate de 5-[(4-amino-3,4-dihydro-2*H*-chromén-8-yl)carbonyl]isoquinoléin-1 (2*H*)-one** |

(suite)

| Produit | Issu de | Nomenclature<br><br>Description analytique |
|---|---|---|
| | | **RMN $^1$H** (400 MHz, DMSO-$d_6$) : δ 11,50 (s large, 1H), 8,61 (s large, 3H), 8,42 (d, 1H), 7,75 (2d, 2H), 7,50 (t, 1H), 7,45 (d, 1H), 7,29 (m, 1H), 7,12 (d + t, 2H), 4,55 (t, 1H), 4,10 (m, 1H), 4,00 (m, 1H), 2,20 (m, 1H), 2,05 (m, 1H)<br>**IR (cm$^{-1}$) :** 3100-2700, 1686, 1667, 1237, 793, 761<br>**HRMS (ESI) :** m/z théorique pour $C_{19}H_{17}N_2O_3$ [M+H]+ 321,1239, mesuré 321,1212. |
| **P96** | **514** | **Chlorhydrate de 5-({5-[(1*R*)-1-aminoéthyl]-3,4-dihydro-2*H*-chromén-8-yl}carbonyl)-4-méthylisoquinoléin-1(2*H*)-one**<br>**RMN $^1$H** (300 MHz, DMSO-$d_6$) : δ 11,35 (d, 1H), 8,50 (m, 3H), 8,36 (dd, 1H), 7,60 (d, 1H), 7,48 (t, 1H), 7,37 (dd, 1H), 7,29 (d, 1H), 7,03 (m, 1H), 4,53 (m, 1H), 3,78 (m, 2H), 2,82+2,71 (m, 2H), 1,91 (s, 3H), 1,82 (m, 2H), 1,49 (d, 3H).<br>**IR (cm$^{-1}$) :** 3500-2300, 3350, 1635<br>**HRMS (ESI) :** m/z théorique pour $C_{22}H_{23}N_2O_3$ [M+H]+ 363,1709, mesuré 363,1706. |
| **P103** | **544** | **Chlorhydrate de 5-{4-[(1*R*)-1-aminoéthyl]-2-méthoxybenzoyl}isoquinoléin-1(2*H*)-one**<br>**RMN $^1$H** (400 MHz, DMSO-$d_6$) : δ 11,50 (s large, 1H), 8,46 (s large, 3H), 8,41 (d, 1H), 7,63 (d, 1H), 7,52 (d, 1H),<br>7,51 (t, 1H), 7,41 (s large, 1H), 7,21 (dl, 1H), 7,27 (m, 1H), 7,03 (d, 1H), 4,47 (quad, 1H), 3,61 (s, 3H), 1,56 (d, 3H)<br>**IR (cm-1) :** 3300-2400, 1665, 1643, 1617, 794<br>**HRMS (ESI) :** m/z théorique pour $C_{19}R_{19}N_2O_3$ [M+H]+ 323,1396, mesuré 323,1386. |
| **P119** | **641** | **Dichlorhydrate de {4-[(1*S*)-1-aminoéthyl]-2-méthoxyphényl}(isoquinoléin-5-yl)méthanone**<br>**RMN $^1$H** (300 MHz, DMSO-$d_6$) : δ 9,92 (s, 1H), 8,88 (s large, 3H), 8,76 (dd, 1H), 8,70 (m, 2H), 8,10 (dd, 1H), 8,00 (t, 1H), 7,64 (d, 1H), 7,60 (d fin, 1H), 7,30 (dd, 1H), 4,50 (m, 1H), 3,55 (s, 3H), 1,60 (d, 3H)<br>**IR (cm$^{-1}$) :** 3000-2000, 1656<br>**HRMS (ESI) :** m/z théorique pour C19H19N2O2 [M+H]+ 307,1447, mesuré 307,1447.<br>**Pureté optique** (Colonne ADH 3μm 4,6x250 mm ; éluant : $CH_3CN$/isopropanol/triéthylamine): 85/15/0,1 ; Détection: 325nm : >99%. (absence de **P3)** |
| **P136** | **736** | **Chlorhydrate de 5-[4-(2-aminopropan-2-yl)-2-chlorobenzoyl]-3-méthylisoquinoléin-1 (2*H*)-one**<br>**RMN $^1$H** (400 MHz, DMSO-$d_6$) : δ 11,66 (s, 1H), 8,80 (m, 3H), 8,43 (d, 1H), 7,81 (d, 1H), 7,69 (dd, 1H), 7,65 (d, 1H), 7,60 (d, 1H), 7,45 (t, 1H), 7,17 (s large, 1H), 2,28 (s, 3H), 1,68 (s, 6H)<br>**IR (cm$^{-1}$) :** 3200-2300, 1639.<br>**HRMS (ESI) :** m/z théorique pour C20H20ClN2O2 [M+H]+ 355,1213, mesuré 355,1212 |
| **P139** | **750** | **Chlorhydrate de 5-{[4-(aminométhyl)-3,4-dihydro-2*H*-chromén-8-yl]carbonyl} isoquinoléin-1(2*H*)-one**<br>**RMN $^1$H** (400 MHz, DMSO-$d_6$) : δ 11,50 (s, 1H), 8,40 (d, 1H), 8,20 (m, 3H), 7,70 (d, 1H), 7,50 (t, 1H), 7,50 (d, 1H), 7,35 (d, 1H), 7,30 (dd, 1H), 7,05 (t, 1H), 7,05 (d, 1H), 3,95 (m, 2H), 3,25 (m, 1H), 3,20-3,05 (m, 2H), 2,00 (m, 2H) |
| | | **IR (cm$^{-1}$) :** 3400, 3100, 2900-2800, 1647-1626<br>**HRMS (ESI) :** m/z théorique pour C20H19N2O3 [M+H]+ 335,1396, mesuré 335,1400 |

**Protocole XXIII : Méthode alternative de préparation des composés de formule (I) pour lesquels X représente -C(=O)**

Les composés de formule (I) pour lesquels X représente -C(=O) peuvent être préparés par réaction de substitution électrophile aromatique.

A titre d'exemple la synthèse de l'intermédiaire **54** est décrite ci après :

**Intermédiaire 54 :**

**2,2,2-trifluoro-*N*-{1-[3-hydroxy-4-(isoquinoléin-5-ylcarbonyl)-5-méthylphényl] éthyl}acétamide**

A un mélange de **53** (8,7 g, 33 mmoles) et de **655** (7,6 g, 33 mmoles) dans le chlorure de méthylène (700 mL) à 30°C, est ajouté de l'$AlCl_3$ (8,8 g). Le mélange est chauffé 1 heure à 50°C, puis après retour à température ambiante de l'$AlCl_3$ (8,8 g) est à nouveau ajouté. Le mélange est chauffé à 50°C pendant 24 heures, de l'$AlCl_3$ (8,8 g) est à nouveau ajouté. Après 24 heures de chauffage à 50°C et deux jours à l'ambiante, le mélange est hydrolysé avec précaution sur un mélange de glace et d'eau. Le produit est extrait au chlorure de méthylène, la phase organique est séchée puis concentrée sous vide. Le produit est purifié par chromatographie sur silice (éluant $CR_2Cl_2$AcOEt 90/1) l'intermédiaire **54** (3,8 g) est ainsi obtenu.
**RMN $^1$H** (400 MHz, DMSO-$d_6$) : δ 9,50, 8,72, 8,70, 8,40, 7,90, 7,75, 6,80, 6,70, 4,97, 2,15, 1,50.
**IR (cm$^{-1}$) :** 3331, 3000-2500, 1692-1663, 1165
Les composés **652, 656, 665** et **673** ont été obtenus selon le même protocole.

**Intermédiaire 652 :**

**2,2,2-trifluoro-*N*-{1-[4-(isoquinoléin-5-ylcarbonyl)-3,5-diméthylphényl] éthyl}acétamide**

Obtenu par réaction de **655** avec **651**
**RMN $^1$H** (300/500 MHz, DMSO-$d_6$) : δ 9,90 (s large, 1H, NH), 9,48 (s, 1H), 8,85 (d large, 1H), 8,72 (d, 1H), 8,45 (d, 1H), 7,82 (d, 1H), 7,75 (t, 1H), 7,15 (s, 2H), 5,03 (quad, 1H), 2,10 (s, 6H), 1,51 (d, 3H)
**IR (cm$^{-1}$) :** 3208, 1712, 1653, 1571, 1145, 1185, 1208

**Intermédiaire 656 :**

**2,2,2-trifluoro-*N*-{(1*R*)-1-[3-hydroxy-4-(isoquinoléin-5-ylcarbonyl)phényl] éthyl}acétamide**

Obtenu par réaction de **655** avec **17**
**RMN $^1$H** (300/500 MHz, DMSO-$d_6$) : δ 9,40 (s, 1H), 8,52 (d, 1H), 8,32 (d, 1H), 7,88 (m, 2H), 7,78 (t, 1H), 7,38 (d, 1H), 6,98 (s, 1H), 6,88 (d, 1H), 5,00 (quint, 1H), 1,78 (d, 3H) **IR (cm$^{-1}$) :** 3295, 1707, 1629, 1147 large

**Intermédiaire 665 :**

**2,2,2-trifluoro-*N*-{(1*R*)-1-[7-(isoquinoléin-5-ylcarbonyl)-2,3-dihydro-1-benzofuran-4-yl]éthyl{acétamide**

Obtenu par réaction de **655** et **664**
**RMN $^1$H** (300MHz, DMSO-$d_6$) : δ 10,00 (NH), 9,40, 8,55, 8,30, 8,00, 7,88, 7,75, 7,45, 6,98, 5,00, 4,45, 3,20, 1,48
**IR (cm$^{-1}$) :** 3300, 1709, 1650, 1230-1150

**Intermédiaire 673 :**

**2,2,2-trifluoro-*N*-{1-[3-hydroxy-4-(isoquinoléin-5-ylcarbonyl)-5-méthoxyphényl] éthyl}acétamide**

Obtenu par réaction de **655** et **672**
**RMN $^1$H** (400 MHz, DMSO-$d_6$) : δ 10,1-9,85 (m, 2H), 8,75 (d, 1H), 8,65 (d, 1H), 8,35 (t, 2H), 7,95 (d, 1H), 7,75 (t, 1H), 6,65-6,55 (2s, 2H), 4,98 (m, 1H), 3,60 (s, 3H), 1,50 (d, 3H)
**IR (cm$^{-1}$) :** 3287, 3120-1980,1706-1625

**Intermédiaire 55 :**

**2,2,2-trifluoro-*N*-{1-[4-(isoquinoléin-5-ylcarbonyl)-3-méthoxy-5-méthylphényl] éthyl}acétamide**

Obtenu par réaction de l'iodure de méthyle sur l'intermédiaire **54** selon le protocole décrit pour l'intermédiaire **93 (Protocole XVII)**
**RMN $^1$H** (300 MHz, DMSO-$d_6$) : δ 9,90 (s large, 1H), 9,40 (s, 1H), 8,80 (d, 1H), 8,70 (d, 1H), 8,40 (d large, 1H), 7,85 (d large, 1H), 7,75 (t, 1H), 7,00 (s large, 1H), 6,95 (s large, 1H), 5,05 (quad, 1H), 3,60 (s, 3H), 2,10 (s, 3H), 1,50 (d, 3H)
**IR (cm$^{-1}$) :** 3215, 1724-1658, 1178

**Intermédiaire 659 :**

***N*-{(1*R*)-1-[3-éthyl-4-(isoquinoléin-5-ylcarbonyl)phényl]éthyl}-2,2,2-trifluoroacétamide**

Obtenu à partir de **656** selon la séquence décrite pour l'obtention de **562**
**RMN $^1$H** (300 MHz, DMSO-$d_6$) : δ 9,80 (s large, 1H, NH), 9,50 (s, 1H), 8,62 (d, 1H), 8,40 (d, 1H), 8,30 (d, 1H), 7,82 (d, 1H), 7,74 (t, 1H), 7,40 (s, 2H), 7,25 (m, 2H), 5,05 (quad., 1H), 2,70 (quad, 2H), 1,52 (d, 3H), 1,12 (t, 3H)
**IR (cm$^{-1}$) :** 3296, 1705, 1657

**Intermédiaire 674 :**

**2,2,2-trifluoro-*N*-{1-[4-(isoquinoléin-5-ylcarbonyl)-3,5-diméthoxyphényl] éthyl}acétamide**

Obtenu par réaction de l'iodure de méthyle sur l'intermédiaire **673** selon le protocole décrit pour l'intermédiaire **93 (Protocole XVII)**
**RMN $^1$H** (400 MHz, DMSO-$d_6$) : δ 9,90 (s large, 1H, NH), 9,45 (s, 1H), 8,82 (d, 1H), 8,68 (d, 1H), 8,40 (d, 1H), 7,92 (d, 1H), 7,75 (t, 1H), 6,85 (s, 2H), 5,10 (quad., 1H), 3,65 (s, 6H), 1,52 (d, 3H)
**IR (cm$^{-1}$) :** 3233, 1726-1664, 1118, 852, 763, 614

Les intermédiaires cétones protégés sous forme de trifluoro-acetamides ont été déprotégés en milieu basique pour conduire aux produits finaux, selon l'exemple de l'intermédiaire **56.**

**Intermédiaire 56 :**

**[4-(1-aminoéthyl)-2-méthoxyphényl](isoquinoléin-5-yl)méthanone**

A une solution de **55** (2,67 g, 6,41 mmoles) dans du méthanol est ajoutée une solution de NaOH 1N (46 mL), le milieu réactionnel est agité à température ambiante jusqu'à conversion compléte. Le solvant est évaporé sous vide, le résidu est repris par de l'eau (150 mL) et extrait par du chlorure de méthylène, la phase organique est séchée puis concentrée sous vide. Le résidu est chromatographié sur gel de silice (éluant $CH_2Cl_2$/EtOH/$NH_4OH$ 28% (95/05/0,5)). L'intermédiaire **56** (0.8 g) est obtenu sous forme d'une huile.
**RMN $^1$H** (300 MHz, DMSO-$d_6$): δ 9,40 (s, 1H), 8,80 (d, 1H), 8,70 (d, 1H), 8,40 (dl, 1H), 7,85 (d large, 1H), 7,70 (t, 1H), 7,00 (s large, 1H), 6,95 (s large, 1H), 4,00 (quad, 1H), 3,55 (s, 3H), 2,10 (s, 3H), 1,90 (m, 2H), 1,30 (d, 3H)
**IR (cm$^{-1}$) :** 3500-3250, 1654, 1610, 1571, 834-672

Les intermédiaires obtenus sont transformés en chlorhydrates par traitement avec une solution 2N d'HCl dans l'éther éthylique.

| Produit | Issu de | Nomenclature<br><br>Description analytique |
|---|---|---|
| P7 | 56 | **Dichlorhydrate de [4-(1-aminoéthyl)-2-méthoxy-6-méthylphényl](isoquinoléin-5-yl) méthanone**<br>**RMN $^1$H** (300 MHz, DMSO-$d_6$) : δ 9,80 (s, 1H), 9,10 (s, 1H), 8,80 (d, 1H), 8,70 (d, 1H), 8,65 (d, 1H), 8,00 (d, 1H), 7,98 (t, 1H), 7,35 (s, 1H), 7,12 (s, 1H), 4,40 (m, 1H), 3,60 (s, 3H), 2,20 (s, 3H), 1,60 (d, 3H)<br>**IR (cm$^{-1}$) :** 2710-2076, 1667<br>**HRMS (ESI) :** m/z calculé pour $C_{20}H_{20}N_2O_2$ $[M+H]^+$ 321,1603, trouvé 321,1612 |
| P121 | 652 | **Dichlorhydrate de [4-(1-aminoéthyl)-2,6-diméthylphényl](isoquinoléin-5-yl)méthanone**<br>**RMN $^1$H** (400 MHz, DMSO-$d_6$) : δ 9,80 (s, 1H), 9,10 (d, 1H), 8,85 (d, 1H), 8,68 (d, 1H), 8,60 (s large, 3H), 7,96 (d, 1H), 7,90 (t, 1H), 7,38 (s, 2H), 4,40 (m, 1H), 2,10 (s, 6H), 1,58 (d, 3H)<br>**IR (cm$^{-1}$) :** 3000-2000, 1659, 910-832<br>**HRMS (ESI) :** m/z théorique pour $C_{20}H_{21}N_2O$ $M^{+\cdot}$ 304.1576 mesuré 304,2 |
| P122 | 659 | **Dichlorhydrate de {4-[(1*R*)-1-aminoéthyl]-2-éthylphényl}(isoquinoléin-5-yl)méthanone**<br>**RMN $^1$H** (400 MHz, DMSO-$d_6$) : δ 9,80 (s, 1H), 8,78 (d, 1H), 8,65 (s large, 3H), 8,65 (m, 2H), 7,95 (m, 2H), 7,65 (s, 1H), 7,48 (dd, 1H), 7,40 (d, 1H), 4,48 (m, 1H), 2,72 (quad, 2H), 1,58 (d, 3H), 1,18 (t, 3H) |

(suite)

| Produit | Issu de | Nomenclature<br><br>Description analytique |
|---|---|---|
| | | **IR (cm$^{-1}$) :** 3000-2000, 1662, 918-821<br>**HRMS (ESI) :** m/z théorique pour $C_{20}H_{21}N_2O$ $M^{+\cdot}$ 304,1576, mesuré 304,2 |
| P123 | 665 | **Dichlorhydrate de {4-[(1*R*)-1-aminoéthyl]-2,3-dihydro-1-benzofuran-7-yl}(isoquinoléin-5-yl)méthanone**<br>**RMN $^1$H** (400 MHz, DMSO-$d_6$) : δ 9,90 (s, 1H), 8,70 (2d, 2H), 8,40 (d, 1H), 8,20 (d large, 1H), 8,00 (t, 1H), 7,60 (d, 1H), 7,30 (d, 1H), 4,40 (t et m, 3H), 3,30 (2m, 2H), 1,50 (d, 3H)<br>**IR (cm$^{-1}$) :** 3250-2000, 1651, 1604, 833, 658<br>**HRMS (ESI) :** m/z théorique pour $C_{20}H_{19}N_2O_2$ $[M+H]^+$ 319,1447, mesuré 319,1442 |
| P124 | 674 | **Dichlorhydrate de [4-(1-aminoéthyl)-2,6-diméthoxyphényl](isoquinoléin-5-yl)méthanone**<br>**RMN $^1$H** (400 MHz, DMSO-$d_6$) : δ 9,80 (s, 1H), 9,10 (d, 1H), 8,80 (d, 1H), 8,65 (d large, 1H), 8,10 (d large, 1H), 7,95 (t, 1H), 7,10 (s, 2H), 4,45 (quint, 1H), 3,70 (s, 6H), 1,60 (d, 3H)<br>**IR (cm$^{-1}$) :** 3340, 1970, 1673, 1607, 1582, 831, 759<br>**HRMS (ESI) :** m/z théorique pour $C_{20}H_{21}N_2O_3$ $[M+H]^+$ 337,1552, mesuré 337,1566 |

**Protocole XXIV : Méthode alternative de préparation des composés de formule (I) pour lesquels X représente -C(=O)**

Les composés de formule (I) pour lesquels X représente -C(=O)peuvent être préparés par réaction d'intermédiaire boroniques avec des intermédiaires carbonylés, l'alcool intermédiaire obtenu est ensuite oxydé en cétone, le groupement protecteur de la fonction amine est enlevé.

A titre d'exemple la synthèse de l'intermédiaire **569** est décrite ci-dessous :

a. $Pin_2B$,
$PdCl_2$-dppf.$CH_2Cl_2$
$CH_3COOK$

**125**
a. Rh-t$Bu_3$P,$K_2CO_3$
b. $MnO_2$

**566** → **567** → **569**

**Intermédiaire 567 :**

**{1-[2-fluoro-3-(4,4,5,5-tétraméthyl-1,3,2-dioxaborolan-2-yl)phényl]éthyl)carbamate de *tert*-butyle**

A un mélange de l'intermédiaire **566** (2 g, 6,2 mmoles), d'acétate de potassium (1,2 g) et de Bis(pinacolato)diborane (1,75 g, 6,9 mmoles) dans du 1,4-dioxane dégazé à l'azote (20 mL) est ajouté du $PdCl_2$-dppf.$CH_2Cl_2$ (0,15 g, 3%). Le mélange réactionnel est chauffé au reflux pendant 4 heures. Après retour à température ambiante, le milieu est hydrolysé puis du toluène est ajouté. Après filtration, le filtrat est lavé par de l'eau et une solution aqueuse saturée en NaCl; la phase organique est séchée sur $MgSO_4$, et concentrée. Le résidu est chromatographié sur gel de silice en utilisant un mélange éluant $iPr_2O$/Cyclohexane (10/90 à 100/0). L'intermédiaire **567** (1,9 g) est obtenu sous forme d'une huile.
**RMN $^1$H** (400 MHz, DMSO-$d_6$) : δ 7,50 (2m, 3H), 7,16 (t, 1H), 4,85 (m, 1H), 1,35 (s large, 9H), 1,3 (s, 12H), 1,28 (d, 3H)
**RMN $^{19}$F:** -108,6
**IR (cm$^{-1}$) :** 3341, 1700, 1361
**GC-EI** (70 eV): M+. = 365,2

**Intermédiaire 569 :**

**(1-{3-[(1-éthoxyisoquinoléin-5-yl)carbonyl]-2-fluorophényl}éthyl)carbamate de *tert*-butyle**

Etape 1 :

A un mélange de l'intermédiaire **567** (1,8 g, 4,9 mmoles), de l'intermédiaire **125** (0.986 g, 4,9 mmoles) et de $K_2CO_3$ (1,36 g, 9,8 mmoles) dans le dioxane (18 mL) dégazé à l'argon, sont ajoutés du dimère de chlorure de bis(éthylène)rhodium(I) (38 mg) et de la tri-tert-butyl-phosphine en solution 1M dans le toluène (0,196 mL). Le mélange réactionnel est chauffé 6 heures à 60 °C puis agité 3 jours à température ambiante. Après hydrolyse et extraction à l'$Et_2O$, la phase organique est lavée successivement à l'eau jusqu'à pH=7 puis par une solution aqueuse saturée en NaCl, séchée sur $MgSO_4$, filtrée et évaporée sous vide. Le résidu est purifié par flash-chromatographie sur silice (éluant : $CH_2Cl_2$-THF : 95-5) pour donner l'intermédiaire attendu (1,1 g) sous la forme d'un solide beige
**RMN $^1$H** (400 MHz, DMSO-$d_6$) : δ 8,15 (d, 1H), 7,95 (d, 1H), 7,80 (d, 1H), 7,60 (t, 1H), 7,50 (d, 1H), 7,45 (m, 1H), 7,30-7,15 (m, 1H), 7,10 (m, 1H), 6,53 (m, 1H), 6,18 (m, 1H), 4,85 (m, 1H), 4,50 (quad, 2H), 1,40 (t, 3H), 1,35 (s large, 9H), 1,25 (d, 3H)
**IR (cm$^{-1}$):** 3315, 1683

Etape 2 :

L'intermédiaire obtenu ci-dessus est oxydé en présence de $MnO_2$ (selon le protocole décrit précédement) pour donner l'intermédiaire **569.**
**RMN $^1$H** (400 MHz, DMSO-$d_6$): δ 8,47 (d, 1H), 8,1 (d, 1H), 7,89 (d, 1H), 7,75-7,6 (3m, 3H), 7,6-7,5 (2m, 2H), 7,37 (t, 1H), 4,85 (m, 1H), 4,57 (quad, 2H), 1,46 (t, 3H), 1,36 (s large, 9H), 1,26 (d, 3H)
**IR (cm$^{-1}$):** 3348, 1680-1661

Intermédiaire **587 :**

**(1-{4-[(1-éthoxyisoquinoléin-5-yl)(hydroxy)méthyl]-2-méthoxy-3-méthylphényl} éthyl)carbamate de *tert*-butyle**

Obtenu à partir de **586** et **125** selon le même protocole.
**RMN $^1$H** (400 MHz, DMSO-$d_6$): δ 8,15 (d, 1H), 7,95 (d, 1H), 7,61 (m, 2H), 7,36-7,29 (2d larges, 1H), 7,34 (d, 1H), 7,14-7,11 (2d, 1H), 6,85-6,81 (m, 1H), 6,4 (s, 1H), 4,93 (m, 1H), 4,51 (quad, 2H), 3,73-3,69 (2s, 3H), 2,2 (s large, 3H), 1,43 (t, 3H), 1,34 (s large, 9H), 1,22-1,2 (2d, 3H)
**IR (cm$^{-1}$):** 3343, 1689
L'intermédiaire **587** est oxydé en présence de $MnO_2$ (selon le protocole décrit précédement) pour donner l'intermédiaire **588:**

**Intermédiaire 588 :**

**(1-{4-[(1-éthoxyisoquinoléin-5-yl)carbonyl]-2-méthoxy-3-méthylphényl}éthyl) carbamate de *tert*-butyle**

**RMN $^1$H** (400 MHz, DMSO-$d_6$) : δ 8,44 (d, 1H), 7,79 (d, 1H), 7,67 (t, 1H), 8,10 (d, 1H), 7,74 (d, 1H), 7,50 (d large, 1H), 7,29 (d, 1H), 7,07 (d, 1H), 5,0 (m, 1H), 4,58 (quad, 2H), 3,80 (s, 3H), 2,27 (s, 3H), 1,46 (t, 3H), 1,36 (s, 9H), 1,27 (d, 3H)
**IR (cm$^{-1}$) :** 3352, 1708, 1660
Les intermédiaires cétones obtenus par le protocole **XXIV** ont été déprotégés en milieu acide pour conduire au produit final, selon les procédures décrites pour les produits **P17** et **P110** lorsqu'ils sont protégés sous forme de tert-butyl-carbamates.

| Produit | Issu de | Nomenclature<br><br>Description analytique |
|---|---|---|
| P108 | 569 | **Chlorhydrate de 5-[3-(1-aminoéthyl)-2-fluorobenzoyl]isoquinoléin-1(2*H*)-one**<br>**RMN $^1$H** (400 MHz, DMSO-$d_6$) : δ 11,57 (d, 1H), 8,80-8,40 (m, 3H), 8,47 (d, 1H), 7,96 (td, 1H), 7,81 (d, 1H), 7,66 (td, 1H), 7,56 (t, 1H), 7,47 (t, 1H), 7,31 (dd, 1H), 6,98 (d, 1H), 4,62 (m, 1H), 1,54 (d, 3H) |

(suite)

| Produit | Issu de | Nomenclature<br><br>Description analytique |
|---|---|---|
| | | **IR ($cm^{-1}$) :** 3300-2000, 1663, 1615<br>**HRMS (ESI) :** m/z théorique pour $C_{18}H_{16}FN_2O_2$ $[M+H]^+$ 311,1190, mesuré 311,1184.<br>**Pureté optique** (SFC: colonne ID 3µM 4,6x250 mm ; éluant: $CO_2$ / (isopropanol/Diéthylamine: 100/0,5) : 70 / 30 ;<br>détection: 253nm) : >99%.<br>(absence de **P100)** |
| **P109** | **588** | **Chlorhydrate de 5-[4-(1-aminoéthyl)-3-méthoxy-2-méthylbenzoyl]isoquinoléin-1(2*H*)-one**<br>**RMN $^1H$** (400 MHz, DMSO-$d_6$) : δ 11,58 (d, 1H), 8,51 (m, 3H), 8,46 (d, 1H), 7,66 (d, 1H), 7,55 (t, 1H), 7,55 (d, 1H), 7,32 (dd, 1H), 7,19 (d, 1H), 7,05 (d, 1H), 4,66 (quad, 1H), 3,79 (s, 3H), 2,25 (s, 3H), 1,53 (d, 3H)<br>**IR ($cm^{-1}$) :** 3400-2300, 1681, 1650<br>**HRMS (ESI) :** m/z théorique pour $C_{20}H_{21}N_2O_3$ $[M+H]^+$ 337,1547, mesuré 337,1541.<br>m/z théorique pour $C_{20}H_{18}NO_3$ $[M+H-NH_3]^+$ 320,1281, mesuré 320,1277.<br>m/z mesuré pour $C_{20}H_{19}N_2O_3$ $[M-H]^-$ 335,30. |

**Protocole XXV : Préparation des composés de formule (I) pour lesquels X représente -CH(OH)-**

Les composés de formule (I) pour lesquels X représente -CH(OH)- peuvent être synthétisés selon le **protocole XX,** sans l'étape d'oxydation finale.

L'intermédiaire **147** a été préparé à partir de l'intermédiaire **3** et l'intermédiaire **145** selon la procédure décrite pour l'obtention du produit **P17.**

**Intermédiaire 147 :**

**[(1*R*)-1-{3,5-difluoro-4-[hydroxy(isoquinoléin-5-yl)méthyl] phényl} ethyl]carbamate de *tert*-butyle**

**RMN $^1H$** (300MHz, DMSO-$d_6$): 9,30 (s, 1H), 8,45 (d, 1H), 8,20 (d, 1H), 8,05 (d large, 1H), 7,75 (t, 1H), 7,65 (d large, 1H), 7,40 (d large, 1H), 6,95 (d, 1H), 6,60 (d, 1H), 6,40 (d, 1H), 4,60 (quint, 1H), 1,35 (s, 9H), 1,20 (d, 3H)

L'intermédiaire **147** (1,44 g) a été chromatographié par chromatographie haute pression sur support chiral (Colonne ChiralCell OJ, éluant : n-propylalcool/heptane/diéthylamine 10/90/0,1, détection 270nm) pour donner les deux antipodes optiques **148** (0,6 g) et **149** (0,53 g).

**Intermédiaire 148 :**

**Pureté optique** (Colonne OJ-H, éluant : n-propylalcool/heptane/diéthylamine 10/90/0,1, détection 270nm) : >99%.

**Intermédiaire 149 :**

**Pureté optique** (Colonne OJ-H, éluant : n-propylalcool/heptane/diéthylamine 10/90/0,1, détection 270nm) : 98%.

A une solution de l'intermédiaire **149** (0,5 g) dans du chlorure de méthylène (30 mL) est ajouté en 10 minutes du TFA (1,7 mL). Le mélange réactionnel est agité à température ambiante pendant 20h avant d'être concentré sous vide. Le résidu repris par de l'eau est traité par de la soude à 20% et extrait par du chlorure de méthylène, la phase organique est séchée sur $MgSO_4$. L'évaporation sous pression réduite conduit à l'obtention de l'intermédiaire **150** (0,25 g).

**Intermédiaire 150 :**

**{4-[(1*R*)-1-aminoéthyl]-2,6-difluorophényl}(isoquinoléin-5-yl)méthanol, énantiomère 1**

**Pureté optique** (électrophorèse capillaire : CE standard, $NaH_2PO_4$ 0,05M, pH2,5 - $H_3PO_4$cc/Cyclodextrine

HS α, détection 233nm) : 99%.

L'intermédiaire **148** traité selon le protocole décrit pour l'intermédiaire **150** a conduit à l'intermédiaire **692** :

**Intermédiaire 692** :

**{4-[(1*R*)-1-aminoéthyl]-2,6-difluorophényl}(isoquinoléin-5-yl)méthanol, énantiomère 2**

**Pureté optique** (électrophorèse capillaire : CE standard, $NaH_2PO_4$ 0,05M, pH2,5 - $H_3PO_4$cc/Cyclodextrine HS α, détection 233nm) : >99%.

L'intermédiaire **151** a été préparé à partir de l'intermédiaire **3** et de l'intermédiaire **146** selon la procédure décrite pour l'obtention du produit **P17.**

**Intermédiaire 151 :**

**[(1*S*)-1-{3,5-difluoro-4-[hydroxy(isoquinoléin-5-yl)méthyl]phényl}éthyl]carbamate de *tert*-butyle**

**RMN $^1$H** (300MHz, DMSO-$d_6$): 9,30 (s, 1H), 8,45 (d, 1H), 8,20 (d, 1H), 8,05 (d large, 1H), 7,75 (t, 1H), 7,65 (dlarge, 1H), 7,40 (d large, 1H), 6,95 (d, 1H), 6,60 (d, 1H), 6,40 (d, 1H), 4,60 (quint, 1H), 1,35 (s, 9H), 1,20 (d, 3H)

L'intermédiaire **151** (1,2 g) a été chromatographié par chromatographie haute pression sur support chiral (Colonne ChiralCell OJ, éluant : n-propylalcool/heptane/diéthylamine 10/100/0,1, détection 270nm) pour donner les deux antipodes optiques **152** (0,42 g) et **153** (0,59 g).

**Intermédiaire 152 :**

**Pureté optique** (Colonne OJ-H, éluant : éthanol/heptane/diéthylamine 70/30/0,1 à 5/95/0,1, détection 275nm) : >98%

**Intermédiaire 153 :**

**Pureté optique** (Colonne OJ-H, éluant : éthanol/heptane/diéthylamine 70/30/0,1 à 5/95/0,1, détection 275nm) : >99%.

L'intermédiaire **153** traité selon le protocole décrit pour l'intermédiaire **150** a conduit à l'intermédiaire **154** (0,25 g).

**Intermédiaire 154 :**

**{4-[(1*S*)-1-aminoéthyl]-2,6-difluorophényl}(isoquinoléin-5-yl)méthanol, énantiomère 1 Pureté optique** (électrophorèse capillaire : CE standard, $NaH_2PO_4$ 0,05M, pH2,5 - $H_3PO_4$cc/Cyclodextrine HS α, détection 233nm) : >99%.

Les intermédiaires **150, 154** et **692** sont transformés en chlorhydrates par traitement avec une solution 2N d'HCl dans l'éthér éthylique :

| Produit | Issu de | Nomenclature<br>Description analytique |
|---|---|---|
| **P20** | **150** | **Dichlorhydrate de {4-[(1*R*)-1-aminoéthyl]-2,6-difluorophényl}(isoquinoléin-5-yl) méthanol, énantiomère 1**<br>**RMN $^1$H** (400 MHz, DMSO-$d_6$) : δ 9,82 (s, 1H), 8,73 (m, 3H), 8,66 (d, 1H), 8,45 (m, 2H), 8,14 (d, 1H), 8,04 (t, 1H), 7,35 (d, 2H), 6,75 (s, 1H), 4,40 (m, 1H), 1,48 (d, 3H).<br>**IR ($cm^{-1}$) :** 3231<br>**HRMS (ESI):** m/z théorique pour $C_{18}H_{17}F_2N_2O$ $[M+H]^+$ 315,1309, mesuré 315,1294.<br>**Pureté optique** (électrophorèse capillaire : CE standard, $NaH_2PO_4$ 0,05M, pH2,5 -$H_3PO_4$cc/ Cyclodextrine HS α, détection 233nm) : 99%. |

(suite)

| Produit | Issu de | Nomenclature<br>Description analytique |
|---|---|---|
| P21 | 154 | **Dichlorhydrate de {4-[(1*S*)-1-aminoéthyl]-2,6-difluorophényl}(isoquinoléin-5-yl) méthanol, énantiomère 1**<br>**RMN $^1$H** (400 MHz, DMSO-$d_6$) : δ 9,78 (s, 1H), 8,68 (s large, 3H), 8,65 (d, 1H), 8,44-8,41 (2d, 2H), 8,10 (d, 1H), 8,02 (t, 1H), 7,34 (d, 2H), 6,75 (s, 1H), 4,40 (m, 1H), 1,47 (d, 3H)<br>**IR (cm$^{-1}$) :** 3300-1900<br>**HRMS (ESI) :** m/z théorique pour $C_{18}H_{17}F_2N_2O$ $[M+H]^+$ 315,1309, mesuré 315,1297.<br>**Pureté optique** (électrophorèse capillaire : CE standard, $NaH_2PO_4$ 0,05M, pH2,5 -$H_3PO_4$cc/ Cyclodextrine HS α, détection 233nm) : 99%. |
| P127 | 692 | **Dichlorhydrate de {4-[(1*R*)-1-aminoéthyl]-2,6-difluorophényl}(isoquinoléin-5-yl) méthanol, énantiomère 2**<br>**RMN $^1$H** (400 MHz, DMSO-$d_6$) : δ 9,82 (s, 1H), 8,73 (m, 3H), 8,66 (d, 1H), 8,45 (m, 2H), 8,14 (d, 1H), 8,04 (t, 1H), 7,35 (d, 2H), 6,75 (s, 1H), 4,40 (m, 1H), 1,48 (d, 3H) **IR (cm$^{-1}$) :** 3231, 3200-2300<br>**HRMS (ESI) :** m/z théorique pour $C_{18}H_{17}F_2N_2O$ $[M+H]^+$ 315,1309, mesuré 315,1309.<br>**Pureté optique** (électrophorèse capillaire : CE standard, $NaH_2PO_4$ 0,05M, pH2,5 -$H_3PO_4$cc/ Cyclodextrine HS α, détection 233nm) : 99%. |

**Protocole XXVI : Préparation des composés de formule (I) pour lesquels X représente -$CH_2$-**

Les composés de formule (I) pour lesquels X représente -$CH_2$- peuvent être synthétisés selon les procédures décrites ci-après :

A titre d'exemple la synthèse de l'intermédiaire **144** (1-[3,5-difluoro-4-(isoquinolin-5-ylméthyl) phényl] éthanamine) :

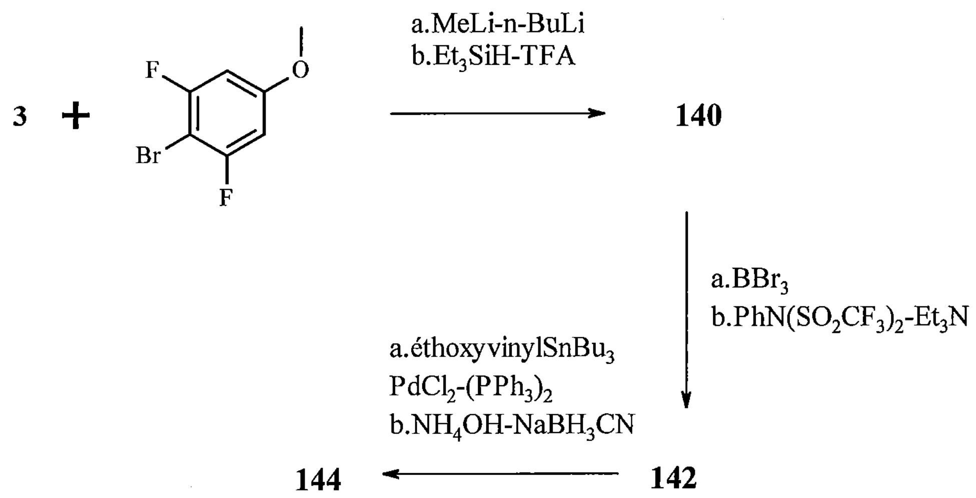


3 + → 140 → 142 → 144

**Intermédiaire 140 :**

**(5-(2,6-difluoro-4-méthoxybenzyl)isoquinoléine)**

Etape 1 :

A une solution de 4-bromo-3,5-difluoroanisole commerciale (25 g, 112 mmoles) dans du THF anhydre (44 mL) refroidie à -70°C, est ajoutée une solution 2,5 N de *n*-BuLi dans le cylcohexane (44 mL, 110 mmoles) en 35 minutes. Le mélange résultant est agité à -70°C pendant 35 minutes puis une solution de (17 g, 108 mmoles) de l'intermédiaire **3** dans du THF (300 mL) est ajoutée en maintenant la température inférieure à -70°C. Le mélange réactionnel est agité une heure à -70°C puis hydrolysé par de l'eau. La phase organique est extraite à l'$Et_2O$, séchée sur $MgSO_4$, filtrée et

évaporée sous vide. Le résidu est purifiée sur Lichroprep RP18 40-60 ($CH_3CN/H_2O$/TFA 95/5/0,1). L'intermédiaire attendu (15,7 g) est obtenu sous la forme d'un solide blanc.
**RMN $^1H$** (300 MHz, DMSO-$d_6$) : δ 9,60 (s, 1H), 8,55 (dd, 1H), 8,35 (m, 1H), 8,25 (m, 1H), 7,85-7,95 (2m, 2H), 6,65-6,75 (d, 2H), 6,60 (s, 1H), 3,75 (s, 3H), 6,0-8,0 (m, 1H)
**IR ($cm^{-1}$) :** 3248, 1050-1200, 1705, 1666

Etape 2 :

L'intermédiaire obtenu ci-dessus (850 mg, 2,82 mmoles) solubilisé dans de l'acide trifluoroacétique (35 mL) est traité par du triéthylsilane (4,5 mL, 28,2 mmoles) à température ambiante. Le mélange résultant est chauffé 1 heure à 70°C. Le milieu réactionnel est versé sur un mélange eau et glace, puis une solution aqueuse à 40% de soude est ajouté. La phase organique extraite à l'acétate d'éthyle est lavée à l'eau, séchée sur $MgSO_4$, filtrée et évaporée sous vide. Le résidu obtenu est purifié par flash-chromatographie sur silice (éluant : $CH_2Cl_2$/AcOEt, gradient 100-0 à 70-30). L'intermédiaire **140** (400 mg) est obtenu sous la forme d'un solide blanc.
**RMN $^1H$** (300 MHz, DMSO-$d_6$) : δ 9,30 (s, 1H), 8,60 (d, 1H), 8,10 (d, 1H), 8,00 (d, 1H), 7,60 (t, 1H), 7,30 (d, 1H), 6,80 (d, 2H), 4,35 (s, 2H), 3,80 (s, 3H)
**IR ($cm^{-1}$) : 1138**

**Intermédiaire 142 :**

**Trifluorométhanesulfonate de 3,5-difluoro-4-(isoquinoléin-5-ylméthyl)phényle**

Etape 1 :

A une solution de l'intermédiaire **140** (8 g, 20 mmoles) dans le chlorure de méthylène (160 mL) refroidie à -70°C. est ajoutée une solution 1N de $BBr_3/CH_2Cl_2$ (48 mL, 48 mmoles). Le mélange réactionnel est agité 2 jours à température ambiante. Le milieu réactionnel refroidi à -70°C et traité par 50 mL de méthanol en 20 minutes est ramené à température ambiante puis concentré sous vide. Le résidu chauffé au reflux dans 160 mL de méthanol pendant 1h30 est alors concentré sous vide. L'intermédiaire attendu (6,9 g) est obtenu sous forme de son bromhydrate.
**RMN $^1H$** (300 MHz, DMSO-$d_6$) : δ 10,35 (s large, 1H), 9,80 (s, 1H), 8,77 (d, 1H), 8,60 (d, 1H), 8,35 (d, 1H), 7,89 (t, 1H), 7,69 (d, 1H), 6,54 (d, 2H), 4,41 (s, 2H)
**IR ($cm^{-1}$)** : 1647-1633,2500-3100

Etape 2 :

A une solution de l'intermédiaire obtenu ci-dessus (500 mg, 1,8 mmoles) et de triéthylamine (1,8 g, 18 mmoles) dans 35 mL de chlorure de méthylène refroidie à -78°C est ajouté du *N*-phényl-bistrifluorométhanesulfonimide (1 g, 2,8 mmoles) en une minute. Le mélange réactionnel est agité 1 heure à -78°C et 1 mL de $Et_3N$ supplémentaire est ajouté. Après agitation pendant 1 heure à -78°C le milieu réactionnel est hydrolysé à -78°C par ajout d'eau. La phase organique est lavée par une solution aqueuse saturée en NaCl, séchée sur $MgSO_4$, filtrée et évaporée sous vide. Le résidu est purifié par flash-chromatographie sur silice (éluant $CH_2Cl_2$-AcOEt: 100-0 à 80-20). On obtient 230 mg de l'intermédiaire **142** sous la forme d'une huile.
**RMN $^1H$** (300 MHz, DMSO-$d_6$) : δ 9,40 (s, 1H), 8,60 (d, 1H), 8,05 (m, 2H), 7,65 (m, 3H), 7,35 (m, 1H), 4,50 (s, 2H)
**IR ($cm^{-1}$):** 1427-1138, 1210

**Intermédiaire 144 :**

**1-[3,5-difluoro-4-(isoquinoléin-5-ylméthyl)phényl]éthanamine**

Etape 1 :

A une solution dégazée à l'azote de l'intermédiaire **142** (500 mg, 1,23 mmoles) dans le DMF (5 mL) sont ajoutés 60 mg (0,22 mmoles) de triphénylphosphine, 15 mg (0,066 mmoles) de $Pd(OAc)_2$, 160 mg (3,77 mmoles) de LiCl et 530 mg (1,46 mmoles) de (1-éthoxy-vinyl)-tributylétain. Le mélange est chauffé à 70°C pendant 5 heures puis agité la nuit à température ambiante. Le milieu traité par une solution aqueuse d'HCl 1N est agité à température ambiante puis basifié par une solution d'$NH_4OH$ à 28%. La phase organique extraite à l'AcOEt est lavée par une solution aqueuse de NaCl, séchée sur $MgSO_4$, filtrée et évaporée à sec. Le résidu est purifié par flash-chromatographie sur silice (éluant : gradient $CH_2Cl_2$-AcOEt : 100-0 à 90-10). L'intermédiaire attendu (150 mg) est obtenu.

**RMN $^1$H (300** MHz, DMSO-$d_6$) : δ 9,35 (s, 1H), 8,60 (d, 1H), 8,10 (d, 1H), 8,05 (d, 1H), 7,70 (d, 2H), 7,60 (t, 1H), 7,35 (d, 1H), 4,50 (s, 2H), 2,60 (s, 3H)
**IR (cm$^{-1}$) :** 1685, 1620, 1579, 1315, 860-831-761

Etape 2 :

A une solution de l'intermédiaire obtenu ci-dessus (650 mg, 2,18 mmoles) dans 13 mL de méthanol est ajouté de l'acétate d'ammonium (2,4 g, 31 mmoles) puis du tamis moléculaire 4 Å en poudre. Après 20 minutes, du cyanoborohydrure de sodium (120 mg, 1,9 mmoles) est ajouté. Le milieu réactionnel est agité une nuit à température ambiante. La solution est filtrée pour éliminer le tamis moléculaire puis le filtrat est évaporé sous vide. Le résidu est traité par HCl 20% auquel est ajouté de l'acétate d'éthyle. La phase aqueuse est décantée, basifiée par ajout de NaOH à 20% et extraite 2 fois au chlorure de méthylène. La phase organique est ensuite séchée sur $MgSO_4$, filtrée et évaporée sous vide. Le résidu est purifiée par flash-chromatographie sur silice (éluant : gradient $CH_2Cl_2$-EtOH : 97-3 à 90-10). L'intermédiaire **144** (260 mg) est obtenu sous forme d'une huile.
**RMN $^1$H** (300 MHz, DMSO-$d_6$) : δ 9,40 (s, 1H), 8,60 (d, 1H), 8,10 (d, 1H), 8,00 (d, 1H), 7,60 (t, 1H), 7,35 (d, 1H), 7,15 (d, 2H), 4,45 (s, 2H), 3,95 (quad., 1H), 1,25 (d, 3H), 1,95 (m, 2H)
**IR (cm$^{-1}$) :** 1309, 3200-3400

**Intermédiaire 530 :**

***N*-[(1*R*)-1-{4-[(1-éthoxyisoquinoléin-5-yl)méthyl]-3-méthoxyphényl}éthyl]-2,2,2-trifluoroacétamide**

Obtenu par deshydroxylation (selon les conditions décrites pour l'intermédiaire **140**) de l'alcool **677,** préparé par couplage de **676** et **3**
**RMN $^1$H** (300 MHz, DMSO-$d_6$) : δ 9,2 (s, 1H), 8,1 (d, 1H), 8,00 (d, 1H), 7,55 (m, 1H), 7,5 (m, 1H), 7,4 (d, 1H), 7,05 (s, 1H), 6,8 (m, 2H), 5,0 (m, 1H), 4,5 (quad., 2H), 4,25 (s, 2H), 3,85 (s, 3H), 1,45 (m, 6H)
**RMN 19F:** -73

**Intermédiaire** 678 :

**2,2,2-trifluoro-*N*-{1-[3-fluoro-4-(isoquinoléin-5-ylméthyl)phényl]éthyl}acétamide**

Obtenu par deshydroxylation (selon les conditions décrites pour l'intermédiaire **140**) de l'alcool **543,** préparé par couplage de **18** et **124**
**RMN $^1$H** (300 MHz, DMSO-$d_6$) : δ 9,80 (d, 1H), 9,30 (s, 1H), 8,50 (d, 1H), 8,00 (d large, 1H), 7,95 (d, 1H), 7,60 (t, 1H), 7,55 (dd, 1H),7,20 (dd, 1H), 7,15 (t, 1H), 7,05 (dd, 1H), 5,0 (quint., 1H), 4,40 (s, 2H), 1,40 (d, 3H)
**IR (cm$^{-1}$):** 3430, 3050, 1703, 1555
Les intermédiaires cétones protégés sous forme de trifluoro-acetamides ont été déprotégés en milieu basique pour conduire aux produits finaux, selon l'exemple de l'intermédiaire **56.** Les intermédiaires obtenus sont transformés en chlorhydrates par traitement avec une solution 2N d'HCl dans l'éther éthylique.

| Produit | Issu de | Nomenclature<br>Description analytique |
|---|---|---|
| P19 | 144 | **Dichlorhydrate de 1-[3,5-difluoro-4-(isoquinoléin-5-ylméthyl)phényl] éthanamine**<br>**RMN $^1$H** (400 MHz, DMSO-$d_6$) : δ 9,90 (s, 1H), 8,80 (m, 3H), 8,80 (d, 1H), 8,35 (d, 1H), 7,90 (t, 1H), 7,70 (d, 1H), 7,45 (d, 2H), 4,60 (s, 2H), 4,45 (m, 1H), 1,55 (d, 3H)<br>**IR (cm$^{-1}$) :** 3000-2500<br>**HRMS (ESI) :** m/z théorique pour $C_{18}H_{17}F_2N_2$ $[M+H]^+$ 299,1360, mesuré 299,1368 |
| P101 | 530 | **Chlorhydrate de 5-{4-[(1*R*)-1-aminoéthyl]-2-méthoxybenzyl}isoquinoléin-1(2*H*)-one**<br>**RMN $^1$H** (400 MHz, DMSO-$d_6$) : δ 11,29 (m, 1H), 8,38 (s large, 3H), 8,11 (d large, 1H), 7,44 (d large, 1H), 7,40 (t, 1H), 7,28 (s large, 1H), 7,16 (m, 1H), 6,94 (d large, 1H), 6,88 (d, 1H), 6,56 (d, 1H), 4,34 (quad, 1H), 4,15 (s, 2H), 3,86 (s, 3H), 1,50 (d, 3H)<br>**IR (cm$^{-1}$) :** 3300-2400, 3173, 1641, 1263, 1036, 782<br>**HRMS (ESI) :** m/z théorique pour $C_{19}H_{21}N_2O_2$ $[M+H]^+$ 309,1603, mesuré 309,1614. |

(suite)

| Produit | Issu de | Nomenclature<br>Description analytique |
|---|---|---|
| P125 | 678 | **Dichlorhydrate de 1-[3-fluoro-4-(isoquinoléin-5-ylméthyl)phényl] éthanamine**<br>**RMN $^1$H** (400 MHz, DMSO-$d_6$) : δ 9,80 (s, 1H), 8,70 (s, 1H), 8,62 (s large, 3H), 8,48 (d, 1H), 8,40 (dd, 1H), 7,90 (m, 2H), 7,48 (d, 1H), 7,28 (m, 2H), 4,58 (s, 2H), 4,38 (m, 1H), 1,50 (d, 3H)<br>**IR (cm$^{-1}$) :** 2552-2505, 2083-1984-1855, 1645-1609, 877-839-815<br>**HRMS (ESI) :** m/z théorique pour $C_{18}H_{18}FN_2$ [M+H]$^+$ 281,1454, mesuré 281,1466 |

**Protocole XXVII**

Lorsque $Ry_3$ représente :

- un groupement -C(=O)-CH$Ry_4$-NH$Ry_5$ avec $Ry_4$ représentant un atome d'hydrogène ou un groupement ($C_1$-$C_6$)alkyle et $Ry_5$ représentant un atome d'hydrogène ou un groupement méthyle, ou
- un groupement -($C_1$-$C_6$)alkyle qui peut être substitué par un groupement hydroxyle, ou
- un groupement -O($C_1$-$C_3$)alkyle, ou
- un groupement cyclohexyle, ou
- un groupement méthylsulfonyle,

les protocoles suivants ont été utilisés.

Les produits finaux ont été préparés à partir d'intermédiaires ou de produits finaux précédement décrits:

**Intermédiaire 418 :**

**(1-amino-4,6-difluoro-2,3-dihydro-1*H*-indén-5-yl)(1-éthoxyisoquinoléin-5-yl)méthanone**

A une solution de l'intermédiaire **417** (2,6 g, 5,5 mmoles) dans le chlorure de méthylène (20 mL) est ajouté de l'acide trifluoroacétique (2 fois 2,07 mL). Le mélange est agité à température ambiante pendant 18h, puis il est dilué par du $CH_2Cl_2$ et traité par de la soude 1N. La phase organique est lavée à l'eau, puis séchée sur $MgSO_4$ et concentrée sous vide. L'intermédiaire **418** (2 g) ainsi obtenu est engagé dans l'étape suivante sans purification supplémentaire.
**RMN $^1$H** (400 MHz, DMSO-$d_6$) : δ 8,50 (d, 1H), 8,20 (2d, 2H), 8,00 (d, 1H), 7,70 (t, 1H), 7,25 (d, 1H), 4,60 (quad, 2H), 4,30 (t et s, 1H), 2,90 (m, 1H), 2,70 (m, 1H), 2,45 (m, 1H), 2,30 (m, 2H), 1,75 (m, 1H), 1,45 (t, 3H)
**IR (cm$^{-1}$) :** 3390, 1667, 1633, 1567, 814-757

**Intermédiaire 421 :**

**[4-(2-aminopropan-2-yl)-2,6-difluorophényl](1-éthoxyisoquinoléin-5-yl)méthanone**

Obtenu à partir de **172** selon le **protocole XXVII**
**RMN $^1$H** (400 MHz, DMSO-$d_6$) : δ 8,50 (d, 1H), 8,2-8,1 (dd, 2H), 8,00 (dd, 1H), 7,75 (dd, 1H), 7,45 (d, 1H), 4,55 (quad, 2H), 2,05 (m, 1H), 2,70 (m, 2H), 1,45 (t, 3H), 1,40 (s, 6H) **IR (cm$^{-1}$) :** 3371, 3302, 1668

**Intermédiaire 426 :**

**(1-amino-4,6-difluoro-2,3-dihydro-1*H*-indén-5-yl)(1-éthoxyisoquinoléin-5-yl)méthanone**

Obtenu à partir de **425** selon le **protocole XXVII**
**RMN $^1$H** (400 MHz, DMSO-$d_6$): δ 8,50 (d, 1H), 8,20 (2d, 2H), 8,00 (d, 1H), 7,70 (t, 1H), 7,25 (d, 1H), 4,60 (quad, 2H), 4,30 (t et s, 1H), 2,90 (m, 2H), 2,70 (m, 1H), 2,45 (m, 1H), 2,30 (m, 2H), 1,75 (m, 1H), 1,45 (t, 3H)
**IR (cm$^{-1}$):** 3390, 1666, 1633, 1567, 814-757

**Intermédiaire 435 :**

**{4-[(1*S*)-1-aminoéthyl]-2,6-difîuorophényl}(1-éthoxyisoquinoléin-5-yl)méthanone** Obtenu à partir de **165** selon le **protocole XXVII**

**RMN $^1$H** (400 MHz, DMSO-$d_6$) : δ 8,55 (d, 1H), 8,20 (d, 1H), 8,15 (d, 1H), 8,00 (d, 1H), 7,70 (t, 1H), 7,30 (d, 2H), 4,60 (quad, 2H), 4,05 (quad, 1H), 2,05 (m, 2H), 1,45 (t, 3H), 1,30 (d, 3H)
**IR ($cm^{-1}$) :** 3380, 3317, 1669, 1633

**Intermédiaire 468 :**

**{4-[(1*R*)-1-aminoéthyl]-2,6-difluorophényl}(1-éthoxyisoquinoléin-5-yl)méthanone**

Obtenu à partir de **170** selon le **protocole XXVII**
**RMN $^1$H** (400 MHz, DMSO-$d_6$) : δ 8,55 (d, 1H), 8,20 (d, 1H), 8,15 (d, 1H), 8,02 (d, 1H), 7,72 (t, 1H), 7,30 (d, 2H), 4,60 (quad, 2H), 4,05 (quad, 1H), 2,02 (m, 2H), 1,45 (t, 3H), 1,30 (d, 3H)
**IR ($cm^{-1}$) :** 3381, 3314, 1670

**Intermédiaire 752 :**

**[4-(1-aminoéthyl)-2,6-difluorophényl](1-éthoxyisoquinoléin-5-yl)méthanone**

Obtenu à partir de **127** selon le **protocole XXVII**
**RMN $^1$H** (400 MHz, DMSO-$d_6$) : δ 8,54 (d large, 1H), 8,19 (d, 1H), 8,13 (d large, 1H), 8,02 (d large, 1H), 7,72 (dd, 1H), 7,31 (m, 2H), 4,57 (quad, 2H), 4,06 (quad, 1H), 2,01 (s large, 2H), 1,46 (t, 3H), 1,30 (d, 3H)
**IR ($cm^{-1}$) :** 3375-3310, 1671
**LCMS [M+H]+=** 356
Les amines obtenues ont été transformées en intermédiaires amides selon les protocoles suivants:

**Intermédiaire 419 :**

**[2-({5-[(1-éthoxyisoquinoléin-5-yl)carbonyl]-4,6-difluoro-2,3-dihydro-1*H*-indén-1-yl}amino)-2-oxoéthyl]carbamate de *tert*-butyle**

A une solution de **418** (0.8 g, 2,1 mmoles) dans $CH_3CN$ (16 mL) sont successivement ajoutés du 1-hydroxybenzotriazole (0,03 g, 0,1éq.), de la *N,N'*-dicyclohexylcarbodiimide (0,71 g), de la *N*-tert-butoxy-carbonyl-glycine (0,38 g). Le mélange est agité à température ambiante pendant 3 jours. Le précipité est filtré, lavé par du $CH_3CN$, le filttrat est concentré sous vide. Le résidu est chromatographié sur gel de silice en utilisant un éluant $CH_2Cl_2$/AcOEt : 80/20 à 60/40. L'intermédiaire **419** (1,16 g) est obtenu sous forme d'un solide amorphe.
**RMN $^1$H** (300 MHz, DMSO-$d_6$) : δ 8,55 (d, 1H), 8,35 (d, 1H), 8,20 (2d, 2H), 8,0 (d, 1H), 7,70 (t, 1H), 7,10 (d, 1H), 7,00 (t, 1H), 5,40 (quad, 1H), 4,60 (quad, 2H), 3,60 (d, 2H), 3,0 (m, 1H), 2,80 (m, 1H), 2,45 (m, 1H), 2,00 (m, 1H), 1,50 (t, 3H), 1,40 (m, 9H)
**IR ($cm^{-1}$) :** 3350, 1720, 1667,1586

**Intermédiaire 427 :**

**[2-({5-[(1-éthoxyisoquinoléin-5-yl)carbonyl]-4,6-difluoro-2,3-dihydro-1*H*-indén-1-yl}amino)-2-oxoéthyl]carbamate de *tert*-butyle**

Obtenu selon le même protocole à partir de **426**
**RMN 1H** (400 MHz, DMSO-$d_6$) : δ 8,55 (d, 1H), 8,35 (d, 1H), 8,20 (2d, 2H), 8,0 (d, 1H), 7,70 (t, 1H), 7,10 (d, 1H), 7,00 (t, 1H), 5,40 (quad, 1H), 4,60 (quad, 2H), 3,60 (d, 2H), 3,0 (m, 1H), 2,80 (m, 1H), 2,45 (m, 1H), 2,00 (m, 1H), 1,50 (t, 3H), 1,40 (m, 9H)
**IR ($cm^{-1}$) :** 3350, 1720, 1667,1586

**Intermédiaire 765 :**

**(2-{[(1*R*)-1-{4-[(1-éthoxyisoquinoléin-5-yl)carbonyl]-3,5-difluorophényl}éthyl]amino}-2-oxoéthyl)carbamate de *tert*-butyle**

A une solution de 468 (1 g, 2,8 mmoles) dans $CH_2Cl_2$ (20 mL) sont successivement ajoutés du 1-hydroxybenzotriazole (0,37 g, 2,8 mmoles), du chlorhydrate de 1-[3-(diméthylamino)propyl]-3-éthylcarbodiimide (0,59 g, 2,8 mmoles), de la N-*tert*-butoxy-carbonyl-glycine (0,49 g, 2,8 mmoles) et de la $Et_3N$ (0,78 mL, 5,6 mmoles). Le mélange est agité à température ambiante pendant 1h. Le mélange dilué par du chlorure de méthylène, est lavée par de la soude 1N, de l'HCl 1N et de l'eau. La phase organique est séchée sur $MgSO_4$ puis concentrée sous vide. Le produit **765** (1,1 g) est obtenu sous forme d'un solide blanc.
**RMN $^1$H** (300 MHz, DMSO-$d_6$) : $\delta$ 8,55 (d, 1H), 8,35 (d, 1H), 8,2 (dd, 2H), 8,15 (d, 1H), 8,02 (d, 1H), 7,70 (d, 1H), 7,25 (d, 2H), 7,0 (t, 1H), 5,02 (quint, 1H), 4,55 (quad, 2H), 3,58 (dd, 2H), 1,48 (t, 3H), 1,45 (d, 3H), 1,4 (s, 9H)
**IR ($cm^{-1}$) :** 3304, 1714, 1675, 1654

**Intermédiaire 436 :**

**(2-{[(1*S*)-1-{4-[(1-éthoxyisoquinoléin-5-yl)carbonyl]-3,5-difluorophényl}éthyl]amino}-2-oxoéthyl)carbamate de *tert*-butyle**

Obtenu selon le même protocole à partir de **435**
**RMN $^1$H** (400 MHz, DMSO-$d_6$) : $\delta$ 8,55 (d, 1H), 8,35 (d, 1H), 8,2-8,1 (dd, 2H), 8,05 (dd, 1H), 7,70 (dd, 1H), 7,25 (d, 2H), 5,00 (m, 1H), 4,55 (quad, 2H), 3,65-3,5 (m, 2H), 1,45 (t, 3H), 1,40 (d, 3H), 1,35 (s, 9H)
**IR ($cm^{-1}$) :** 3311, 1717, 1672, 1637

**Intermédiaire 742 :**

**{2-[(2-{4-[(1-éthoxyisoquinoléin-5-yl)carbonyl]-3,5-difluorophényl}propan-2-yl)amino]-2-oxoéthyl}carbamate de *tert*-butyle**

Obtenu selon le même protocole à partir de **421**
**RMN $^1$H** (400 MHz, DMSO-$d_6$) : $\delta$ 8,55 (m, 1H), 8,20 (d, 1H), 8,15 (d, 1H), 8,00 (d, 1H), 7,75 (dd, 1H), 7,25 (d, 2H), 4,55 (quad, 2H), 3,55 (d, 2H), 1,60 (s, 6H), 1,45 (t, 3H), 1,35 (s, 9H)
**IR ($cm^{-1}$) :** 3500-3200, 1667

Les intermédiaires **419, 427, 468, 436, 742** obtenus ont été déprotégés en milieu acide pour conduire aux produits finaux, selon la procédure décrite pour le produit **P17.**

Les amides ont été obtenus également par réaction selon les protocoles suivants:

**Intermédiaire 224 :**

**{2-[(1-{3,5-difluoro-4-[(1-oxo-1,2-dihydroisoquinoléin-5-yl)carbonyl] phényl}éthyl)amino]-2-oxoéthyl}carbamate de *tert*-butyle**

A une solution -10°C, de *N-tert*-butoxy-carbonyl-glycine (0,48 g, 2,8 mmoles), de $Et_3N$ (0,38 mL, 2,7 mmoles) dans THF (5 mL) traitée par du chloroformiate d'éthyle (0,26mL, 2,7 mmoles) est ajoutée lentement une solution de **P17** (1 g, 2,7 mmoles) et de $Et_3N$ (0,42 mL, 3 mmoles) dans un mélange DMF/THF (13 mL/7,6 mL). Le mélange est agité à température ambiante pendant 20h. Le mélange réactionnel est versé sur de l'eau et extrait à l'AcOEt, séché puis concentré. Le résidu est chromatographié sur gel de silice *via* un dépôt solide en utilisant un éluant $CH_2Cl_2$/EtOH : 97/3. Le produit **224** (0,46 g) est obtenu.
**RMN $^1$H** (400 MHz, DMSO-$d_6$) : $\delta$ 11,62 (m, 1H), 8,51 (d, 1H), 8,36 (d, 1H), 7,90 (d, 1H), 7,57 (t, 1H), 7,37 (m, 2H), 7,24 (m, 2H), 6,97 (t, 1H), 4,99 (quint, 1H), 3,58 (d, 1H), 1,38 (s large, 12H)
**IR ($cm^{-1}$) :** 3305
**LCMS [M+H]+=** 485

**Intermédiaire 320 :**

**{(2*S*,3*S*)-1-[(1-3,5-difluoro-4-[(1-oxo-1,2-dihydroisoquinoléin-5-yl)carbonyl]phényl}éthyl)amino]-3-méthyl-1-oxopentan-2-yl}carbamate de *tert*-butyle**

Obtenu selon le même protocole à partir du **P17** en utilisant la N-*tert*-butoxy-carbonyl-(L)-isoleucine. L'intermédiaire **320** a été directement transformé en **P55** par traitement selon le protocole décrit pour **P17.**

**Intermédiaire 392 :**

**{2-[(1-{3,5-difluoro-4-[(1-oxo-1,2-dihydroisoquinoléin-5-yl)carbonyl]phényl}éthyl)amino]-2-oxoéthyl}méthylcarbamate de *tert*-butyle**

Obtenu selon le même protocole à partir du **P17** en utilisant la méthyl-N-*tert*-butoxy-carbonyl-glycine
**RMN $^1$H** (300 MHz, DMSO-$d_6$) : δ 11,32 (m, 1H), 8,52 (d, 1H), 8,15 (d large, 1H), 7,87 (d, 1H), 7,55 (t, 1H), 7,32 (dd, 2H), 7,21 (d, 2H), 5,03 (m, 1H), 3,83 (dd, 1H), 2,85 (s, 3H), 1,44 (d, 3H), 1,38 (s, 9H)
**IR ($cm^{-1}$) :** 3303, 3200-2500, 1699, 1672, 1660, 1632

**Intermédiaire 420 :**

**(2-{[(1*S*)-1-{3-méthyl-4-[(1-oxo-1,2-dihydroisoquinoléin-5-yl)carbonyl]phényl}éthyl]amino}-2-oxoéthyl)carbamate de *tert*-butyle**

Obtenu selon le même protocole à partir du **P53** en utilisant la N-*tert*-butoxy-carbonyl-glycine
**RMN $^1$H** (400 MHz, DMSO-$d_6$) : δ 11,50 (m, 1H), 8,40 (d, 1H), 8,35 (d, 1H), 7,65 (dd, 1H), 7,50 (t, 1H), 7,30 (d, 1H), 7,25 (d, 1H), 7,20 (m, 2H), 6,90 (t, 1H), 6,75 (d, 1H), 4,95 (m, 1H), 3,60 (d, 2H), 1,40 (s, 3H), 1,35 (m, 12H)
**IR ($cm^{-1}$) :** 3600-2500, 1695, 1633, 1505
**LCMS [M+H]+=** 463

Les intermédiaires ainsi obtenus ont été déprotégés en milieu acide pour conduire aux produits finaux, selon la procédure décrite pour le produit **P110** lorsqu'ils sont protégés sous forme de tert-butyl-carbamates.

Les amines obtenues ont été transformées en intermédiaires alkyles selon les protocoles suivants:

**Intermédiaire 469 :**

**{2,6-difluoro-4-[(1*R*)-1-{[2-(tétrahydro-2*H*-pyran-2-yloxy)éthyl]amino}éthyl] phényl}(1-éthoxyisoquinoléin-5-yl)méthanone**

A une solution de **468** (1 g, 2,8 mmoles) dans le DMF (10 mL) sont ajoutés du $K_2CO_3$ (1,2 g, 8,4 mmoles) et du 2(2-bromoéthoxy)-tetrahydro-2H-pyrane (0,46 mL, 3,08 mmoles). Le mélange est chauffé à 80°C pendant 20h. Le solvant est évaporé sous vide, le résidu repris par de l'eau est extrait par du chlorure de méthylène, la phase organique est séchée sur $MgSO_4$ puis concentrée sous vide. Le résidu est chromatographié sur gel de silice en utilisant un éluant $CH_2Cl_2$/EtOH : 98-2. Le produit **469** (0,59 g) est obtenu sous forme d'une huile.
**RMN $^1$H** (400 MHz, DMSO-$d_6$) : δ 8,55 (d, 1H), 8,20 (d, 1H), 8,15 (d, 1H), 8,05 (d, 1H), 7,72 (d, 1H), 7,30 (d, 2H), 4,58 (quad et m, 3H), 3,85 (quad, 1H), 3,75-3,45 (2m, 2H), 3,68-3,45 (2m, 2H), 2,65-2,5 (m, 2H), 2,30 (m, 1H), 1,75-1,65 (2m, 2H), 1,50 (t et m, 7H), 1,30 (d, 3H)
**IR ($cm^{-1}$) :** 3333, 1674

**Intermédiaire 437 :**

**{2,6-difluoro-4-[(1*S*)-1-{[2-(tétrahydro-2*H*-pyran-2-yloxy)éthyl]amino}éthyl] phényl}(1-éthoxyisoquinoléin-5-yl)méthanone**

Obtenu selon le même protocole à partir de l'intermédiaire **435**
**RMN $^1$H** (400 MHz, DMSO-$d_6$) : δ 8,55 (d, 1H), 8,20 (d, 1H), 8,15 (d, 1H), 8,05 (d, 1H), 7,72 (d, 1H), 7,30 (d, 2H), 4,58 (quad et m, 3H), 3,85 (quad, 1H), 3,75-3,45 (2m, 2H), 3,68-3,45 (2m, 2H), 2,65-2,5 (m, 2H), 2,30 (m, 1H), 1,75-1,65 (2m, 2H), 1,50 (t et m, 7H), 1,30 (d, 3H)
**IR ($cm^{-1}$) :** 3328, 1671

**Intermédiaire 285 :**

**[2,6-difluoro-4-(1-{[2-(tétrahydro-2*H*-pyran-2-yloxy)éthyl]amino}éthyl)phényl](1-éthoxyisoquinoléin-5-yl)méthanone**

Cette procédure a également été utilisée pour la préparation de l'intermédiaire **285,** mélange racémique des intermédiaires **469** et **437.**

**Intermédiaire 422 :**

**[2,6-difluoro-4-(2-{[2-(tétrahydro-2*H*-pyran-2-yloxy)éthyl]amino}propan-2-yl)phényl] (1-éthoxyisoquinoléin-5-yl)méthanone**

Obtenu selon le même protocole à partir de **421**
**RMN $^1$H** (400 MHz, DMSO-$d_6$) : δ 8,55 (d, 1H), 8,20 (d, 1H), 8,15 (d, 1H), 8,05 (d, 1H), 7,72 (t, 1H), 7,35 (d, 2H), 4,57 (quad et m, 3H), 3,75-3,45 (2m, 2H), 3,65-3,45 (2m, 2H), 2,48 (m, 2H), 2,30 (m, 1H), 1,8-1,4 (m, 4H), 1,6-1,45 (2m, 2H), 1,50 (t, 3H), 1,4 (s, 6H)

**Intermédiaire 516 :**

**(2,6-difluoro-4-{(1*R*)-1-[(2-méthoxyéthyl)amino]éthyl}phényl)(1-éthoxyisoquinoléin-5-yl)méthanone**

A une solution de **468** (1 g, 2,8 mmoles) dans le DMF (15 mL) sont ajoutés de l'$Et_3N$ (1,18 mL, 8,4 mmoles) et du 2-bromoéthyl-méthyl-éther (0,29 mL, 3,1 mmoles). Le mélange est chauffé à 70°C pendant 4 jours. Le milieu est décanté en présence d'eau et de chlorure de méthylène, la phase organique est lavée à l'eau, puis par une solution saturée en NaCl. Après séchage sur $MgSO_4$ et concentration sous vide, le résidu est chromatographié sur gel de silice en utilisant un éluant $CH_2Cl_2$/AcOEt : 50/50. Le produit **516** (0,46 g) est obtenu sous forme d'une huile.
**RMN $^1$H** (400 MHz, DMSO-$d_6$) : δ 8,55 (d, 1H), 8,20 (d, 1H), 8,15 (d, 1H), 8,05 (d, 1H), 7,75 (m, 1H), 7,30 (m, 2H), 4,6 (quad, 2H), 3,85 (m, 1H), 3,4 (t, 2H), 3,25 (s, 3H), 2,55 (m, 2H), 2,25 (s, 1H), 1,45 (t, 3H), 1,30 (d, 3H)
**IR ($cm^{-1}$) :** 3325
**LCMS [M+H]+=** 414
**Pureté optique** (Colonne OJ-H, éluant : méthanol/diéthylamine 100/0,1, détection 254nm) : >98,8%.

**Intermédiaire 517 :**

**(2,6-difluoro-4-{(1*S*)-1-[(2-méthoxyéthyl)amino]éthyl}phényl)(1-éthoxyisoquinoléin-5-yl)méthanone**

Obtenu selon le même protocole à partir de **435**
**RMN $^1$H** (400 MHz, DMSO-$d_6$) : δ 8,55 (d, 1H), 8,20 (d, 1H), 8,15 (d, 1H), 8,05 (d, 1H), 7,75 (m, 1H), 7,30 (m, 2H), 4,6 (quad, 2H), 3,85 (m, 1H), 3,4 (t, 2H), 3,25 (s, 3H), 2,55 (m, 2H), 2,25 (s, 1H), 1,45 (t, 3H), 1,30 (d, 3H)
**IR ($cm^{-1}$) :** 3325
**LCMS [M+H]+=** 414
**Pureté optique** (Colonne OJ-H, éluant : méthanol/diéthylamine 100/0,1, détection 254nm) : >99%.

**Intermédiaire 753 :**

**[2,6-difluoro-4-(1-{[2-(méthylsulfonyl)éthyl]amino}éthyl)phényl](1-éthoxyisoquinoléin-5-yl)méthanone**

A une solution de **752** (0,7 g, 2,09 mmoles) dans 1,4-dioxane (7,5 mL) sont ajoutés de la diisopropyl-éthylamine (0,47 mL, 3,3 mmoles) et de la méthyl-vinyl-sulfone (1,09 mL, 1,2 mmoles). Le mélange est chauffé à 90°C pendant 8 jours. Le milieu est décanté en présence d'eau et de chlorure de méthylène, la phase organique est lavée par une solution saturée en $NH_4Cl$ puis par une solution saturée de $Na_2CO_3$ Après séchage sur $MgSO_4$ et concentration sous vide, le résidu est chromatographié sur gel de silice en utilisant un éluant $CH_2Cl_2$/EtOH : 100/0 à 95/5. Le produit **753** (0,46 g) est obtenu sous forme d'une huile.
**RMN $^1$H** (400 MHz, DMSO-$d_6$) : δ 8,53 (d, 1H), 8,20 (d, 1H), 8,12 (d, 1H), 8,06 (d, 1H), 7,72 (t, 1H), 7,30 (d, 2H), 4,58 (quad, 2H), 3,88 (quad, 1H), 3,23 (quad, 2H), 3,03 (s, 3H), 2,78 (t, 2H), 1,47 (t, 3H), 1,29 (d, 3H)
**IR ($cm^{-1}$) :** 3339, 1671, 1371, 1119
Les intermédiaires **422, 437, 469, 516, 517, 753** obtenus ont été déprotégés en milieu acide pour conduire aux

produits finaux, selon la procédure décrite pour le produit **P17.**

| Produit | Issu de | Nomenclature<br>Description analytique |
|---|---|---|
| **P38** | **P17** | **Chlorhydrate de *N*-(1-{3,5-difluoro-4-[(1-oxo-1,2-dihydroisoquinoléin-5-yl)carbonyl] phényl}éthyl)glysinamide**<br>**RMN $^1$H** (400 MHz, DMSO-$d_6$) : δ 10,70 (m, 1H), 9,20 (d, 1H), 8,55 (d, 1H), 8,20 (m, 3H), 7,90 (d, 1H), 7,60 (t, 1H), 7,40 (m, 2H), 7,35 (m, 2H), 5,05 (m, 1H), 3,70 (m, 2H), 1,45 (d, 3H)<br>**IR (cm$^{-1}$) :** 3100-3000, 3000-2800, 1672<br>**HRMS (ESI) :** m/z théorique pour $C_{20}H_{18}F_2N_3O_3$ [M+H]$^+$ 386,1316, mesuré 386,1315 |
| **P48** | **285** | **Chlorhydrate de 5-(2,6-difluoro-4-{1-[(2-hydroxyéthyl)amino]éthyl}benzoyl)isoquinoléin-1 (2*H*)-one**<br>**RMN $^1$H** (400 MHz, DMSO-$d_6$) : δ 11,70-11,60 (s, 1H), 10,0-9,0 (2m, 2H), 8,55 (m, 1H), 8,00 (m, 1H), 7,77-7,55 (m, 3H), 7,40 (m, 2H), 5,5-5,0 (m, 1H), 4,55 (m, 1H), 3,70 (m, 2H), 2,95-2,80 (2m, 2H)<br>**IR (cm$^{-1}$) :** 3350, 2844-2400, 1687-1674, 1633<br>**HRMS (ESI) :** m/z théorique pour $C_{10}H_{19}F_2N_2O_3$ [M+H]$^+$ 373,1364, mesuré 373,1362 |
| **P54** | **P17** | **Chlorhydrate de *N*-(1-{3,5-difluoro-4-[(1-oxo-1,2-dihydroisoquinoléin-5-yl)carbonyl] phényl}éthyl)-L-isoleucinamide**<br>**RMN $^1$H** (400 MHz, DMSO-$d_6$) : δ 11,65 (s large, 1H), 9,25 (2d, 1H), 8,55 (d, 1H), 8,12 (massif, 3H), 7,90 (2d, 1H), 7,60 (2t, 1H), 5,10 (quint, 1H), 3,72 (m, 1H), 1,90 (m, 1H), 1,60-1,05 (m, 3H), 0.95-0.85 (m, 6H)<br>**IR (cm$^{-1}$) :** 3600-2300, 1689, 1661, 1630<br>**HRMS (ESI) :** m/z théorique pour $C_{24}H_{26}F_2N_3O_3$ [M+H]$^+$ 442,194223, mesuré 442,1939 |
| **P69** | **P17** | **Chlorhydrate de *N*-(1-{3,5-difluoro-4-[(1-oxo-1,2-dihydroisoquinoléin-5-yl)carbonyl] phényl}éthyl)-*N*$^2$-méthylglysinamide**<br>**RMN $^1$H** (400 MHz, DMSO-$d_6$) : δ 11,70 (s, 1H), 9,15 (d, 1H), 8,80 (m, 2H), 8,52 (d, 1H), 7,90 (d, 1H), 7,60 (t, 1H), 7,40 (m, 2H), 7,30 (d, 2H), 5,05 (quint, 1H), 3,80 (AB, 2H), 2,60 (s, 3H), 1,45 (d, 3H)<br>**IR (cm$^{-1}$) :** 3700-2000, 1689, 1672, 1632<br>**HRMS (ESI) :** m/z théorique pour $C_{21}H_{20}F_2N_3O_3$ [M+H]$^+$ 400,1473, mesuré 400,1456 |
| **P76** | **419** | **Chlorhydrate de *N*-{4,6-difluoro-5-[(1-oxo-1,2-dihydroisoquinoléin-5-yl)carbonyl]-2,3-dihydro-1*H*-indén-1-yl}glysinamide**<br>**RMN $^1$H** (300 MHz, DMSO-$d_6$) : δ 11,60 (m, 1H), 8,90 (d, 1H), 8,50 (dd, 1H), 8,00 (m, 3H), 7,90 (d, 1H), 7,60 (t, 1H), 7,40 (m, 2H), 7,15 (d, 1H), 5,40 (quad, 1H), 3,65 (2d, 2H), 3 (m, 1H), 2,85 (m, 1H), 2,55 (m, 1H), 2,00 (m, 1H)<br>**IR (cm$^{-1}$)** : 3300-2300, 3289, 1652, 1628, 1544<br>**HRMS (ESI) :** m/z théorique pour $C_{21}H_{18}F_2N_3O_3$ [M+H]$^+$ 398,1316, trouvé 398,1304.<br>Pureté optique (SFC: colonne AD 5μM 4,6x250 mm ; éluant: $CO_2$ / (méthanol/butylamine: 100/0,5) : 65/35 ; détection: 255nm) : >99%.<br>(absence de **P79**) |
| **P77** | **P53** | **Chlorhydrate de *N*-[(1*S*)-1-{3-méthyl-4-[(1-oxo-1,2-dihydroisoquinoléin-5-yl)carbonyl] phényl}éthyl]glysinamide**<br>**RMN $^1$H** (400 MHz, DMSO-$d_6$) : δ 11,50 (m, 1H), 9,00 (d, 1H), 8,45 (d, 1H), 8,10 (m, 3H), 7,65 (dd, 1H), 7,55 (t, 1H), 7,40 (d, 1H), 7,30 (m, 2H), 7,30 (m, 1H), 6,80 (d, 1H), 5,00 (quint, 1H), 3,60 (s, 2H), 2,40 (s, 3H), 1,40 (d, 3H) |
| | | **IR (cm$^{-1}$) :** 3650-2080, 1675-1660, 1600, 1557, 832-688<br>**HRMS (ESI) :** m/z théorique pour $C_{21}H_{22}N_3O_3$ [M+H]$^+$ 364,1661, mesuré 364,1648 |

(suite)

| Produit | Issu de | Nomenclature<br>Description analytique |
|---|---|---|
| **P78** | **422** | **Chlorhydrate de 5-(2,6-difluoro-4-{2-[(2-hydroxyéthyl)amino]propan-2-yl}benzoyl) isoquinoléin-1(2*H*)-one**<br>**RMN $^1$H** (400 MHz, DMSO-$d_6$) : $\delta$ 11,70 (s large, 1H), 9,80-9,30 (m, 2H), 8,55 (m, 1H), 8,00 (m, 1H), 7,65 (d, 2H), 7,60 (t, 1H), 7,40 (m, 2H), 5,25 (t, 1H), 3,65 (quad, 2H), 2,75 (m, 2H), 1,80 (s large, 6H)<br>**IR (cm$^{-1}$) :** 3500-2000, 1660, 1627<br>**HRMS (ESI) :** m/z théorique pour $C_{21}H_{21}F_2N_2O_3$ [M+H]$^+$ 387,1520, mesuré 387,1517. |
| **P79** | **427** | **Chlorhydrate de *N*-{4,6-difluoro-5-[(1-oxo-1,2-dihydroisoquinoléin-5-yl)carbonyl]-2,3-dihydro-1*H*-indén-1-yl}glysinamide**<br>**RMN $^1$H** (400 MHz, DMSO-$d_6$) : $\delta$ 11,60 (m, 1H), 9,10 (d, 1H), 8,50 (dd, 1H), 8,20 (m, 3H), 7,90 (d, 1H), 7,60 (t, 1H), 7,40 (m, 2H), 7,15 (d, 1H), 5,40 (quad, 1H), 3,65 (2d, 2H), 3,00 (m, 1H), 2,85 (m, 1H), 2,55 (m, 1H), 2,00 (m,1H)<br>**IR (cm$^{-1}$) :** 3300-2300, 3289, 1652, 1628, 1544<br>**HRMS (ESI) :** m/z théorique pour $C_{21}H_{18}F_2N_3O_3$ [M+H]$^+$ 398,1316, mesuré 398,1334.<br>**Pureté optique** (SFC: colonne AD 5$\mu$M 4,6x250 mm ; éluant: $CO_2$ / (méthanol/butylamine: 100/0,5) : 65/35 ; détection: 255nm) : >99%.<br>(absence de **P76**) |
| **P83** | **436** | **Chlorhydrate de *N*-[(1*S*)-1-{-3,5-difluoro-4-[(1-oxo-1,2-dihydroisoquinoléin-5-yl)carbonyl] phényl}éthyl]glysinamide**<br>**RMN $^1$H** (400 MHz, DMSO-$d_6$) : $\delta$ 11,65 (s large, 1H), 9,10 (d, 1H), 8,50 (dd, 1H), 8,30-7,90 (s large, 3H), 7,90 (dd, 1H), 7,55 (t, 1H), 7,35 (dd, 2H), 7,30 (d, 2H), 5,05 (m, 1H), 3,65 (m, 2H), 1,45 (d, 3H)<br>**IR (cm$^{-1}$) :** 3318, 3200-2500, 1692, 1674<br>**HRMS (ESI) :** m/z théorique pour $C_{20}H_{18}F_2N_3O_3$ [M+H]$^+$ 386,1316, mesuré 386,1300.<br>$\alpha_D$ (589nM) = -59,93 (c = 1, DMSO) à 20°C |
| **P84** | **437** | **Chlorhydrate de 5-(2,6-difluoro-4-{(1*S*)-1-[(2-hydroxyéthyl)amino]éthyl}benzoyl) isoquinoléin-1(2*H*)-one**<br>**RMN $^1$H** (400 MHz, DMSO-$d_6$) : $\delta$ 11,70 (d, 1H), 8,53 (d, 1H), 7,99 (d, 1H), 7,60 (m, 3H), 7,41 (m, 2H), 5,24 (t, 1H), 4,53 (q, 1H), 3,69 (m, 2H), 2,95 (m, 1H), 2,80 (m, 1H), 1,63 (d, 3H)<br>**IR (cm$^{-1}$) :** 3500-3200, 3200-2200, 1685, 1620<br>**HRMS (ESI) :** m/z théorique pour $C_{20}H_{19}F_2N_2O_3$ [M+H]$^+$ 373,1364, mesuré 373,1350.<br>$\alpha_D$ (589nM) = -1,7 (c = 0,01, DMSO) à 20°C |
| **P89** | **469** | **Chlorhydrate de 5-(2,6-difluoro-4-{(1*R*)-1-[(2-hydroxyéthyl)amino]éthyl}benzoyl) isoquinoléin-1(2*H*)-one**<br>**RMN $^1$H** (400 MHz, DMSO-$d_6$) : $\delta$ 11,80 (s large, 1H), 10.80-10,30 (2s large, 2H), 8,52 (d, 1H), 8,00 (d, 1H), 7,60 (d+t, 3H), 7,40 (d, 2H), 5,25 (t, 1H), 4,50 (m, 1H), 3,70 (m, 2H), 2,95-2,70 (m, 2H), 1,65 (d, 3H)<br>**IR (cm$^{-1}$) :** 3500-2700, 3200-2700, 1685-1672<br>**HRMS (ESI) :** m/z théorique pour $C_{20}H_{19}F_2N_2O_3$ [M+H]$^+$ 373,1364, mesuré 373,1348.<br>$\alpha_D$ (589nM) = 1,29 (c = 1, DMSO) à 20°C |
| **P97** | **468** | **Chlorhydrate de 5-(2,6-difluoro-4-{(1*R*)-1-[(2-méthoxyéthyl)amino]éthyl}benzoyl) isoquinoléin-1(2*H*)-one**<br>**RMN $^1$H** (400 MHz, DMSO-$d_6$) : $\delta$ 11,66 (m, 1H), 9,67-9,38 (2m, 2H), 8,54 (dd, 1H), 7,98 (d large, 1H), 7,58 (t, 1H), 7,56 (d, 2H), 7,42 (m, 2H), 4,50 (m, 1H), 3,60 (m, 2H), 3,30 (s, 3H), 3,05-2,90 (2m, 2H), 1,62 (d, 3H)<br>**IR (cm$^{-1}$) :** 3500-2000, 1671, 1630<br>**HRMS (ESI) :** m/z théorique pour $C_{21}H_{21}F_2N_2O_3$ [M+H]$^+$ 387,1520, mesuré 387,1524.<br>**Pureté optique** (SFC: colonne OZ-H 5$\mu$M 4,6x250 mm ; éluant: $CO_2$ / (méthanol/diéthylamine: 100/0,5) : 73/27 ; détection: 254nm) : 98,7%. |

(suite)

| Produit | Issu de | Nomenclature<br>Description analytique |
|---|---|---|
| **P98** | **495** | **Chlorhydrate de 5-(2,6-difluoro-4-{(1*S*)-1-[(2-méthoxyéthyl)amino]éthyl}benzoyl) isoquinoléin-1(2*H*)-one**<br>**RMN $^1$H** (400 MHz, DMSO-$d_6$) : $\delta$ 11,68 (m, 1H), 9,90-9,56 (2m, 2H), 8,54 (dd, 1H), 7,99 (d large, 1H), 7,61 (d, 2H), 7,57 (t, 1H), 7,42 (m, 2H), 4,51 (m, 1H), 3,63 (m, 2H), 3,30 (s, 3H), 3,05-2,90 (2m, 2H), 1,64 (d, 3H)<br>**IR (cm$^{-1}$) :** 3500-2000, 1693, 1662, 1627<br>**HRMS (ESI) :** m/z théorique pour $C_{21}H_{21}F_2N_2O_3$ [M+H]$^+$ 387,1520, mesuré 387,1524.<br>**Pureté optique** (SFC: colonne OZ-H 5$\mu$M 4,6x250 mm ; éluant: $CO_2$ / (méthanol/diéthylamine: 100/0,5) : 73/27 ; détection: 254nm) : >99%. (absence de **P97**) |
| **P138** | **742** | **Chlorhydrate de *N*-(2-{3,5-difluoro-4-[(1-oxo-1,2-dihydroisoquinoléin-5-yl)carbonyl] phényl}propan-2-yl)glysinamide**<br>**RMN $^1$H** (400 MHz, DMSO-$d_6$) : $\delta$ 11,90-11,50 (m, 1H), 8,85 (m, 1H), 8,50 (dd, 1H), 8,20-7,85 (m, 3H), 7,90 (dd, 1H), 7,60 (dd, 1H), 7,40 (m, 2H), 7,25 (d, 2H), 3,65 (s, 2H), 1,65 (s, 6H)<br>**IR (cm$^{-1}$) :** 3600-2000, 3303, 1672, 1628<br>**HRMS (ESI) :** m/z théorique pour $C_{21}H_{20}F_2N_3O_3$ [M+H]$^+$ 400,1473, mesuré 400,14518 |
| **P140** | **753** | **Chlorhydrate de 5-[2,6-difluoro-4-(1-{[2-(méthylsulfonyl)éthyl)amino}éthyl)benzoyl] isoquinoléin-1(2*H*)-one**<br>**RMN $^1$H** (400 MHz, DMSO-$d_6$) : $\delta$ 10,70 (m, 1H), 10,50-10,00 (m, 2H), 8,55 (d, 1H), 7,95 (d, 1H), 7,65 (d, 2H), 7,60 (t, 1H), 7,40 (m, 2H), 4,60 (m, 1H), 3,65 (m, 2H), 3,40-3,10 (m, 2H), 3,15 (s, 3H), 1,65 (d, 3H)<br>**RMN $^{19}$F:** -111,7<br>**IR (cm$^{-1}$) :** 3100, 2800-2600, 1674-1634, 1276-1139<br>**HRMS (ESI) :** m/z théorique pour $C_{21}H_{21}F_2N_2O_4S$ [M+H]$^+$ 435,1190, mesuré 435,1152. |
| **P142** | **765** | **Chlorhydrate de *N*-[(1*R*)-1-{3,5-difluoro-4-[(1-oxo-1,2-dihydroisoquinoléin-5-yl)carbonyl] phényl}éthyl]glysinamide**<br>**RMN $^1$H** (400 MHz, DMSO-$d_6$) : $\delta$ 12,0-11. 5 (m, 1H), 9,0 (d, 1H), 8,50 (dd, 1H), 7,90 (dd, 1H), 8,2-7,8 (m, 3H), 7,55 (t, 1H), 7,35 (dd, 2H), 7,30 (d, 2H), 5,05 (m, 1H), 3,65 (m, 2H), 1,45 (d, 3H)<br>**IR (cm$^{-1}$)** : 3307, 3300-2000, 1691, 1673<br>**HRMS (ESI) :** m/z théorique pour $C_{20}H_{18}F_2N_3O_3$ [M+H]$^+$ 386,1316, mesuré 386,1300.<br>$\alpha_D$ (589nM) = +62,5 (c = 1, DMSO) à 20°C |

## Etudes pharmacologiques

### Test enzymatique ROCK1

Evaluation des effets de composés sur l'activité du ROCK1 humain quantifiés en mesurant la phosphorylation du substrat Ulight-RRRSLLE (PLK) en utilisant une enzyme recombinante humaine et le procédé de détection LANCE®.

Protocole expérimental

On mélange le composé test, le composé de référence ou de l'eau (témoin) avec l'enzyme (8,2 ng) dans un tampon contenant 40 mM d'Hepes/Tris (pH 7,4), 0,8 mM d'EGTA/Tris, 8 mM de $MgCl_2$, 1,6 mM de DTT et 0,008% de Tween 20.

Ensuite, on amorce la réaction en ajoutant 50 nM de substrat Ulight-RRRSLLE (PLK) et 1 $\mu$M d'ATP et on laisse le mélange incuber pendant 30 min à température ambiante. Pour les mesures basales de contrôle, on exclut l'enzyme du mélange réactionnel.

Après l'incubation, on arrête la réaction en ajoutant 13 mM d'EDTA. Après 5 min, on ajoute l'anticorps anti-phospho-PLK marqué avec du chélate d'europium. Après 60 autres min, on mesure le transfert de fluorescence à lex=337 nm, lem=620 nm et lem=665 nm en utilisant un lecteur de microplaques (Envision, Perkin Elmer). On détermine l'activité enzymatique en divisant le signal mesuré à 665 nm par celui mesuré à 620 nm (rapport). Les résultats sont

exprimés sous forme d'un pourcentage d'inhibition de l'activité enzymatique témoin. Le composé de référence d'inhibition standard est la staurosporine, qui est testée dans chaque expérience à plusieurs concentrations pour obtenir une courbe d'inhibition à partir de laquelle on calcule : la valeur de l'$IC_{50}$ (concentration induisant 50 % d'inhibition)

Référence bibliographique

Doe, C., Bentley, R., Behm, D.J., Lafferty, R., Stavenger, R., Jung, D., Bamford, M., Panchal, T., Grygielko, E., Wright, L.L., Smith, G.K., Chen, Z., Webb, C., Khandekar, S., YI, T., Kirkpatrick, R., Dul, E., Jolivette, L., Marino, J.P. JR., Willette, R., Lee, D. et Hu, E. (2007), Novel Rho kinase inhibitors with anti-inflammatory and vasodilatory activities. J. Pharmacol. Exp. Ther., 320: 89.

| Composé de formule (I) | ROCK1(h), $IC_{50}$ (M) |
|---|---|
| **P9** | 5,87E-09 |
| **P17** | 4,20E-09 |
| **P19** | 6,30E-08 |
| **P24** | 2,30E-08 |
| **P27** | 8,70E-09 |
| **P42** | 9,60E-09 |
| **P45** | 5,00E-09 |
| **P47** | 6,78E-09 |
| **P48** | 7,45E-09 |
| **P50** | 5,40E-09 |
| **P56** | 1,65E-08 |
| **P58** | 3,85E-09 |
| **P59** | 1,95E-09 |
| **P61** | 5,35E-09 |
| **P75** | 1,04E-08 |
| **P129** | 2,05E-08 |
| **P139** | 8,75E-08 |

**Test fonctionnel (aorte de rat)**

Etude de la réactivité vasculaire sur segments aortiques de rat.

Après anesthésie de l'animal, l'aorte thoracique est prélevée et immédiatement placée dans une solution saline physiologique (SSP). L'aorte thoracique proximale est nettoyée du tissu conjonctif adhérent et 4 anneaux aortiques (3-4 mm) sont découpés. L'endothélium est enlevé mécaniquement sans léser les cellules musculaires lisses.

Ces anneaux sans endothélium sont placés en milieu SSP dans des cuves à organe isolé maintenues à 37°C en présence de carbogène. La tension isométrique des anneaux est enregistrée à l'aide d'un capteur de force. A leur tension optimale, les anneaux sont soumis à une période d'équilibration, période durant laquelle le milieu physiologique est remplacé régulièrement.

Les préparations sont ensuite contractées 2 fois à l'aide d'une solution hyperpotassique (KCl 60 mM), chacune des 2 contractions étant suivie de lavages successifs pour revenir à la tension initiale. L'absence d'endothélium est vérifiée après contraction par un agoniste des récepteurs $\alpha_1$-adrénergiques, la phényléphrine (PHE, $10^{-6}$M), suivi de l'addition de carbachol ($10^{-5}$M), agoniste des récepteurs muscariniques, qui induit une relaxation uniquement en présence d'endothélium. Puis, les anneaux sont lavés régulièrement avec du SSP durant 60 minutes afin d'éliminer les agents pharmacologiques.

Les anneaux sont recontractés avec la PHE ($10^{-6}$M) puis après la stabilisation de la contraction, le produit ou son solvant est ajouté en concentrations cumulatives, 5 concentrations sont testées: $10^{-8}$M, $10^{-7}$M, $10^{-6}$M, $10^{-5}$M et $3x10^{-5}$M (si le produit est solubilisé dans le DMSO) ou $10^{-4}$M (si $H_2O$ est le solvant du produit).

Analyse des résultats

La réponse contractile est obtenue en milligramme (mg). Les résultats sont exprimés par la moyenne ± SEM des réponses contractiles obtenues sur au moins 2 rats. Les variations de tension de chaque produit sont calculées en pourcentage de la contraction maximale induite par la PHE avant ajout du produit, *selon la formule :*

*% de contraction B(t) = [x (mg) de tension (composé) B(t) / y (mg) de tension maximale (PHE) B] x100.*

Les courbes concentration-réponses obtenues sont analysées et permettent de déterminer la valeur $IC_{50}$ de chaque produit ($IC_{50}$: concentration de produit nécessaire pour inhiber 50 % de la contraction maximale induite par la PHE), l'estimation de la valeur $IC_{50}$ des courbes concentration-réponses étant obtenue par régression non linéaire.

| Composé de formule (I) | Relaxation (aorte de rat+PHE), $IC_{50}$ (M) |
|---|---|
| **P6** | 2.00E-07 |
| **P9** | 3.30E-08 |
| **P17** | 1.40E-08 |
| **P19** | 2.30E-07 |
| **P21** | 1.00E-06 |
| **P24** | 1.00E-08 |
| **P27** | 5.00E-08 |
| **P42** | 2,00E-07 |
| **P45** | 1,00E-07 |
| **P47** | 4,00E-08 |
| **P48** | 1,50E-07 |
| **P50** | 1,00E-07 |
| **P56** | 1,50E-07 |
| **P58** | 7,00E-08 |
| **P59** | 5,50E-07 |
| **P61** | 1,70E-07 |
| **P75** | 2,00E-07 |
| **P129** | 2,00E-06 |
| **P139** | 2.00E-06 |

**Evaluation sur la pression artérielle (rats RSH)**

L'effet des inhibiteurs de ROCK a été testé par la diminution de la pression artérielle (PA) qu'ils induisent chez des rats spontanément hypertendus (RSH) après administration intraveineuse (i.v.) et/ou orale.En résumé, les RSH ont été anesthésiés à l'isoflurane 2 % et ont subi l'implantation d'une sonde de télémétrie (PAC40, Data Science International) dans l'aorte abdominale pour enregistrer la PA et d'un cathéter de polyéthylène dans la veine jugulaire pour réaliser l'administration i.v.

Après récupération de la chirurgie (2 à 3 semaines), la PA a été enregistrée continûment pendant 24 heures après l'administration des inhibiteurs de ROCK aux doses de 1, 3, 10 et 30 mg/kg par la voie i.v. et/ou par la voie orale. L'effet sur la PA a été exprimé en pourcentage de réduction par rapport à la pression artérielle basale avant administration du produit.

| Composé de formule (I) | Rat RSH ∆PA% max, IV 3mg/kg | Rat RSH ∆PA% max, PO 3mg/kg |
|---|---|---|
| **P6** | -73.4 | -35.6 |
| **P9** | -67 | -53.7 |
| **P17** | -72.8 | -56.6 |
| **P19** | -39.6 | - |
| **P24** | -68.2 | -52.5 |
| **P27** | -69 | -54 |
| **P42** | -59.9 | -12.3 |
| **P45** | -60.6 | -24 |
| **P47** | -62.7 | -26.4 |
| **P48** | -67.3 | -29.2 |
| **P50** | -58 | -16.8 |
| **P56** | -54.7 | - |
| **P58** | -65.1 | -52.7 |
| **P59** | -40.3 | - |
| **P61** | -59 | - |
| **P75** | -49.8 | -12.4 |
| **P129** | -30.2 | -18.4 |

## Compositions pharmaceutiques

Comprimés obtenus par granulation humide

| Constituants | Quantité % |
|---|---|
| Composé de formule (I) | 10 |
| Lactose poudre normale | qsp 100 |
| Amidon de maïs | 20 |
| PVP K30 | 7 |
| Carboxyméthylamidon de pomme de terre, sel de sodium faiblement réticulé | 3 |
| Silice colloïdale | 0.2 |
| Stéarate de magnésium | 0.5 |

Comprimés obtenus par compression directe :

| Constituants | Quantité % |
|---|---|
| Composé de formule (I) | 10 |
| Lactose aggloméré | qsp 100 |
| Cellulose microcristalline | 25 |
| Carboxyméthylamidon de pomme de terre, sel de sodium faiblement réticulé | 3 |
| Silice colloïdale | 0.2 |
| Stéarate de magnésium | 0.5 |

## Revendications

**1.** Composé de formule (I) :

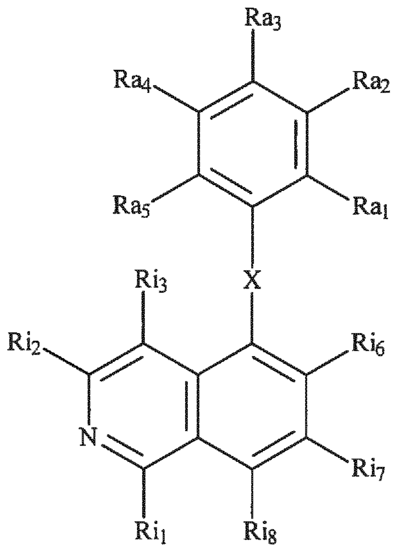

(I)

dans laquelle :

- X représente un groupement -C(=O), -CH(OH)- ou $-CH_2-$,
- $Ri_1$ représente un atome d'hydrogène ou un groupement hydroxyle,
étant entendu que le composé de formule (I) pour lequel $Ri_1$ représente un groupement hydroxyle peut être représenté sous la forme tautomère suivante :

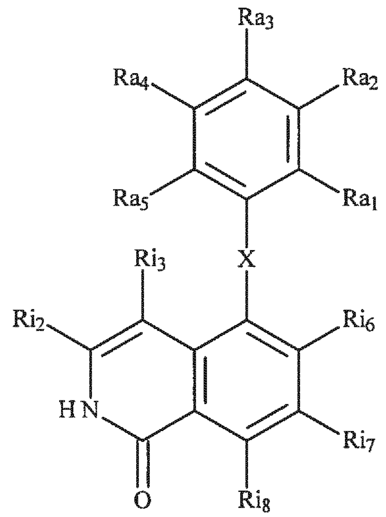

- $Ri_2$ et $Ri_3$, identiques ou différents, représentent chacun un atome d'hydrogène, un groupement $(C_1-C_6)$alkyle ou un atome d'halogène,
- $Ri_6$, $Ri_7$ et $Ri_8$, identiques ou différents, représentent chacun un atome d'hydrogène ou un atome d'halogène,
- $Ra_1$ et $Ra_5$, identiques ou différents, représentent chacun un atome d'hydrogène ou d'halogène, un groupement $-O(C_1-C_6)$alkyle ou un groupement $(C_1-C_6)$alkyle,
- $Ra_2$ représente un atome d'hydrogène ou d'halogène, un groupement hydroxyle, un groupement $-O(C_1-C_6)$alkyle, un groupement $-(C_1-C_6)$alkyle, un hétérocycle azoté possédant de 3 à 7 chaînons ou un groupement $-O-(CH2)_m-NR'R''$,
- $Ra_3$ représente un atome d'hydrogène, un groupement $-O(C_1-C_6)$alkyle, un groupement $-(C_1-C_6)$alkyle, un hétérocycle azoté possédant de 3 à 7 chaînons ou un groupement $-CRy_1Ry_2NH(Ry_3)$

- $Ra_4$ représente un atome d'hydrogène ou d'halogène, un groupement -O($C_1$-$C_6$)alkyle, un groupement -($C_1$-$C_6$)alkyle, ou un groupement -$CRy_1Ry_2NH(Ry_3)$,
étant entendu que :

  - $Ra_1$, $Ra_2$, $Ra_3$, $Ra_4$ et $Ra_5$ ne peuvent représenter simultanément un atome d'hydrogène,
  - $Ra_3$ et $Ra_4$ ne peuvent représenter simultanément un groupement -$CRy_1Ry_2NH(Ry_3)$,
  - $Ra_1$ et $Ra_2$ peuvent former ensemble avec les atomes de carbone qui les portent un hétérocycle possédant de 4 à 7 chaînons choisi parmi le tétrahydrofurane, le 1,4-dioxane, le tétrahydropyrane, la tétrahydro-2*H*-pyran-4-amine ou la 1-(tétrahydro-2*H*-pyran-4-yl)méthanamine, et
  - $Ra_2$ et $Ra_3$ peuvent former ensemble avec les atomes de carbone qui les portent un cycle hydrogénocarboné possédant de 4 à 7 chaînons choisi parmi le cyclopentane, la cyclopentanamine, le *N*-cyclopentylglysinamide ou la 1-méthylcyclopentanamine,

- m est un nombre entier dont la valeur est fixée à 1, 2 ou 3,
- R' et R", identiques ou différents, représentent chacun des groupements -(Ci-$C_6$)alkyle, ou bien forment ensemble avec l'atome d'azote qui les porte un hétérocycle possédant de 3 à 7 chaînons,
- $Ry_1$ représente un atome d'hydrogène, un groupement -($C_1$-$C_6$)alkyle, un groupement - $CH_2$-cyclohexyle ou un groupement 3-méthoxyphényle,
- $Ry_2$ représente un atome d'hydrogène ou un groupement -($C_1$-$C_6$)alkyle,
- $Ry_3$ représente :

  - un atome d'hydrogène,
  - un groupement -C(=O)-$CHRy_4$-$NHRy_5$ avec $Ry_4$ représentant un atome d'hydrogène ou un groupement ($C_1$-$C_6$)alkyle et $Ry_5$ représentant un atome d'hydrogène ou un groupement méthyle, ou
  - un groupement -($C_1$-$C_6$)alkyle qui peut être substitué par un groupement hydroxyle, un groupement -O($C_1$-$C_3$)alkyle, un groupement cyclohexyle ou un groupement méthylsulfonyle,

ou $Ry_1$ et $Ry_2$ forment ensemble avec l'atome de carbone qui les porte un groupement cyclopropane, cyclobutane ou tétrahydropyrane,
ou $Ry_2$ et $Ry_3$ forment ensemble avec les atomes de carbone et d'azote qui les portent respectivement un groupement pyrrolidine ou pipéridine,
ses isomères optiques lorsqu'ils existent, ainsi que leurs sels d'addition à un acide pharmaceutiquement acceptable et leurs hydrates.

2. Composé de formule (I) selon la revendication 1, **caractérisé en ce que** X représente un groupement -C(=O), ses isomères optiques lorsqu'ils existent, ainsi que leurs sels d'addition à un acide pharmaceutiquement acceptable et leurs hydrates.

3. Composé de formule (I) selon l'une quelconque des revendications 1 ou 2, **caractérisé en ce que** $Ri_1$ représente un groupement hydroxyle, étant entendu que ledit composé peut être représenté sous sa forme tautomère, ses isomères optiques lorsqu'ils existent, ainsi que leurs sels d'addition à un acide pharmaceutiquement acceptable et leurs hydrates.

4. Composé de formule (I) selon l'une quelconque des revendications 1 à 3, **caractérisé en ce que** $Ri_2$, $Ri_6$, $Ri_7$ et $Ri_8$ représentent chacun un atome d'hydrogène, ses isomères optiques lorsqu'ils existent, ainsi que leurs sels d'addition à un acide pharmaceutiquement acceptable et leurs hydrates.

5. Composé de formule (I) selon l'une quelconque des revendications 1 à 4, **caractérisé en ce que** $Ra_1$ et $Ra_5$ représentent chacun un atome de fluor, ses isomères optiques lorsqu'ils existent, ainsi que leurs sels d'addition à un acide pharmaceutiquement acceptable et leurs hydrates.

6. Composé de formule (I) selon l'une quelconque des revendications 1 à 5, **caractérisé en ce que** $Ra_3$ ou $Ra_4$ représente un groupement -$CRy_1Ry_2NH(Ry_3)$, ses isomères optiques lorsqu'ils existent, ainsi que leurs sels d'addition à un acide pharmaceutiquement acceptable et leurs hydrates.

7. Composé de formule (I) selon la revendication 6, **caractérisé en ce que** :

   - $Ry_1$ représente un atome d'hydrogène ou un groupement -($C_1$-$C_6$)alkyle,

- $Ry_2$ représente un groupement -($C_1$-$C_6$)alkyle,
- $Ry_3$ représente un atome d'hydrogène,

ses isomères optiques lorsqu'ils existent, ainsi que leurs sels d'addition à un acide pharmaceutiquement acceptable et leurs hydrates.

**8.** Composé de formule (I) selon la revendication 1, **caractérisé en ce que** :

- X représente un groupement -C(=O)-,
- $Ri_1$ représente un atome d'hydrogène ou un groupement hydroxyle,
- $Ri_2$, $Ri_6$, $Ri_7$ et $Ri_8$ représentent chacun un atome d'hydrogène et $Ri_3$ représente un atome d'hydrogène ou un groupement ($C_1$-$C_6$)alkyle,
- $Ra_1$ et $Ra_5$, identiques ou différents, représentent chacun un atome d'hydrogène ou de fluor, ou un groupement ($C_1$-$C_6$)alkyle,
- $Ra_2$ représente un atome d'hydrogène ou un groupement -($C_1$-$C_6$)alkyle,
- $Ra_3$ représente un atome d'hydrogène, un groupement pipéridine, ou un groupement - $CRy_1Ry_2NH(Ry_3)$,
- $Ra_4$ représente un atome d'hydrogène ou un groupement -$CRy_1Ry_2NH(Ry_3)$, étant entendu que $Ra_3$ et $Ra_4$ ne peuvent représenter simultanément un groupement - $CRy_1Ry_2NH(Ry_3)$, et que :

  • $Ra_3$ représentant un groupement -$CRy_1Ry_2NH(Ry_3)$, $Ra_1$ et $Ra_2$ peuvent former ensemble avec les atomes de carbone qui les portent un groupement tétrahydrofurane, 1,4-dioxane ou tétrahydropyrane, ou
  • $Ra_3$ représentant un atome d'hydrogène, $Ra_1$ et $Ra_2$ peuvent former ensemble avec les atomes de carbone qui les portent un groupement tétrahydro-2*H*-pyran-4-amine ou 1-(tétrahydro-2*H*-pyran-4-yl)méthanamine, ou
  • $Ra_2$ et $Ra_3$ peuvent former ensemble avec les atomes de carbone qui les portent un groupement cyclopentanamine ou 1-méthylcyclopentanamine,

- $Ry_1$ représente un atome d'hydrogène, un groupement -($C_1$-$C_6$)alkyle, ou un groupement -$CH_2$-cyclohexyle,
- $Ry_2$ représente un atome d'hydrogène ou un groupement -($C_1$-$C_6$)alkyle,
- $Ry_3$ représente un atome d'hydrogène ou un groupement -($C_1$-$C_6$)alkyle qui peut être substitué par un groupement hydroxyle,

ses isomères optiques lorsqu'ils existent, ainsi que leurs sels d'addition à un acide pharmaceutiquement acceptable et leurs hydrates.

**9.** Composé de formule (I) selon la revendication 1 choisi parmi :

- la [4-(1-aminoéthyl)-2,6-difluorophényl](isoquinoléin-5-yl)méthanone ainsi que ses isomères optiques et leurs sels d'addition à un acide pharmaceutiquement acceptable et leurs hydrates,
- la [4-((1*R*)-1-aminoéthyl)-2,6-difluorophényl](isoquinoléin-5-yl)méthanone ainsi que ses sels d'addition à un acide pharmaceutiquement acceptable et leurs hydrates,
- la [4-(1-aminoéthyl)-2,6-difluorophényl](1-hydroxyisoquinoléin-5-yl)méthanone ainsi que ses isomères optiques et leurs sels d'addition à un acide pharmaceutiquement acceptable et leurs hydrates,
- la 1-[3,5-difluoro-4-(isoquinoléin-5-ylméthyl)phényl]éthanamine ainsi que ses isomères optiques et ses sels d'addition à un acide pharmaceutiquement acceptable et leurs hydrates,
- le {4-[(1*S*)-1-aminoéthyl]-2,6-difluorophényl}(isoquinoléin-5-yl)méthanol ainsi que ses sels d'addition à un acide pharmaceutiquement acceptable et leurs hydrates,
- la [4-(2-aminopropan-2-yl)-2,6-difluorophényl](isoquinoléin-5-yl)méthanone ainsi que ses sels d'addition à un acide pharmaceutiquement acceptable et leurs hydrates,
- la 5-[4-(2-aminopropan-2-yl)-2,6-difluorobenzoyl]isoquinoléin-1(2*H*)-one ainsi que ses sels d'addition à un acide pharmaceutiquement acceptable et leurs hydrates,
- la 5-[4-(1-aminoéthyl)-2-fluoro-3-méthoxybenzoyl]isoquinoléin-1(2*H*)-one ainsi que ses isomères optiques et leur sels d'addition à un acide pharmaceutiquement acceptable et leurs hydrates,
- la 5-({5-[(1*R*)-1-aminoéthyl]-3,4-dihydro-2*H*-chromén-8-yl}carbonyl)isoquinoléin-1(2*H*)-one ainsi que ses sels d'addition à un acide pharmaceutiquement acceptable et leurs hydrates,
- la 5-{4-[(1*R*)-1-aminoéthyl]-2-méthylbenzoyl}isoquinoléin-1(2*H*)-one ainsi que ses sels d'addition à un acide pharmaceutiquement acceptable et leurs hydrates,
- la 5-(2,6-difluoro-4-{1-[(2-hydroxyéthyl)amino]éthyl}benzoyl)isoquinoléin-1(2*H*)-one ainsi que ses isomères

optiques et leurs sels d'addition à un acide pharmaceutiquement acceptable et leurs hydrates,
- la 5-{4-[(1*R*)-1-aminoéthyl]-2,6-difluorobenzoyl}-4-méthylisoquinoléin-1(2*H*)-one ainsi que ses sels d'addition à un acide pharmaceutiquement acceptable et leurs hydrates,
- la 5-{3-[(1*R*)-1-aminoéthyl]-2,6-difluorobenzoyl}isoquinoléin-1(2*H*)-one ainsi que ses sels d'addition à un acide pharmaceutiquement acceptable et leurs hydrates,
- la 5-[(1-amino-4,6-difluoro-2,3-dihydro-1*H*-indén-5-yl)carbonyl]isoquinoléin-1(2*H*)-one ainsi que ses isomères optiques et leurs sels d'addition à un acide pharmaceutiquement acceptable et leurs hydrates,
- la 5-{[(3*R*)-3-amino-4,6-difluoro-2,3-dihydro-1*H*-indén-5-yl]carbonyl} isoquinoléin-1(2*H*)-one ainsi que ses sels d'addition à un acide pharmaceutiquement acceptable et leurs hydrates,
- la 5-({8-[(1*R*)-1-aminoéthyl]-2,3-dihydro-1,4-benzodioxin-5-yl}carbonyl) isoquinoléin-1(2*H*)-one ainsi que ses sels d'addition à un acide pharmaceutiquement acceptable,
- la 5-[2,6-difluoro-4-(pipéridin-2-yl)benzoyl]isoquinoléin-1(2*H*)-one ainsi que ses isomères optiques et leurs sels d'addition à un acide pharmaceutiquement acceptable et leurs hydrates,
- la 5-[4-(1-amino-2-cyclohexyléthyl)-2,6-difluorobenzoyl]isoquinoléin-1(2*H*)-one ainsi que ses isomères optiques et leurs sels d'addition à un acide pharmaceutiquement acceptable et leurs hydrates,
- la 5-{[4-(aminométhyl)-3,4-dihydro-2*H*-chromén-8-yl]carbonyl}isoquinoléin-1(2*H*)-one ainsi que ses isomères optiques et leurs sels d'addition à un acide pharmaceutiquement acceptable et leurs hydrates.

**10.** Procédé de synthèse des composés de formule (Ia), cas particuliers des composés de formule (I) selon la revendication 1 pour lesquels X représente un groupement -C(=O), **caractérisé en ce que** les composés de formule (Ia) sont préparés à partir d'un composé de formule (II) :

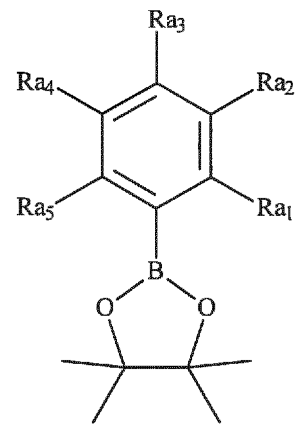

(II)

qui est soumis à une réaction de couplage avec le composé de formule (III) :

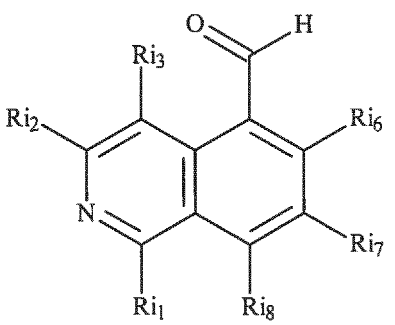

(III)

en présence d'un catalyseur au rhodium ou au palladium, d'une phosphine et d'une base dans un solvant organique, pour conduire au composé de formule (Ia) :

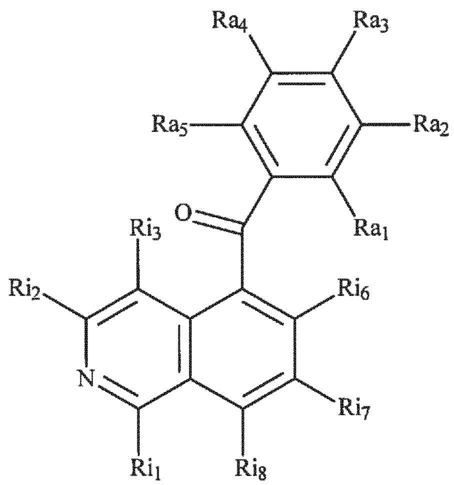

(Ia)

11. Procédé de synthèse des composés de formule (Ib), cas particuliers des composés de formule (I) selon la revendication 1 pour lesquels X représente un groupement -CH(OH)-:

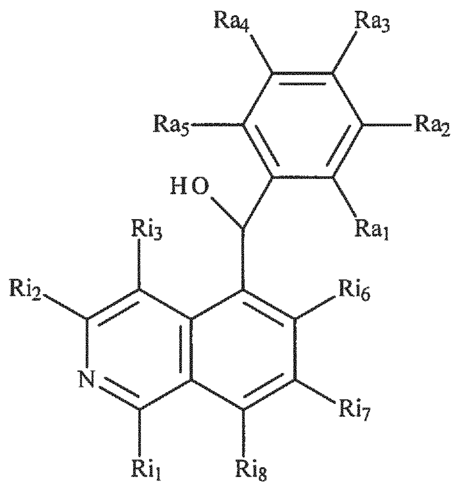

(Ib)

**caractérisé en ce que** les composés de formules (Ib) sont préparés à partir des composés de formule (Ia) selon la revendication 10 par une réaction de réduction en présence de tétraborohydrure de sodium.

12. Procédé de synthèse des composés de formule (Ic), cas particuliers des composés de formule (I) selon la revendication 1 pour lesquels X représente un groupement $-CH_2-$

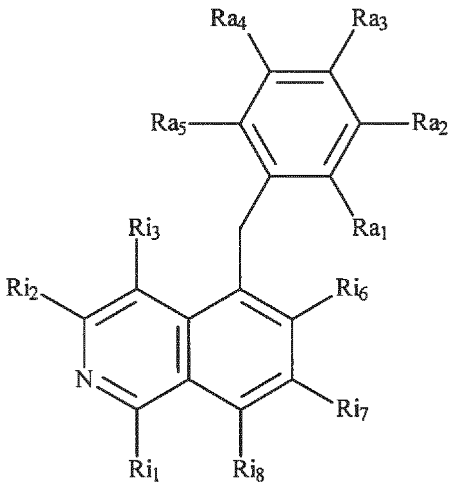

(Ic)

**caractérisé en ce que** les composés de formules (Ic) sont préparés à partir des composés de formule (Ib) selon la revendication 11 par une réaction de réduction en présence d'acide trifluoroacétique et de triéthylsilane.

**13.** Composition pharmaceutique contenant comme principe actif un composé de formule (I) selon l'une quelconque des revendications 1 à 9, en combinaison avec un ou plusieurs excipients ou véhicules inertes, non toxiques, pharmaceutiquement acceptables.

**14.** Composition pharmaceutique selon la revendication 13, pour utilisation dans le traitement ou la prévention des pathologies qui résultent de l'activation de la voie RhoA/ROCK et de la phosphorylation de la chaîne légère de la myosine.

**15.** Composition pharmaceutique pour utilisation selon la revendication 14, **caractérisée en ce qu'**elle est utilisée dans le traitement ou la prévention de l'hypertension artérielle systémique, de l'hypertension artérielle pulmonaire, de l'angor, de l'infarctus du myocarde, de la resténose post-angioplastie, de l'anévrisme aortique, de l'occlusion des artères périphériques, de l'athérosclérose, de la fibrose cardiaque et de l'insuffisance cardiaque.

**16.** Composition pharmaceutique pour utilisation selon la revendication 15, **caractérisée en ce qu'**elle est utilisée dans le traitement ou la prévention de l'hypertension artérielle systémique.

**17.** Composition pharmaceutique pour utilisation selon la revendication 14, **caractérisée en ce qu'**elle est utilisée dans le traitement ou la prévention du glaucome et des pathologies de la cornée.

**18.** Composition pharmaceutique pour utilisation selon la revendication 14, **caractérisée en ce qu'**elle est utilisée dans le traitement ou la prévention de la dysfonction érectile, des maladies pulmonaires broncho-obstructives, de la fibrose intestinale post-radiation, de la sclérose systémique cutanée, de la fibrose pulmonaire associée à l'hypertension artérielle pulmonaire, des maladies hépatiques, de la fibrose et la glomérulo-sclérose rénale, du diabète, de l'hyperglycémie, de l'insulino-résistance, des néphropathies diabétiques induites ou non par l'hypertension, des maladies thrombotiques, du vaso-spasme cérébral et de l'ischémie cérébrale résultante.

**Patentansprüche**

**1.** Verbindung der Formel (I):

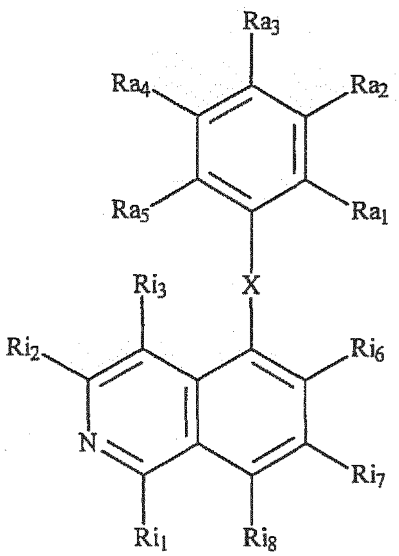

(I)

in der:

- X eine Gruppe -C(=O), -CH(OH)- oder $-CH_2-$ bedeutet,
- $Ri_1$ ein Wasserstoffatom oder eine Hydroxylgruppe bedeutet,
wobei es sich versteht, dass die Verbindung der Formel (I), worin $Ri_1$ eine Hydroxylgruppe darstellt, durch die folgende tautomere Form dargestellt werden kann:

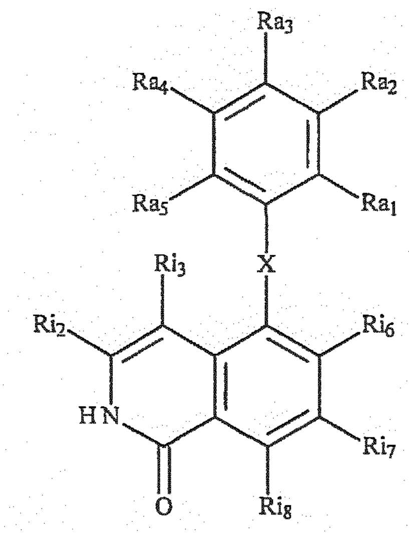

- $Ri_2$ und $Ri_3$, die gleichartig oder verschieden sind, jeweils ein Wasserstoffatom, eine $(C_1\text{-}C_6)$-Alkylgruppe oder ein Halogenatom bedeuten,
- $Ri_6$, $Ri_7$ und $Ri_8$, die gleichartig oder verschieden sind, jeweils ein Wasserstoffatom oder ein Halogenatom bedeuten,
- $Ra_1$ und $Ra_5$, die gleichartig oder verschieden sind, jeweils ein Wasserstoffatom oder ein Halogenatom, eine $-O(C_1\text{-}C_6)$-Alkylgruppe oder eine $(C_1\text{-}C_6)$-Alkylgruppe bedeuten,
- $Ra_2$ ein Wasserstoffatom oder ein Halogenatom, eine Hydroxylgruppe, eine - $O(C_1\text{-}C_6)$-Alkylgruppe, eine $-(C_1\text{-}C_6)$-Alkylgruppe, einen Stickstoff-haltigen Heterocyclus mit 3 bis 7 Kettengliedern oder eine Gruppe $-O-(CH_2)_m-NR'R''$ bedeutet,
- $Ra_3$ ein Wasserstoffatom, eine $-O(C_1\text{-}C_6)$-Alkylgruppe, eine $-(C_1\text{-}C_6)$-Alkylgruppe, einen Stickstoff-haltigen Heterocyclus mit 3 bis 7 Kettengliedern oder eine Gruppe $-CRy_1Ry_2NH(Ry_3)$ bedeutet,
- $Ra_4$ ein Wasserstoffatom oder ein Halogenatom, eine $-O(C_1\text{-}C_6)$-Alkylgruppe, eine $-(C_1\text{-}C_6)$-Alkylgruppe oder eine Gruppe $-CRy_1Ry_2NH(Ry_3)$ bedeutet,
wobei es sich versteht, dass:

• $Ra_1$, $Ra_2$, $Ra_3$, $Ra_4$ und $Ra_5$ nicht gleichzeitig ein Wasserstoffatom bedeuten können,
• $Ra_3$ und $Ra_4$ nicht gleichzeitig eine Gruppe $-CRy_1Ry_2NH(Ry_3)$ bedeuten können,
• $Ra_1$ und $Ra_2$ gemeinsam mit den sie tragenden Kohlenstoffatomen einen Heterocyclus mit 4 bis 7 Kettengliedern bilden können, ausgewählt aus Tetrahydrofuran, 1,4-Dioxan, Tetrahydropyran, Tetrahydro-2*H*-pyran-4-amin oder 1-(Tetrahydro-2*H*-pyran-4-yl)-methanamin und
• $Ra_2$ und $Ra_3$ gemeinsam mit den sie tragenden Kohlenstoffatomen einen Kohlenwasserstoffcyclus mit 4 bis 7 Kettengliedern bilden können, ausgewählt aus Cyclopentan, Cyclopentanamin, *N*-Cyclopentylglysinamid oder 1-Methylcyclopentanamin,

- m eine ganze Zahl mit einem Wert von 1, 2 oder 3 bedeutet,
- R' und R", die gleichartig oder verschieden sind, jeweils $-(C_1-C_6)$-Alkylgruppen bedeuten oder gemeinsam mit dem sie tragenden Stickstoffatom einen Heterocyclus mit 3 bis 7 Kettengliedern bilden,
- $Ry_1$ ein Wasserstoffatom, eine $-(C_1-C_6)$-Alkylgruppe, eine $-CH_2$-Cyclohexylgruppe oder eine 3-Methoxyphenylgruppe bedeutet,
- $Ry_2$ ein Wasserstoffatom oder eine $-(C_1-C_6)$-Alkylgruppe bedeutet,
- $Ry_3$:

• ein Wasserstoffatom,
• eine Gruppe $-C(=O)-CHRy_4-NHRy_5$, worin $Ry_4$ ein Wasserstoffatom oder eine $(C_1-C_6)$-Alkylgruppe darstellt und $Ry_5$ ein Wasserstoffatom oder eine Methylgruppe darstellt, oder
• eine $-(C_1-C_6)$-Alkylgruppe, die durch eine Hydroxylgruppe substituiert sein kann, eine $-O(C_1-C_3)$-Alkylgruppe, eine Cyclohexylgruppe oder eine Methylsulfonylgruppe bedeutet,

oder $Ry_1$ und $Ry_2$ gemeinsam mit dem sie tragenden Kohlenstoffatom eine Cyclopropan-, Cyclobutan- oder Tetrahydropyrangruppe bilden,
oder $Ry_2$ und $Ry_3$ gemeinsam mit den sie tragenden Kohlenstoff- bzw. Stickstoffatomen eine Pyrrolidin- oder Piperidingruppe bilden,
ihre optischen Isomeren, falls diese existieren, sowie ihre Additionssalze mit einer pharmazeutisch annehmbaren Säure und ihre Hydrate.

2. Verbindung der Formel (I) nach Anspruch 1, **dadurch gekennzeichnet, dass** X eine Gruppe -C(=O) bedeutet, ihre optischen Isomeren, falls diese existieren, sowie ihre Additionssalze mit einer pharmazeutisch annehmbaren Säure und ihre Hydrate.

3. Verbindung der Formel (I) nach einem der Ansprüche 1 oder 2, **dadurch gekennzeichnet, dass** $Ri_1$ eine Hydroxylgruppe bedeutet, wobei es sich versteht, dass die Verbindung in ihrer tautomeren Form dargestellt werden kann, ihre optischen Isomeren, falls diese existieren, sowie ihre Additionssalze mit einer pharmazeutisch annehmbaren Säure und ihre Hydrate.

4. Verbindung der Formel (I) nach einem der Ansprüche 1 bis 3, **dadurch gekennzeichnet, dass** $Ri_2$, $Ri_6$, $Ri_7$ und $Ri_8$ jeweils ein Wasserstoffatom bedeuten, ihre optischen Isomeren, falls diese existieren, sowie ihre Additionssalze mit einer phannazeutisch annehmbaren Säure und ihre Hydrate.

5. Verbindung der Formel (I) nach einem der Ansprüche 1 bis 4, **dadurch gekennzeichnet, dass** $Ra_1$ und $Ra_5$ jeweils ein Fluoratom bedeuten, ihre optischen Isomeren, falls diese existieren, sowie ihre Additionssalze mit einer pharmazeutisch annehmbaren Säure und ihre Hydrate.

6. Verbindung der Formel (I) nach einem der Ansprüche 1 bis 5, **dadurch gekennzeichnet, dass** $Ra_3$ oder $Ra_4$ eine Gruppe $-CRy_1Ry_2NH(Ry_3)$ bedeuten, ihre optischen Isomeren, falls diese existieren, sowie ihre Additionssalze mit einer pharmazeutisch annehmbaren Säure und ihre Hydrate.

7. Verbindung der Formel (I) nach Anspruch 6, **dadurch gekennzeichnet, dass**:

- $Ry_1$ ein Wasserstoffatom oder eine $-(C_1-C_6)$-Alkylgruppe bedeutet,
- $Ry_2$ eine $-(C_1-C_6)$-Alkylgruppe bedeutet,
- $Ry_3$ ein Wasserstoffatom bedeutet

ihre optischen Isomeren, falls diese existieren, sowie ihre Additionssalze mit einer pharmazeutisch annehmbaren

Säure und ihre Hydrate.

**8.** Verbindung der Formel (I) nach Anspruch 1, **dadurch gekennzeichnet, dass**:

- X eine Gruppe -C(=O)- bedeutet,
- $Ri_1$ ein Wasserstoffatom oder eine Hydroxylgruppe bedeutet,
- $Ri_2$, $Ri_6$, $Ri_7$ und $Ri_8$ jeweils ein Wasserstoffatom bedeuten und $Ri_3$ ein Wasserstoffatom oder eine $(C_1-C_6)$-Alkylgruppe darstellt,
- $Ra_1$ und $Ra_5$, die gleichartig oder verschieden sind, jeweils ein Wasserstoff- oder Fluoratom oder eine $(C_1-C_6)$-Alkylgruppe bedeuten,
- $Ra_2$ ein Wasserstoffatom oder eine $-(C_1-C_6)$-Alkylgruppe bedeutet,
- $Ra_3$ ein Wasserstoffatom, eine Piperidingruppe oder eine Gruppe - $CRy_1Ry_2NH(Ry_3)$ bedeutet,
- $Ra_4$ ein Wasserstoffatom oder eine Gruppe $-CRy_1)Ry_2NH(Ry_3)$ bedeutet, wobei es sich versteht, dass $Ra_3$ und $Ra_4$ nicht gleichzeitig eine Gruppe - $CRy_1Ry_2NH(Ry_3)$ bedeuten können, und dass:

  • $Ra_3$ eine Gruppe $-CRy_1Ry_2NH(Ry_3)$ bedeutet, $Ra_1$ und $Ra_2$ gemeinsam mit den sie tragenden Kohlenstoffatomen eine Tetrahydrofuran-, 1,4-Dioxan- oder Tetrahydropyrangruppe bilden können, oder
  • $Ra_3$ ein Wasserstoffatom bedeutet, $Ra_1$ und $Ra_2$ gemeinsam mit den sie tragenden Kohlenstoffatomen eine Tetrahydro-2*H*-pyran-4-amin- oder 1-(Tetrahydro-2*H*-pyran-4-yl)-methanamingruppe bilden können, oder
  • $Ra_2$ und $Ra_3$ gemeinsam mit den sie tragenden Kohlenstoffatomen eine Cyclopentanamin- oder 1-Methylcyclopentanamin-gruppe bilden können,

- $Ry_1$ ein Wasserstoffatom, eine $-(C_1-C_6)$-Alkylgruppe oder eine $-CH_2$-Cyclohexylgruppe bedeutet,
- $Ry_2$ ein Wasserstoffatom oder eine $-(C_1-C_6)$-Alkylgruppe bedeutet,
- $Ry_3$ ein Wasserstoffatom oder eine $-(C_1-C_6)$-Alkylgruppe, die durch eine Hydroxylgruppe substituiert sein kann, bedeutet,

ihre optischen Isomeren, falls diese existieren, sowie ihre Additionssalze mit einer pharmazeutisch annehmbaren Säure und ihre Hydrate.

**9.** Verbindung der Formel (I) nach Anspruch 1, ausgewählt aus:

- [4-(1-Aminoethyl)-2,6-difluorphenyl](isochinolinin-5-yl)-methanon sowie seine optischen Isomeren und seine Additionssalze mit einer pharmazeutisch annehmbaren Säure und seine Hydrate,
- [4-((1*R*)-1-Aminoethyl)-2,6-difluorphenyl](isochinolin-5-yl)-methanon sowie seine Additionssalze mit einer pharmazeutisch annehmbaren Säure und seine Hydrate,
- 4-(1-Aminoethyl)-2,6-difluorphenyl](1-hydroxyisochinolin-5-yl)-methanon sowie seine optischen Isomeren und seine Additionssalze mit einer pharmazeutisch annehmbaren Säure und seine Hydrate,
- 1-[3,5-Difluor-4-(isochinolin-5-ylmetyl)-phenyl]-ethanamin sowie seine optischen Isomeren und seine Additionssalze mit einer pharmazeutisch annehmbaren Säure und seine Hydrate,
- {4-[(1*S*)-1-Aminoethyl]-2,6-difluorphenyl}(isochinolin-5-yl)-methanol sowie seine Additionssalze mit einer pharmazeutisch annehmbaren Säure und seine Hydrate,
- [4-(2-Aminopropan-2-yl)-2,6-difluorphenyl](isochinolin-5-yl)-methanon sowie seine Additionssalze mit einer pharmazeutisch annehmbaren Säure und seine Hydrate,
- 5-[4-(2Aminopropan-2-yl)-2,6-difluorbenzoyl]-isochinolin-1(2*H*)-on sowie seine Additionssalze mit einer pharmazeutisch annehmbaren Säure und seine Hydrate,
- 5-[4-(1-Aminoethyl)-2-fluor-3-methoxybenzoyl]-isochinolin-1(2*H*)-on sowie seine optischen Isomeren und seine Additionssalze mit einer pharmazeutisch annehmbaren Säure und seine Hydrate,
- 5-({5-[(1*R*)-1-Aminoethyl]-3,4-dihydro-2*H*-chromen-8-yl}-carbonyl)-isochinolin-1(2*H*)-on sowie seine Additionssalze mit einer pharmazeutisch annehmbaren Säure und seine Hydrate,
- 5-{4-[(1*R*)-1-Aminoethyl]-2-methylbenzoyl}-isochinolin-1(2*H*)-on sowie seine Additionssalze mit einer pharmazeutisch annehmbaren Säure und seine Hydrate,
- 5-(2,6-Difluor-4-{1-[(2-hydroxyethyl)-amino]-ethyll-benzoyl)-isochinolin-1-(2*H*)-on sowie seine optischen Isomeren und seine Additionssalze mit einer pharmazeutisch annehmbaren Säure und seine Hydrate,
- 5-{4-[(1*R*)-1-Aminoethyl]-2,6-difluorbenzoyl}-4-methylisochinolin-1(2*H*)-on sowie seine Additionssalze mit einer pharmazeutisch annehmbaren Säure und seine Hydrate,
- 5-{3-[(1*R*)-1-Aminoethyl]-2,6-difluorbenzoyl}-isochinolin-1(2*H*)-on sowie seine Additionssalze mit einer pharmazeutisch annehmbaren Säure und seine Hydrate,
- 5-[(1-Amino-4,6-difluor-2,3-dihydro-1*H*-inden-5-yl)-carbonyl]-isochinolin-1(2*H*)-on sowie seine optischen Isomeren und seine Additionssalze mit einer pharmazeutisch annehmbaren Säure und seine Hydrate,
- 5-{[(3*R*)-3-Amino-4,6-difluor-2,3-dihydro-1*H*-inden-5-yl]-carbonyl}-isochinolin-1(2*H*)-on sowie seine Additionssalze mit einer pharmazeutisch annehmbaren Säure und seine Hydrate,
- 5-({8-[(1*R*)-1-Aminoethyl]-2,3-dihydro-1,4-benzodioxin-5-yl}-carbonyl)-isochinolin-1(2*H*)-on sowie seine Additionssalze mit einer pharmazeutisch annehmbaren Säure,
- 5-[2,6-Difluor-4-(piperidin-2-yl)-benzoyl]-isochinolin-1(2*H*)-on sowie seine optischen Isomeren und seine Additionssalze mit einer pharmazeutisch annehmbaren Säure und seine Hydrate,
- 5-[4-(1-Amino-2-cyclohexylethyl)-2,6-difluorbenzoyl]-isochinolin-1(2*H*)-on sowie seine optischen Isomeren und seine Additionssalze mit einer pharmazeutisch annehmbaren Säure und seine Hydrate,
- 5-{[4-(Aminomethyl)-3,4-dihydro-2*H*-chromen-8-yl]-carbonyl}-isochinolin-1(2*H*)-on sowie seine optischen Isomeren und seine Additionssalze mit einer pharmazeutisch annehmbaren Säure und seine Hydrate.

**10.** Verfahren zur Synthese der Verbindungen der Formel (Ia), einem Sonderfall der Verbindungen der Formel (I) nach Anspruch 1, worin X eine Gruppe -C(=O) bedeutet, **dadurch gekennzeichnet, dass** die Verbindungen der Formel (Ia) hergestellt werden ausgehend von der Verbindung der Formel (II):

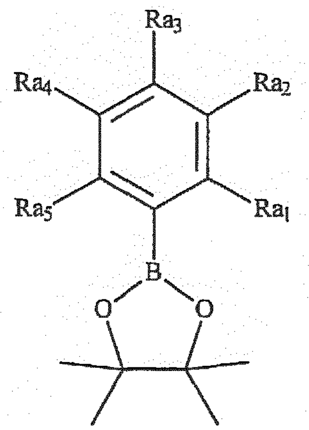

(II)

die einer Kupplungsreaktion mit der Verbindung der Formel (III):

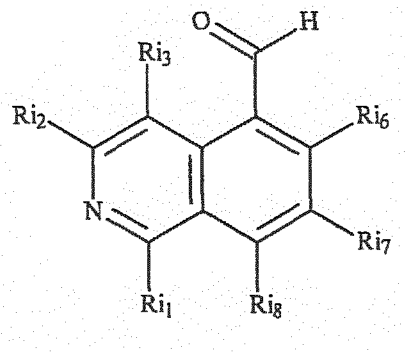

(III)

in Gegenwart eines Rhodium- oder Palladium-Katalysators, eines Phosphins und einer Base in einem organischen Lösungsmittel unterworfen wird,
zur Bildung der Verbindung der Formel (Ia):

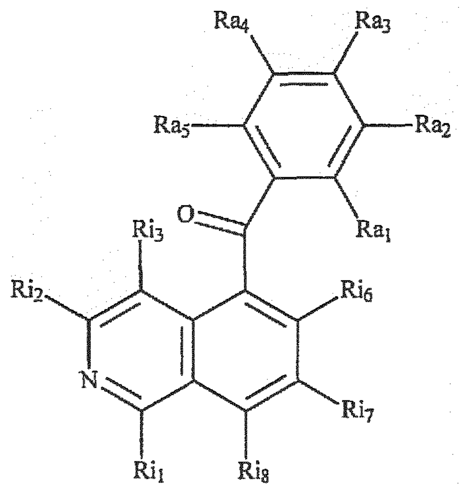

(Ia)

11. Verfahren zur Synthese der Verbindungen der Formel (Ib), einem Sonderfall der Verbindungen der Formel (I) nach Anspruch 1, worin X eine Gruppe -CH(OH)-bedeutet:

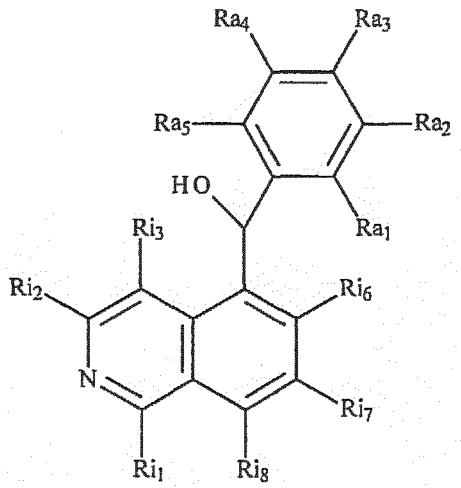

(Ib)

**dadurch gekennzeichnet, dass** die Verbindungen der Formeln (Ib) hergestellt werden ausgehend von den Verbindungen der Formel (Ia) nach Anspruch 10 durch eine Reduktionsreaktion in Gegenwart von Natriumtetraborhydrid.

12. Verfahren zur Synthese der Verbindungen der Formel (Ic), einem Sonderfall der Verbindungen der Formel (I) nach Anspruch 1, worin X eine Gruppe $-CH_2-$ bedeutet:

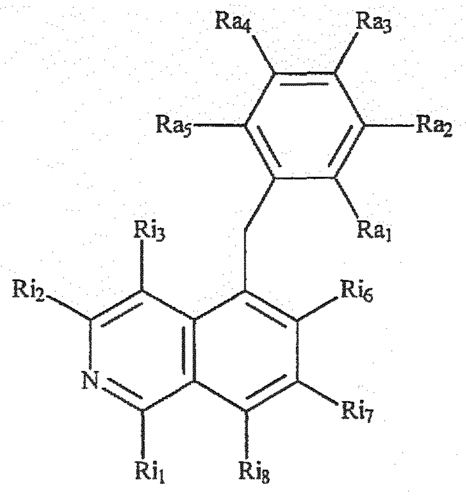

(Ic)

**dadurch gekennzeichnet, dass** die Verbindungen der Formeln (Ic) hergestellt werden ausgehend von den Verbindungen der Formel (Ib) nach Anspruch 11 durch eine Reduktionsreaktion in Gegenwart von Trifluoressigsäure und Triethylsilan.

**13.** Pharmazeutische Zubereitung enthaltend als Wirkstoff eine Verbindung der Formel (I) nach einem der Ansprüche 1 bis 9 in Kombination mit einem oder mehreren inerten, nichttoxischen und pharmazeutisch annehmbaren Trägermaterialien oder Hilfsstoffen.

**14.** Pharmazeutische Zubereitung nach Anspruch 13 zur Verwendung bei der Behandlung oder der Vorbeugung von Erkrankungen, die sich als Folge der Aktivierung des RhoA/ROCK-Wegs und der Phosphorylierung der leichten Kette von Myosin ergeben.

**15.** Pharmazeutische Zubereitung zur Verwendung gemäß Anspruch 14, **dadurch gekennzeichnet, dass** sie zur Behandlung oder Vorbeugung von arterieller systemischer Hypertension, pulmonaler arterieller Hypertension, Angina pectoris, Myocardinfarkt, post-angioplastischer Restenose, Aortenaneurysma, Verschluss der peripheren Arterien, Atherosklerose, Herzfibrose und Herzinsuffizienz verwendet wird.

**16.** Pharmazeutische Zubereitung zur Verwendung gemäß Anspruch 15, **dadurch gekennzeichnet, dass** sie zur Behandlung oder Vorbeugung von systemischer arterieller Hypertension verwendet wird.

**17.** Pharmazeutische Zubereitung zur Verwendung gemäß Anspruch 14, **dadurch gekennzeichnet, dass** sie zur Behandlung oder Vorbeugung von Glaukom und Hornhauterkrankungen verwendet wird.

**18.** Pharmazeutische Zubereitung zur Verwendung gemäß Anspruch 14, **dadurch gekennzeichnet, dass** sie zur Behandlung oder Vorbeugung von erektiler Dysfunktion, broncho-obstruktiven Lungenerkrankungen, Darmfibrose nach Strahlenbehandlung, systemischer Hautsklerose, mit pulmonaler arterieller Hypertension verbundener Lungenfibrose, Lebererkrankungen, Fibrose und Glomerulosklerose der Nieren, Diabetes, Hyperglykämie, Insulinresistenz, diabetischen Nierenerkrankungen, die durch Hypertension verursacht sind oder auch nicht, thrombotischen Erkrankungen, zerebralen Gefäßspasmen und sich daraus ergebender Zerebralischämie.

## Claims

**1.** Compound of formula (I) :

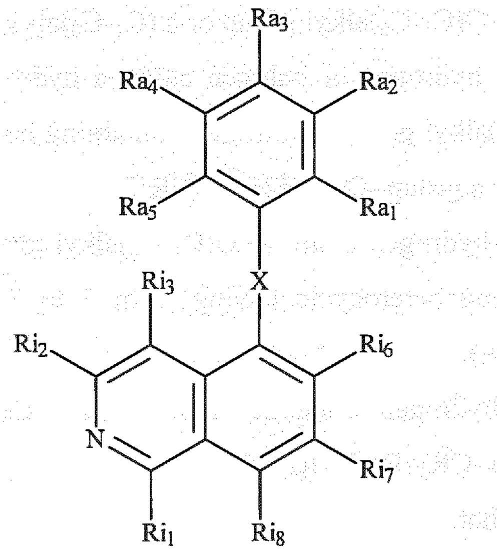

(I)

wherein:

- X represents a group -C(=O), -CH(OH)- or $-CH_2-$,
- $Ri_1$ represents a hydrogen atom or a hydroxyl group,

it being understood that the compound of formula (I) wherein $Ri_1$ represents a hydroxyl group may be represented by the following tautomeric form:

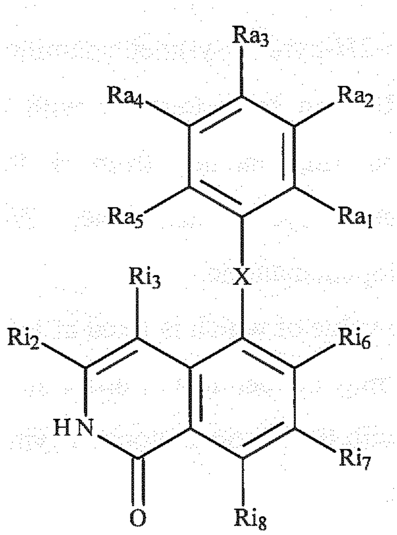

- $Ri_2$ and $Ri_3$, which may be identical or different, each represent a hydrogen atom, a $(C_1-C_6)$alkyl group or a halogen atom,
- $Ri_6$, $Ri_7$ and $Ri_8$, which may be identical or different, each represent a hydrogen atom or a halogen atom,
- $Ra_1$ and $Ra_5$, which may be identical or different, each represent a hydrogen or halogen atom, a $-O(C_1-C_6)$alkyl group or a $(C_1-C_6)$alkyl group,
- $Ra_2$ represents a hydrogen or halogen atom, a hydroxyl group, a $-O(C_1-C_6)$alkyl group, a $-(C_1-C_6)$alkyl group, a nitrogen-containing heterocycle having from 3 to 7 ring members, or a group $-O-(CH2)_m-NR'R''$,
- $Ra_3$ represents a hydrogen atom, a $-O(C_1-C_6)$alkyl group, a $-(C_1-C_6)$alkyl group, a nitrogen-containing heterocycle having from 3 to 7 ring members, or a group $-CRy_1Ry_2NH(Ry_3)$,
- $Ra_4$ represents a hydrogen or halogen atom, a $-O(C_1-C_6)$alkyl group, a $-(C_1-C_6)$alkyl group, or a group $-CRy_1Ry_2NH(Ry_3)$,

it being understood that:

- $Ra_1$, $Ra_2$, $Ra_3$, $Ra_4$ and $Ra_5$ may not simultaneously represent a hydrogen atom,

• $Ra_3$ and $Ra_4$ may not simultaneously represent a group $-CRy_1Ry_2NH(Ry_3)$,
• $Ra_1$ and $Ra_2$ can form together with the carbon atoms carrying them a heterocycle having from 4 to 7 ring members chosen from tetrahydrofuran, 1,4-dioxane, tetrahydropyran, tetrahydro-2*H*-pyran-4-amine and 1-(tetrahydro-2*H*-pyran-4-yl)methanamine, and
• $Ra_2$ and $Ra_3$ can form together with the carbon atoms carrying them a hydrocarbon ring having from 4 to 7 ring members chosen from cyclopentane, cyclopentanamine, *N*-cyclopentylglycinamide and 1-methylcyclopentanamine,

- m is an integer the value of which is fixed at 1, 2 or 3,
- R' and R", which may be identical or different, each represent $-(C_1-C_6)$alkyl groups or form together with the nitrogen atom carrying them a heterocycle having from 3 to 7 ring members,
- $Ry_1$ represents a hydrogen atom, a $-(C_1-C_6)$alkyl group, a $-CH_2$-cyclohexyl group, or a 3-methoxyphenyl group,
- $Ry_2$ represents a hydrogen atom or a $-(C_1-C_6)$alkyl group,
- $Ry_3$ represents:

• a hydrogen atom,
• a group $-C(=O)-CHRy_4-NHRy_5$ wherein $Ry_4$ represents a hydrogen atom or a $(C_1-C_6)$alkyl group and $Ry_5$ represents a hydrogen atom or a methyl group, or
• a $-(C_1-C_6)$alkyl group which can be substituted by a hydroxyl group, a $-O(C_1-C_3)$alkyl group, a cyclohexyl group or a methylsulphonyl group,

or $Ry_1$ and $Ry_2$ form together with the carbon atom carrying them a cyclopropane, cyclobutane or tetrahydropyran group,
or $Ry_2$ and $Ry_3$ form together with the carbon and nitrogen atoms carrying them, respectively, a pyrrolidine or piperidine group,
its optical isomers, where they exist, and addition salts thereof with a pharmaceutically acceptable acid and hydrates thereof.

2. Compound of formula (I) according to claim 1, **characterised in that** X represents a group -C(=O), its optical isomers, where they exist, and addition salts thereof with a pharmaceutically acceptable acid and hydrates thereof.

3. Compound of formula (I) according to either claim 1 or claim 2, **characterised in that** $Ri_1$ represents a hydroxyl group, it being understood that said compound may be represented in its tautomeric form, its optical isomers, where they exist, and addition salts thereof with a pharmaceutically acceptable acid and hydrates thereof.

4. Compound of formula (I) according to any one of claims 1 to 3, **characterised in that** $Ri_2$, $Ri_6$, $Ri_7$ and $Ri_8$ each represent a hydrogen atom, its optical isomers, where they exist, and addition salts thereof with a pharmaceutically acceptable acid and hydrates thereof.

5. Compound of formula (I) according to any one of claims 1 to 4, **characterised in that** $Ra_1$ and $Ra_5$ each represent a fluorine atom, its optical isomers, where they exist, and addition salts thereof with a pharmaceutically acceptable acid and hydrates thereof.

6. Compound of formula (I) according to any one of claims 1 to 5, **characterised in that** $Ra_3$ or $Ra_4$ represents a group $-CRy_1Ry_2NH(Ry_3)$, its optical isomers, where they exist, and addition salts thereof with a pharmaceutically acceptable acid and hydrates thereof.

7. Compound of formula (I) according to claim 6, **characterised in that**:

- $Ry_1$ represents a hydrogen atom or a $-(C_1-C_6)$alkyl group,
- $Ry_2$ represents a $-(C_1-C_6)$alkyl group,
- $Ry_3$ represents a hydrogen atom,

its optical isomers, where they exist, and addition salts thereof with a pharmaceutically acceptable acid and hydrates thereof.

8. Compound of formula (I) according to claim 1, **characterised in that**:

- X represents a group -C(=O)-,
- $Ri_1$ represents a hydrogen atom or a hydroxyl group,
- $Ri_2$, $Ri_6$, $Ri_7$ and $Ri_8$ each represent a hydrogen atom and $Ri_3$ represents a hydrogen atom or a ($C_1$-$C_6$)alkyl group,
- $Ra_1$ and $Ra_5$, which may be identical or different, each represent a hydrogen or fluorine atom or a ($C_1$-$C_6$)alkyl group,
- $Ra_2$ represents a hydrogen atom or a -($C_1$-$C_6$)alkyl group,
- $Ra_3$ represents a hydrogen atom, a piperidine group, or a group -$CRy_1Ry_2NH(Ry_3)$,
- $Ra_4$ represents a hydrogen atom or a group -$CRy_1Ry_2NH(Ry_3)$, it being understood that $Ra_3$ and $Ra_4$ may not simultaneously represent a group -$CRy_1Ry_2NH(Ry_3)$, and that:

  • when $Ra_3$ represents a group -$CRy_1Ry_2NH(Ry_3)$, $Ra_1$ and $Ra_2$ can form together with the carbon atoms carrying them a tetrahydrofuran, 1,4-dioxane or tetrahydropyran group, or
  • when $Ra_3$ represents a hydrogen atom, $Ra_1$ and $Ra_2$ can form together with the carbon atoms carrying them a tetrahydro-2*H*-pyran-4-amine or 1-(tetrahydro-2*H*-pyran-4-yl)methanamine group, or
  • $Ra_2$ and $Ra_3$ can form together with the carbon atoms carrying them a cyclopentanamine or 1-methylcyclopentanamine group,

- $Ry_1$ represents a hydrogen atom, a -($C_1$-$C_6$)alkyl group or a -$CH_2$-cyclohexyl group,
- $Ry_2$ represents a hydrogen atom or a -($C_1$-$C_6$)alkyl group,
- $Ry_3$ represents a hydrogen atom or a -($C_1$-$C_6$)alkyl group which can be substituted by a hydroxyl group,

its optical isomers, where they exist, and addition salts thereof with a pharmaceutically acceptable acid and hydrates thereof.

**9.** Compound of formula (I) according to claim 1 chosen from:

- [4-(1-aminoethyl)-2,6-difluorophenyl](isoquinolin-5-yl)methanone and its optical isomers and addition salts thereof with a pharmaceutically acceptable acid and hydrates thereof,
- [4-((1*R*)-1-aminoethyl)-2,6-difluorophenyl](isoquinolin-5-yl)methanone and its addition salts with a pharmaceutically acceptable acid and hydrates thereof,
- [4-(1-aminoethyl)-2,6-difluorophenyl](1-hydroxyisoquinolin-5-yl)methanone and its optical isomers and addition salts thereof with a pharmaceutically acceptable acid and hydrates thereof,
- 1-[3,5-difluoro-4-(isoquinolin-5-ylmethyl)phenyl]ethanamine and its optical isomers and addition salts thereof with a pharmaceutically acceptable acid and hydrates thereof,
- {4-[(1*S*)-1-aminoethyl]-2,6-difluorophenyl}(isoquinolin-5-yl)methanol and its addition salts with a pharmaceutically acceptable acid and hydrates thereof,
- [4-(2-aminopropan-2-yl)-2,6-difluorophenyl](isoquinolin-5-yl)methanone and its addition salts with a pharmaceutically acceptable acid and hydrates thereof,
- 5-[4-(2-aminopropan-2-yl)-2,6-difluorobenzoyl]isoquinolin-1(2*H*)-one and its addition salts with a pharmaceutically acceptable acid and hydrates thereof,
- 5-[4-(1-aminoethyl)-2-fluoro-3-methoxybenzoyl]isoquinolin-1(2*H*)-one and its optical isomers and addition salts thereof with a pharmaceutically acceptable acid and hydrates thereof,
- 5-({5-[(1*R*)-1-aminoethyl]-3,4-dihydro-2*H*-chromen-8-yl}carbonyl)isoquinolin-1(2*H*)-one and its addition salts with a pharmaceutically acceptable acid and hydrates thereof,
- 5-{4-[(1*R*)-1-aminoethyl]-2-methylbenzoyl}isoquinolin-1(2*H*)-one and its addition salts with a pharmaceutically acceptable acid and hydrates thereof,
- 5-(2,6-difluoro-4-{1-[(2-hydroxyethyl)amino]ethyl}benzoyl)isoquinolin-1(2*H*)-one and its optical isomers and addition salts thereof with a pharmaceutically acceptable acid and hydrates thereof,
- 5-{4-[(1*R*)-1-aminoethyl]-2,6-difluorobenzoyl}-4-methylisoquinolin-1(2*H*)-one and its addition salts with a pharmaceutically acceptable acid and hydrates thereof,
- 5-{3-[(1*R*)-1-aminoethyl]-2,6-difluorobenzoyl}isoquinolin-1(2*H*)-one and its addition salts with a pharmaceutically acceptable acid and hydrates thereof,
- 5-[(1-amino-4,6-difluoro-2,3-dihydro-1*H*-inden-5-yl)carbonyl]isoquinolin-1(2*H*)-one and its optical isomers and addition salts thereof with a pharmaceutically acceptable acid and hydrates thereof,
- 5-{[(3*R*)-3-amino-4,6-difluoro-2,3-dihydro-1*H*-inden-5-yl]carbonyl}isoquinolin-1(2*H*)-one and its addition salts with a pharmaceutically acceptable acid and hydrates thereof,
- 5-({8-[(1*R*)-1-aminoethyl]-2,3-dihydro-1,4-benzodioxin-5-yl}carbonyl)isoquinolin-1(2*H*)-one and its addition

salts with a pharmaceutically acceptable acid,
- 5-[2,6-difluoro-4-(piperidin-2-yl)benzoyl]isoquinolin-1(2*H*)-one and its optical isomers and addition salts thereof with a pharmaceutically acceptable acid and hydrates thereof,
- 5-[4-(1-amino-2-cyclohexylethyl)-2,6-difluorobenzoyl]isoquinolin-1(2*H*)-one and its optical isomers and addition salts thereof with a pharmaceutically acceptable acid and hydrates thereof,
- 5-{[4-(aminomethyl)-3,4-dihydro-2*H*-chromen-8-yl]carbonyl}isoquinolin-1(2*H*)-one and its optical isomers and addition salts thereof with a pharmaceutically acceptable acid and hydrates thereof.

**10.** Process for the synthesis of compounds of formula (Ia), particular cases of the compounds of formula (I) according to claim 1 wherein X represents a group -C(=O), **characterised in that** the compounds of formula (Ia) are prepared starting from a compound of formula (II) :

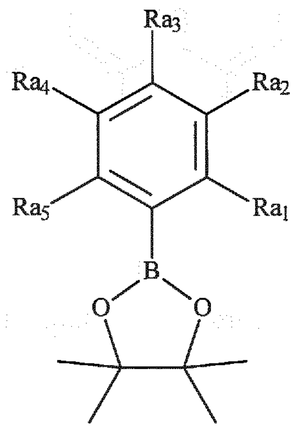

(II)

which is subjected to a coupling reaction with the compound of formula (III) :

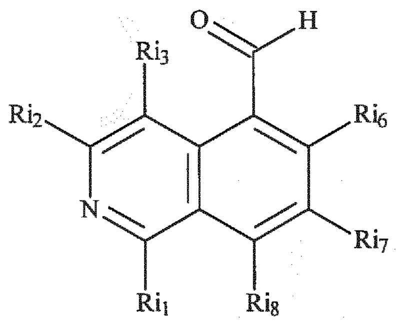

(III)

in the presence of a rhodium or palladium catalyst, of a phosphine and of a base in an organic solvent, to yield the compound of formula (Ia) :

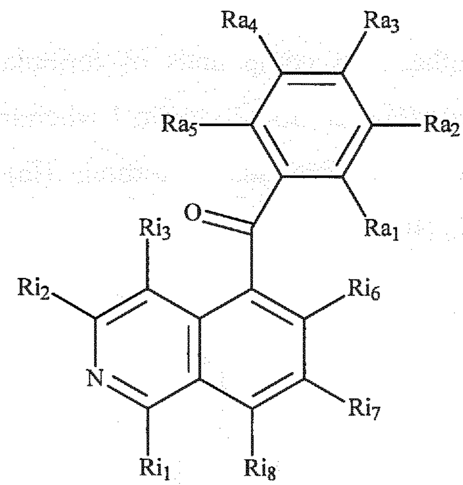

(Ia)

**11.** Process for the synthesis of compounds of formula (Ib), particular cases of the compounds of formula (I) according to claim 1 wherein X represents a group -CH(OH)- :

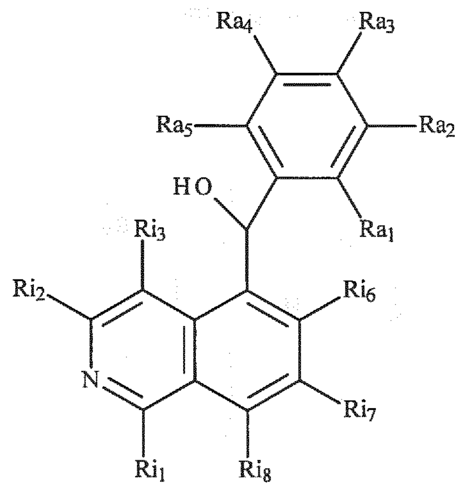

(Ib)

**characterised in that** the compounds of formula (Ib) are prepared starting from the compounds of formula (Ia) according to claim 10 by a reduction reaction in the presence of sodium tetraborohydride.

**12.** Process for the synthesis of compounds of formula (Ic), particular cases of the compounds of formula (I) according to claim 1 wherein X represents a group $-CH_2-$

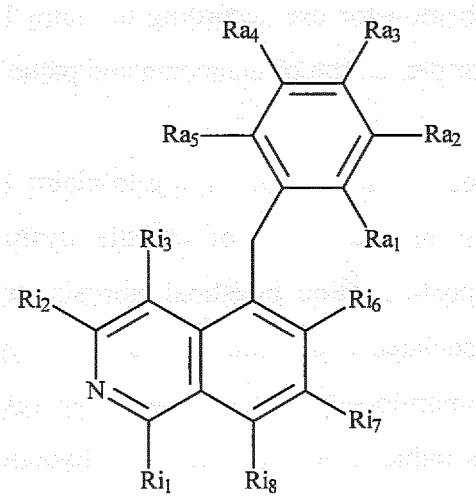

(Ic)

**characterised in that** the compounds of formula (Ic) are prepared starting from the compounds of formula (Ib) according to claim 11 by a reduction reaction in the presence of trifluoroacetic acid and of triethylsilane.

**13.** Pharmaceutical composition comprising as active ingredient a compound of formula (I) according to any one of claims 1 to 9, in combination with one or more inert, non-toxic, pharmaceutically acceptable excipients or carriers.

**14.** Pharmaceutical composition according to claim 13, for use in the treatment or prevention of pathologies which are the result of activation of the RhoA/ROCK pathway and phosphorylation of the myosin light chain.

**15.** Pharmaceutical composition for use according to claim 14, **characterised in that** it is used in the treatment or prevention of systemic arterial hypertension, pulmonary arterial hypertension, angina, myocardial infarction, post-angioplasty restenosis, aortic aneurysm, occlusion of the peripheral arteries, atherosclerosis, cardiac fibrosis and heart failure.

**16.** Pharmaceutical composition for use according to claim 15, **characterised in that** it is used in the treatment or prevention of systemic arterial hypertension.

**17.** Pharmaceutical composition for use according to claim 14, **characterised in that** it is used in the treatment or prevention of glaucoma and pathologies of the cornea.

**18.** Pharmaceutical composition for use according to claim 14, **characterised in that** it is used in the treatment or prevention of erectile dysfunction, broncho-obstructive pulmonary diseases, post-radiation intestinal fibrosis, cutaneous systemic sclerosis, pulmonary fibrosis associated with pulmonary arterial hypertension, hepatic diseases, renal fibrosis and glomerulo-sclerosis, diabetes, hyperglycaemia, insulin resistance, diabetic nephropathies induced or not induced by hypertension, thrombotic diseases, cerebral vasospasm and resulting cerebral ischaemia.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2000064478 A1
- WO 2005035503 A
- EP 0187371 A
- WO 2007000240 A
- WO 2007012421 A
- WO 2007012422 A
- WO 2008077550 A
- WO 2008077552 A
- WO 2008077553 A
- WO 2008077554 A
- WO 2008077555 A
- WO 2008077556 A
- WO 2009156092 A
- WO 2009156099 A
- WO 2009156100 A

### Littérature non-brevet citée dans la description


- **RATTAN et al.** *Pharmacological Sciences,* 2011, vol. 880, 1-10
- **KAIBUCHI et al.** *Sciences,* 1997, vol. 275, 1308
- **UEHATA et al.** *Nature,* 1997, vol. 389, 990-993
- **PACAUD et al.** *P. Nat. Rev. Cardiol,* 2010, vol. 7 (11), 637-647
- **JANKOV et al.** *Am J Physiol Heart Circ Physiol,* 2010, vol. 299, H1854-H1864
- **FUKUMOTO et al.** *Heart,* 2005, vol. 91, 391-392
- **DUONG-QUY et al.** *J. Fran. Viet. Pneu.,* 2012, vol. 03 (08), 1-74
- **ACOTT et al.** *Curr Opin Ophtalmol,* 2012, vol. 23 (2), 135-43
- **TANIHARA et al.** *Curr Eye Res,* 2011, vol. 36 (10), 964-70
- **ROSSETTI et al.** *Expert.Opin.Investig.Drugs,* 2011, vol. 20 (7), 947-959
- **CHEN et al.** *Clin. Ophtalmol,* 2011, vol. 5, 667-677
- **RAO et al.** *J Glaucoma,* 2012, vol. 21, 530-538
- **ZHONG et al.** *Int J Oncol,* 2013, vol. 43 (5), 1357-67
- **VAN DE VELDE.** *Acta Ophtalmologica,* 2013, vol. 91, s252
- **KINOSHITA et al.** *Cornea,* 2013, vol. 32 (8), 1167-1170
- **KANDABASHI et al.** *Circulation,* 2000, vol. 101, 1319-1323
- **SHIMOKAWA et al.** *Am. J. Physiol. Heart Circ. Physiol,* 2011, vol. 301, H287-H296
- **SHIMOKAWA et al.** *Cardiovasc . Res.,* 2001, vol. 51, 169-177
- **SHIMOKAWA et al.** *Am J Physiol Heart Circ Physiol,* 2011, vol. 301, H287-H296
- **CHITALEY et al.** *Int J Impot Res,* 2012, vol. 24 (2), 49-60
- **IMAMURA et al.** *Biochem, Biophys Res,* 2000, vol. 269 (2), 633-640
- **ZHANG et al.** *Central South Pharmacy,* 2008, vol. 3, 035
- **KALLURI et al.** *J. Cell. Physiol.,* 2010, vol. 225, 631-637
- **SABBADINI et al.** *Circ.Res.,* 2009, vol. 82, 303-312
- **ROHR.** *Heart Rhythm,* 2009, vol. 6 (6), 848-856
- **HATTORI et al.** *Circulation,* 2004, vol. 109, 2234-2239
- **KRUM et al.** *Am J Physiol Heart Circ Physiol,* 2008, vol. 294, H1804-H1814
- **ENTMAN et al.** *Cardiovasc.Res.,* 2009, vol. 83, 511-518
- **LIU et al.** *Toxicology Letters,* 2012, vol. 211, 91-97
- **KISHI et al.** *Circulation,* 2005, vol. 111, 2741-2747
- **KIKUCHI et al.** *J. Endocrinol.,* 2007, vol. 192, 595-603
- **KOLAVENNU et al.** *Diabetes,* 2008, vol. 57, 714-723
- **MATSUOKA et al.** *J Hypertens,* 2008, vol. 26 (9), 1837-48
- **DISTLER et al.** *Arthritis and Rheumatism,* 2008, vol. 58 (8), 2553-2564
- **VOZENIN-BROTONS et al.** *Gut,* 2005, vol. 54 (3), 336-343
- **SCHWARTZ et al.** *EMBO Journal,* 2007, vol. 26, 505-515
- **DOE et al.** *J.Pharmacol.Exp.Ther.,* 2007, vol. 320, 89-98

- **SHIBUYA et al.** *J.Neurol.Science,* 2005, vol. 238, 31-39
- **ZHOU et al.** *Science,* 2003, vol. 302, 1215-1217
- **SONG et al.** *CNS Neurosci. Ther.,* 2013, vol. 19, 603-610
- **XIAO et al.** *Brain, Behaviour and Immunity,* 2009, vol. 23 (8), 1083-88
- **HARA et al.** *J. Neurosurg.,* 2000, vol. 93 (1), 94-101
- **DERGHAM et al.** *J. Neurosci.,* 2002, vol. 22, 6570-6577
- **YAMASHITA et al.** *Ther. Clin. Risk Manag.,* 2008, vol. 4 (3), 605-615
- **FENG et al.** *Current Topics in Medical Chemistry,* 2009, vol. 9, 704-723
- **UTSUNOMIYA et al.** *Biochemical and Biophysical Research Communication,* 2010, vol. 402, 725-730
- **LIU et al.** *Cancer Res,* 2009, vol. 69, 8742-8751
- **LI et al.** *FEBS Lett.,* 2006, vol. 580, 4252-4260
- **VISHNUBHOTLA et al.** *Lab.Invest.,* 2007, vol. 87, 1149-1158
- **ZOHRABIAN et al.** *Anticancer Res.,* 2009, vol. 29, 119-123
- **TORRE et al.** *Arch. Otolaryngol. Head Neck Surg.,* 2010, vol. 136, 493-501
- **YING et al.** *Mol.Cancer Ther.,* 2006, vol. 5, 2158-2164
- **HRUSKA et al.** *J Biol chem,* 2003, vol. 278 (31), 29086-97
- **MORI et al.** *Am. J. Resp. Cell. Mol. Biol.,* 1999, vol. 20 (6), 1190-1200
- **KANAIDE et al.** *Br J Pharmacol,* 2001, vol. 132, 111-118
- **LIN et al.** *Cir. Res.,* 2003, vol. 92 (12), 1296-1304
- **BROWN, W. D. ; GOULIAEV, A. H.** *Synthesis,* 2002, vol. 1, 83-86
- *Organic Syntheses,* 2004, vol. 81, 98-104
- **JOHN T. COLYER ; NEIL G. ANDERSEN ; JASON S. TEDROW ; TROY S. SOUKUP ; MARGARET M. FAUL.** *J. Org. Chem.,* 2006, vol. 71, 6859-6862
- **DOE, C. ; BENTLEY, R. ; BEHM, D.J. ; LAFFERTY, R. ; STAVENGER, R. ; JUNG, D. ; BAMFORD, M. ; PANCHAL, T. ; GRYGIELKO, E. ; WRIGHT, L.L.** Novel Rho kinase inhibitors with anti-inflammatory and vasodilatory activities. *J. Pharmacol. Exp. Ther.,* 2007, vol. 320, 89